# EUROPEAN PATENT APPLICATION

(11) **EP 4 818 157 A1**
(43) Date of publication of application: **07.10.2026**
(21) Application number: 24912981.8
(22) Date of filing: 26.12.2024
(51) Int. Cl.: C07D 211/76, A61K 31/435

(54) **N-SUBSTITUTED CYCLIC AMIDE COMPOUND**

(30) Priority: 29.12.2023 JP 2023223738
(71) Applicant: Kissei Pharmaceutical Co., Ltd., Matsumoto-Shi, Nagano 399-8710 (JP)
(72) Inventor: MUTAI, Yosuke, Azumino-shi, Nagano 399-8304 (JP); KIYOSAWA, Seina, Azumino-shi, Nagano 399-8304 (JP); KAMIYA, Akinobu, Azumino-shi, Nagano 399-8304 (JP); TANADA, Fumiya, Azumino-shi, Nagano 399-8304 (JP); ITOU, Tatsuya, Azumino-shi, Nagano 399-8304 (JP); MATSUMOTO, Tsutomu, Azumino-shi, Nagano 399-8304 (JP); OKI, Tomoya, Azumino-shi, Nagano 399-8304 (JP); MATSUMOTO, Shunsuke, Azumino-shi, Nagano 399-8304 (JP)
(74) Representative: Isarpatent
(86) International application number: PCT/JP2024/046100
(87) International publication number: WO 2025/143095

(57) **Abstract**

An object of the present invention is to provide a novel compound which has thyroid-stimulating hormone receptor antagonist activity and is useful for the treatment of thyroid-related diseases. The present invention relates to an N-substituted cyclic amide compound represented by the following formula or a pharmacologically acceptable salt thereof. The compound of the present invention or a pharmacologically acceptable salt thereof has antagonist activity against a thyroid-stimulating hormone receptor and is useful as a therapeutic agent for thyroid-related diseases (for example, hyperthyroidism, Graves' disease, thyroid eye disease and thyroid cancer).

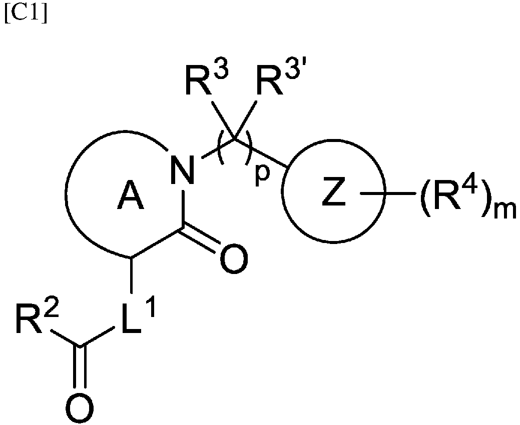

## Description

### [Technical Field]

The present invention relates to an N-substituted cyclic amide compound useful as a pharmaceutical. More specifically, the present invention relates to an N-substituted cyclic amide compound or a pharmacologically acceptable salt thereof, which has antagonist activity against thyroid-stimulating hormone receptor (TSHR) and is useful as a therapeutic agent for thyroid-related diseases.

### [Background Art]

The thyroid hormones triiodothyronine (T3) and thyroxine (T4) play important roles in development, growth, and metabolism, and their synthesis and secretion are precisely regulated by the thyroid-stimulating hormone (TSH) secreted by the pituitary gland.

In hyperthyroidism, these thyroid hormones are excessively secreted for some reason, and the hormone action thereof causes various undesirable effects on the mind and body, such as goiter, tachycardia, high blood pressure, fatigue, weight loss, palpitations, sleep disorders, and menstrual irregularities.

There are various causes of hyperthyroidism, and among these, Graves' disease (Basedow's disease) is the most common. In Graves' disease, due to the autoimmune mechanism, one's own thyroid gland is recognized as a foreign body, and autoantibodies against TSHR present on thyroid follicular cells, called TSHR antibodies (TRAb), are produced. It is thought that this TRAb acts as an agonist on TSHR, and when TSHR is excessively stimulated, more of thyroid hormones than necessary is secreted, and hyperthyroidism develops.

In addition, thyroid eye disease is also known as a disease associated with Graves' disease. Thyroid eye disease is an autoimmune inflammatory disease that exhibits various ocular symptoms, and is thought to be mainly caused by the agonist action of TRAb on TSHR in the orbital tissue. It often develops at roughly the same time as hyperthyroidism, but may not be associated with thyroid dysfunction.

Currently, antithyroid drugs such as thiamazole and propylthiouracil, which inhibit biosynthesis of thyroid hormones, are used for drug therapy for Graves' disease, but they have problems of a low remission rate, a long treatment period until remission is achieved, and a high frequency of side effects. Therefore, there is currently a need for a therapeutic agent for Graves' disease with a new mechanism of action.

Blocking TSHR or inhibiting signal transduction induced through TSHR inhibits thyroid hormone production, secretion, and thyroid cell proliferation. Therefore, a TSHR antagonist is thought to have a therapeutic effect for Graves' disease and thyroid eye disease caused by the agonist action of TRAb on TSHR (PTL 1 and PTL 2). As a TSHR antagonist, NCGC00242364 is known, and it has been clarified that the TSHR antagonist has an effect of lowering a blood T4 concentration in a TSH-releasing hormone (TRH)-administered mouse and a thyroid-stimulating antibody (M22)-administered mouse (NPL 1).

Compounds having TSHR antagonist activity are described in PTL 1 to PTL 3, and NPL 1.

However, an N-substituted cyclic amide compound of the present invention is not described in any of PTL 1 to PTL 3 or NPL 1.

### [Citation List]

### [Patent Literature]

[PTL 1] U.S. Patent Application Publication No. 2011/0172267
[PTL 2] U.S. Patent Application Publication No. 2012/0315217
[PTL 3] U.S. Patent Application Publication No. 2019/0134024

### [Non Patent Literature]

[NPL 1] Susanne Neumann et al., "Endocrinology" 2014, Vol. 155, No. 1, p. 310-314

### [Summary of Invention]

### [Technical Problem]

An object of the present invention is to provide a novel compound which has TSHR antagonist activity and is useful for treating thyroid-related diseases.

### [Solution to Problem]

The present invention relates to a compound represented by the following Formula (A-I) or a pharmacologically acceptable salt thereof.

In other words, the present invention relates to the following [A-1] to [A-51], [B-1] to [B-51], and the like.
[A-1] A compound represented by Formula (A-I): 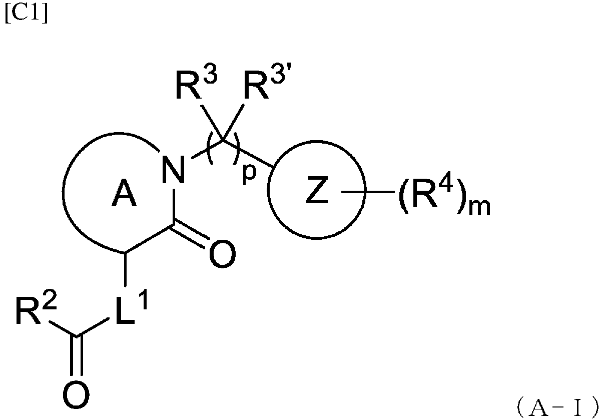 [wherein,
   ring A is a group represented by Formula (1), (2), or (3) below: 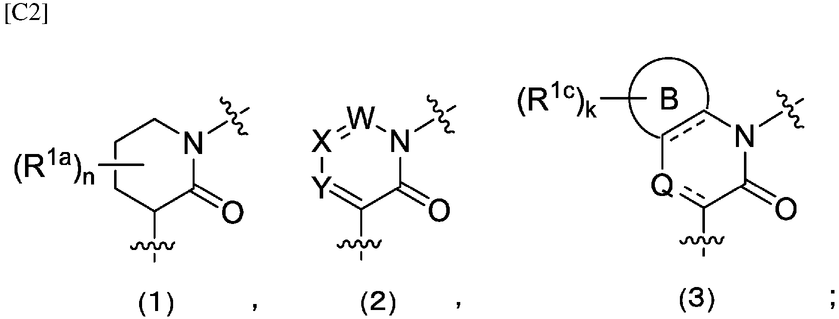
   W, X, and Y are each independently -CR^{1b}= or -N=;
   Q is -CR^{Q1}=, -CR^{Q1}R^{Q1'}-, -O-, -N=, or -NR^{Q2}-;
   R^{Q1} and R^{Q1'} are each independently a hydrogen atom, a halogen atom, hydroxy, C₁₋₆ alkyl, or halo C₁₋₆ alkyl;
   R^{Q2} is a hydrogen atom, C₁₋₆ alkyl, or halo C₁₋₆ alkyl;
   ring B is 5- or 6-membered heteroaryl or benzene;
   R^{1a} and R^{1c} are each independently a halogen atom, hydroxy, carboxy, cyano, C₁₋₆ alkyl, C₁₋₆ alkoxy, halo C₁₋₆ alkyl, halo C₁₋₆ alkoxy, C₁₋₆ alkoxy C₁₋₆ alkyl, C₃₋₈ cycloalkyl, C₃₋₈ cycloalkoxy, hydroxy C₁₋₆ alkyl, carboxy C₁₋₆ alkyl, amino C₁₋₆ alkyl, cyano C₁₋₆ alkyl, - NR⁷COR⁸, -NR⁷COOR⁹, -NR⁷CONR¹⁰R¹¹, -CONR¹²R¹³, -(C₁₋₆ alkylene)-(3- to 8-membered heterocycloalkyl), which is unsubstituted or substituted with 1 to 6 substituents selected from substituent group A, -(C₁₋₆ alkylene)-NR⁷COR⁸, -(C₁₋₆ alkylene)-NR⁷COOR⁹, -(C₁₋₆ alkylene)-NR⁷CONR¹⁰R¹¹, or -(C₁₋₆ alkylene)-CONR¹²R¹³;
   R^{1b} is a hydrogen atom, a halogen atom, hydroxy, carboxy, cyano, C₁₋₆ alkyl, C₂₋₆ alkenyl, C₁₋₆ alkoxy, halo C₁₋₆ alkyl, halo C₁₋₆ alkoxy, C₁₋₆ alkoxy C₁₋₆ alkyl, C₃₋₈ cycloalkyl, C₃₋₈ cycloalkoxy, hydroxy C₁₋₆ alkyl, carboxy C₁₋₆ alkyl, amino C₁₋₆ alkyl, cyano C₁₋₆ alkyl, - NR⁷COR⁸, -NR⁷COOR⁹, -NR⁷CONR¹⁰R¹¹, -CONR¹²R¹³, -(C₁₋₆ alkylene)-(3- to 8-membered heterocycloalkyl), which is unsubstituted or substituted with 1 to 6 groups selected from substituent group A, -(C₁₋₆ alkylene)-NR⁷COR⁸, -(C₁₋₆ alkylene)-NR⁷COOR⁹, -(C₁₋₆ alkylene)-NR⁷CONR¹⁰R¹¹, or -(C₁₋₆ alkylene)-CONR¹²R¹³;
   R⁷ is a hydrogen atom or C₁₋₆ alkyl;
   R⁸, R⁹, R¹⁰, R¹¹, R¹², and R¹³ are each independently a hydrogen atom or a group selected from the group consisting of the following (i) to (v):
      (i) C₁₋₆ alkyl which is unsubstituted or substituted with 1 to 6 groups selected from substituent group A,
      (ii) C₃₋₈ cycloalkyl which is unsubstituted or substituted with 1 to 6 groups selected from substituent group A,
      (iii) C₃₋₈ cycloalkyl C₁₋₆ alkyl which is unsubstituted or substituted with 1 to 6 groups selected from substituent group A,
      (iv) C₆₋₁₀ aryl which is unsubstituted or substituted with 1 to 6 groups selected from substituent group A,
      (v) C₆₋₁₀ aryl C₁₋₆ alkyl which is unsubstituted or substituted with 1 to 6 groups selected from substituent group A,
   substituent group A is a group consisting of a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkoxy, C₃₋₈ cycloalkyl C₁₋₆ alkyl, and C₃₋₈ cycloalkyl C₁₋₆ alkoxy;
   when a group is substituted with two or more groups selected from substituent group A, the respective groups may be the same or different from each other;
   R¹⁰ and R¹¹ may be taken together to form a 3- to 10-membered saturated heterocycle or a 3- to 10-membered unsaturated heterocycle;
   R¹² and R¹³ may be taken together to form a 3- to 10-membered saturated heterocycle or a 3- to 10-membered unsaturated heterocycle;
   n is an integer of 1 to 3;
   when n is 2 or 3, respective R^{1a}s may be the same as or different from each other;
   when two or more R^{1b}s are present, respective R^{1b}s may be the same or different from each other;
   k is an integer of 0 to 3;
   when k is 2 or 3, respective R^{1c}s may be the same or different from each other;
   two R^{1a}s may be taken together to form a 3- to 8-membered saturated carbocycle, or a 3- to 8-membered saturated heterocycle;

      [C3] - - -
   represents a single bond or a double bond;
   ring Z is C₆₋₁₀ aryl, 5- to 10-membered heteroaryl or C₃₋₈ cycloalkyl;
   R² is C₁₋₆ alkyl, C₁₋₆ alkoxy, halo C₁₋₆ alkyl, halo C₁₋₆ alkoxy, C₁₋₆ alkoxy C₁₋₆ alkyl, C₃₋₈ cycloalkyl, C₃₋₈ cycloalkoxy, 3- to 8-membered heterocycloalkyl, or -NR⁵R^{5'};
   provided that, when ring A is a group represented by Formula (2) or Formula (3), R² is halo C₁₋₆ alkyl, 3- to 8-membered heterocycloalkyl, or -NHR⁵;
   R⁵ and R^{5'} are each independently a hydrogen atom, C₁₋₆ alkyl, halo C₁₋₆ alkyl, or C₃₋₈ cycloalkyl;
   R³ and R^{3'} are each independently a hydrogen atom, a halogen atom, cyano, C₁₋₆ alkyl, C₁₋₆ alkoxy, C₁₋₆ alkoxy C₁₋₆ alkyl, hydroxy C₁₋₆ alkyl, amino C₁₋₆ alkyl, or cyano C₁₋₆ alkyl;
   R⁴ is a halogen atom, cyano, C₁₋₆ alkyl, C₂₋₆ alkenyl, C₁₋₆ alkoxy, halo C₁₋₆ alkyl, halo C₁₋₆ alkoxy, C₁₋₆ alkoxy C₁₋₆ alkyl, C₃₋₈ cycloalkyl, C₃₋₈ cycloalkoxy, C₃₋₈ cycloalkyl C₁₋₆ alkyl, C₃₋₈ cycloalkyl C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₁₋₆ alkyl, 5- or 6-membered heteroaryl C₂₋₆ alkynyl, C₃₋₈ cycloalkyl C₂₋₆ alkynyl, hydroxy C₁₋₆ alkyl, amino C₁₋₆ alkyl, cyano C₁₋₆ alkyl, halohydroxy C₁₋₆ alkyl, or -SF₅;
   R^{1a}, R^{1b}, or R^{1c} may together with R³ form a 5- to 8-membered saturated heterocycle or a 5- to 8-membered unsaturated heterocycle;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, respective R⁴s may be the same as or different from each other;
   provided that when ring A is a group represented by Formula (2), m is an integer of 1 to 3;
   p is an integer of 0 to 3; and
   when p is 2 or 3, respective R³ and R^{3'} may be the same as or different from each other]
   or a pharmacologically acceptable salt thereof.
[A-2] The compound according to the above [A-1], which is a compound represented by Formula (A-II): 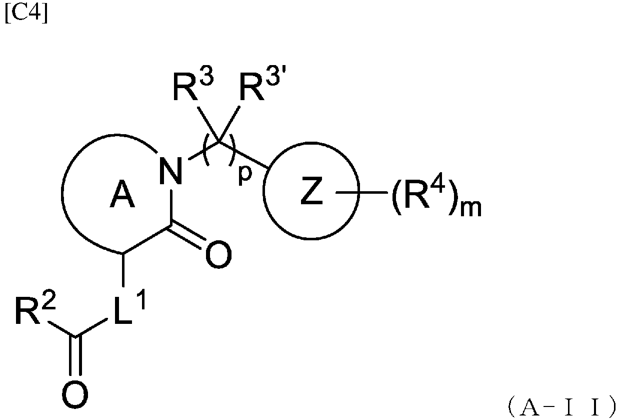 [wherein,
   ring A is a group represented by Formula (1), (2), or (3) below: 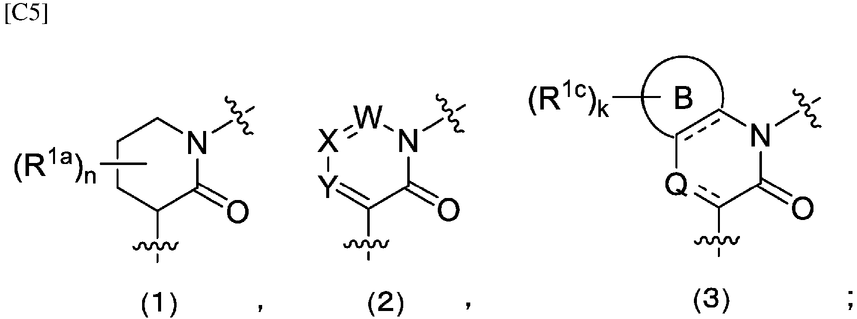
   W, X, and Y are each independently -CR^{1b}= or -N=;
   Q is -CR^{Q1}=, -CR^{Q1}R^{Q1'}-, -O-, -N=, or -NR^{Q2}-;
   R^{Q1} and R^{Q1'} are each independently a hydrogen atom, a halogen atom, hydroxy, C₁₋₆ alkyl, or halo C₁₋₆ alkyl;
   R^{Q2} is a hydrogen atom, C₁₋₆ alkyl, or a halo C₁₋₆ alkyl;
   ring B is 5- or 6-membered heteroaryl or benzene;
   R^{1a} and R^{1c} are each independently a halogen atom, hydroxy, carboxy, cyano, C₁₋₆ alkyl, C₁₋₆ alkoxy, halo C₁₋₆ alkyl, C₁₋₆ alkoxy C₁₋₆ alkyl, hydroxy C₁₋₆ alkyl, amino C₁₋₆ alkyl, carboxy C₁₋₆ alkyl, -NR⁷COR⁸, -NR⁷COOR⁹, -NR⁷CONR¹⁰R¹¹, -CONR¹²R¹³, -(C₁₋₆ alkylene)-(3- to 8-membered heterocycloalkyl), which is unsubstituted or substituted with 1 to 6 groups selected from substituent group A, -(C₁₋₆ alkylene)-NR⁷COR⁸, -(C₁₋₆ alkylene)-NR⁷COOR⁹, -(C₁₋₆ alkylene)-NR⁷CONR¹⁰R¹¹, or -(C₁₋₆ alkylene)-CONR¹²R¹³;
   R^{1b} is a hydrogen atom, a halogen atom, hydroxy, carboxy, cyano, C₁₋₆ alkyl, C₂₋₆ alkenyl, C₁₋₆ alkoxy, halo C₁₋₆ alkyl, hydroxy C₁₋₆ alkyl, carboxy C₁₋₆ alkyl, amino C₁₋₆ alkyl, -NR⁷COR⁸, -NR⁷COOR⁹, -NR⁷CONR¹⁰R¹¹, -CONR¹²R¹³, -(C₁₋₆ alkylene)-(3- to 8-membered heterocycloalkyl), which is unsubstituted or substituted with 1 to 6 groups selected from substituent group A, -(C₁₋₆ alkylene)-NR⁷COR⁸, -(C₁₋₆ alkylene)-NR⁷COOR⁹, -(C₁₋₆ alkylene)-NR⁷CONR¹⁰R¹¹, or -(C₁₋₆ alkylene)-CONR¹²R¹³;
   R⁷ is a hydrogen atom or C₁₋₆ alkyl;
   R⁸, R⁹, R¹⁰, R¹¹, R¹², and R¹³ are each independently a hydrogen atom or a group selected from the group consisting of the following (i) to (v):
      (i) C₁₋₆ alkyl which is unsubstituted or substituted with 1 to 6 groups selected from substituent group A,
      (ii) C₃₋₈ cycloalkyl which is unsubstituted or substituted with 1 to 6 groups selected from substituent group A,
      (iii) C₃₋₈ cycloalkyl C₁₋₆ alkyl which is unsubstituted or substituted with 1 to 6 groups selected from substituent group A,
      (iv) C₆₋₁₀ aryl which is unsubstituted or substituted with 1 to 6 groups selected from substituent group A, and
      (v) C₆₋₁₀ aryl C₁₋₆ alkyl which is unsubstituted or substituted with 1 to 6 groups selected from substituent group A,
   substituent group A is a group consisting of a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkoxy, C₃₋₈ cycloalkyl C₁₋₆ alkyl, and C₃₋₈ cycloalkyl C₁₋₆ alkoxy;
   when substituted with two or more groups selected from substituent group A, the respective groups may be the same as or different from each other;
   R¹⁰ and R¹¹ may be taken together to form a 3- to 10-membered saturated heterocycle or a 3- to 10-membered unsaturated heterocycle;
   R¹² and R¹³ may be taken together to form a 3- to 10-membered saturated heterocycle or a 3- to 10-membered unsaturated heterocycle;
   n is an integer of 1 to 3;
   when n is 2 or 3, respective R^{1a}s may be the same as or different from each other;
   when two or more R^{1b}s are present, respective R^{1b}s may be the same or different from each other;
   k is an integer of 0 to 3;
   when k is 2 or 3, respective R^{1c}s may be the same or different from each other;
   two R^{1a}s may be taken together to form a 3- to 8-membered saturated carbocycle, or a 3- to 8-membered saturated heterocycle;

      [C6] - - -
   represents a single bond or a double bond;
   ring Z is C₆₋₁₀ aryl, 5- to 10-membered heteroaryl, or C₃₋₈ cycloalkyl;
   R² is C1₋₆ alkyl, halo C₁₋₆ alkyl, C₁₋₆ alkoxy C₁₋₆ alkyl, C₃₋₈ cycloalkyl, a 3- to 8-membered heterocycloalkyl, or -NR⁵R^{5'};
   provided that, when ring A is a group represented by Formula (2) or Formula (3), R² is halo C₁₋₆ alkyl, 3- to 8-membered heterocycloalkyl, or -NHR⁵;
   R⁵ and R^{5'} are each independently a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, C₂₋₆ alkenyl, C₁₋₆ alkoxy, halo C₁₋₆ alkyl, halo C₁₋₆ alkoxy, C₃₋₈ cycloalkyl, C₃₋₈ cycloalkyl C₁₋₆ alkyl, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₁₋₆ alkyl, 5- or 6-membered heteroaryl C₂₋₆ alkynyl, C₃₋₈ cycloalkyl C₂₋₆ alkynyl, halo hydroxy C₁₋₆ alkyl, or -SF₅;
   R^{1a}, R^{1b}, or R^{1c} may be taken together with R³ to form a 5- to 8-membered saturated heterocycle or a 5- to 8-membered unsaturated heterocycle;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, respective R⁴s may be the same as or different from each other;
   provided that when ring A is a group represented by Formula (2), m is an integer of 1 to 3;
   p is an integer of 0 to 3; and
   when p is 2 or 3, respective R³ and R^{3'} may be the same as or different from each other]
   or a pharmacologically acceptable salt thereof.
[A-3] The compound according to the above [A-1] or [A-2], which is a compound represented by Formula (A-III): 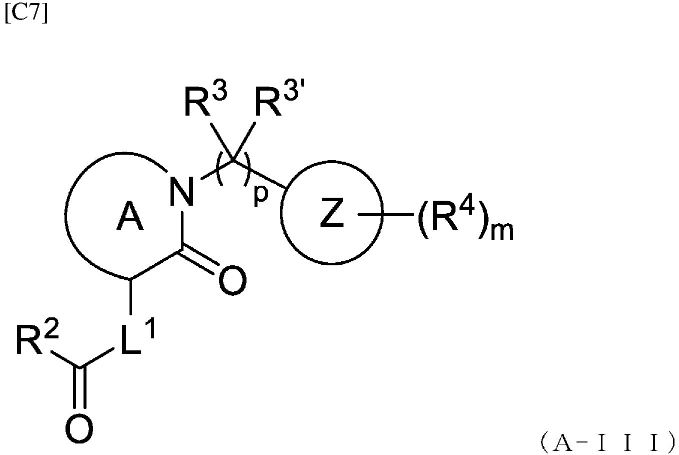 [wherein,
   ring A is a group represented by Formula (1), (2), or (3) below: 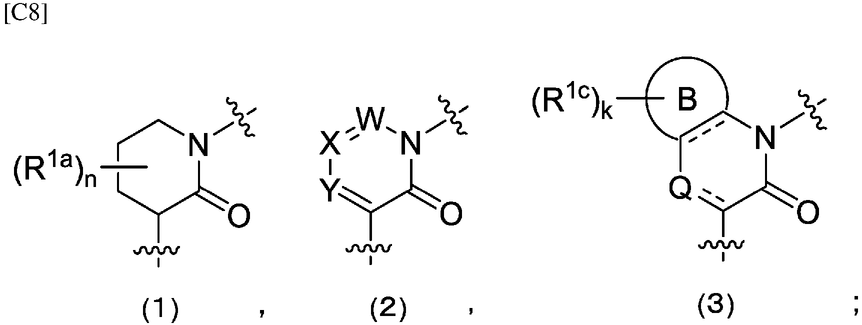
   Q is -CH=, -CH₂-, -O-, or -N=;
   R^{1a} is a halogen atom, C₁₋₆ alkyl, halo C₁₋₆ alkyl, C₁₋₆ alkoxy C₁₋₆ alkyl, hydroxy C₁₋₆ alkyl, amino C₁₋₆ alkyl, -(C₁₋₆ alkylene)-(3- to 8-membered heterocycloalkyl), which is unsubstituted or substituted with 1 to 6 groups selected from substituent group A, -(C₁₋₆ alkylene)-NH-COO-(C₁₋₆ alkyl), -(C₁₋₆ alkylene)-NH-CO-(C₁₋₆ alkyl), or -(C₁₋₆ alkylene)-CON(C₁₋₆ alkyl)₂;
   substituent group A is a group consisting of a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkoxy, C₃₋₈ cycloalkyl C₁₋₆ alkyl, and C₃₋₈ cycloalkyl C₁₋₆ alkoxy;
   when substituted with two or more groups selected from substituent group A, the respective groups may be the same as or different from each other;
   R^{1c} is a halogen atom, hydroxy, carboxy, cyano, C₁₋₆ alkyl, halo C₁₋₆ alkyl, hydroxy C₁₋₆ alkyl, amino C₁₋₆ alkyl, C₁₋₆ alkoxy, -CONH₂, -NH-COO-(C₁₋₆ alkyl), or -NH-CO-NH-(C₁₋₆ alkyl);
   n is an integer of 1 to 3;
   when n is 2 or 3, respective R^{1a}s may be the same as or different from each other;
   two R^{1a}s may be taken together to form a 3- to 8-membered saturated carbocycle, or a 3- to 8-membered saturated heterocycle;
   R^{1a} may be taken together with R³ to form a 5- to 8-membered saturated heterocycle or a 5- to 8-membered unsaturated heterocycle;
   W, X, and Y are each independently -CR^{1b}= or -N=;
   R^{1b} is a hydrogen atom, a halogen atom, cyano, C₁₋₆ alkyl, C₂₋₆ alkenyl, hydroxy C₁₋₆ alkyl, carboxy C₁₋₆ alkyl, amino C₁₋₆ alkyl, halo C₁₋₆ alkyl, C₁₋₆ alkoxy, C₁₋₆ alkoxy C₁₋₆ alkyl, -(C₁₋₆ alkylene)-(3- to 8-membered heterocycloalkyl), or -(C₁₋₆ alkylene)-NH-COO-(C₁₋₆ alkyl);
   at least one R^{1b} is other than a hydrogen atom;
   when two or more R^{1b}s are present, respective R^{1b}s may be the same or different from each other;
   R^{1b} may be taken together with R³ to form a 5- to 8-membered saturated heterocycle or a 5- to 8-membered unsaturated heterocycle;
   k is an integer of 0 to 3;
   when k is 2 or 3, respective R^{1c}s may be the same or different from each other;
   ring B is a group represented by the following formula: 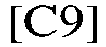 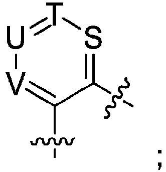
   thiazole or thiophene;
   S, T, U, and V are each independently -CH=, -CR^{1c}=, or -N=;

      [C10] - - -
   represents a single bond or a double bond;
   ring Z is C₆₋₁₀ aryl, 5- or 6-membered heteroaryl, C₃₋₈ cycloalkyl, or a ring represented by the following formula: 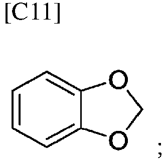
   R² is C₁₋₆ alkyl, C₃₋₈ cycloalkyl, C₁₋₆ alkoxy, C₁₋₆ alkoxy C₁₋₆ alkyl, or -NR⁵R^{5'};
   provided that when ring A is a group represented by Formula (2) or Formula (3), R² is -NHR⁵;
   R⁵ and R^{5'} are each independently a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, C₂₋₆ alkenyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, respective R⁴s may be the same as or different from each other;
   provided that when ring A is a group represented by Formula (2), m is an integer of 1 to 3;
   p is an integer of 1 to 3; and
   when p is 2 or 3, respective R³ and R^{3'} may be the same as or different from each other]
   or a pharmacologically acceptable salt thereof.
[A-4] The compound according to any one of the above [A-1] to [A-3], which is a compound represented by Formula (A-IV): 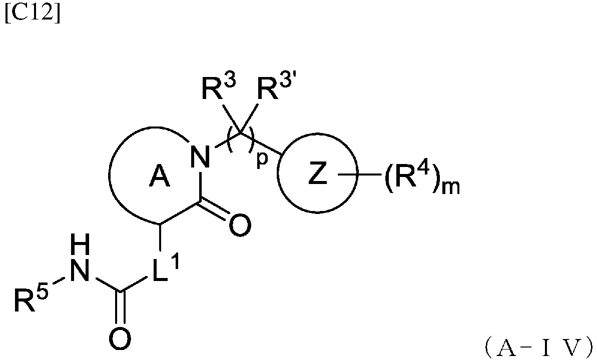 [wherein,
   ring A is a group represented by Formula (1), (2), or (3) below: 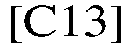 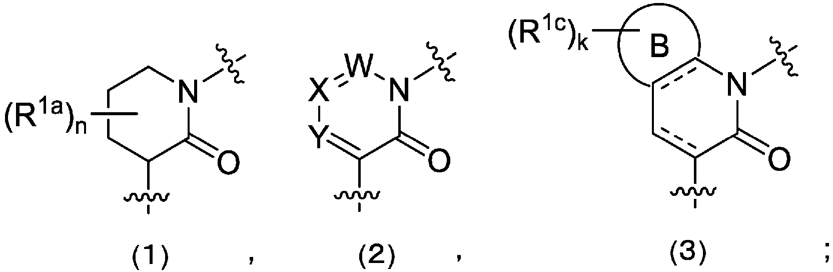
   R^{1a} is a halogen atom, C₁₋₆ alkyl, halo C₁₋₆ alkyl, C₁₋₆ alkoxy C₁₋₆ alkyl, hydroxy C₁₋₆ alkyl, amino C₁₋₆ alkyl, -(C₁₋₆ alkylene)-(3- to 8-membered heterocycloalkyl), which is unsubstituted or substituted with 1 to 6 groups selected from substituent group A, -(C₁₋₆ alkylene)-NH-COO-(C₁₋₆ alkyl), -(C₁₋₆ alkylene)-NH-CO-(C₁₋₆ alkyl), or -(C₁₋₆ alkylene)-CON(C₁₋₆ alkyl)₂;
   substituent group A is a group consisting of a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkoxy, C₃₋₈ cycloalkyl C₁₋₆ alkyl, and C₃₋₈ cycloalkyl C₁₋₆ alkoxy;
   when substituted with two or more groups selected from substituent group A, the respective groups may be the same as or different from each other;
   R^{1c} is a halogen atom, hydroxy, carboxy, cyano, C₁₋₆ alkyl, halo C₁₋₆ alkyl, hydroxy C₁₋₆ alkyl, amino C₁₋₆ alkyl, C₁₋₆ alkoxy, -CONH₂, -NH-COO-(C₁₋₆ alkyl), or -NH-CO-NH-(C₁₋₆ alkyl);
   n is an integer of 1 to 3;
   when n is 2 or 3, respective R^{1a}s may be the same as or different from each other;
   two R^{1a}s may be taken together to form a 3- to 8-membered saturated carbocycle, or a 3- to 8-membered saturated heterocycle;
   R^{1a} may be taken together with R³ to form a 5- to 8-membered saturated heterocycle or a 5- to 8-membered unsaturated heterocycle;
   W, X, and Y are each independently -CR^{1b}= or -N=;
   R^{1b} is a hydrogen atom, a halogen atom, cyano, C₁₋₆ alkyl, C₂₋₆ alkenyl, hydroxy C₁₋₆ alkyl, carboxy C₁₋₆ alkyl, amino C₁₋₆ alkyl, halo C₁₋₆ alkyl, C₁₋₆ alkoxy, C₁₋₆ alkoxy C₁₋₆ alkyl, -(C₁₋₆ alkylene)-(3- to 8-membered heterocycloalkyl), or -(C₁₋₆ alkylene)-NH-COO-(C₁₋₆ alkyl);
   at least one R^{1b} is other than a hydrogen atom;
   when two or more R^{1b}s are present, respective R^{1b}s may be the same or different from each other;
   k is an integer of 0 to 3;
   when k is 2 or 3, respective R^{1c}s may be the same or different from each other;
   ring B is a group represented by the following formula: 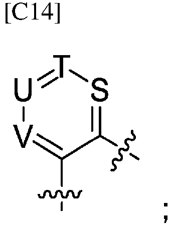
   thiazole or thiophene;
   S, T, U, and V are each independently -CH=, -CR^{1c}=, or -N=;

      [C15] - - -
   represents a single bond or a double bond;
   ring Z is C₆₋₁₀ aryl, 5- or 6-membered heteroaryl, C₃₋₈ cycloalkyl, or a ring represented by the following formula: 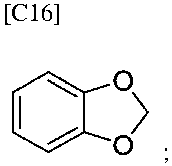
   R⁵ is a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, C₂₋₆ alkenyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, respective R⁴s may be the same as or different from each other;
   provided that when ring A is a group represented by Formula (2), m is an integer of 1 to 3;
   p is an integer of 1 to 3; and
   when p is 2 or 3, respective R³ and R^{3'} may be the same as or different from each other]
   or a pharmacologically acceptable salt thereof.
[A-5] The compound according to any one of the above [A-1] to [A-4], which is a compound in which R⁵ is a hydrogen atom, or a pharmacologically acceptable salt thereof.
[A-6] The compound according to any one of the above [A-1] to [A-5], which is a compound in which L¹ is -NH-, or a pharmacologically acceptable salt thereof.
[A-7] The compound according to any one of the above [A-1] to [A-6], wherein
   W is -CR^{1b}=;
   R^{1b} is a hydrogen atom, a halogen atom, cyano, C₁₋₆ alkyl, C₂₋₆ alkenyl, hydroxy C₁₋₆ alkyl, carboxy C₁₋₆ alkyl, amino C₁₋₆ alkyl, halo C₁₋₆ alkyl, C₁₋₆ alkoxy C₁₋₆ alkyl, -(C₁₋₆ alkylene)-(3- to 8-membered heterocycloalkyl), or -(C₁₋₆ alkylene)-NH-COO-(C₁₋₆ alkyl);
   S, U, and V are each independently -CH= or -CR^{1c}=; and
   R^{1c} is a halogen atom, carboxy, cyano, C₁₋₆ alkyl, halo C₁₋₆ alkyl, hydroxy C₁₋₆ alkyl, amino C₁₋₆ alkyl, C₁₋₆ alkoxy, -CONH₂, -NH-COO-(C₁₋₆ alkyl), or -NH-CO-NH-(C₁₋₆ alkyl), or a pharmacologically acceptable salt thereof.
[A-8] The compound according to any one of the above [A-1] to [A-7], wherein
   X and Y are each independently -CR^{1b}=;
   T is -CH= or -CR^{1c}=; and
   p is 1 or 2, or a pharmacologically acceptable salt thereof.
[A-9] The compound according to any one of the above [A-1] to [A-8], wherein
   n is 1 or 2;
   k is an integer of 0 to 2, or a pharmacologically acceptable salt thereof.
[A-10] The compound according to any one of the above [A-1] to [A-9], wherein
   R³ and R^{3'} are hydrogen atoms;
   p is 1, or a pharmacologically acceptable salt thereof.
[A-11] The compound according to any one of the above [A-1] to [A-10], wherein
   m is an integer of 1 to 3, or a pharmacologically acceptable salt thereof.
[A-12] The compound according to any one of the above [A-1] to [A-11], which is a compound represented by Formula (A-V): 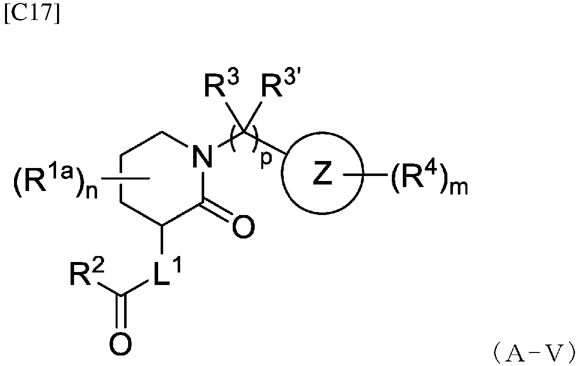 [wherein,
   R^{1a} is a halogen atom, C₁₋₆ alkyl, halo C₁₋₆ alkyl, C₁₋₆ alkoxy C₁₋₆ alkyl, hydroxy C₁₋₆ alkyl, amino C₁₋₆ alkyl, -(C₁₋₆ alkylene)-(3- to 8-membered heterocycloalkyl), which is unsubstituted or substituted with 1 to 6 groups selected from substituent group A, -(C₁₋₆ alkylene)-NH-COO-(C₁₋₆ alkyl), -(C₁₋₆ alkylene)-NH-CO-(C₁₋₆ alkyl), or -(C₁₋₆ alkylene)-CON(C₁₋₆ alkyl)₂;
   substituent group A is a group consisting of a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkoxy, C₃₋₈ cycloalkyl C₁₋₆ alkyl, and C₃₋₈ cycloalkyl C₁₋₆ alkoxy;
   when substituted with two or more groups selected from substituent group A, the respective groups may be the same as or different from each other;
   n is an integer of 1 to 3;
   when n is 2 or 3, respective R^{1a}s may be the same as or different from each other;
   two R^{1a}s may be taken together to form a 3- to 8-membered saturated carbocycle, or a 3- to 8-membered saturated heterocycle;
   R^{1a} may be taken together with R³ to form a 5- to 8-membered saturated heterocycle or a 5- to 8-membered unsaturated heterocycle;
   ring Z is C₆₋₁₀ aryl, 5- or 6-membered heteroaryl, C₃₋₈ cycloalkyl, or a ring represented by the following formula: 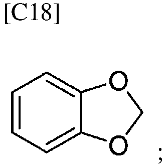
   R² is C₁₋₆ alkyl, C₃₋₈ cycloalkyl, C₁₋₆ alkoxy, C₁₋₆ alkoxy C₁₋₆ alkyl, or -NR⁵R^{5'};
   R⁵ and R^{5'} are each independently a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, C₂₋₆ alkenyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, respective R⁴s may be the same as or different from each other;
   p is an integer of 1 to 3;
   when p is 2 or 3, respective R³ and R^{3'} may be the same as or different from each other]
   or a pharmacologically acceptable salt thereof.
[A-13] The compound according to any one of the above [A-1] to [A-12], which is a compound represented by Formula (A-VI): 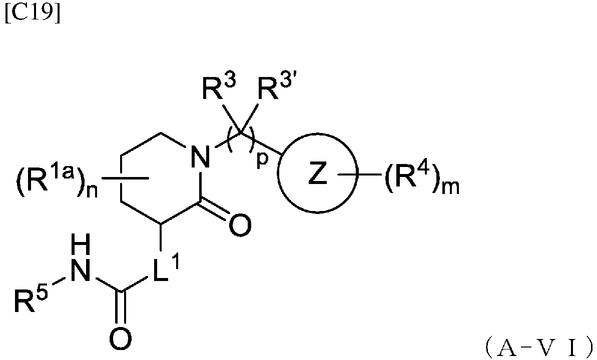 [wherein,
   R^{1a} is a halogen atom, C₁₋₆ alkyl, halo C₁₋₆ alkyl, C₁₋₆ alkoxy C₁₋₆ alkyl, hydroxy C₁₋₆ alkyl, amino C₁₋₆ alkyl, -(C₁₋₆ alkylene)-(3- to 8-membered heterocycloalkyl), which is unsubstituted or substituted with 1 to 6 groups selected from substituent group A, -(C₁₋₆ alkylene)-NH-COO-(C₁₋₆ alkyl), -(C₁₋₆ alkylene)-NH-CO-(C₁₋₆ alkyl), or -(C₁₋₆ alkylene)-CON(C₁₋₆ alkyl)₂;
   substituent group A is a group consisting of a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkoxy, C₃₋₈ cycloalkyl C₁₋₆ alkyl, and C₃₋₈ cycloalkyl C₁₋₆ alkoxy;
   when substituted with two or more groups selected from substituent group A, the respective groups may be the same as or different from each other;
   n is an integer of 1 to 3;
   when n is 2 or 3, respective R^{1a}s may be the same as or different from each other;
   two R^{1a}s may be taken together to form a 3- to 8-membered saturated carbocycle, or a 3- to 8-membered saturated heterocycle;
   R^{1a} may be taken together with R³ to form a 5- to 8-membered saturated heterocycle or a 5- to 8-membered unsaturated heterocycle;
   ring Z is C₆₋₁₀ aryl, 5- or 6-membered heteroaryl, C₃₋₈ cycloalkyl, or a ring represented by the following formula: 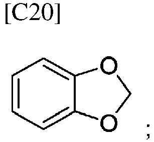
   R⁵ is a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, C₂₋₆ alkenyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, respective R⁴s may be the same as or different from each other;
   p is an integer of 1 to 3; and
   when p is 2 or 3, respective R³ and R^{3'} may be the same or different from each other]
   or a pharmacologically acceptable salt thereof.
[A-14] The compound according to any one of the above [A-1] to [A-11], which is a compound represented by Formula (A-VII): 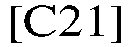 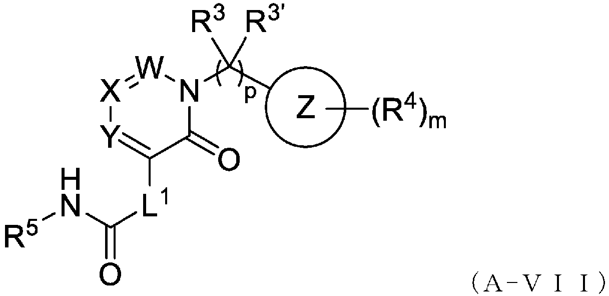 [wherein,
   W, X, and Y are each independently -CR^{1b}= or -N=;
   R^{1b} is a hydrogen atom, a halogen atom, cyano, C₁₋₆ alkyl, C₂₋₆ alkenyl, hydroxy C₁₋₆ alkyl, carboxy C₁₋₆ alkyl, amino C₁₋₆ alkyl, halo C₁₋₆ alkyl, C₁₋₆ alkoxy, C₁₋₆ alkoxy C₁₋₆ alkyl, -(C₁₋₆ alkylene)-(3- to 8-membered heterocycloalkyl) unsubstituted or substituted with 1 to 6 groups selected from substituent group A, or -(C₁₋₆ alkylene)-NH-COO-(C₁₋₆ alkyl);
   substituent group A is a group consisting of a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkoxy, C₃₋₈ cycloalkyl C₁₋₆ alkyl, and C₃₋₈ cycloalkyl C₁₋₆ alkoxy;
   when substituted with two or more groups selected from substituent group A, the respective groups may be the same as or different from each other;
   at least one R^{1b} is other than a hydrogen atom;
   when two or more R^{1b}s are present, respective R^{1b}s may be the same or different from each other;
   ring Z is C₆₋₁₀ aryl, 5- or 6-membered heteroaryl, C₃₋₈ cycloalkyl, or a ring represented by the following formula: 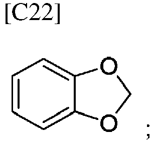
   R⁵ is a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, C₂₋₆ alkenyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
   L¹ is -CR⁶R^{6'}-, -O-, or -NR⁶-;
   R⁶ and R^{6'} are each independently a hydrogen atom or C₁₋₆ alkyl;
   m is an integer of 1 to 3;
   when m is 2 or 3, respective R⁴s may be the same as or different from each other;
   p is an integer of 1 to 3; and
   when p is 2 or 3, respective R³ and R^{3'} may be the same as or different from each other]
   or a pharmacologically acceptable salt thereof.
[A-15] The compound according to any one of the above [A-1] to [A-11], which is a compound represented by Formula (A-VIII): 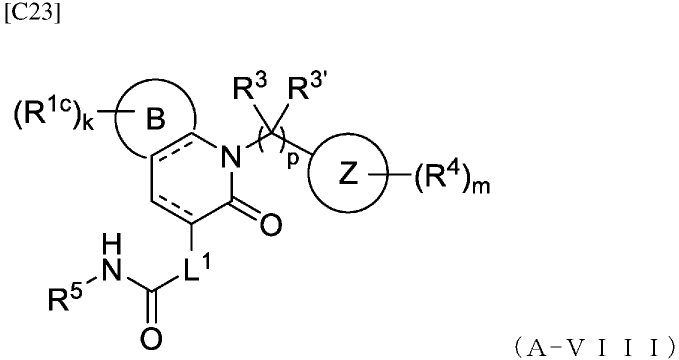 [wherein,
   R^{1c} is a halogen atom, hydroxy, carboxy, cyano, C₁₋₆ alkyl, halo C₁₋₆ alkyl, hydroxy C₁₋₆ alkyl, amino C₁₋₆ alkyl, C₁₋₆ alkoxy, -CONH₂, -NH-COO-(C₁₋₆ alkyl), or -NH-CO-NH-(C₁₋₆ alkyl);
   k is an integer of 0 to 3;
   when k is 2 or 3, respective R^{1c}s may be the same or different from each other;
   ring B is a group represented by the following formula: 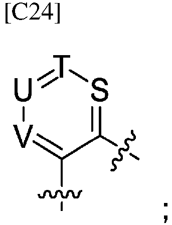
   thiazole or thiophene;
   S, T, U, and V are each independently -CH=, -CR^{1c}=, or -N=;

      [C25] - - -
   represents a single bond or a double bond;
   ring Z is C₆₋₁₀ aryl, 5- or 6-membered heteroaryl, C₃₋₈ cycloalkyl, or a ring represented by the following formula: 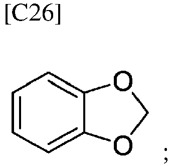
   R⁵ is a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, C₂₋₆ alkenyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, respective R⁴s may be the same as or different from each other;
   p is an integer of 1 to 3; and
   when p is 2 or 3, respective R³ and R^{3'} may be the same as or different from each other]
   or a pharmacologically acceptable salt thereof.
[A-16] The compound according to any one of the above [A-1] to [A-11], wherein
   when ring A is a group represented by Formula (2) or Formula (3), R⁵ is a hydrogen atom, methyl or cyclopropyl, or a pharmacologically acceptable salt thereof.
[A-17] The compound according to any one of the above [A-1] to [A-16], wherein
   R⁵ and R^{5'} are each independently a hydrogen atom, methyl, or cyclopropyl, or a pharmacologically acceptable salt thereof.
[A-18] The compound according to any one of the above [A-1] to [A-17], wherein
   R² is -NHR⁵, or a pharmacologically acceptable salt thereof.
[A-19] The compound according to any one of the above [A-1] to [A-18], which is a compound in which R⁵ is a hydrogen atom, or a pharmacologically acceptable salt thereof.
[A-20] The compound according to any one of the above [A-1] to [A-19], which is a compound in which L¹ is -NH-, or a pharmacologically acceptable salt thereof.
[A-21] The compound according to any one of the above [A-1] to [A-20], wherein
   W is -CR^{1b}=, or a pharmacologically acceptable salt thereof.
[A-22] The compound according to any one of the above [A-1] to [A-21], wherein
   X and Y are each independently -CR^{1b}=, or a pharmacologically acceptable salt thereof.
[A-23] The compound according to any one of the above [A-1] to [A-22], wherein
   R^{1b} is a hydrogen atom, a halogen atom, cyano, C₁₋₆ alkyl, carboxy C₁₋₆ alkyl, halo C₁₋₆ alkyl, or -(C₁₋₆ alkylene)-NH-COO-(C₁₋₆ alkyl), or a pharmacologically acceptable salt thereof.
[A-24] The compound according to any one of the above [A-1] to [A-23], wherein
   R^{1b} is a hydrogen atom, a halogen atom, C₁₋₆ alkyl, halo C₁₋₆ alkyl, or -(C₁₋₆ alkylene)-NH-COO-(C₁₋₆ alkyl), or a pharmacologically acceptable salt thereof.
[A-25] The compound according to any one of the above [A-1] to [A-24], wherein
   S, U, and V are each independently -CH= or -CR^{1c}=, or a pharmacologically acceptable salt thereof.
[A-26] The compound according to any one of the above [A-1] to [A-25], wherein
   T is -CH= or -CR^{1c}=, or a pharmacologically acceptable salt thereof.
[A-27] The compound according to any one of the above [A-1] to [A-26], wherein
   R^{1c} is a halogen atom, carboxy, cyano, C₁₋₆ alkyl, halo C₁₋₆ alkyl, hydroxy C₁₋₆ alkyl, C₁₋₆ alkoxy, or -CONH₂, or a pharmacologically acceptable salt thereof.
[A-28] The compound according to any one of the above [A-1] to [A-27], wherein
   R^{1c} is a halogen atom, C₁₋₆ alkyl, halo C₁₋₆ alkyl, or hydroxy C₁₋₆ alkyl, or a pharmacologically acceptable salt thereof.
[A-29] The compound according to any one of the above [A-1] to [A-28], wherein
   R^{1a} is a halogen atom, C₁₋₆ alkyl, halo C₁₋₆ alkyl, C₁₋₆ alkoxy C₁₋₆ alkyl, -(C₁₋₆ alkylene)-(3- to 8-membered heterocycloalkyl), which is unsubstituted or substituted with 1 to 6 groups selected from substituent group A, or -(C₁₋₆ alkylene)-NH-COO-(C₁₋₆ alkyl);
   substituent group A has the same meaning as in the above [A-1], or a pharmacologically acceptable salt thereof.
[A-30] The compound according to any one of the above [A-1] to [A-29], wherein
   R^{1a} is C₁₋₆ alkyl, halo C₁₋₆ alkyl, C₁₋₆ alkoxy C₁₋₆ alkyl, -(C₁₋₆ alkylene)-(3- to 8-membered heterocycloalkyl) unsubstituted or substituted with 1 to 6 groups selected from substituent group A, or -(C₁₋₆ alkylene)-NH-COO-(C₁₋₆ alkyl);
   substituent group A has the same meaning as in [A-1], or a pharmacologically acceptable salt thereof.
[A-31] The compound according to any one of the above [A-1] to [A-30], wherein
   n is 1 or 2, or a pharmacologically acceptable salt thereof.
[A-32] The compound according to any one of the above [A-1] to [A-31], wherein
   k is an integer of 0 to 2, or a pharmacologically acceptable salt thereof.
[A-33] The compound according to any one of the above [A-1] to [A-32], wherein
   k is 1 or 2, or a pharmacologically acceptable salt thereof.
[A-34] The compound according to any one of the above [A-1] to [A-33], wherein
   R³ and R^{3'} are each a hydrogen atom or C₁₋₆ alkyl;
   p is 1 or 2, or a pharmacologically acceptable salt thereof.
[A-35] The compound according to any one of the above [A-1] to [A-34], wherein
   R³ and R^{3'} are hydrogen atoms;
   p is 1 or 2, or a pharmacologically acceptable salt thereof.
[A-36] The compound according to any one of the above [A-1] to [A-35], wherein
   p is 1 or 2, or a pharmacologically acceptable salt thereof.
[A-37] The compound according to any one of the above [A-1] to [A-36], wherein
   p is 1, or a pharmacologically acceptable salt thereof.
[A-38] The compound according to any one of the above [A-1] to [A-37], wherein
   ring Z is C₆₋₁₀ aryl, or a pharmacologically acceptable salt thereof.
[A-39] The compound according to any one of the above [A-1] to [A-38], wherein
   R⁴ is a halogen atom, cyano, C₁₋₆ alkyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅, or a pharmacologically acceptable salt thereof.
[A-40] The compound according to any one of the above [A-1] to [A-39], wherein
   R⁴ is a halogen atom, C₁₋₆ alkyl, halo C₁₋₆ alkyl, halohydroxy C₁₋₆ alkyl, halo C₁₋₆ alkoxy, C₆₋₁₀ aryl C₁₋₆ alkyl, C₆₋₁₀ aryl C₂₋₆ alkynyl, 5- or 6-membered heteroaryl C₂₋₆ alkynyl, or -SF₅, or a pharmacologically acceptable salt thereof.
[A-41] The compound according to any one of the above [A-1] to [A-40], which is a compound in which m is an integer of 1 to 3, or a pharmacologically acceptable salt thereof.
[A-42] The compound according to any one of the above [A-1] to [A-41], selected from the group consisting of the following compounds: 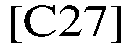 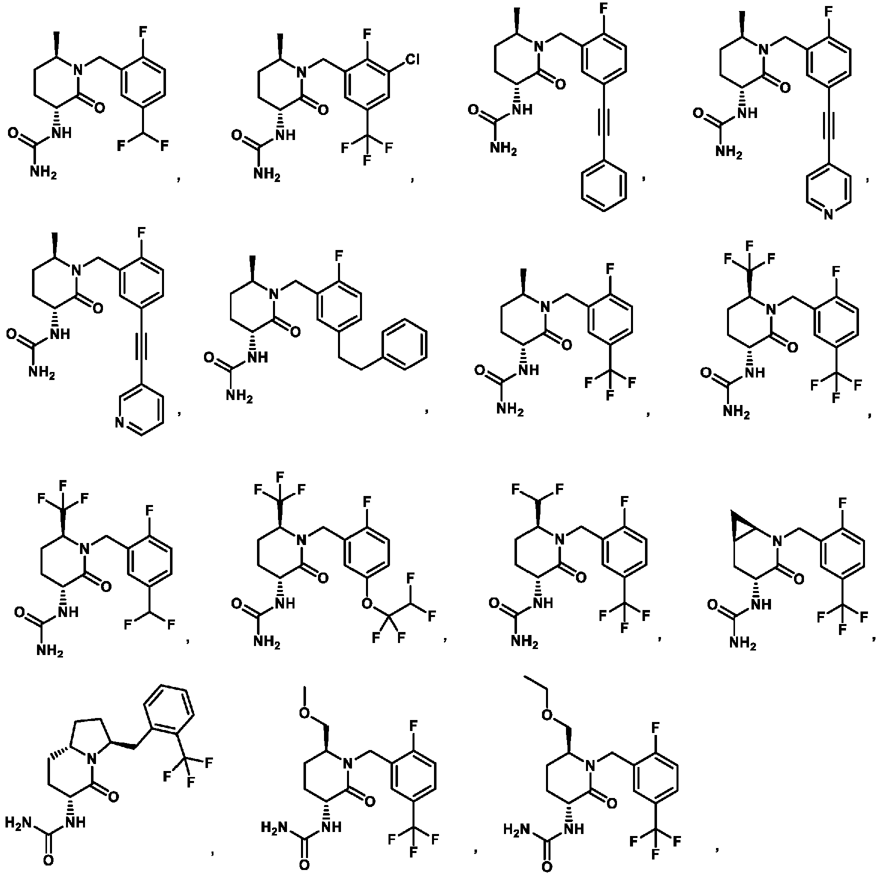 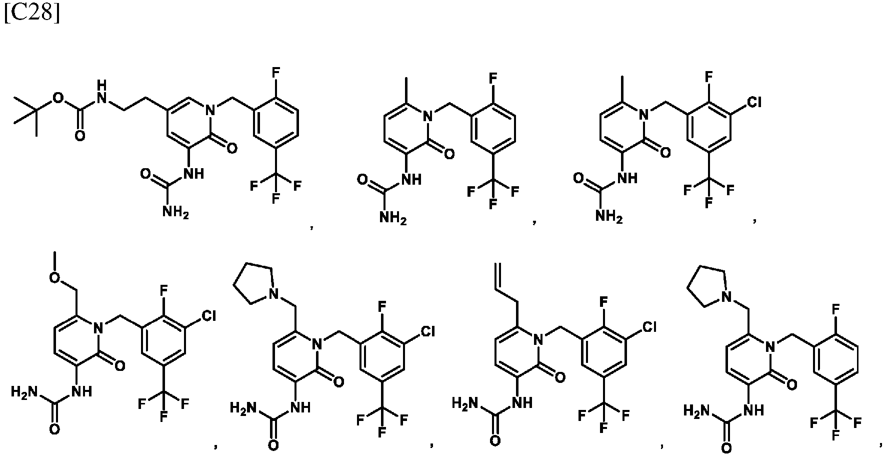 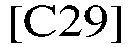 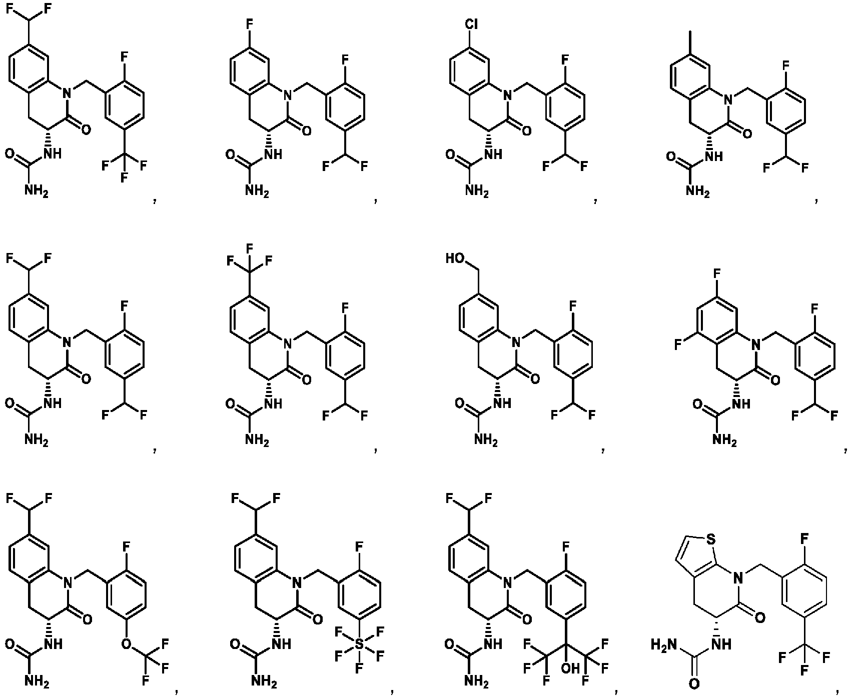 and 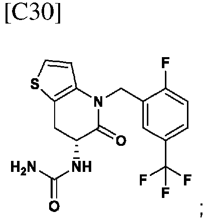 or a pharmacologically acceptable salt thereof.
[A-43] The compound according to any one of the above [A-1] to [A-42], selected from the group consisting of the following compounds: 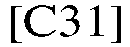 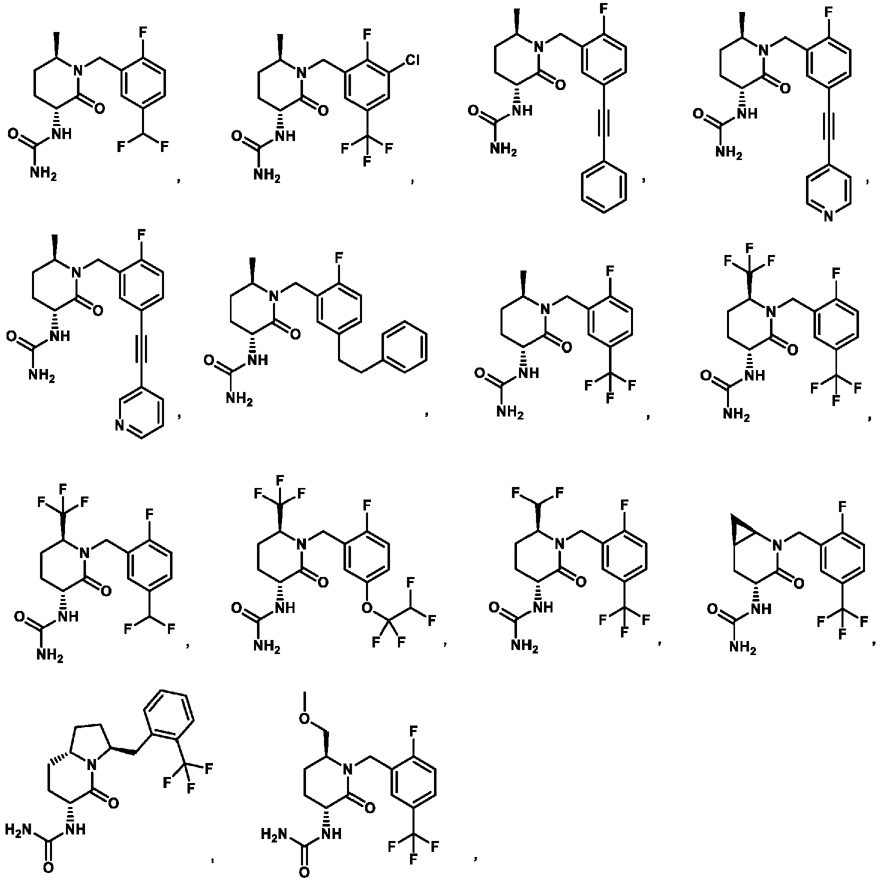 and 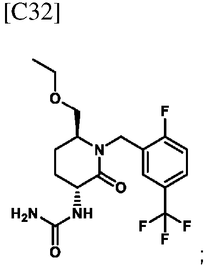 or a pharmacologically acceptable salt thereof.
[A-44] The compound according to any one of the above [A-1] to [A-42], selected from the group consisting of the following compounds: 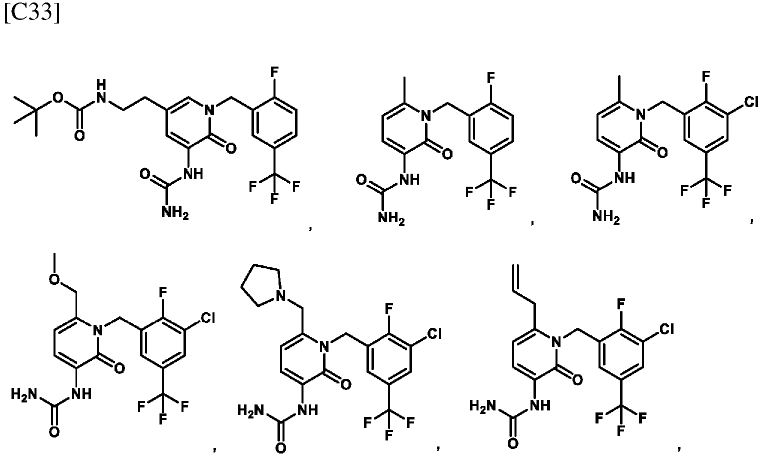 and 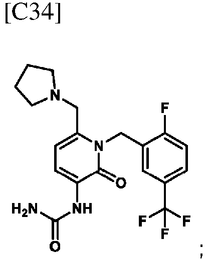 or a pharmacologically acceptable salt thereof.
[A-45] The compound according to any one of the above [A-1] to [A-42], selected from the group consisting of the following compounds: 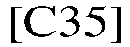 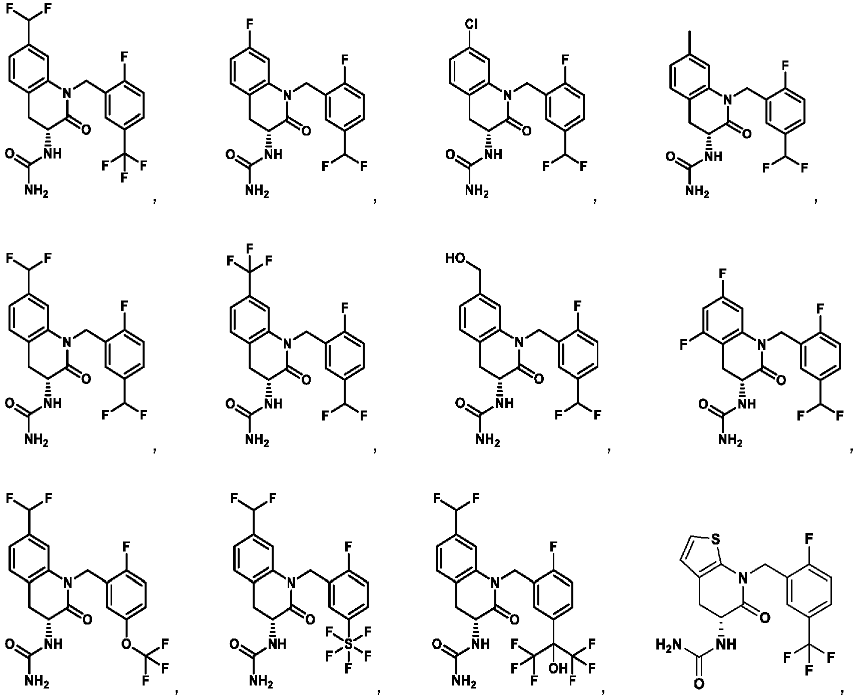 and 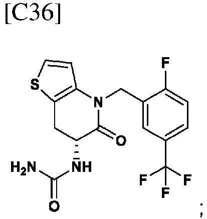 or a pharmacologically acceptable salt thereof.
[A-46] The compound according to any one of the above [A-1] to [A-41], selected from the group consisting of the following compounds: 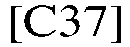 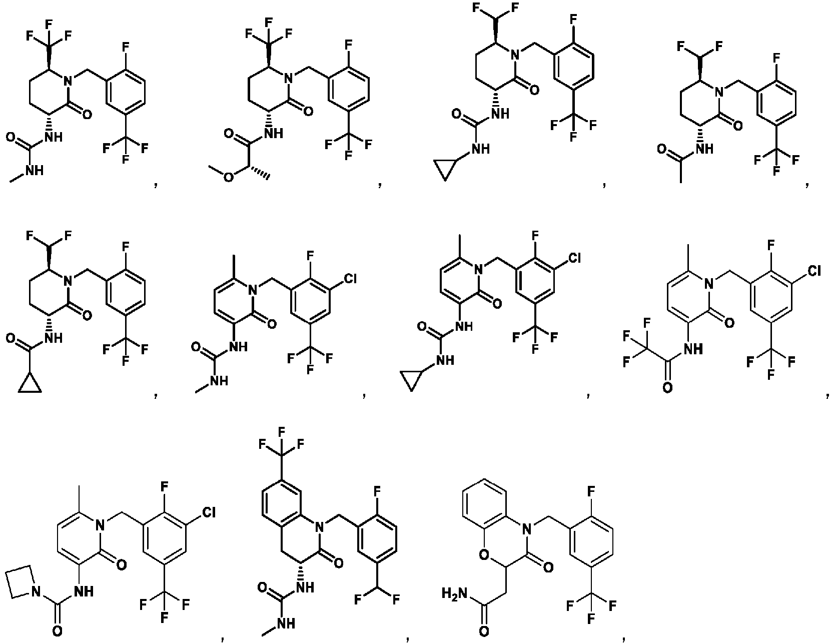 and 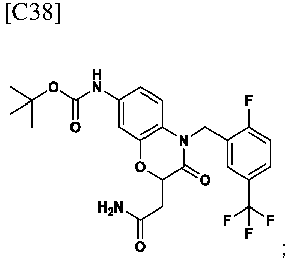 or a pharmacologically acceptable salt thereof.
[A-47] A pharmaceutical composition comprising the compound according to any one of the above [A-1] to [A-46] or a pharmacologically acceptable salt thereof, and a pharmaceutical additive.
[A-48] The pharmaceutical composition according to the above [A-47], which is a pharmaceutical composition for treating thyroid-related diseases.
[A-49] The pharmaceutical composition according to the above [A-48], wherein the thyroid-related diseases are hyperthyroidism, Graves' disease, or thyroid eye disease.
[A-50] A TSHR inhibitor comprising, as an active ingredient, the compound according to any one of the above [A-1] to [A-46], or a pharmacologically acceptable salt thereof.
[A-51] A therapeutic agent for thyroid-related diseases, comprising, as an active ingredient, the compound according to any one of the above [A-1] to [A-46], or a pharmacologically acceptable salt thereof.

As one embodiment of the compounds represented by Formulas (A-I) to (A-VI), for example, when two R^{1a}s are taken together to form a 3- to 8-membered saturated carbocycle, compounds that form a ring represented by the following Formula (a) or (b): 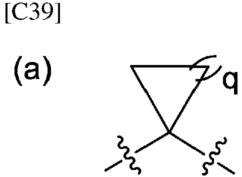 (wherein, q is an integer of 1 to 4), 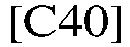 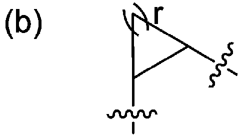
(wherein, r is an integer of 1 to 4);
are exemplified.

As one embodiment, the present invention relates to a method for treating thyroid-related diseases, comprising administering a required amount of the pharmaceutical composition according to the above [A-47] to a patient.

As one embodiment, the present invention relates to a use of the compound according to any one of the above [A-1] to [A-46] or a pharmacologically acceptable salt thereof for manufacturing a pharmaceutical composition for treating thyroid-related diseases.
[B-1] A compound represented by Formula (I): 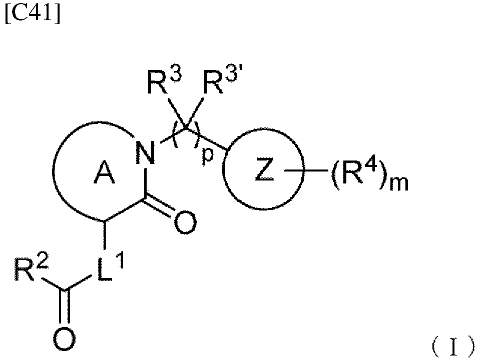 [wherein,
   ring A is a group represented by Formula (1), (2), or (3) below: 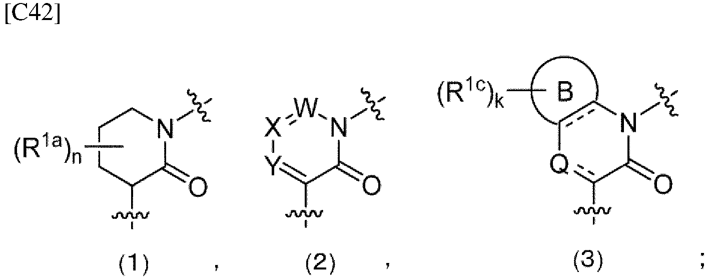
   W, X, and Y are each independently -CR^{1b}= or -N=;
   Q is -CR^{Q1}=, -CR^{Q1}R^{Q1'}-, -O-, or -NR^{Q2}-;
   R^{Q1} and R^{Q1'} are each independently a hydrogen atom, a halogen atom, hydroxy, C₁₋₆ alkyl, or halo C₁₋₆ alkyl;
   R^{Q2} is a hydrogen atom, C₁₋₆ alkyl, or a halo C₁₋₆ alkyl;
   ring B is 5- or 6-membered heteroaryl or benzene;
   R^{1a} and R^{1c} are each independently a halogen atom, hydroxy, carboxy, cyano, C₁₋₆ alkyl, C₁₋₆ alkoxy, halo C₁₋₆ alkyl, halo C₁₋₆ alkoxy, C₁₋₆ alkoxy C₁₋₆ alkyl, C₃₋₈ cycloalkyl, C₃₋₈ cycloalkoxy, hydroxy C₁₋₆ alkyl, carboxy C₁₋₆ alkyl, amino C₁₋₆ alkyl, cyano C₁₋₆ alkyl, - NR⁷COR⁸, -NR⁷COOR⁹, -NR⁷CONR¹⁰R¹¹, -CONR¹²R¹³, -(C₁₋₆ alkylene)-NR⁷COR⁸, -(C₁₋₆ alkylene)-NR⁷COOR⁹, -(C₁₋₆ alkylene)-NR⁷CONR¹⁰R¹¹, or -(C₁₋₆ alkylene)-CONR¹²R¹³;
   R^{1b} is a hydrogen atom, a halogen atom, hydroxy, carboxy, cyano, C₁₋₆ alkyl, C₁₋₆ alkoxy, halo C₁₋₆ alkyl, halo C₁₋₆ alkoxy, C₁₋₆ alkoxy C₁₋₆ alkyl, C₃₋₈ cycloalkyl, C₃₋₈ cycloalkoxy, hydroxy C₁₋₆ alkyl, carboxy C₁₋₆ alkyl, amino C₁₋₆ alkyl, cyano C₁₋₆ alkyl, - NR⁷COR⁸, -NR⁷COOR⁹, -NR⁷CONR¹⁰R¹¹, -CONR¹²R¹³, -(C₁₋₆ alkylene)-NR⁷COR⁸, -(C₁₋₆ alkylene)-NR⁷COOR⁹, -(C₁₋₆ alkylene)-NR⁷CONR¹⁰R¹¹, or -(C₁₋₆ alkylene)-CONR¹²R¹³;
   R⁷ is a hydrogen atom or C₁₋₆ alkyl;
   R⁸, R⁹, R¹⁰, R¹¹, R¹², and R¹³ are each independently a hydrogen atom or a group selected from the group consisting of the following (i) to (v):
      (i) C₁₋₆ alkyl which is unsubstituted or substituted with 1 to 6 groups selected from substituent group A,
      (ii) C₃₋₈ cycloalkyl which is unsubstituted or substituted with 1 to 6 groups selected from substituent group A,
      (iii) C₃₋₈ cycloalkyl C₁₋₆ alkyl which is unsubstituted or substituted with 1 to 6 groups selected from substituent group A,
      (iv) C₆₋₁₀ aryl which is unsubstituted or substituted with 1 to 6 groups selected from substituent group A, and
      (v) C₆₋₁₀ aryl C₁₋₆ alkyl which is unsubstituted or substituted with 1 to 6 groups selected from substituent group A,
   substituent group A is a group consisting of a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkoxy, C₃₋₈ cycloalkyl C₁₋₆ alkyl, and C₃₋₈ cycloalkyl C₁₋₆ alkoxy;
   when substituted with two or more groups selected from substituent group A, the respective groups may be the same as or different from each other;
   R¹⁰ and R¹¹ may be taken together to form a 3- to 10-membered saturated heterocycle or a 3- to 10-membered unsaturated heterocycle;
   R¹² and R¹³ may be taken together to form a 3- to 10-membered saturated heterocycle or a 3- to 10-membered unsaturated heterocycle;
   n is an integer of 1 to 3;
   when n is 2 or 3, respective R^{1a}s may be the same as or different from each other;
   when two or more R^{1b}s are present, respective R^{1b}s may be the same or different from each other;
   k is an integer of 0 to 3;
   when k is 2 or 3, respective R^{1c}s may be the same or different from each other;
   two R^{1a}s may be taken together to form C₃₋₈ cycloalkyl;

      [C43] - - -
   represents a single bond or a double bond;
   ring Z is C₆₋₁₀ aryl, 5- to 10-membered heteroaryl, C₃₋₈ cycloalkyl, or 3- to 8-membered heterocycloalkyl;
   R² is C₁₋₆ alkyl, C₁₋₆ alkoxy, halo C₁₋₆ alkyl, halo C₁₋₆ alkoxy, C₁₋₆ alkoxy C₁₋₆ alkyl, C₃₋₈ cycloalkyl, C₃₋₈ cycloalkoxy, 3- to 8-membered heterocycloalkyl, or -NR⁵R^{5'};
   provided that when ring A is a group represented by Formula (2) or Formula (3), R² is -NHR⁵;
   R⁵ and R^{5'} are each independently a hydrogen atom, C₁₋₆ alkyl, halo C₁₋₆ alkyl, or C₃₋₈ cycloalkyl;
   R³ and R^{3'} are each independently a hydrogen atom, a halogen atom, cyano, C₁₋₆ alkyl, C₁₋₆ alkoxy, C₁₋₆ alkoxy C₁₋₆ alkyl, hydroxy C₁₋₆ alkyl, amino C₁₋₆ alkyl, cyano C₁₋₆ alkyl, -NR¹⁴COR¹⁵, -NR¹⁴COOR¹⁶, -NR¹⁴CONR¹⁷R¹⁸, -CONR¹⁹R²⁰, -(C₁₋₆ alkylene)-NR¹⁴COR¹⁵, -(C₁₋₆ alkylene)-NR¹⁴COOR¹⁶, -(C₁₋₆ alkylene)-NR¹⁴CONR¹⁷R¹⁸, or -(C₁₋₆ alkylene)-CONR¹⁹R²⁰;
   R¹⁴, R¹⁵, R¹⁶, R¹⁷, R¹⁸, R¹⁹, and R²⁰ are each independently a hydrogen atom or C₁₋₆ alkyl;
   R³ and R^{3'} may be taken together to form an oxo group;
   R⁴ is a halogen atom, cyano, C₁₋₆ alkyl, C₁₋₆ alkoxy, halo C₁₋₆ alkyl, halo C₁₋₆ alkoxy, C₁₋₆ alkoxy C₁₋₆ alkyl, C₃₋₈ cycloalkyl, C₃₋₈ cycloalkoxy, C₃₋₈ cycloalkyl C₁₋₆ alkyl, C₃₋₈ cycloalkyl C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₁₋₆ alkyl, 5- or 6-membered heteroaryl C₂₋₆ alkynyl, C₃₋₈ cycloalkyl C₂₋₆ alkynyl, hydroxy C₁₋₆ alkyl, amino C₁₋₆ alkyl, cyano C₁₋₆ alkyl, -NR²¹COR²², -NR²¹COOR²³, -NR²¹CONR²⁴R²⁵, -CONR²⁶R²⁷, -(C₁₋₆ alkylene)-NR²¹COR²², -(C₁₋₆ alkylene)-NR²¹COOR²³, -(C₁₋₆ alkylene)-NR²¹CONR²⁴R²⁵, -(C₁₋₆ alkylene)-CONR²⁶R²⁷, a halohydroxy C₁₋₆ alkyl, or -SF₅;
   R²¹, R²², R²³, R²⁴, R²⁵, R²⁶, and R²⁷ are each independently a hydrogen atom or C₁₋₆ alkyl;
   R^{1a}, R^{1b}, or R^{1c} may be taken together with R³ to form a 5- to 8-membered saturated heterocycle or a 5- to 8-membered unsaturated heterocycle;
   R⁴ and R³ may be taken together to form a 5- to 8-membered ring;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
      when m is 2 or 3, each R ⁴ may be the same as or different from each other;
   provided that when ring A is a group represented by Formula (2), m is an integer of 1 to 3;
   p is an integer of 0 to 3; and
   when p is 2 or 3, each R³ and R^{3'} may be the same as or different from each other]
   or a pharmacologically acceptable salt thereof.
[B-2] The compound according to the above [B-1], which is a compound represented by Formula (II): 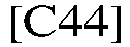 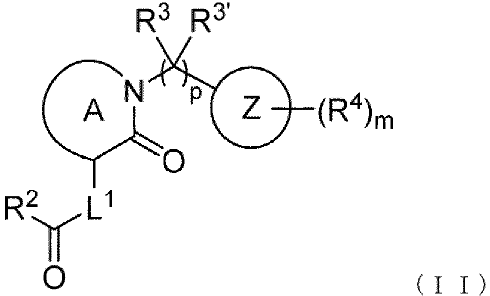 [wherein,
   ring A is a group represented by Formula (1), (2), or (3) below: 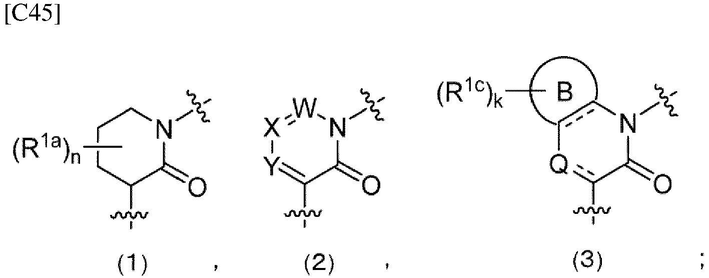
   W, X, and Y are each independently -CR^{1b}= or -N=;
   Q is -CR^{Q1}=, -CR^{Q1}R^{Q1'}-, -O-, or -NR^{Q2}-;
   R^{Q1} and R^{Q1'} are each independently a hydrogen atom, a halogen atom, hydroxy, C₁₋₆ alkyl, or halo C₁₋₆ alkyl;
   R^{Q2} is a hydrogen atom, C₁₋₆ alkyl, or a halo C₁₋₆ alkyl;
   ring B is 5- or 6-membered heteroaryl or benzene;
   R^{1a} and R^{1c} are each independently a halogen atom, hydroxy, carboxy, cyano, C₁₋₆ alkyl, C₁₋₆ alkoxy, halo C₁₋₆ alkyl, hydroxy C₁₋₆ alkyl, carboxy C₁₋₆ alkyl, -NR⁷COR⁸, - NR⁷COOR⁹, -NR⁷CONR¹⁰R¹¹, -CONR¹²R¹³, -(C₁₋₆ alkylene)-NR⁷COR⁸, -(C₁₋₆ alkylene)-NR⁷COOR⁹, -(C₁₋₆ alkylene)-NR⁷CONR¹⁰R¹¹, or -(C₁₋₆ alkylene)-CONR¹²R¹³;
   R^{1b} is a hydrogen atom, a halogen atom, hydroxy, carboxy, cyano, C₁₋₆ alkyl, C₁₋₆ alkoxy, halo C₁₋₆ alkyl, hydroxy C₁₋₆ alkyl, carboxy C₁₋₆ alkyl, -NR⁷COR⁸, -NR⁷COOR⁹, - NR⁷CONR¹⁰R¹¹, -CONR¹²R¹³, -(C₁₋₆ alkylene)-NR⁷COR⁸, -(C₁₋₆ alkylene)-NR⁷COOR⁹, -(C₁₋₆ alkylene)-NR⁷CONR¹⁰R¹¹, or -(C₁₋₆ alkylene)-CONR¹²R¹³;
   R⁷ is a hydrogen atom or C₁₋₆ alkyl;
   R⁸, R⁹, R¹⁰, R¹¹, R¹², and R¹³ are each independently a hydrogen atom or a group selected from the group consisting of the following (i) to (v):
      (i) C₁₋₆ alkyl which is unsubstituted or substituted with 1 to 6 groups selected from substituent group A,
      (ii) C₃₋₈ cycloalkyl which is unsubstituted or substituted with 1 to 6 groups selected from substituent group A,
      (iii) C₃₋₈ cycloalkyl C₁₋₆ alkyl which is unsubstituted or substituted with 1 to 6 groups selected from substituent group A,
      (iv) C₆₋₁₀ aryl which is unsubstituted or substituted with 1 to 6 groups selected from substituent group A, and
      (v) C₆₋₁₀ aryl C₁₋₆ alkyl which is unsubstituted or substituted with 1 to 6 groups selected from substituent group A,
   substituent group A is a group consisting of a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkoxy, C₃₋₈ cycloalkyl C₁₋₆ alkyl, and C₃₋₈ cycloalkyl C₁₋₆ alkoxy;
   when a group is substituted with two or more groups selected from substituent group A, the respective groups may be the same or different from each other;
   R¹⁰ and R¹¹ may be taken together to form a 3- to 10-membered saturated heterocycle or a 3- to 10-membered unsaturated heterocycle;
   R¹² and R¹³ may be taken together to form a 3- to 10-membered saturated heterocycle or a 3- to 10-membered unsaturated heterocycle;
   n is an integer of 1 to 3;
   when n is 2 or 3, respective R^{1a}s may be the same as or different from each other;
   when two or more R^{1b}s are present, respective R^{1b}s may be the same or different from each other;
   k is an integer of 0 to 3;
   when k is 2 or 3, respective R^{1c}s may be the same or different from each other;
   two R^{1a}s may be taken together to form C₃₋₈ cycloalkyl;

      [C46] - - -
   represents a single bond or a double bond;
   ring Z is C₆₋₁₀ aryl, 5- or 6-membered heteroaryl, or C₃₋₈ cycloalkyl;
   R² is C₁₋₆ alkyl, C₁₋₆ alkoxy C₁₋₆ alkyl, C₃₋₈ cycloalkyl, or -NR⁵R^{5'};
   provided that when ring A is a group represented by Formula (2) or Formula (3), R² is -NHR⁵;
   R⁵ and R^{5'} are each independently a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkyl C₁₋₆ alkyl, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₁₋₆ alkyl, 5- or 6-membered heteroaryl C₂₋₆ alkynyl, C₃₋₈ cycloalkyl C₂₋₆ alkynyl, halo hydroxy C₁₋₆ alkyl, or - SF₅;
   R^{1a}, R^{1b}, or R^{1c} may be taken together with R³ to form a 5- to 8-membered saturated heterocycle or a 5- to 8-membered unsaturated heterocycle;
   R⁴ and R³ may be taken together to form a 5- to 8-membered ring;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, each R⁴ may be the same as or different from each other;
   provided that when ring A is a group represented by Formula (2), m is an integer of 1 to 3;
   p is an integer of 0 to 3; and
   when p is 2 or 3, respective R³ and R^{3'} may be the same as or different from each other]
   or a pharmacologically acceptable salt thereof.
[B-3] The compound according to the above [B-1] or [B-2], which is a compound represented by Formula (III): 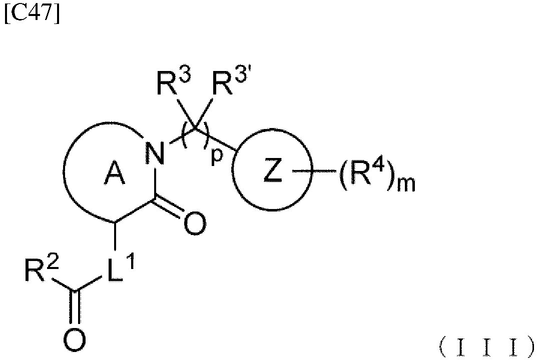 [wherein,
   ring A is a group represented by Formula (1), (2), or (3) below: 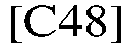 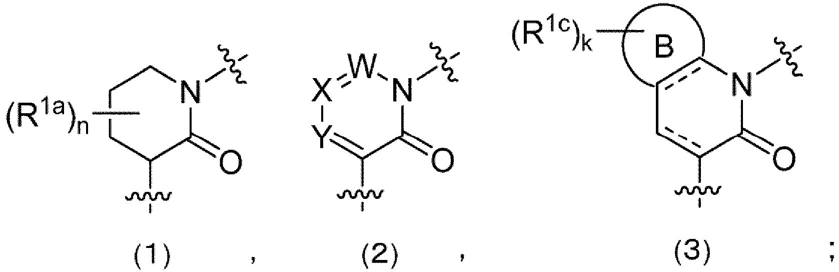
   R^{1a} is a halogen atom, C₁₋₆ alkyl, halo C₁₋₆ alkyl, or -(C₁₋₆ alkylene)-NH-COO-(C₁₋₆ alkyl);
   R^{1c} is a halogen atom, hydroxy, carboxy, cyano, C₁₋₆ alkyl, halo C₁₋₆ alkyl, hydroxy C₁₋₆ alkyl, C₁₋₆ alkoxy, or -CONH₂;
   n is an integer of 1 to 3;
   when n is 2 or 3, respective R^{1a}s may be the same as or different from each other;
   two R^{1a}s may be taken together to form C₃₋₈ cycloalkyl;
   W, X, and Y are each independently -CR^{1b}= or -N=;
   R^{1b} is a hydrogen atom, a halogen atom, cyano, C₁₋₆ alkyl, carboxy C₁₋₆ alkyl, halo C₁₋₆ alkyl, C₁₋₆ alkoxy, or -(C₁₋₆ alkylene)-NH-COO-(C₁₋₆ alkyl);
   at least one R^{1b} is other than a hydrogen atom;
   when two or more R^{1b}s are present, respective R^{1b}s may be the same or different from each other;
   k is an integer of 0 to 3;
   when k is 2 or 3, respective R^{1c}s may be the same or different from each other;
   ring B is a group represented by the following formula: 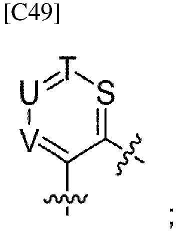
   thiazole or thiophene;
   S, T, U, and V are each independently -CH=, -CR^{1c}=, or -N=;

      [C50] - - -
   represents a single bond or a double bond;
   ring Z is C₆₋₁₀ aryl, 5- or 6-membered heteroaryl, C₃₋₈ cycloalkyl, or a ring represented by the following formula: 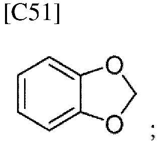
   R² is C₁₋₆ alkyl, C₃₋₈ cycloalkyl, C₁₋₆ alkoxy, C₁₋₆ alkoxy C₁₋₆ alkyl, or -NR⁵R^{5'};
   provided that when ring A is a group represented by Formula (2) or Formula (3), R² is -NHR⁵;
   R⁵ and R^{5'} are each independently a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, each R⁴ may be the same as or different from each other;
   provided that when ring A is a group represented by Formula (2), m is an integer of 1 to 3;
   p is an integer of 1 to 3; and
   when p is 2 or 3, respective R³ and R^{3'} may be the same as or different from each other]
   or a pharmacologically acceptable salt thereof.
[B-4] The compound according to any one of the above [B-1] to [B-3], which is a compound represented by Formula (IV): 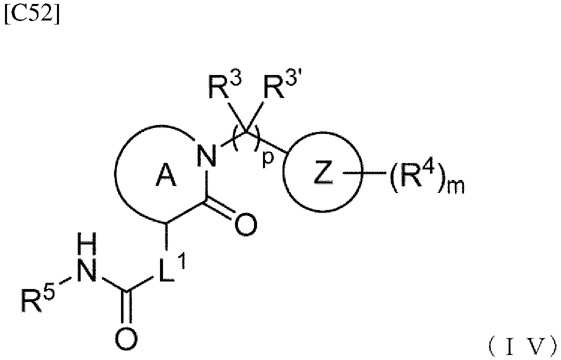 [wherein,
   ring A is a group represented by Formula (1), (2), or (3) below: 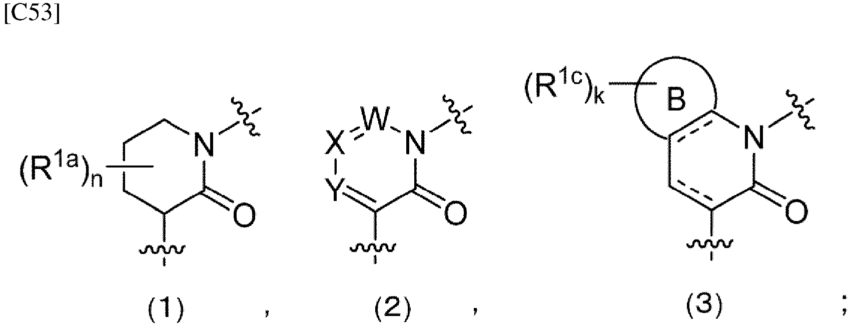
   R^{1a} is a halogen atom, C₁₋₆ alkyl, halo C₁₋₆ alkyl, or -(C₁₋₆ alkylene)-NH-COO-(C₁₋₆ alkyl);
   R^{1c} is a halogen atom, hydroxy, carboxy, cyano, C₁₋₆ alkyl, halo C₁₋₆ alkyl, hydroxy C₁₋₆ alkyl, C₁₋₆ alkoxy, or -CONH₂;
   n is an integer of 1 to 3;
   when n is 2 or 3, respective R^{1a}s may be the same as or different from each other;
   two R^{1a}s may be taken together to form C₃₋₈ cycloalkyl;
   W, X, and Y are each independently -CR^{1b}= or -N=;
   R^{1b} is a hydrogen atom, a halogen atom, cyano, C₁₋₆ alkyl, carboxy C₁₋₆ alkyl, halo C₁₋₆ alkyl, C₁₋₆ alkoxy, or -(C₁₋₆ alkylene)-NH-COO-(C₁₋₆ alkyl);
   at least one R^{1b} is other than a hydrogen atom;
   when two or more R^{1b}s are present, respective R^{1b}s may be the same or different from each other;
   k is an integer of 0 to 3;
   when k is 2 or 3, respective R^{1c}s may be the same or different from each other;
   ring B is a group represented by the following formula: 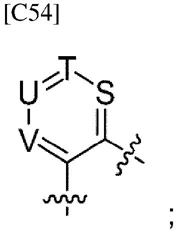
   thiazole or thiophene;
   S, T, U, and V are each independently -CH=, -CR^{1c}=, or -N=;

      [C55] - - -
   represents a single bond or a double bond;
   ring Z is C₆₋₁₀ aryl, 5- or 6-membered heteroaryl, C₃₋₈ cycloalkyl, or a ring represented by the following formula: 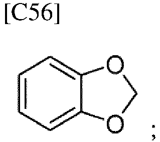
   R⁵ is a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, each R⁴ may be the same as or different from each other;
   provided that when ring A is a group represented by Formula (2), m is an integer of 1 to 3;
   p is an integer of 1 to 3; and
   when p is 2 or 3, respective R³ and R^{3'} may be the same as or different from each other]
   or a pharmacologically acceptable salt thereof.
[B-5] The compound according to any one of the above [B-1] to [B-4], which is a compound in which R⁵ is a hydrogen atom, or a pharmacologically acceptable salt thereof.
[B-6] The compound according to any one of [B-1] to [B-5], which is a compound in which L¹ is -NH-, or a pharmacologically acceptable salt thereof.
[B-7] The compound according to any one of the above [B-1] to [B-6], wherein
   W is -CR^{1b}=;
   R^{1b} is a hydrogen atom, a halogen atom, cyano, C₁₋₆ alkyl, carboxy C₁₋₆ alkyl, halo C₁₋₆ alkyl, or -(C₁₋₆ alkylene)-NH-COO-(C₁₋₆ alkyl);
   S, U, and V are each independently -CH= or -CR^{1c}=; and
   R^{1c} is a halogen atom, carboxy, cyano, C₁₋₆ alkyl, halo C₁₋₆ alkyl, hydroxy C₁₋₆ alkyl, C₁₋₆ alkoxy, or -CONH₂, or a pharmacologically acceptable salt thereof.
[B-8] The compound according to any one of the above [B-1] to [B-7], wherein
   X and Y are each independently -CR^{1b}=;
   T is -CH= or -CR^{1c}=; and
   p is 1 or 2, or a pharmacologically acceptable salt thereof.
[B-9] The compound according to any one of the above [B-1] to [B-8], wherein
   n is 1 or 2;
   k is an integer of 0 to 2, or a pharmacologically acceptable salt thereof.
[B-10] The compound according to any one of the above [B-1] to [B-9], wherein
   R³ and R^{3'} are hydrogen atoms;
   p is 1, or a pharmacologically acceptable salt thereof.
[B-11] The compound according to any one of the above [B-1] to [B-10], wherein
   m is an integer of 1 to 3, or a pharmacologically acceptable salt thereof.
[B-12] The compound according to any one of the above [B-1] to [B-11], which is a compound represented by Formula (V): 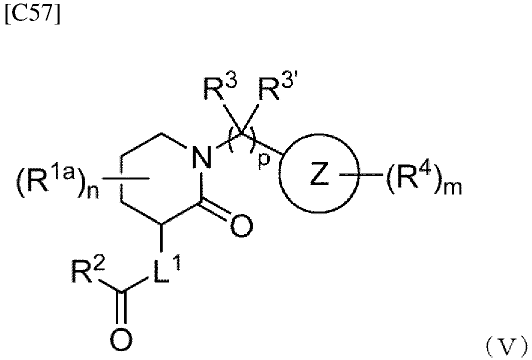 [wherein,
   R^{1a} is a halogen atom, C₁₋₆ alkyl, halo C₁₋₆ alkyl, or -(C₁₋₆ alkylene)-NH-COO-(C₁₋₆ alkyl);
   n is an integer of 1 to 3;
   when n is 2 or 3, respective R^{1a}s may be the same as or different from each other;
   two R^{1a}s may be taken together to form C₃₋₈ cycloalkyl;
   ring Z is C₆₋₁₀ aryl, 5- or 6-membered heteroaryl, C₃₋₈ cycloalkyl, or a ring represented by the following formula: 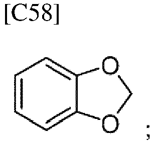
   R² is C₁₋₆ alkyl, C₃₋₈ cycloalkyl, C₁₋₆ alkoxy, C₁₋₆ alkoxy C₁₋₆ alkyl, or -NR⁵R^{5'};
   R⁵ and R^{5'} are each independently a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, each R⁴ may be the same as or different from each other;
   p is an integer of 1 to 3; and
   when p is 2 or 3, respective R³ and R^{3'} may be the same as or different from each other]
   or a pharmacologically acceptable salt thereof.
[B-13] The compound according to any one of the above [B-1] to [B-12], which is a compound represented by Formula (VI): 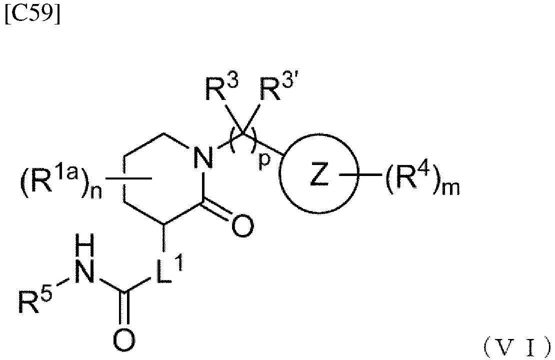 [wherein,
   R^{1a} is a halogen atom, C₁₋₆ alkyl, halo C₁₋₆ alkyl, or -(C₁₋₆ alkylene)-NH-COO-(C₁₋₆ alkyl);
   n is an integer of 1 to 3;
   when n is 2 or 3, respective R^{1a}s may be the same as or different from each other;
   two R^{1a}s may be taken together to form C₃₋₈ cycloalkyl;
   ring Z is C₆₋₁₀ aryl, 5- or 6-membered heteroaryl, C₃₋₈ cycloalkyl, or a ring represented by the following formula: 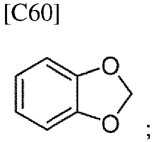
   R⁵ is a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, each R⁴ may be the same as or different from each other;
   p is an integer of 1 to 3; and
   when p is 2 or 3, respective R³ and R^{3'} may be the same as or different from each other]
   or a pharmacologically acceptable salt thereof.
[B-14] The compound according to any one of the above [B-1] to [B-11], which is a compound represented by Formula (VII): 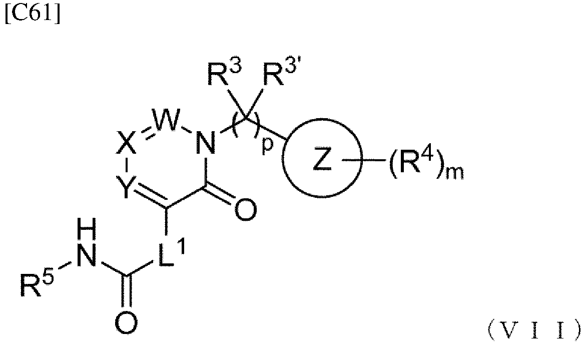 [wherein,
   W, X, and Y are each independently -CR^{1b}= or -N=;
   R^{1b} is a hydrogen atom, a halogen atom, cyano, C₁₋₆ alkyl, carboxy C₁₋₆ alkyl, halo C₁₋₆ alkyl, C₁₋₆ alkoxy, or -(C₁₋₆ alkylene)-NH-COO-(C₁₋₆ alkyl);
   at least one R^{1b} is other than a hydrogen atom;
   when two or more R^{1b}s are present, respective R^{1b}s may be the same or different from each other;
   ring Z is C₆₋₁₀ aryl, 5- or 6-membered heteroaryl, C₃₋₈ cycloalkyl, or a ring represented by the following formula: 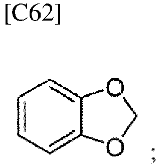
   R⁵ is a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
   L¹ is -CR⁶R^{6'}-, -O-, or -NR⁶-;
   R⁶ and R^{6'} are each independently a hydrogen atom or C₁₋₆ alkyl;
   m is an integer of 1 to 3;
   when m is 2 or 3, respective R⁴s may be the same as or different from each other;
   p is an integer of 1 to 3; and
   when p is 2 or 3, respective R³ and R^{3'} may be the same as or different from each other]
   or a pharmacologically acceptable salt thereof.
[B-15] The compound according to any one of the above [B-1] to [B-11], which is a compound represented by Formula (VIII): 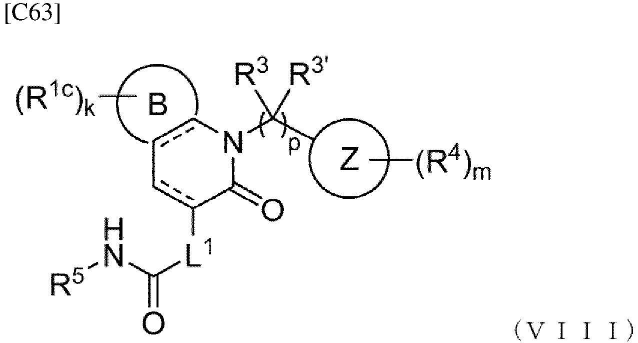 [wherein,
   R^{1c} is a halogen atom, hydroxy, carboxy, cyano, C₁₋₆ alkyl, halo C₁₋₆ alkyl, hydroxy C₁₋₆ alkyl, C₁₋₆ alkoxy, or -CONH₂;
   k is an integer of 0 to 3;
   when k is 2 or 3, respective R^{1c}s may be the same or different from each other;
   ring B is a group represented by the following formula: 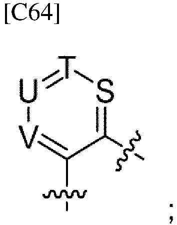
   thiazole or thiophene;
   S, T, U, and V are each independently -CH=, -CR^{1c}=, or -N=;

      [C65] - - -
   represents a single bond or a double bond;
   ring Z is C₆₋₁₀ aryl, 5- or 6-membered heteroaryl, C₃₋₈ cycloalkyl, or a ring represented by the following formula: 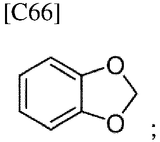
   R⁵ is a hydrogen atom, C₁₋₆ alkyl, or C₃₋₈ cycloalkyl;
   R³ and R^{3'} are each independently a hydrogen atom or C₁₋₆ alkyl;
   R⁴ is a halogen atom, cyano, C₁₋₆ alkyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
   L¹ is -CR⁶R^{6'}-, -O-, or -NR⁶-;
   R⁶ and R^{6'} are each independently a hydrogen atom or C₁₋₆ alkyl;
   m is an integer of 0 to 3;
   when m is 2 or 3, each R⁴ may be the same as or different from each other;
   p is an integer of 1 to 3; and
   when p is 2 or 3, respective R³ and R^{3'} may be the same as or different from each other]
   or a pharmacologically acceptable salt thereof.
[B-16] The compound according to any one of the above [B-1] to [B-11], wherein
   when ring A is a group represented by Formula (2) or Formula (3), R⁵ is a hydrogen atom, methyl or cyclopropyl, or a pharmacologically acceptable salt thereof.
[B-17] The compound according to any one of the above [B-1] to [B-16], wherein
   R⁵ and R^{5'} are each independently a hydrogen atom, methyl, or cyclopropyl, or a pharmacologically acceptable salt thereof.
[B-18] The compound according to any one of the above [B-1] to [B-17], wherein
   R² is -NHR⁵, or a pharmacologically acceptable salt thereof.
[B-19] The compound according to any one of the above [B-1] to [B-18], which is a compound in which R⁵ is a hydrogen atom, or a pharmacologically acceptable salt thereof.
[B-20] The compound according to any one of the above [B-1] to [B-19], which is a compound in which L¹ is -NH-, or a pharmacologically acceptable salt thereof.
[B-21] The compound according to any one of the above [B-1] to [B-20], wherein
   W is -CR^{1b}=, or a pharmacologically acceptable salt thereof.
[B-22] The compound according to any one of the above [B-1] to [B-21], wherein
   X and Y are each independently -CR^{1b}=, or a pharmacologically acceptable salt thereof.
[B-23] The compound according to any one of the above [B-1] to [B-22], wherein
   R^{1b} is a hydrogen atom, a halogen atom, cyano, C₁₋₆ alkyl, carboxy C₁₋₆ alkyl, halo C₁₋₆ alkyl, or -(C₁₋₆ alkylene)-NH-COO-(C₁₋₆ alkyl), or a pharmacologically acceptable salt thereof.
[B-24] The compound according to any one of the above [B-1] to [B-23], wherein
   R^{1b} is a hydrogen atom, a halogen atom, C₁₋₆ alkyl, halo C₁₋₆ alkyl, or -(C₁₋₆ alkylene)-NH-COO-(C₁₋₆ alkyl), or a pharmacologically acceptable salt thereof.
[B-25] The compound according to any one of the above [B-1] to [B-24], wherein
   S, U, and V are each independently -CH= or -CR^{1c}=, or a pharmacologically acceptable salt thereof.
[B-26] The compound according to any one of the above [B-1] to [B-25], wherein
   T is -CH= or -CR^{1c}=, or a pharmacologically acceptable salt thereof.
[B-27] The compound according to any one of the above [B-1] to [B-26], wherein
   R^{1c} is a halogen atom, carboxy, cyano, C₁₋₆ alkyl, halo C₁₋₆ alkyl, hydroxy C₁₋₆ alkyl, C₁₋₆ alkoxy, or -CONH₂, or a pharmacologically acceptable salt thereof.
[B-28] The compound according to any one of the above [B-1] to [B-27], wherein
   R^{1c} is a halogen atom, C₁₋₆ alkyl, halo C₁₋₆ alkyl, or hydroxy C₁₋₆ alkyl, or a pharmacologically acceptable salt thereof.
[B-29] The compound according to any one of the above [B-1] to [B-28], wherein
   R^{1a} is a halogen atom, C₁₋₆ alkyl, halo C₁₋₆ alkyl, or -(C₁₋₆ alkylene)-NH-COO-(C₁₋₆ alkyl), or a pharmacologically acceptable salt thereof.
[B-30] The compound according to any one of the above [B-1] to [B-29], wherein
   R^{1a} is C₁₋₆ alkyl, halo C₁₋₆ alkyl, or -(C₁₋₆ alkylene)-NH-COO-(C₁₋₆ alkyl), or a pharmacologically acceptable salt thereof.
[B-31] The compound according to any one of the above [B-1] to [B-30], wherein
   n is 1 or 2, or a pharmacologically acceptable salt thereof.
[B-32] The compound according to any one of the above [B-1] to [B-31], wherein
   k is an integer of 0 to 2, or a pharmacologically acceptable salt thereof.
[B-33] The compound according to any one of the above [B-1] to [B-32], wherein
   k is 1 or 2, or a pharmacologically acceptable salt thereof.
[B-34] The compound according to any one of the above [B-1] to [B-33], wherein
   R³ and R^{3'} are each a hydrogen atom or C₁₋₆ alkyl;
   p is 1 or 2, or a pharmacologically acceptable salt thereof.
[B-35] The compound according to any one of the above [B-1] to [B-34], wherein
   R³ and R^{3'} are hydrogen atoms;
   p is 1 or 2, or a pharmacologically acceptable salt thereof.
[B-36] The compound according to any one of the above [B-1] to [B-35], wherein
   p is 1 or 2, or a pharmacologically acceptable salt thereof.
[B-37] The compound according to any one of the above [B-1] to [B-36], wherein
   p is 1, or a pharmacologically acceptable salt thereof.
[B-38] The compound according to any one of the above [B-1] to [B-37], wherein
   ring Z is C₆₋₁₀ aryl, or a pharmacologically acceptable salt thereof.
[B-39] The compound according to any one of the above [B-1] to [B-38], wherein
   R⁴ is a halogen atom, cyano, C₁₋₆ alkyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅, or a pharmacologically acceptable salt thereof.
[B-40] The compound according to any one of the above [B-1] to [B-39], wherein
   R⁴ is a halogen atom, C₁₋₆ alkyl, halo C₁₋₆ alkyl, halohydroxy C₁₋₆ alkyl, halo C₁₋₆ alkoxy, C₆₋₁₀ aryl C₁₋₆ alkyl, C₆₋₁₀ aryl C₂₋₆ alkynyl, 5- or 6-membered heteroaryl C₂₋₆ alkynyl, or -SF₅, or a pharmacologically acceptable salt thereof.
[B-41] The compound according to any one of the above [B-1] to [B-40], which is a compound in which m is an integer of 1 to 3, or a pharmacologically acceptable salt thereof.
[B-42] The compound according to any one of the above [B-1] to [B-41], selected from the group consisting of the following compounds: 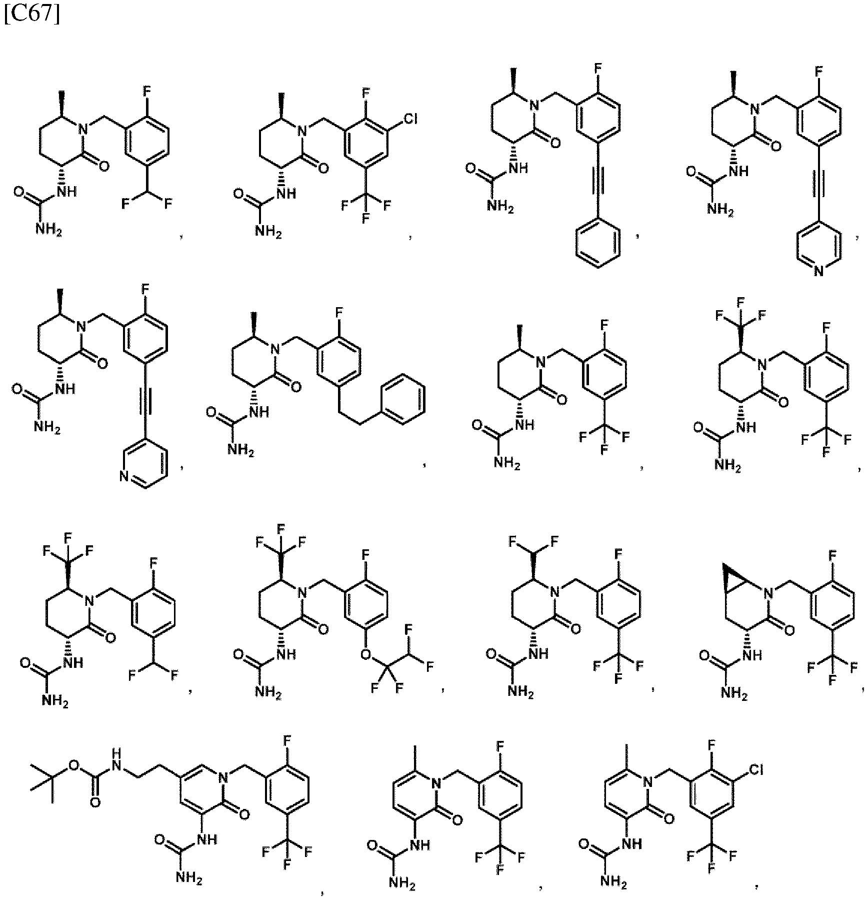 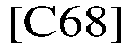 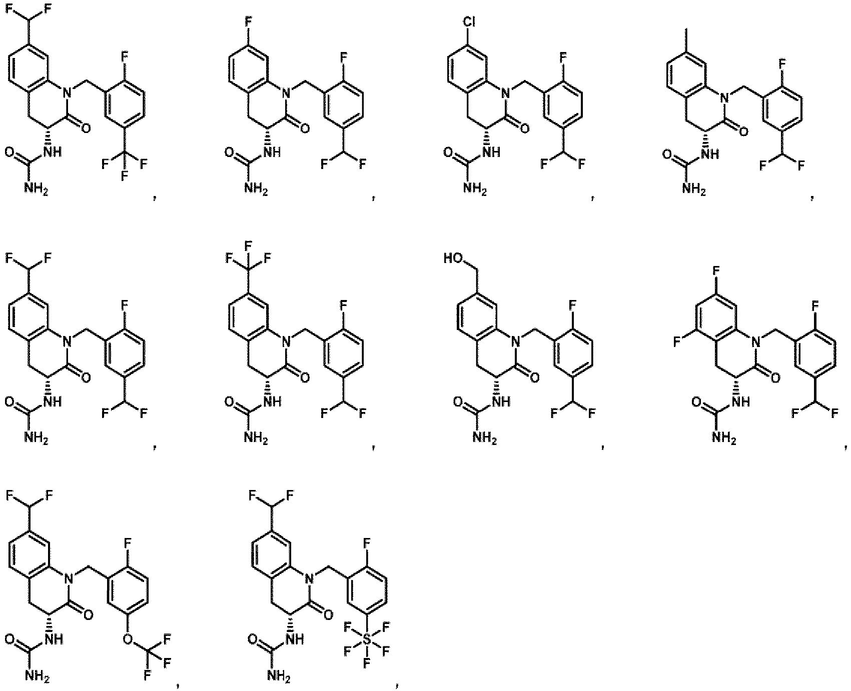 and 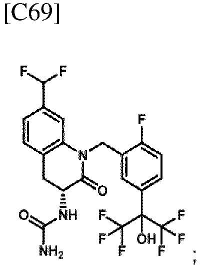 or a pharmacologically acceptable salt thereof.
[B-43] The compound according to any one of the above [B-1] to [B-42], selected from the group consisting of the following compounds: 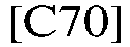 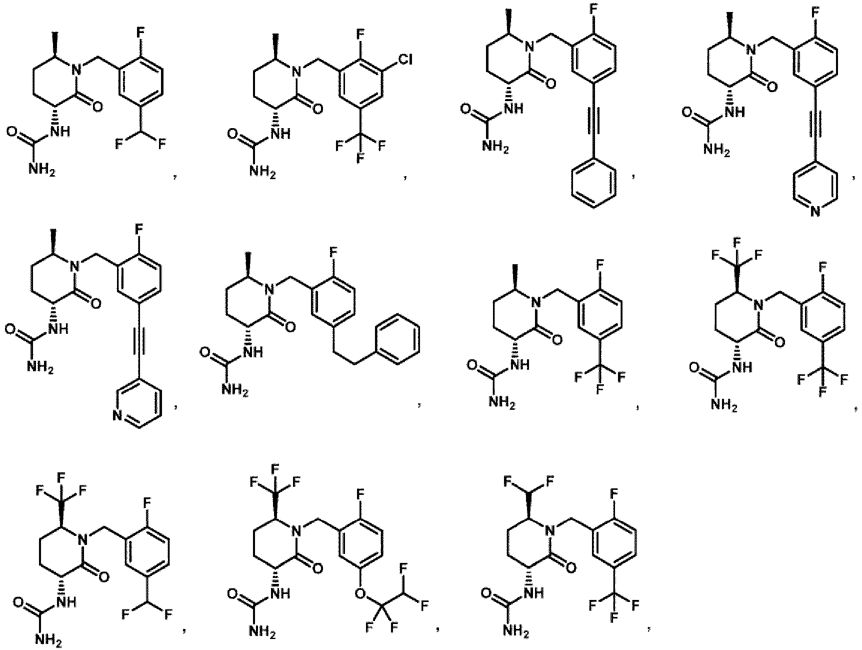 and 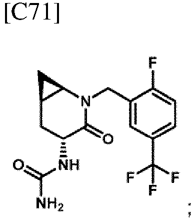 or a pharmacologically acceptable salt thereof.
[B-44] The compound according to any one of the above [B-1] to [B-42], selected from the group consisting of the following compounds: 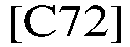 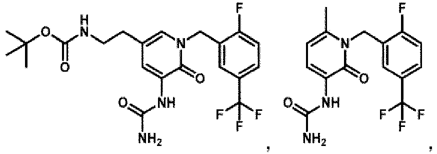 and 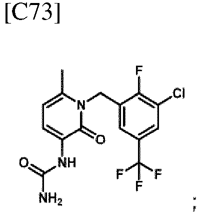 or a pharmacologically acceptable salt thereof.
[B-45] The compound according to any one of the above [B-1] to [B-42], selected from the group consisting of the following compounds: 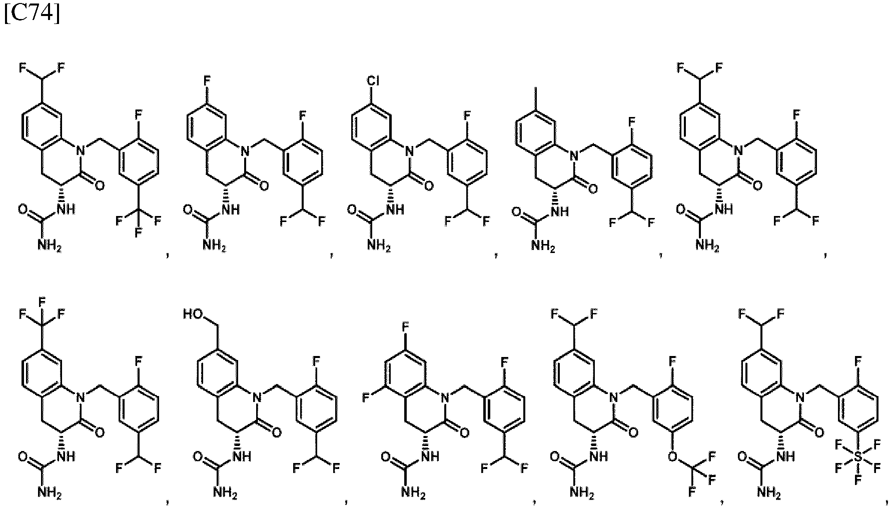 and 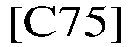 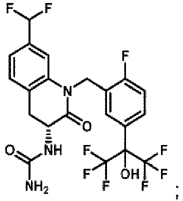 or a pharmacologically acceptable salt thereof.
[B-46] The compound according to any one of the above [B-1] to [B-41], selected from the group consisting of the following compounds: 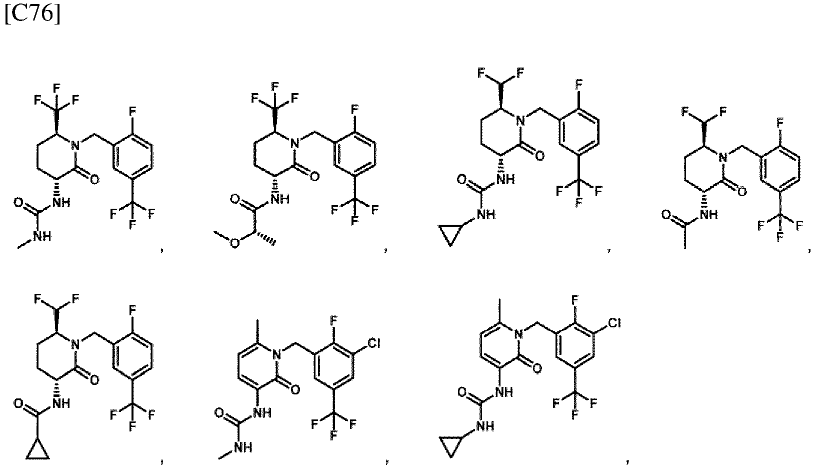 and 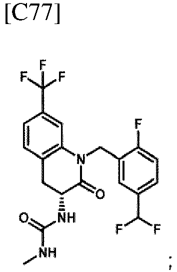 or a pharmacologically acceptable salt thereof.
[B-47] A pharmaceutical composition comprising the compound according to any one of the above [B-1] to [B-46] or a pharmacologically acceptable salt thereof, and a pharmaceutical additive.
[B-48] The pharmaceutical composition according to the above [B-47], which is a pharmaceutical composition for treating thyroid-related diseases.
[B-49] The pharmaceutical composition according to the above [B-48], wherein the thyroid-related diseases are hyperthyroidism, Graves' disease, or thyroid eye disease.
[B-50] A TSHR inhibitor comprising, as an active ingredient, the compound according to any one of the above [B-1] to [B-46], or a pharmacologically acceptable salt thereof.
[B-51] A therapeutic agent for thyroid-related diseases, comprising, as an active ingredient, the compound according to any one of the above [B-1] to [B-46], or a pharmacologically acceptable salt thereof.

As one embodiment of the compounds represented by Formulas (I) to (VI), for example, when two R^{1a}s are taken together to form a C₃₋₈ cycloalkyl, compounds that form a ring represented by the following Formula (a) or (b): 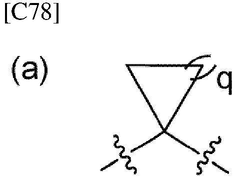
(wherein, q is an integer of 1 to 4), 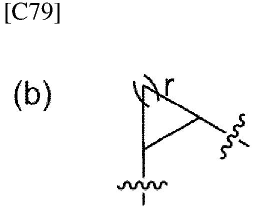
(wherein, r is an integer 1 to 4);
are exemplified.

As one embodiment, the present invention relates to a method for treating thyroid-related diseases, comprising administering a required amount of the pharmaceutical composition according to the above [B-47] to a patient.

As one embodiment, the present invention relates to a use of the compound according to any one of the above [B-1] to [B-46] or a pharmacologically acceptable salt thereof for manufacturing a pharmaceutical composition for treating thyroid-related diseases.

### [Advantageous Effects of Invention]

The compound of the present invention has excellent TSHR antagonist activity. Therefore, the compound of the present invention or a pharmacologically acceptable salt thereof are useful as a therapeutic agent for thyroid-related diseases.

### [Description of Embodiments]

Hereinafter, embodiments of the present invention will be described in more detail.

In the present invention, unless otherwise specified, respective terms have the following meanings.

"Halogen atom" refers to a fluorine atom, a chlorine atom, a bromine atom or an iodine atom.

"C₁₋₆ alkyl" refers to a linear or branched alkyl group having 1 to 6 carbon atoms. Examples thereof include methyl, ethyl, propyl, isopropyl, butyl, isobutyl, sec-butyl, and tert-butyl.

"C₁₋₆ alkylene" refers to a linear or branched alkylene group having 1 to 6 carbon atoms. For example, methylene, ethylene, 1-propylene, and the like are exemplified.

"C₂₋₆ alkenyl" refers to an alkenyl group having 2 to 6 carbon atoms and having at least one double bond. For example, ethenyl, 1-propenyl, 2-propenyl, and the like can be illustrated.

"C₂₋₆ alkynyl" refers to an alkynyl group having 2 to 6 carbon atoms and having at least one triple bond. Examples thereof include ethynyl, 1-propynyl, 2-propynyl, and 1-butynyl.

"C₁₋₆ alkoxy" refers to a linear or branched alkoxy group having 1 to 6 carbon atoms. Examples thereof include methoxy, ethoxy, propoxy, and isopropoxy.

"Hydroxy C₁₋₆ alkyl" refers to C₁₋₆ alkyl substituted with one or two hydroxy groups. Examples thereof include hydroxymethyl, 2-hydroxyethyl, 3-hydroxypropyl, and 2-hydroxypropan-2-yl.

"Halo C₁₋₆ alkyl" refers to C₁₋₆ alkyl substituted with 1 to 5 identical or different halogen atoms. Examples thereof include monofluoromethyl, 2-fluoroethyl, difluoromethyl, trifluoromethyl, 1,1-difluoroethyl, 2,2,2-trifluoroethyl, 3,3,3-trifluoropropyl, 4,4,4-trifluorobutyl, and pentafluoroethyl.

"Halo C₁₋₆ alkoxy" refers to C₁₋₆ alkoxy substituted with 1 to 5 identical or different halogen atoms. Examples thereof include monofluoromethoxy, difluoromethoxy, trifluoromethoxy, 1,1,2,2-tetrafluoroethoxy, and pentafluoroethoxy.

"C₁₋₆ alkoxy C₁₋₆ alkyl" refers to C₁₋₆ alkyl substituted by one C₁₋₆ alkoxy. Example thereof include methoxymethyl, methoxyethyl, ethoxymethyl, and ethoxyethyl.

"Carboxy C₁₋₆ alkyl" refers to C₁₋₆ alkyl substituted with one carboxy. Example thereof include carboxymethyl and carboxyethyl.

"Amino C₁₋₆ alkyl" refers to C₁₋₆ alkyl substituted with one amino group that may be substituted with one or two C₁₋₆ alkyl groups. Examples thereof include aminomethyl, N-methylaminoethyl, and N,N-dimethylaminoethyl.

"Cyano C₁₋₆ alkyl" refers to C₁₋₆ alkyl substituted with one cyano group. Examples thereof include cyanomethyl and cyanoethyl.

"Halohydroxy C₁₋₆ alkyl" refers to C₁₋₆ alkyl substituted with 1 to 6 identical or different halogen atoms and 1 or 2 hydroxy groups. Examples thereof include 1,1,1,3,3,3-hexafluoro-2-hydroxypropan-2-yl.

"C₆₋₁₀ aryl" refers to a phenyl group or a naphthyl group.

"5- or 6-membered heteroaryl" refers to a 5- or 6-membered aromatic heterocyclic group including 1 to 4 heteroatoms selected from among an oxygen atom, a nitrogen atom and a sulfur atom in the ring. Examples thereof include pyridyl, furyl, pyrrolyl, thienyl, imidazolyl, pyrazolyl, 1,2,4-triazolyl, isothiazolyl, isoxazolyl, oxazolyl, thiazolyl, 1,3,4-oxadiazolyl, and 1,2,4-oxadiazolyl.

"5- to 10-membered heteroaryl" refers to a 5- to 10-membered aromatic heterocyclic group containing 1 to 4 heteroatoms selected from an oxygen atom, a nitrogen atom, and a sulfur atom in a ring, in which a part of the ring may be partially saturated. Examples thereof include pyridyl, furyl, pyrrolyl, thienyl, imidazolyl, pyrazolyl, 1,2,4-triazolyl, isothiazolyl, isoxazolyl, oxazolyl, thiazolyl, 1,3,4-oxadiazolyl, 1,2,4-oxadiazolyl, 2,3-dihydrobenzofuranyl, 1,2,3,4-tetrahydroquinolyl, and 1,2,3,4-tetrahydroisoquinolyl.

"C₃₋₈ cycloalkyl" refers to a 3- to 8-membered saturated hydrocarbon group, and includes those having a partially crosslinked structure. Examples thereof include cyclopropyl, cyclobutyl, cyclopentyl, cyclohexyl, bicyclo[1.1.1]pentyl, and bicyclo[2.2.2]octyl.

"C₃₋₈ cycloalkoxy" refers to a group represented by (C₃₋₈ cycloalkyl)-O-. Examples thereof include cyclopropyloxy.

"3- to 8-membered heterocycloalkyl" refers to a cycloalkyl group in which carbon atoms in the ring are substituted with 1 or 2 heteroatoms selected from among an oxygen atom, a nitrogen atom and a sulfur atom, and includes those having a partially crosslinked structure. Examples thereof include aziridinyl, azetidinyl, pyrrolidinyl, piperidinyl, piperazinyl, morpholinyl, thiomorpholinyl, azepanyl, azabicyclo[2.2.2]octyl, 2-oxa-5-azabicyclo[2.2.1]heptanyl, 6-oxa-3-azabicyclo[2.2.1]heptanyl, 8-oxa-3-azabicyclo[3.2.1]octanyl, 3-oxa-8-azabicyclo[3.2.1]octanyl, 2-azaspiro[3.3]heptanyl, 2-oxa-6-azaspiro[3.3]heptanyl, pyrrolidonyl, piperidonyl, oxiranyl, oxetyl, tetrahydrofuranyl, and tetrahydropyranyl.

"C₆₋₁₀ aryloxy" refers to a group represented by (C₆₋₁₀ aryl)-O-. Examples thereof include phenoxy.

"C₆₋₁₀ aryl C₁₋₆ alkoxy" refers to C₁₋₆ alkoxy substituted with one C₆₋₁₀ aryl. Examples thereof include benzyloxy.

"C₃₋₈ cycloalkyl C₁₋₆ alkoxy" refers to C₁₋₆ alkoxy substituted with one C₃₋₈ cycloalkyl. Examples thereof include cyclopropylmethyloxy.

"C₆₋₁₀ aryl C₁₋₆ alkyl" refers to C₁₋₆ alkyl substituted with one C₆₋₁₀ aryl. Examples thereof include benzyl.

"5- or 6-membered heteroaryl C₂₋₆ alkyl" refers to C₂₋₆ alkyl substituted with one 5- or 6-membered heteroaryl. Examples thereof include pyridin-4-ylmethyl, thiophen-2-ylethyl, and thiophen-3-ylethyl.

"C₃₋₈ cycloalkyl C₁₋₆ alkyl" refers to C₁₋₆ alkyl substituted with one C₃₋₈ cycloalkyl. Examples thereof include cyclopropylmethyl, cyclopropylethan-1-yl, cyclobutylmethyl, cyclopentylmethyl, and cyclohexylmethyl.

"C₆₋₁₀ aryl C₂₋₆ alkynyl" refers to C₂₋₆ alkynyl substituted with one C₆₋₁₀ aryl. Examples thereof include phenylethynyl.

"5- or 6-membered heteroaryl C₁₋₆ alkyl" refers to C₁₋₆ alkyl substituted with one 5- or 6-membered heteroaryl.

"5- or 6-membered heteroaryl C₂₋₆ alkynyl" refers to C₂₋₆ alkynyl substituted with one 5- or 6-membered heteroaryl. Examples thereof include pyridin-4-ylethynyl and pyridin-3-ylethynyl.

"C₃₋₈ cycloalkyl C₂₋₆ alkynyl" refers to C₂₋₆ alkynyl substituted with one C₃₋₈ cycloalkyl. Examples thereof include cyclopropylethynyl.

"3- to 8-membered saturated carbocycle" refers to a monocyclic or polycyclic saturated cyclic hydrocarbon having 3 to 8 carbon atoms.

"5- to 8-membered saturated carbocycle" refers to a monocyclic or polycyclic saturated cyclic hydrocarbon having 5 to 8 carbon atoms.

"5- to 8-membered unsaturated carbocycle" refers to a monocyclic or polycyclic cyclic hydrocarbon having 5 to 8 carbon atoms and being fully unsaturated or partially unsaturated.

"3- to 8-membered saturated heterocycle" refers to a 3- to 8-membered saturated heterocycle that is monocyclic or polycyclic and has 1 to 5 heteroatoms selected from a nitrogen atom, an oxygen atom, and a sulfur atom in a ring.

"3- to 10-membered saturated heterocycle" refers to a 3- to 10-membered saturated heterocycle that is monocyclic or polycyclic and has 1 to 5 heteroatoms selected from a nitrogen atom, an oxygen atom, and a sulfur atom in a ring.

"5- to 8-membered saturated heterocycle" refers to a 5- to 8-membered saturated heterocycle that is monocyclic or polycyclic and has 1 to 3 heteroatoms selected from a nitrogen atom, an oxygen atom, and a sulfur atom in a ring.

"3- to 10-membered unsaturated heterocycle" refers to a 3- to 10-membered heterocycle that is monocyclic or polycyclic, is fully unsaturated or partially unsaturated, and has 1 to 5 heteroatoms selected from a nitrogen atom, an oxygen atom, and a sulfur atom in a ring.

"5- to 8-membered unsaturated heterocycle" refers to a 5- to 8-membered heterocycle that is monocyclic or polycyclic, is fully unsaturated or partially unsaturated, and has 1 to 3 heteroatoms selected from a nitrogen atom, an oxygen atom, and a sulfur atom in a ring.

"5- to 8-membered ring" refers to a 5- to 8-membered saturated carbocycle, a 5- to 8-membered saturated heterocycle, a 5- to 8-membered unsaturated carbocycle, or a 5- to 8-membered unsaturated heterocycle.

The following abbreviations in the description, figures, and tables have the following meanings, respectively.
AIBN: azobisisobutyronitrile
BH₃-THF: borane-tetrahydrofuran complex
Boc: tert-butoxycarbonyl
Boc₂O: di-tert-butyl dicarbonate
DBU: diazabicycloundecene
DCM: dichloromethane
DEAD: diethyl azodicarboxylate
DIBAL-H: diisobutylaluminum hydride
DIPEA: N,N-diisopropylethylamine
DMAP: 4-dimethylaminopyridine
DME: 1,2-dimethoxyethane
DMF: N,N-dimethylformamide
DMP: Dess-Martin periodinane
DPPA: diphenylphosphoryl azide
DMT-MM: 4-(4,6-dimethoxy-1,3,5-triazin-2-yl)-4-methylmorpholinium chloride
HATU: O-(7-azabenzotriazol-1-yl)-N,N,N',N'-tetramethyluronium hexafluorophosphate
LDA: lithium diisopropylamide
MeCN: acetonitrile
MTBE: methyl tert-butyl ether
NBS: N-bromosuccinimide
NMP: N-methylpyrrolidone
PdCl₂(dppf)-DCM: [1,1'-bis(diphenylphosphino)ferrocene]palladium(II) dichloride DCM adduct
TBAF: tetrabutylammonium fluoride
TBAI: tetrabutylammonium iodide
TBDPS: tert-butyldiphenylsilyl
TBDPSCl: tert-butyldiphenylsilyl chloride
TBS: tert-butyldimethylsilyl
TEA: triethylamine
TFA: trifluoroacetic acid
THF: tetrahydrofuran
TMEDA: N,N,N',N'-tetramethylethylenediamine
TMS: trimethylsilyl
TMSCl: trimethylsilyl chloride
Xphos: 2-dicyclohexylphosphino-2',4',6'-triisopropylbiphenyl
10% Pd/C: 10% palladium on carbon (approximately 55% water-wet)
APS: aminopropylated silica gel
Method A: column chromatography using a silica gel column connected to the lower portion of an aminopropylated silica gel column
Method B: column chromatography using an aminopropylated silica gel column connected to the lower portion of a silica gel column
ODS: octadecylsilylated silica gel
PLC: preparative thin-layer chromatography
Ref. No.: Reference Example Number
Str.: structural formula
Ex. No.: Example Number
Phys. data: physical property values
IC₅₀: 50% inhibitory concentration
¹H-NMR: proton nuclear magnetic resonance spectrum
CD₃OD: methanol-d4
DMSO: dimethyl sulfoxide
DMSO-d6: dimethyl sulfoxide-d6
CDCl₃: chloroform-d1
MS: mass spectrometry
(The measured values in the tables were measured by an electrospray ionization method or a multi-ionization method of an electrospray ionization-atmospheric pressure chemical ionization method.)
cAMP: adenosine 3',5'-cyclic monophosphate
CHO: Chinese hamster ovary
FBS: fetal bovine serum
HEPES: 2-(4-(2-hydroxyethyl)-1-piperazinyl)ethanesulfonic acid
IBMX: 3-isobutyl-1-methylxanthine

In the compounds represented by Formulas (A-I) to (A-VIII) or Formulas (I) to (VIII), when one or more asymmetric carbon atoms are present, the present invention also encompasses a compound in which each asymmetric carbon atom has an R-configuration, a compound in which each asymmetric carbon atom has an S-configuration, and a compound having any combination thereof. In addition, the scope of the present invention also includes racemic compounds, racemic mixtures, single enantiomers and diastereomeric mixtures thereof.

In the compounds represented by Formulas (A-I) to (A-VIII) or Formulas (I) to (VIII), when cis-trans isomers are present, the present invention encompasses any of the cis-trans isomers.

In the compounds represented by Formulas (A-I) to (A-VIII) or Formulas (I) to (VIII), when tautomers are present, the present invention includes any of the tautomers.

In the present invention, the determination of stereochemistry can be performed by a method known in the art.

The compounds represented by Formulas (A-I) to (A-VIII) or Formulas (I) to (VIII) can also be converted into pharmacologically acceptable salts thereof according to a general method, if necessary. Examples of such salts include acid addition salts and salts with bases.

Examples of acid addition salts include acid addition salts with mineral acids such as hydrochloric acid, hydrobromic acid, hydroiodic acid, sulfuric acid, nitric acid, and phosphoric acid; and acid addition salts with organic acids such as formic acid, acetic acid, TFA, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, benzoic acid, glutamic acid, and aspartic acid.

Examples of salts with bases include salts with inorganic bases such as lithium salts, sodium salts, potassium salts, calcium salts, and magnesium salts, and salts with organic bases such as N-methyl-D-glucamine, N,N'-dibenzylethylenediamine, TEA, piperidine, morpholine, pyrrolidine, arginine, lysine, and choline.

Unless otherwise specified, for example, suffixes "hydrochloride," or "HCl" to chemical names or structural formulas for salts are not stoichiometric descriptions, but simply refer to salt forms.

When the compounds represented by Formulas (A-I) to (A-VIII) or Formulas (I) to (VIII) or pharmacologically acceptable salts thereof are present as, for example, a crystal, the present invention also includes any crystal form. For example, a pharmacologically acceptable salt also includes a solvate thereof with a pharmacologically acceptable solvent such as water or ethanol, a cocrystal thereof with an appropriate cocrystal former (Coformer) and the like.

In the compounds represented by Formulas (A-I) to (A-VIII) or Formulas (I) to (VIII), a part of each of atoms may be replaced by a corresponding isotope thereof. The present invention also includes such isotope-replaced compounds. Examples of isotopes include isotopes of a hydrogen atom, a carbon atom, a chlorine atom, a fluorine atom, an iodine atom, a nitrogen atom, an oxygen atom, and a sulfur atom represented by ²H, ³H, ¹¹C, ¹³C, ¹⁴C, ³⁶Cl, ¹⁸F, ¹²³I, ¹²⁵I, ¹³N, ¹⁵N, ¹⁵O, ¹⁷O, ¹⁸O, and ³⁵S, respectively. As one embodiment, a compound in which a part of the hydrogen atoms in the compounds represented by Formulas (A-I) to (A-VIII) or Formulas (I) to (VIII) is replaced by ²H (D: deuterium atom) is exemplified.

In the compounds represented by Formulas (A-I) to (A-VIII) or Formulas (I) to (VIII), compounds in which some atoms are substituted with isotopes can be manufactured by the same method as a manufacturing method to be described below using commercially available building blocks into which isotopes are introduced. In addition, methods described in the literature (for example, refer to Journal of Synthetic Organic Chemistry, Vol. 65, No. 12, p. 1179-1190, 2007 and refer to RADIOISOTOPES, Vol. 56, No. 11, p. 741-750, 2007) can be used for manufacturing.

The compounds represented by Formulas (A-I) to (A-VIII) or Formulas (I) to (VIII) of the present invention can be manufactured, for example, by methods shown in Schemes 1 to 7 or similar methods, or by methods described in the literature or similar methods. In the Schemes, the compounds represented by Formulas (A-I) to (A-VIII) or Formulas (I) to (VIII) correspond to compounds represented by Formulas (I-1) to (I-5).

The compounds represented by Formulas (A-I) to (A-VIII) or Formulas (I) to (VIII) of the present invention can be manufactured by methods shown below, but the following manufacturing methods are exemplifications of general manufacturing methods and do not limit the manufacturing methods.

In the reaction of each step, when raw materials and reagents are commercially available, commercially available products can be used.

In the reaction of each step, the reaction time varies depending on the raw materials used, solvent, reaction temperature, and the like, but is generally 30 minutes to 3 days unless otherwise specified.

In the reaction of each step, the reaction temperature varies depending on the raw materials used, solvent, and the like, but is generally -78°C to a reflux temperature unless otherwise specified.

In the reaction of each step, the pressure varies depending on the raw materials used, solvent, reaction temperature, and the like, but is generally 1 to 20 atm unless otherwise specified.

In the reaction of each step, a microwave reaction device such as an Initiator (commercially available from Biotage) can be used. When the reaction is performed using a microwave reaction device, the reaction can be performed under varying conditions depending on the raw materials used, solvent, and model, pressure range: 1 to 30 bar, power range: 1 to 400 W, reaction temperature: room temperature to 300°C, and reaction time: 1 minute to 1 day.

Unless otherwise specified, the reaction in each step is performed without a solvent or using an appropriate solvent. Examples of appropriate solvents include solvents inert to the reaction. Specific examples of solvents used include solvents described in reference examples and examples corresponding to the steps and the following solvents. Two or more of the following solvents may be used in combination at an appropriate ratio.
Alcohols: methanol, ethanol, tert-butyl alcohol, 2-propanol, and the like;
ethers: diethyl ether, THF, DME, 1,4-dioxane, cyclopentyl methyl ether, MTBE, and the like;
aromatic hydrocarbons: benzene, chlorobenzene, 1,2-dichlorobenzene, toluene, xylene, and the like;
saturated hydrocarbons: cyclohexane, n-hexane, n-pentane, and the like;
amides: DMF, N,N-dimethylacetamide, NMP, and the like;
halogenated hydrocarbons: DCM, 1,2-dichloroethane, carbon tetrachloride, and the like;
nitriles: MeCN, and the like;
sulfoxides: DMSO, and the like;
aromatic organic bases: pyridine, and the like;
acid anhydrides: acetic anhydride, and the like;
organic acids: formic acid, acetic acid, TFA, methanesulfonic acid, and the like;
esters: ethyl acetate, methyl acetate, isopropyl acetate, and the like;
ketones: acetone, methyl ethyl ketone, and the like; and
water.

In the reaction of each step, when a base is used, the reaction is performed using a base suitable for the reaction. Specific examples of bases used include bases described in reference examples and examples corresponding to the steps and the following bases.
Inorganic bases: sodium hydroxide, lithium hydroxide, potassium hydroxide, and the like;
basic salts: sodium carbonate, sodium hydrogen carbonate, potassium carbonate, cesium carbonate, and the like;
organic bases: TEA, DIPEA, diethylamine, pyridine, DMAP, 2,6-lutidine, DBU, imidazole, piperidine, and the like;
metal alkoxides: sodium ethoxide, sodium methoxide, potassium tert-butoxide, and the like;
alkali metal hydrides: sodium hydride, and the like;
metal amides: sodium amide, LDA, lithium bis(trimethylsilyl)amide, sodium bis(trimethylsilyl)amide, potassium bis(trimethylsilyl)amide, and the like;
organomagnesiums: isopropylmagnesium chloride, and the like;
organolithiums: methyllithium, n-butyllithium, sec-butyllithium, tert-butyllithium, and the like

In the reaction of each step, when an acid is used, the reaction is performed using an acid suitable for the reaction. Specific examples of acids used include acids described in reference examples and examples corresponding to the steps and the following acids.
Inorganic acids: hydrochloric acid, sulfuric acid, nitric acid, hydrobromic acid, phosphoric acid, and the like;
organic acids: acetic acid, TFA, citric acid, methanesulfonic acid, p-toluenesulfonic acid, 10-camphorsulfonic acid, and the like; and
Lewis acids: boron trifluoride diethyl ether complex, zinc iodide, aluminum chloride, zinc chloride, titanium(IV) chloride, and the like

In the reaction of each step, when a condensing agent is used, the reaction is performed using a condensing agent suitable for the reaction. Specific examples of condensing agents used include condensing agents described in reference examples and examples corresponding to the steps and the following condensing agents.
Carbodiimides: 1-ethyl-3-(3-dimethylaminopropyl)carbodiimide hydrochloride, N,N'-dicyclohexylcarbodiimide, and the like;
imidazoles: carbonyldiimidazole, and the like;
uronium salts, phosphonium salts: HATU, 1H-benzotriazol-1-yloxytris(dimethylamino)phosphonium hexafluorophosphate, and the like;
triazines: DMT-MM, and the like;
others: propylphosphonic anhydride (cyclic trimer), and the like.

In the reaction of each step, when a reducing agent is used, the reaction is performed using a reducing agent suitable for the reaction. Specific examples of reducing agents used include reducing agents described in reference examples and examples corresponding to the steps and the following reducing agents.
Metal hydrides: lithium aluminum hydride, lithium borohydride, sodium borohydride, sodium triacetoxyborohydride, sodium cyanoborohydride, DIBAL-H, and the like;
boranes: BH₃-THF complex, picoline borane complex, decaborane, and the like.

In the reaction of each step, when a carbonyl group introduction reagent is used, the reaction is performed using a carbonyl group introduction reagent suitable for the reaction. Specific examples of carbonyl group introduction reagents used include carbonyl group introduction reagents described in reference examples and examples corresponding to the steps and the following carbonyl group introduction reagents.
Phosgenes: phosgene, diphosgene, triphosgene, and the like;
chloroformates: 4-nitrophenyl chloroformate, and the like;
imidazoles: carbonyldiimidazole, and the like.

In each step, when a protecting group is required according to the type of functional groups, introduction and removal operations can be performed in an appropriate combination according to a conventional method. Regarding the type of protecting groups, protection, and deprotection, for example, the methods described in "Greene's Protective Groups in Organic Synthesis," fifth edition, Wiley-Interscience, 2014 edited by Peter G.M.Wuts may be referred to.

In each step, when a hydrolysis reaction is performed, the reaction can be performed in the presence of an acid or a base. Examples of acids and bases used include those exemplified above.

In each step, when a catalytic reduction reaction is performed, the reaction can be performed in the presence of hydrogen and a catalyst. Examples of catalysts used include palladium on carbon powder, Pearlman's catalyst, platinum carbon powder, and Raney nickel. Here, as necessary, an acid may be used in the reaction.

In each step, when a reduction reaction is performed, the reaction can be performed in the presence of a reducing agent. Examples of reducing agents used include those exemplified above.

In each step, when a metal reduction reaction is performed, the reaction can be performed in the presence of a metal or the like. Examples of metals used or the like include iron powder, zinc powder, tin chloride, and titanium trichloride. Here, as necessary, an acid may be used in the reaction.

In each process, when amidation is conducted, the reaction can be conducted using a condensing reagent in the presence or absence of a base. Examples of condensing agents and bases used include those exemplified above. Here, when a carbodiimide is used as the condensing agent, the reaction may be performed by adding additives such as 1-hydroxybenzotriazole and DMAP as necessary. In addition, the reaction can be performed using an acyl halide or an acid anhydride in the presence or absence of a base.

In each process, when reductive amination is conducted, the reaction can be conducted in the presence of a reducing reagent. Examples of reducing agents used include those exemplified above. In addition, the reaction can be performed in the presence of hydrogen and a catalyst. Examples of catalysts used include palladium on carbon powder, a Perlman catalyst, platinum carbon powder, and Raney nickel.

In each step, when a Negishi cross coupling reaction is performed, the reaction can be performed in the presence of an organozinc compound, a palladium catalyst and a ligand. Examples of palladium catalysts used include palladium(II) acetate and tris(dibenzylideneacetone)palladium(0). Examples of ligands include Xphos, 2-dicyclohexylphosphino-2',6'-dimethoxybiphenyl, and tris(2-methylphenyl)phosphine.

In each step, when a Suzuki-Miyaura cross-coupling reaction is performed, the reaction can be performed in the presence of a palladium catalyst and a base. Examples of palladium catalysts used include bis(di-tert-butyl(4-dimethylaminophenyl)phosphine)dichloropalladium(II), and tetrakis(triphenylphosphine)palladium(0). Examples of bases used include those exemplified above.

In each step, when Goldberg amination is carried out, the reaction can be carried out in the presence of a copper catalyst, a ligand, and a base. Examples of the copper catalyst to be used include copper(I) iodide, copper(I) bromide, copper(I) chloride, copper(II) chloride, copper(I) oxide, copper(II) oxide, copper(II) acetate, and copper(II) sulfate. Examples of the ligand to be used include 1,10-phenanthroline, TMEDA, and 2,2'-bipyridine. Examples of bases to be used include those exemplified above.

In each step, when a Sonogashira coupling reaction is carried out, the reaction can be carried out in the presence of a palladium catalyst, a copper catalyst, and a base. Examples of the palladium catalyst to be used include bis(di-tert-butyl(4-dimethylaminophenyl)phosphine)dichloropalladium(II) and bis(acetonitrile)palladium(II) chloride. Examples of the copper catalyst to be used include copper(I) iodide and copper(I) bromide. Examples of the base to be used include those exemplified above.

In each step, when a Curtius rearrangement reaction is carried out, the reaction can be carried out in the presence of an azide source. Examples of azide sources to be used include sodium azide and DPPA.

In each step, when a carbamate formation or a urea formation is carried out, the reaction can be carried out using a carbonyl group introduction reagent in the presence or absence of a base. Examples of the carbonyl group introduction reagent and the base to be used include those exemplified above.

The compounds represented by Formulas (I-1a) to (I-1d) can be manufactured, for example, according to the method described in Scheme 1. 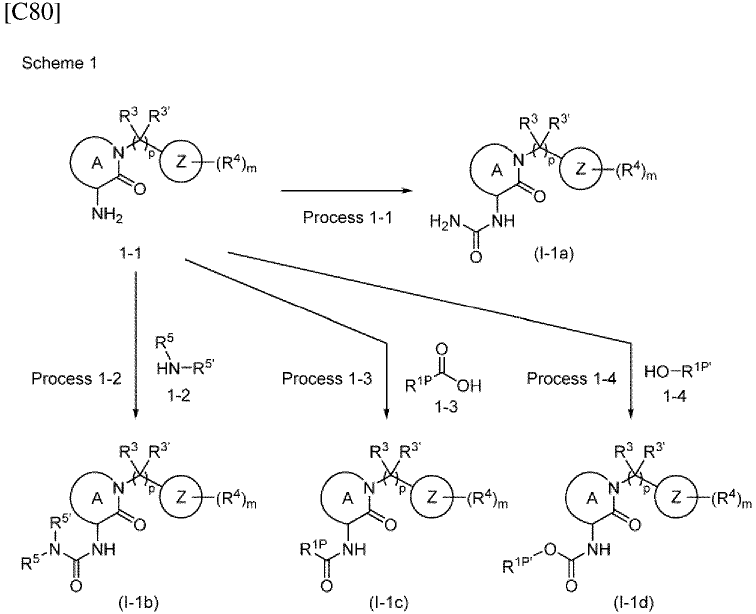 The symbols in the formulas have the same meanings as above. R^{1P} is C₁₋₆ alkyl, C₁₋₆ haloalkyl, C₃₋₈ cycloalkyl, or C₁₋₆ alkoxy C₁₋₆ alkyl. R1^{P'} is C₁₋₆ alkyl, C₁₋₆ haloalkyl, or C₃₋₈ cycloalkyl.

### Process 1-1

Compound (I-1a) can be manufactured by reacting Compound (1-1) with trimethylsilyl isocyanate. As another method, Compound (I-1a) can be manufactured by reacting Compound (1-1) with potassium cyanate or sodium cyanate in the presence of an acid.

### Process 1-2

Compound (I-1b) can be manufactured by urea formation of Compound (1-1) and Compound (1-2).

### Process 1-3

Compound (I-1c) can be manufactured by amidation of Compound (1-1) and Compound (1-3).

### Process 1-4

Compound (I-1d) can be manufactured by carbamate formation of Compound (1-1) and Compound (1-4).

The compound represented by Formula (1-1a) can be manufactured, for example, according to the method described in Scheme 2.

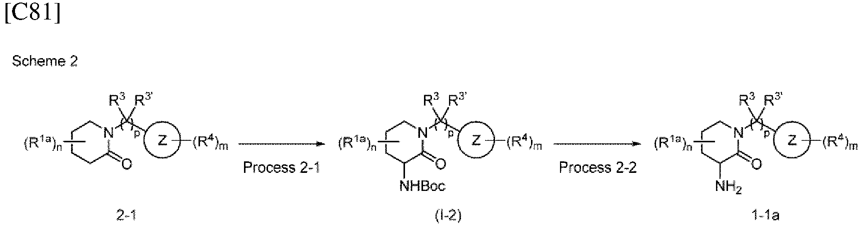

The symbols in the formulas have the same meanings as above.

### Process 2-1

Compound (I-2) can be manufactured by reacting Compound (2-1) with DPPA and Boc₂O in the presence of a base.

### Process 2-2

Compound (1-1a) can be manufactured by removing the Boc group of Compound (I-2).

The compound represented by Formula (2-1) can be manufactured, for example, according to the method described in Scheme 3.

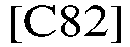

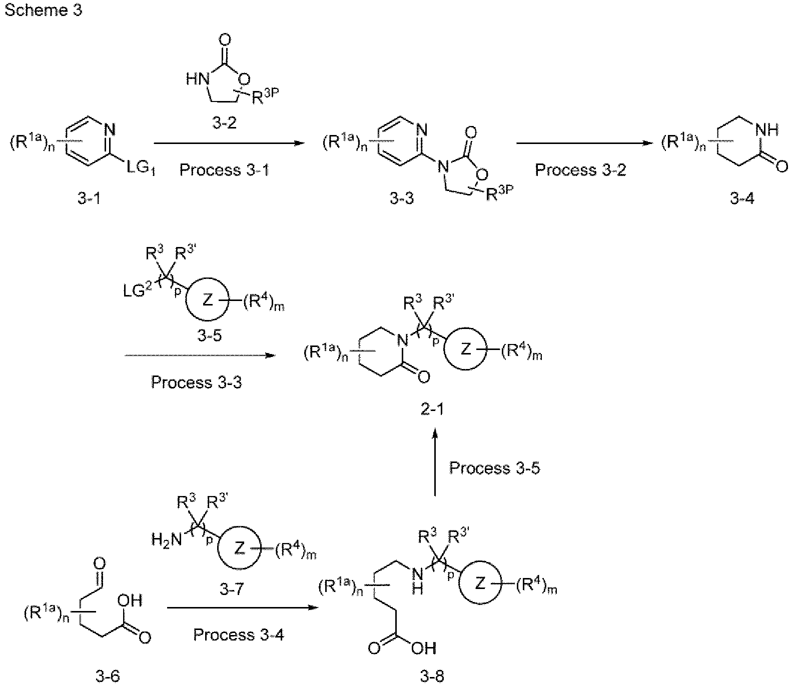

The symbols in the formulas have the same meanings as above. LG¹ is a chlorine atom, a bromine atom, or an iodine atom, R^{3P} is C₁₋₆ alkyl, C₆₋₁₀ aryl, or C₆₋₁₀ aryl C₁₋₆ alkyl, and LG² is a chlorine atom, a bromine atom, an iodine atom, or a methanesulfonyloxy group.

### Process 3-1

Compound (3-3) can also be manufactured by Goldberg amination of Compound (3-1) and Compound (3-2).

### Process 3-2

Compound (3-4) can also be manufactured by catalytic reduction of Compound (3-3).

### Process 3-3

Compound (2-1) can be manufactured by reacting Compound (3-4) with Compound (3-5) in the presence of a base.

### Process 3-4

Compound (3-8) can also be manufactured by reductive amination of Compound (3-6) and Compound (3-7).

### Process 3-5

Compound (2-1) can also be manufactured by amidation of Compound (3-8).

The compound represented by Formula (1-4b) can be manufactured, for example, according to the method described in Scheme 4.

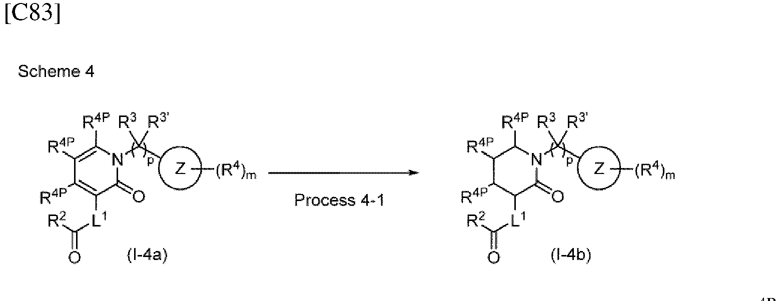

The symbols in the formulas have the same meanings as above. R^{4P} is a hydrogen atom, a fluorine atom, C₁₋₆ alkyl, halo C₁₋₆ alkyl, or -(C₁₋₆ alkylene)-NH-COO-(C₁₋₆ alkyl).

### Process 4-1

Compound (I-4b) can also be manufactured by catalytic reduction of Compound (I-4a).

The compound represented by Formula (1-1b) can be manufactured, for example, according to the method described in Scheme 5.

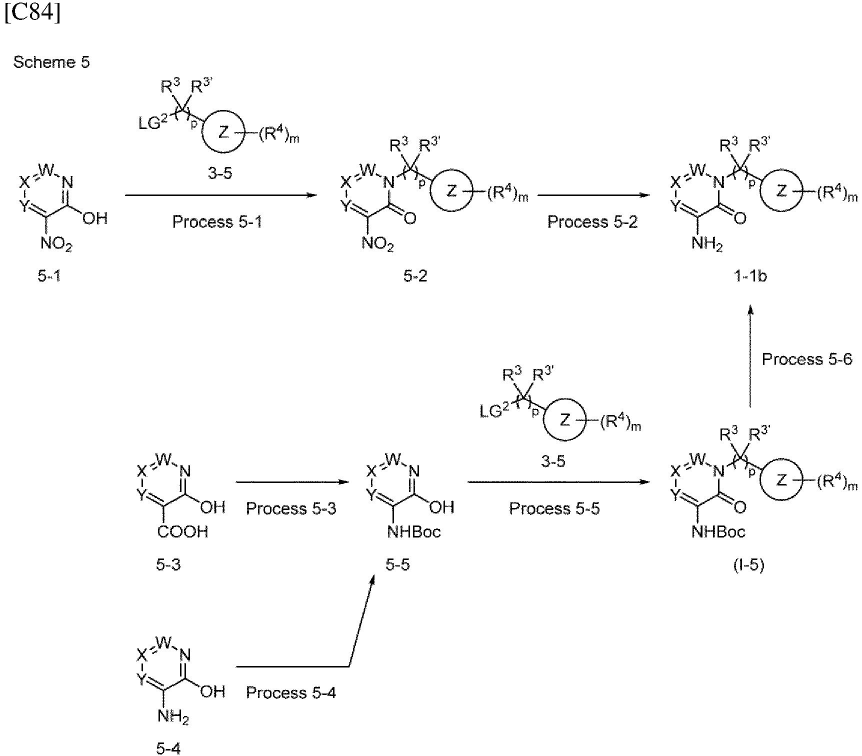

The symbols in the formulas have the same meanings as above.

### Process 5-1

Compound (5-2) can be manufactured by reacting Compound (5-1) with Compound (3-5) in the presence of a base.

### Process 5-2

Compound (1-1b) can also be manufactured by reducing a nitro group of Compound (5-2).

### Process 5-3

Compound (5-5) can be manufactured by Curtius rearrangement of Compound (5-3).

### Process 5-4

Compound (5-5) can also be manufactured by carbamate formation of Compound (5-4).

### Process 5-5

Compound (I-5) can be manufactured by reacting Compound (5-5) with Compound (3-5) in the presence of a base.

### Process 5-6

Compound (1-1b) can be manufactured by removing the Boc group of Compound (I-5).

The compound represented by Formula (1-1c) can be manufactured, for example, according to the method described in Scheme 6.

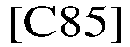

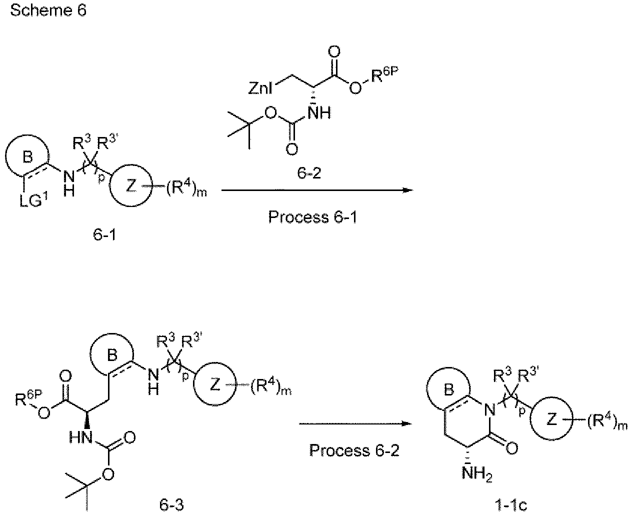

The symbols in the formulas have the same meanings as above. R^{6P} is C₁₋₆ alkyl.

### Process 6-1

Compound (6-3) can be manufactured by a Negishi coupling reaction of Compound (6-1) and Compound (6-2).

### Process 6-2

Compound (1-1c) can be manufactured by reacting Compound (6-3) with an acid.

The compound represented by Formula (6-1) and Formula (6-1a) can be manufactured, for example, according to the method described in Scheme 7.

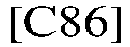

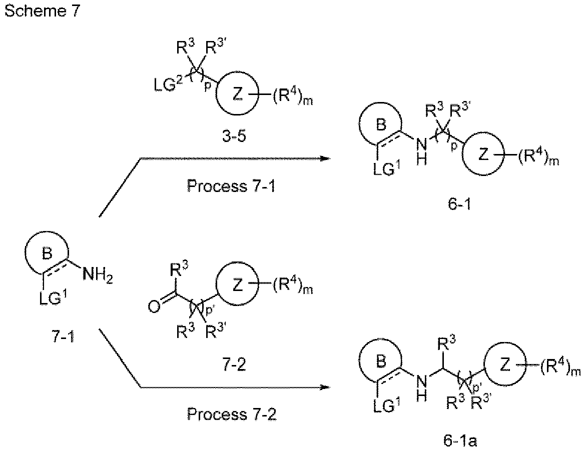

The symbols in the formulas have the same meanings as above. p' is an integer of 0 to 2.

### Process 7-1

Compound (6-1) can be manufactured by reacting Compound (7-1) with Compound (3-5) in the presence of a base.

### Process 7-2

Compound (6-1a) can also be manufactured by reductive amination of Compound (7-1) and Compound (7-2).

Each of the above-mentioned Schemes is exemplifications of methods for manufacturing the compounds represented by Formulas (A-I) to (A-VIII) or Formulas (I) to (VIII), or a manufacturing intermediate thereof. Each of the above-mentioned Schemes can be variously modified into Schemes that can be easily understood by those skilled in the art.

The compounds represented by Formulas (A-I) to (A-VIII) or Formulas (I) to (VIII) and manufacturing intermediates thereof can also be isolated and purified, if required, according to isolation and purification means well known to those skilled in the art in the technical field, such as solvent extraction, crystallization, recrystallization, chromatography or preparative high performance liquid chromatography.

The compound of the present invention can be used as a therapeutic agent for thyroid-related diseases because it has excellent TSHR antagonist activity. In the present invention, the thyroid-related diseases include, for example, hyperthyroidism, Graves' disease, thyroid eye disease and thyroid cancer. Preferably, the compound of the present invention can be used as a therapeutic agent for hyperthyroidism, Graves' disease, or thyroid eye disease (refer to Endocrinology, 2014, 155 (1), p. 310-314). More preferably, the compound of the present invention can be used as a therapeutic agent for hyperthyroidism or Graves' disease.

In addition, as one embodiment, hyperthyroidism includes, for example, hyperthyroidism caused by any of Graves' disease, thyroiditis, Plummer's disease, toxic multinodular goiter, TSH-producing pituitary adenoma, hyperemesis gravidarum, ovarian goiter, gestational trophoblastic tumor, and germ cell tumor. Preferably, the compound of the present invention can be used as a therapeutic agent for hyperthyroidism caused by Graves' disease.

In addition, as one embodiment, the thyroid-related diseases are diseases and symptoms associated with abnormalities in thyroid hormone levels. The diseases and symptoms associated with abnormalities in thyroid hormone levels include, for example, a disease and symptom caused by TRAb.

In the present invention, "treatment" includes the meaning of "prevention". For example, the treatment of hyperthyroidism, Graves' disease, or thyroid eye disease includes the meanings of "prevention of relapse/recurrence" and "maintenance of remission". In addition, as one embodiment, the compound of the present invention can be used to prevent the development of thyroid eye disease in patients with Graves' disease.

In the present invention, "antagonist" refers to a drug that inhibits or blocks the function of a target protein, regardless of a binding site thereof. For example, "antagonist" includes the meanings of "allosteric antagonist" and "negative allosteric modulator (NAM)".

The therapeutic effect of the compound of the present invention on thyroid-related diseases can be confirmed according to methods well known in the related art. Examples of methods for confirming the effect of hyperthyroidism or Graves' disease in model animals include methods described in Endocrinology 2007, 148(5), p. 2335-2344 and similar methods.

The pharmaceutical composition of the present invention may be used in various dosage forms depending on the usage. Examples of such dosage forms include powdered drugs, granules, fine granules, dry syrups, tablets, capsules, injections, liquid drugs, ointments, suppositories, patches, eye drops, and enema agents.

The pharmaceutical composition of the present invention includes the compounds represented by Formulas (A-I) to (A-VIII) or Formulas (I) to (VIII) or pharmacologically acceptable salts thereof as an active ingredient.

The pharmaceutical composition of the present invention is prepared by using the compounds represented by Formulas (A-I) to (A-VIII) or Formulas (I) to (VIII) or pharmacologically acceptable salts thereof, and at least one pharmaceutical additive. These pharmaceutical compositions can also be prepared by being appropriately mixed, diluted or dissolved with appropriate pharmaceutical additives such as an excipient, a disintegrant, a binder, a lubricant, a diluent, a buffer, an isotonizing agent, a preservative, a wetting agent, an emulsifying agent, a dispersing agent, a stabilizer, and a solubilizing agent according to a pharmaceutically known method depending on a dosage form.

When the pharmaceutical composition of the present invention is used in the treatment, the dosage of the compounds represented by Formulas (A-I) to (A-VIII) or Formulas (I) to (VIII) or the pharmacologically acceptable salts thereof is appropriately decided depending on the age, sex, body weight, degree of disorders and treatment of each patient, and the like. The daily dosage may be administered separately once, twice, three times or four times a day.

In the case of oral administration, the dosage for adults can be set, for example, in a range of 0.1 to 5,000 mg/day. As one embodiment, in the case of oral administration, the dosage can be set in a range of 1 to 1,500 mg/day, and preferably in a range of 1 to 500 mg/day. In the case of parenteral administration, the dosage for adults can be set to, for example, 0.01 to 5,000 mg/day. As one embodiment, in the case of parenteral administration, the dosage can be set in a range of 0.1 to 1,500 mg/day and preferably in a range of 0.1 to 500 mg/day.

As one embodiment, the pharmaceutical composition of the present invention can also be used in combination with drugs other than TSHR antagonist. Examples of other drugs that can be used in combination in the treatment of thyroid-related diseases include antithyroid drugs (for example, thiamazole, propylthiouracil, and the like), inorganic iodine, lithium carbonate, and thyroid hormone formulations.

When the compounds represented by Formulas (A-I) to (A-VIII) or Formulas (I) to (VIII) or pharmacologically acceptable salts thereof are used in combination with other drugs, a formulation containing these active components together or a formulation in which each of these active components is separately formulated can be administered. When the components are formulated separately, the formulations can be administered separately or simultaneously. In addition, the dosage of the compounds represented by Formulas (A-I) to (A-VIII) or Formulas (I) to (VIII) or pharmacologically acceptable salts thereof can be appropriately reduced depending on the dosage of other drugs used in combination.

The compounds represented by Formulas (A-I) to (A-VIII) or Formulas (I) to (VIII) may be converted into prodrugs as appropriate for use. For example, the prodrugs of the compounds represented by Formulas (A-I) to (A-VIII) or Formulas (I) to (VIII) can be manufactured by introducing a prodrug-constituting group using a corresponding prodrug formation reagent such as a halide and performing purification. Examples of prodrug-constituting groups include groups described in "Development of Medicine" (Hirokawa Shoten, 1990), Vol.. 7, p. 163-198.

### [Examples]

Hereinafter, the present invention will be described in more detail with reference to reference examples, examples and test examples, but the present invention is not limited to the contents thereof.

The compounds described in the following examples, except for commercially available reagents, were named using ChemDraw Professional (PerkinElmer), MarvinSketch (ChemAxon), and the like.

The stereo configuration of the chiral center marked with "*" in a compound name means that it is a relative configuration.

### Reference Example A-1

### 5-(difluoromethyl)-2-fluorobenzaldehyde

To a mixture of 5-(difluoromethyl)-2-fluorobenzonitrile (2.30 g) and DCM (40 mL), DIBAL-H (1.02 mol/L in n-hexane) (27 mL) was added under an ice-salt bath, and the mixture was stirred under an ice-salt bath for 1 hour. To the reaction mixture, 2 mol/L hydrochloric acid (40 mL) was added, and the mixture was stirred at room temperature for 1 hour. The mixture was extracted with DCM, and the extract was concentrated under reduced pressure. The residue was purified by silica gel column chromatography (eluent: n-hexane/ethyl acetate = 98/2 to 85/15) to give the title compound (1.62 g).

### Reference Example A-2

### 5-(difluoromethyl)-2-fluorobenzyl methanesulfonate

To a mixture of Reference Example A-1 (0.500 g) and methanol (9.6 mL), sodium borohydride (0.119 g) was added under ice cooling, and the mixture was stirred under ice cooling for 1 hour. Water was added to the reaction mixture, and the mixture was extracted with DCM. The extract was dried over anhydrous magnesium sulfate and concentrated under reduced pressure. The residue was purified by silica gel column chromatography (eluent: n-hexane/ethyl acetate = 100/0 to 80/20) to give (5-(difluoromethyl)-2-fluorophenyl)methanol (0.375 g).

To a mixture of the obtained compound (0.374 g), TEA (0.258 g), and DCM (7.1 mL), methanesulfonyl chloride (0.268 g) was added under ice cooling, and the mixture was stirred under ice cooling for 10 minutes and at room temperature for 30 minutes. Water was added under ice cooling to the reaction mixture, and the mixture was extracted with DCM. The extract was concentrated under reduced pressure. The residue was purified by silica gel column chromatography (eluent: n-hexane/ethyl acetate = 85/15 to 60/40) to give the title compound (0.383 g).

### Reference Example A-3

### 3-chloro-2-fluoro-5-(trifluoromethyl)benzyl methanesulfonate

Reference Example A-3 was synthesized in the same method as in Reference Example A-2, except that 3-chloro-2-fluoro-5-(trifluoromethyl)benzaldehyde was used instead of Reference Example A-1.

### Reference Example A-4

### 4-(benzyloxy)-2-(bromomethyl)-1-fluorobenzene

To a mixture of (5-(benzyloxy)-2-fluorophenyl)methanol (0.125 g) and toluene (1.3 mL), phosphorus tribromide (0.219 g) was added at room temperature, and the mixture was stirred at 50°C for 1 hour. Phosphorus tribromide (0.029 g) was added at room temperature to the reaction mixture, and the mixture was stirred at 60°C for 1 hour. The reaction mixture was concentrated under reduced pressure to give the title compound (0.159 g).

### Reference Example A-5

### 2-fluoro-5-(1,1,2,2-tetrafluoroethoxy)benzaldehyde

To a mixture of 3-bromo-4-fluorophenol (1.15 g), 1-bromo-1,1,2,2-tetrafluoro-2-iodoethane (3.68 g), and DMSO (6.0 mL), cesium carbonate (2.93 g) was added at room temperature, and the mixture was stirred at 60°C for 1 hour. After the reaction mixture was allowed to cool to room temperature, water was added thereto. The mixture was extracted twice with n-hexane. The combined extract was washed with water and saturated brine, dried over anhydrous magnesium sulfate, and concentrated under reduced pressure.

A mixture of the residue, zinc powder (1.18 g), and acetic acid (1.0 mL) was stirred at 50°C for 4 hours. After the reaction mixture was allowed to cool to room temperature, water was added, and the mixture was extracted with DCM. The extract was washed with a saturated aqueous sodium hydrogen carbonate solution and then concentrated under reduced pressure. The residue was purified by silica gel column chromatography (eluent: n-hexane/ethyl acetate = 100/0 to 90/10) to give 2-bromo-1-fluoro-4-(1,1,2,2-tetrafluoroethoxy)benzene (0.618 g).

Under an argon atmosphere, to a mixture of the obtained compound (0.617 g) and diethyl ether (7.1 mL), n-butyllithium (1.57 mol/L in n-hexane) (1.35 mL) was added at - 78°C, and the mixture was stirred at the same temperature for 20 minutes. DMF (0.197 mL) was added at -78°C to the reaction mixture, and the mixture was stirred at the same temperature for 1 hour. A saturated aqueous ammonium chloride solution was added to the reaction mixture, and the mixture was extracted with diethyl ether. The extract was washed with water and saturated brine, dried over anhydrous magnesium sulfate, and concentrated under reduced pressure to give the title compound (0.559 g).

### Reference Example A-6

### 2-fluoro-5-(1,1,2,2-tetrafluoroethoxy)benzyl methanesulfonate

Reference Example A-6 was synthesized by the same method as in Reference Example A-2, except that Reference Example A-5 was used instead of Reference Example A-1.

### Reference Example A-7

### 2-fluoro-5-(trifluoromethyl)benzyl methanesulfonate

Reference Example A-7 was synthesized by the same method as in Reference Example A-2, except that 2-fluoro-5-(trifluoromethyl)benzaldehyde was used instead of Reference Example A-1.

### Reference Example A-8

### 1-(bromomethyl)-3-chloro-2-fluoro-5-(trifluoromethyl)benzene

To a mixture of 3-chloro-2-fluoro-5-(trifluoromethyl)benzaldehyde (1.00 g) and ethanol (10 mL), sodium borohydride (0.250 g) was added under ice cooling, and the mixture was stirred at room temperature for 20 minutes. The reaction mixture was poured into a saturated aqueous ammonium chloride solution, and the mixture was extracted with ethyl acetate. The extract was washed with water and saturated brine, and then concentrated under reduced pressure.

To a mixture of the residue and 1,2-dichloroethane (10 mL), phosphorus tribromide (1.79 g) was added at room temperature, and the mixture was stirred at the same temperature for 40 minutes. The reaction mixture was poured into a saturated aqueous sodium hydrogen carbonate solution, and the mixture was extracted with ethyl acetate. The extract was washed with saturated brine and a saturated aqueous sodium hydrogen carbonate solution, dried over anhydrous sodium sulfate, and concentrated under reduced pressure. The residue was purified by column chromatography on silica gel (eluent: n-hexane/ethyl acetate = 98/2 to 85/15) to give the title compound (0.484 g).

### Reference Example A-9

### Ethyl 2-amino-5-(1,1,1,3,3,3-hexafluoro-2-hydroxypropan-2-yl)benzoate

A mixture of ethyl 2-aminobenzoate (2.00 g), hexafluoroacetone hydrate (2.93 g), and p-toluenesulfonic acid hydrate (0.115 g) was stirred at 140°C for 20 hours under microwave irradiation. The reaction mixture was poured into a saturated aqueous sodium hydrogen carbonate solution, and the mixture was extracted with ethyl acetate. The extract was washed with saturated brine, dried over anhydrous magnesium sulfate, and concentrated under reduced pressure. The residue was purified by silica gel column chromatography (eluent: n-hexane/ethyl acetate = 95/5 to 80/20). The obtained product was suspended in water, and an insoluble material was collected by filtration. The obtained solid was dried under reduced pressure to give the title compound (2.30 g).

### Reference Example A-10

### Ethyl 2-fluoro-5-(1,1,1,3,3,3-hexafluoro-2-hydroxypropan-2-yl)benzoate

To a mixture of Reference Example A-9 (0.579 g) and DME (15 mL), boron trifluoride diethyl ether complex (0.310 g) was added under an ice-salt bath, and the mixture was stirred under an ice-salt bath for 15 minutes. A mixture of tert-butyl nitrite (0.189 g) and DME (7.5 mL) was added under an ice-salt bath to the reaction mixture, and the mixture was stirred under ice cooling for 4 hours. The reaction mixture was concentrated under reduced pressure.

A mixture of the residue and chlorobenzene (15 mL) was refluxed for 2 hours. After the reaction mixture was allowed to cool to room temperature, the mixture was concentrated under reduced pressure. The residue was purified by silica gel column chromatography (eluent: n-hexane/ethyl acetate = 98/2 to 85/15) to give the title compound (0.504 g).

### Reference Example A-11

### 2-fluoro-5-(1,1,1,3,3,3-hexafluoro-2-hydroxypropan-2-yl)benzaldehyde

To a mixture of Reference Example A-10 (0.855 g), THF (6.4 mL), and methanol (0.64 mL), lithium borohydride (4 mol/L in THF) (3.20 mL) was added under ice cooling, and the mixture was stirred at room temperature for 19 hours. Lithium borohydride (4 mol/L in THF) (1.28 mL) was added at room temperature to the reaction mixture, and the mixture was stirred at the same temperature for 1.5 hours. Methanol (0.32 mL) was added at room temperature to the reaction mixture, and the mixture was stirred at the same temperature for 2.5 hours and at 60°C for 45 minutes. To the reaction mixture, water and a saturated aqueous ammonium chloride solution were added under ice cooling, and the mixture was extracted with ethyl acetate. The extract was washed with water and saturated brine, dried over anhydrous magnesium sulfate, and concentrated under reduced pressure. The residue was purified by silica gel column chromatography (eluent: n-hexane/ethyl acetate = 90/10 to 70/30) to give 1,1,1,3,3,3-hexafluoro-2-(4-fluoro-3-(hydroxymethyl)phenyl)propan-2-ol (0.696 g).

To a mixture of the obtained compound (0.696 g) and DCM (6.2 mL), manganese (IV) oxide (1.61 g) was added at room temperature, and the mixture was stirred at the same temperature for 65 hours. The reaction mixture was filtered through Celite, and the filtrate was concentrated under reduced pressure. The residue was purified by column chromatography on silica gel (eluent: n-hexane/ethyl acetate = 98/2 to 70/30) to give the title compound (0.261 g).

### Reference Example A-12

### 2-(1-bromoethyl)-1-fluoro-4-(trifluoromethyl)benzene

Reference Example A-12 was synthesized by the same method as in Reference Example A-8, except that 1-(2-fluoro-5-(trifluoromethyl)phenyl)ethan-1-one was used instead of 3-chloro-2-fluoro-5-(trifluoromethyl)benzaldehyde.

### Reference Example B-1

### (S)-6-(trifluoromethyl)piperidin-2-one

Under an argon atmosphere, a mixture of 2-bromo-6-(trifluoromethyl)pyridine (4.00 g), (R)-4-benzyloxazolidin-2-one (3.76 g), potassium carbonate (4.89 g), copper(I) iodide (0.169 g), 1,10-phenanthroline (0.319 g), and toluene (30 mL) was stirred at 140°C for 14 hours under microwave irradiation. The reaction mixture was filtered through Celite, and the filtrate was concentrated under reduced pressure. The residue was purified by silica gel column chromatography (eluent: n-hexane/ethyl acetate = 95/5 to 85/15) to give (R)-4-benzyl-3-(6-(trifluoromethyl)pyridin-2-yl)oxazolidin-2-one (5.80 g).

To a mixture of the obtained compound (0.872 g), THF (4.4 mL), water (4.4 mL), and methanesulfonic acid (1.04 g), 10% Pd/C (0.548 g) was added at room temperature, and the mixture was stirred at 50°C for 35 hours under a hydrogen atmosphere. The reaction mixture was filtered through Celite, and a saturated aqueous sodium hydrogen carbonate solution was added to the filtrate. The mixture was extracted with ethyl acetate, and the extract was concentrated under reduced pressure.

A mixture of the residue and toluene (9.0 mL) was stirred at 100°C for 2 hours. The reaction mixture was concentrated under reduced pressure. Ethyl acetate (2.7 mL), n-hexane (16 mL), and a small amount of (R)-4-benzyloxazolidin-2-one were added to the residue, and the mixture was stirred at room temperature for 15 minutes. The mixture was filtered, the insoluble material was washed with n-hexane/ethyl acetate (8/1), and the filtrate was concentrated under reduced pressure. The residue was purified by silica gel column chromatography (eluent: n-hexane/ethyl acetate/methanol = 50/50/0 to 0/100/0 to 0/90/10) to give the title compound (0.321 g).

### Reference Example B-2

### (S)-6-(difluoromethyl)piperidin-2-one

A mixture of 2-bromo-6-(difluoromethyl)pyridine (3.14 g), (R)-4-benzyloxazolidin-2-one (3.21 g), potassium carbonate (4.17 g), copper(I) iodide (0.144 g), 1,10-phenanthroline (0.272 g), and toluene (25 mL) was stirred at 140°C for 14 hours under microwave irradiation. The reaction mixture was filtered through Celite, and the filtrate was concentrated under reduced pressure. The residue was purified by silica gel column chromatography (eluent: n-hexane/ethyl acetate = 98/2 to 75/25) to give (R)-4-benzyl-3-(6-(difluoromethyl)pyridin-2-yl)oxazolidin-2-one (4.57 g).

A mixture of the obtained compound (4.57 g), 10% Pd/C (1.29 g), THF (40 mL), water (40 mL), and concentrated hydrochloric acid (1.23 g) was stirred at 40°C for 90 hours under hydrogen pressure (0.4 MPa). After the reaction mixture was allowed to cool to room temperature, it was filtered through Celite. Concentrated hydrochloric acid (1.23 g) was added to the filtrate, and the mixture was stirred at 70°C for 1.5 hours. After the reaction mixture was allowed to cool to room temperature, sodium carbonate (8.95 g) was added. The mixture was filtered through Celite, and the filtrate was extracted with ethyl acetate. The extract was washed with saturated brine, dried over anhydrous magnesium sulfate, and concentrated under reduced pressure. The residue was purified by silica gel column chromatography (eluent: n-hexane/ethyl acetate/methanol = 50/50/0 to 0/100/0 to 0/90/10) to give the title compound (0.880 g).

### Reference Example B-3

### (S)-5-fluoropiperidin-2-one

Reference Example B-3 was synthesized by the same method as in Reference Example B-2, except that 2-bromo-5-fluoropyridine was used instead of 2-bromo-6-(difluoromethyl)pyridine.

### Reference Example B-4

### (4aS^{*},8aR^{*})octahydroquinolin-2(1H)-one

To a mixture of 3,4,5,6,7,8-hexahydroquinolin-2(1H)-one (0.500 g) and formic acid (2.5 mL), sodium formate (1.40 g) was added at room temperature, and the mixture was refluxed for 40 hours. After the reaction mixture was allowed to cool to room temperature, a 5 mol/L aqueous sodium hydroxide solution was added, and the mixture was extracted with ethyl acetate. The extract was washed with saturated brine, dried over anhydrous magnesium sulfate, and concentrated under reduced pressure. The residue was purified by silica gel column chromatography (eluent: n-hexane/ethyl acetate/methanol = 30/70/0 to 0/100/0 to 0/90/10) to give the title compound (0.108 g).

### Reference Example C-1

### 1-(5-bromo-2-fluorobenzyl)-6-methylpiperidin-2-one

Under an argon atmosphere, to a mixture of 6-methylpiperidin-2-one (0.320 g) and DMF (6.9 mL), sodium hydride (approximately 60%) (0.170 g) was added under ice cooling, and the mixture was stirred under ice cooling for 10 minutes. To the reaction mixture, 4-bromo-2-(bromomethyl)-1-fluorobenzene (0.833 g) was added under ice cooling, and the mixture was stirred at room temperature for 1.5 hours. To the reaction mixture, a saturated aqueous ammonium chloride solution was added under ice cooling, and the mixture was extracted with n-hexane/ethyl acetate (1/1). The extract was washed with water and saturated brine, dried over anhydrous magnesium sulfate, and concentrated under reduced pressure. The residue was purified by silica gel column chromatography (eluent: n-hexane/ethyl acetate = 70/30 to 20/80) to give the title compound (0.688 g).

### Reference Example C-2

### 1-(5-(difluoromethyl)-2-fluorobenzyl)-6-methylpiperidin-2-one

Reference Example C-2 was synthesized by the same method as in Reference Example C-1, except that Reference Example A-2 was used instead of 4-bromo-2-(bromomethyl)-1-fluorobenzene.

### Reference Example C-3

### 1-(3-chloro-2-fluoro-5-(trifluoromethyl)benzyl)-6-methylpiperidin-2-one

Reference Example C-3 was synthesized by the same method as in Reference Example C-1, except that Reference Example A-3 was used instead of 4-bromo-2-(bromomethyl)-1-fluorobenzene.

### Reference Example C-4

### 1-((4-fluoro-(1,1'-biphenyl)-3-yl)methyl)-6-methylpiperidin-2-one

To a mixture of Reference Example C-1 (0.210 g), phenylboronic acid (0.094 g), potassium carbonate (0.145 g), THF (1.1 mL), and water (1.1 mL), bis(triphenylphosphine)palladium(II) chloride (0.049 g) was added at room temperature, and the mixture was stirred at 70°C for 30 minutes. After the reaction mixture was allowed to cool to room temperature, it was poured into water, and the mixture was extracted with ethyl acetate. The extract was washed with water and saturated brine, and then concentrated under reduced pressure. The residue was purified by silica gel column chromatography (eluent: n-hexane/ethyl acetate/methanol = 100/0/0 to 0/100/0 to 0/88/12) to give the title compound (0.209 g).

### Reference Example C-5

### 1-(5-benzyl-2-fluorobenzyl)-6-methylpiperidin-2-one

A mixture of Reference Example C-1 (0.160 g), potassium benzyltrifluoroborate (0.211 g), cesium carbonate (0.347 g), bis(di-tert-butyl(4-dimethylaminophenyl)phosphine)dichloropalladium(II) (0.038 g), 1,4-dioxane (1.6 mL), and water (0.16 mL) was stirred at 120°C for 1 hour under microwave irradiation. At room temperature, the reaction mixture was poured into a saturated aqueous ammonium chloride solution, and the mixture was extracted with ethyl acetate. The extract was washed with water and saturated brine, dried over anhydrous magnesium sulfate, and concentrated under reduced pressure. The residue was purified by column chromatography on silica gel (eluent: n-hexane/ethyl acetate = 100/0 to 60/40) to give the title compound (0.056 g).

### Reference Example C-6

### 6-methyl-1-(3-phenoxybenzyl)piperidin-2-one

Reference Example C-6 was synthesized by the same method as in Reference Example C-1, except that 1-(bromomethyl)-3-phenoxybenzene was used instead of 4-bromo-2-(bromomethyl)-1-fluorobenzene.

### Reference Example C-7

### 1-(5-(benzyloxy)-2-fluorobenzyl)-6-methylpiperidin-2-one

Reference Example C-7 was synthesized by the same method as in Reference Example C-1, except that Reference Example A-4 was used instead of 4-bromo-2-(bromomethyl)-1-fluorobenzene.

### Reference Example C-8

### (R)-1-(2-fluoro-5-(trifluoromethyl)benzyl)-6-methylpiperidin-2-one

To a mixture of (R)-6-methylpiperidin-2-one (0.150 g), 2-(bromomethyl)-1-fluoro-4-(trifluoromethyl)benzene (0.409 g), and DMF (2.0 mL), sodium hydride (approximately 60%) (0.064 g) was added under water cooling, and the mixture was stirred at room temperature for 1 hour. To the reaction mixture, a saturated aqueous ammonium chloride solution and water were added under water cooling, and the mixture was extracted with n-hexane/ethyl acetate (1/1). The extract was washed with water and then concentrated under reduced pressure. The residue was purified by column chromatography on silica gel (eluent: n-hexane/ethyl acetate = 90/10 to 15/85) to give the title compound (0.352 g).

### Reference Example C-9

### (S)-1-(2-fluoro-5-(trifluoromethyl)benzyl)-6-(trifluoromethyl)piperidin-2-one

Under an argon atmosphere, to a mixture of Reference Example B-1 (0.113 g) and DMF (1.8 mL), sodium hydride (approximately 60%) (0.041 g) was added under ice cooling, and the mixture was stirred under ice cooling for 10 minutes. To the reaction mixture, 2-(bromomethyl)-1-fluoro-4-(trifluoromethyl)benzene (0.226 g) was added under ice cooling, and the mixture was stirred at room temperature for 2 hours. To the reaction mixture, a saturated aqueous ammonium chloride solution and water were added under ice cooling, and the mixture was extracted with n-hexane/ethyl acetate (1/1). The extract was washed with water and saturated brine, dried over anhydrous magnesium sulfate, and concentrated under reduced pressure. The residue was purified by silica gel column chromatography (eluent: n-hexane/ethyl acetate = 70/30 to 20/80) to give the title compound (0.175 g).

### Reference Example C-10

### (S)-1-(5-(difluoromethyl)-2-fluorobenzyl)-6-(trifluoromethyl)piperidin-2-one

Under an argon atmosphere, to a mixture of Reference Example B-1 (0.117 g) and DMF (1.8 mL), sodium hydride (approximately 60%) (0.040 g) was added under ice cooling, and the mixture was stirred under ice cooling for 10 minutes. To the reaction mixture, Reference Example A-2 (0.204 g) was added under ice cooling, and the mixture was stirred at room temperature for 2 hours. To the reaction mixture, a saturated aqueous ammonium chloride solution was added under ice cooling, and the mixture was extracted with n-hexane/ethyl acetate (1/1). The extract was washed with water and saturated brine, dried over anhydrous magnesium sulfate, and concentrated under reduced pressure. The residue was purified by silica gel column chromatography (eluent: n-hexane/ethyl acetate = 70/30 to 20/80) to give the title compound (0.112 g).

### Reference Example C-11

### (S)-1-(2-fluoro-5-(1,1,2,2-tetrafluoroethoxy)benzyl)-6-(trifluoromethyl)piperidin-2-one

Under an argon atmosphere, to a mixture of Reference Example B-1 (0.100 g) and DMF (1.6 mL), sodium hydride (approximately 60%) (0.036 g) was added under ice cooling, and the mixture was stirred under ice cooling for 15 minutes. To the reaction mixture, Reference Example A-6 (0.220 g) was added under ice cooling, and the mixture was stirred at room temperature for 65 hours. To the reaction mixture, a saturated aqueous ammonium chloride solution and water were added under ice cooling, and the mixture was extracted with n-hexane/ethyl acetate (1/1). The extract was washed with water and saturated brine, dried over anhydrous magnesium sulfate, and concentrated under reduced pressure. The residue was purified by Method A (eluent: n-hexane/ethyl acetate = 85/15 to 50/50) to give the title compound (0.166 g).

### Reference Example C-12

### (S)-6-(difluoromethyl)-1-(2-fluoro-5-(trifluoromethyl)benzyl)piperidin-2-one

Under an argon atmosphere, to a mixture of Reference Example B-2 (0.101 g) and DMF (1.8 mL), sodium hydride (approximately 60%) (0.041 g) was added under ice cooling, and the mixture was stirred under ice cooling for 10 minutes. To the reaction mixture, 2-(bromomethyl)-1-fluoro-4-(trifluoromethyl)benzene (0.226 g) was added under ice cooling, and the mixture was stirred at room temperature for 2 hours. To the reaction mixture, a saturated aqueous ammonium chloride solution and water were added under ice cooling, and the mixture was extracted with n-hexane/ethyl acetate (1/1). The extract was washed with water and saturated brine, dried over anhydrous magnesium sulfate, and concentrated under reduced pressure. The residue was purified by silica gel column chromatography (eluent: n-hexane/ethyl acetate = 70/30 to 20/80) to give the title compound (0.157 g).

### Reference Example C-13

### (S)-5-fluoro-1-(2-fluoro-5-(trifluoromethyl)benzyl)piperidin-2-one

Reference Example C-13 was synthesized by the same method as in Reference Example C-9, except that Reference Example B-3 was used instead of Reference Example B-1.

### Reference Example C-14

### 4-(3-(trifluoromethyl)benzyl)-4-azaspiro[2.5]octan-5-one

Reference Example C-14 was synthesized by the same method as in Reference Example C-1, except that 4-azaspiro[2.5]octan-5-one was used instead of 6-methylpiperidin-2-one and 1-(bromomethyl)-3-(trifluoromethyl)benzene was used instead of 4-bromo-2-(bromomethyl)-1-fluorobenzene.

### Reference Example C-15

### 2-(2-fluoro-5-(trifluoromethyl)benzyl)-2-azabicyclo[4.1.0]heptan-3-one

Reference Example C-15 was synthesized by the same method as in Reference Example C-1, except that 2-azabicyclo[4.1.0]heptan-3-one was used instead of 6-methylpiperidin-2-one and Reference Example A-7 was used instead of 4-bromo-2-(bromomethyl)-1-fluorobenzene.

### Reference Example C-16

### (6S)-1-(1-(2-fluoro-5-(trifluoromethyl)phenyl)ethyl)-6-(trifluoromethyl)piperidin-2-one

Reference Example C-16 was synthesized by the same method as in Reference Example C-1, except that Reference Example B-1 was used instead of 6-methylpiperidin-2-one and Reference Example A-12 was used instead of 4-bromo-2-(bromomethyl)-1-fluorobenzene.

### Reference Example C-17

### 6-methyl-1-(3-(trifluoromethyl)phenethyl)piperidin-2-one

To a mixture of 5-oxohexanoic acid (0.200 g), 2-(3-(trifluoromethyl)phenyl)ethan-1-amine (0.291 g), and methanol (4.0 mL), decaborane (0.056 g) was added under water cooling, and the mixture was stirred at room temperature for 30 minutes. APS was added to the reaction mixture, and the mixture was stirred at room temperature for 15 minutes. The mixture was filtered, and the filtrate was concentrated under reduced pressure.

A mixture of the residue, DIPEA (0.397 g), HATU (0.701 g), and MeCN (6.0 mL) was stirred at room temperature for 30 minutes. A saturated aqueous ammonium chloride solution and water were added to the reaction mixture, and the mixture was extracted with ethyl acetate. The extract was concentrated under reduced pressure. The residue was purified by APS column chromatography (eluent: n-hexane/ethyl acetate = 90/10 to 0/100) to give the title compound (0.269 g).

### Reference Example C-18

### (4aS^{*},8aR^{*})-1-(3-(trifluoromethyl)benzyl)octahydroquinolin-2(1H)-one

Reference Example C-18 was synthesized by the same method as in Reference Example C-1, except that Reference Example B-4 was used instead of 6-methylpiperidin-2-one and 1-(bromomethyl)-3-(trifluoromethyl)benzene was used instead of 4-bromo-2-(bromomethyl)-1-fluorobenzene.

### Reference Example D-1

### tert-butyl (6-bromo-2-hydroxypyridin-3-yl)carbamate

A mixture of 3-amino-6-bromopyridin-2-ol hydrobromide (1.00 g), Boc₂O (0.970 g), TEA (1.13 g), DMAP (0.045 g), and DCM (10 mL) was stirred at room temperature for 2 hours. A saturated aqueous ammonium chloride solution was added to the reaction mixture, and the mixture was extracted with DCM. The extract was concentrated under reduced pressure. The residue was purified by silica gel column chromatography (eluent: n-hexane/ethyl acetate = 100/0 to 80/20) to give the title compound (0.402 g).

### Reference Example D-2

### tert-butyl (4-hydroxy-2-methylpyrimidin-5-yl)carbamate

To a mixture of 4-hydroxy-2-methylpyrimidine-5-carboxylic acid (0.354 g), TEA (0.465 g), tert-butyl alcohol (0.851 g), and toluene (10 mL), DPPA (0.695 g) was added at room temperature, and the mixture was refluxed for 3.5 hours. After the reaction mixture was allowed to cool to room temperature, water and a saturated aqueous ammonium chloride solution were added, and the mixture was extracted with DCM. The extract was concentrated under reduced pressure. The residue was purified by silica gel column chromatography (eluent: n-hexane/ethyl acetate/methanol = 70/30/0 to 0/100/0 to 0/85/15) to give the title compound (0.068 g).

### Reference Example D-3

### tert-butyl (3-hydroxy-5-methylpyrazin-2-yl)carbamate

Reference Example D-3 was synthesized by the same method as in Reference Example D-2, except that 3-hydroxy-5-methylpyrazine-2-carboxylic acid was used instead of 4-hydroxy-2-methylpyrimidine-5-carboxylic acid.

### Reference Example E-1

### 5-bromo-1-(2-fluoro-5-(trifluoromethyl)benzyl)-3-nitropyridin-2(1H)-one

To a mixture of 5-bromo-3-nitropyridin-2-ol (0.222 g) and DMF (4.0 mL), cesium carbonate (0.380 g) and 2-(bromomethyl)-1-fluoro-4-(trifluoromethyl)benzene (0.200 g) were added at room temperature, and the mixture was stirred at 70°C for 1 hour. After the reaction mixture was allowed to cool to room temperature, water, n-hexane, and ethyl acetate were added, and an organic layer was separated. The organic layer was washed with water and saturated brine, dried over anhydrous sodium sulfate, and concentrated under reduced pressure. The residue was purified by Method A (eluent: n-hexane/ethyl acetate = 70/30 to 20/80) to give the title compound (0.180 g).

### Reference Example E-2

### 3-amino-5-bromo-1-(2-fluoro-5-(trifluoromethyl)benzyl)pyridin-2(1H)-one

To a mixture of Reference Example E-1 (0.180 g) and DMF (2.0 mL), tetrahydroxydiboron (0.123 g) and 4,4'-dipyridyl (0.0004 g) were added at room temperature, and the mixture was stirred at the same temperature for 10 minutes. Water, n-hexane, and ethyl acetate were added to the reaction mixture, and an organic layer was separated. The organic layer was washed with water and saturated brine, dried over anhydrous sodium sulfate, and concentrated under reduced pressure. The residue was purified by Method A (eluent: n-hexane/ethyl acetate = 90/10 to 60/40) to give the title compound (0.140 g).

### Reference Example E-3

### 1-(2-fluoro-5-(trifluoromethyl)benzyl)-6-methyl-3-nitropyridin-2(1H)-one

To a mixture of 6-methyl-3-nitropyridin-2-ol (0.156 g) and DMF (2.0 mL), cesium carbonate (0.380 g) and 2-(bromomethyl)-1-fluoro-4-(trifluoromethyl)benzene (0.200 g) were added at room temperature, and the mixture was stirred at 70°C for 30 minutes. After the reaction mixture was allowed to cool to room temperature, water, n-hexane, and ethyl acetate were added, and an organic layer was separated. The organic layer was washed with water and saturated brine, dried over anhydrous sodium sulfate, and concentrated under reduced pressure. The residue was purified by Method A (eluent: n-hexane/ethyl acetate = 70/30 to 20/80) to give the title compound (0.068 g).

### Reference Example E-4

### 3-amino-1-(2-fluoro-5-(trifluoromethyl)benzyl)-6-methylpyridin-2(1H)-one

A mixture of Reference Example E-3 (0.065 g), 10% Pd/C (0.050 g), THF (1.0 mL), and methanol (2.0 mL) was stirred at room temperature for 1 hour under a hydrogen atmosphere. The reaction mixture was filtered through Celite, and the filtrate was concentrated under reduced pressure. The residue was purified by APS column chromatography (eluent: n-hexane/ethyl acetate = 80/20 to 20/80) to give the title compound (0.056 g).

### Reference Example E-5

### 1-(3-chloro-2-fluoro-5-(trifluoromethyl)benzyl)-6-methyl-3-nitropyridin-2(1H)-one

To a mixture of 6-methyl-3-nitropyridin-2-ol (0.350 g) and DMF (5.0 mL), cesium carbonate (0.805 g) and Reference Example A-8 (0.480 g) were added at room temperature, and the mixture was stirred at 70°C for 30 minutes. After the reaction mixture was allowed to cool to room temperature, water, n-hexane, and ethyl acetate were added, and an organic layer was separated. The organic layer was washed with water and saturated brine, dried over anhydrous sodium sulfate, and concentrated under reduced pressure. The residue was purified by Method A (eluent: n-hexane/ethyl acetate = 90/10 to 30/70) to give the title compound (0.170 g).

### Reference Example E-6

### 3-amino-1-(3-chloro-2-fluoro-5-(trifluoromethyl)benzyl)-6-methylpyridin-2(1H)-one

A mixture of Reference Example E-5 (0.170 g), 10% Pd/C (0.120 g), THF (1.0 mL), and methanol (5.0 mL) was stirred at room temperature for 2.5 hours under a hydrogen atmosphere. The reaction mixture was filtered through Celite, and the filtrate was concentrated under reduced pressure. The residue was purified by APS column chromatography (eluent: n-hexane/ethyl acetate = 80/20 to 20/80) to give the title compound (0.113 g).

### Reference Example E-7

### 1-(2-fluoro-5-(trifluoromethyl)benzyl)-3-nitro-5-(trifluoromethyl)pyridin-2(1H)-one

Reference Example E-7 was synthesized by the same method as in Reference Example E-1, except that 3-nitro-5-(trifluoromethyl)pyridin-2-ol was used instead of 5-bromo-3-nitropyridin-2-ol.

### Reference Example E-8

### 3-amino-1-(2-fluoro-5-(trifluoromethyl)benzyl)-5-(trifluoromethyl)pyridin-2(1H)-one

A mixture of Reference Example E-7 (0.153 g), 10% Pd/C (0.120 g), THF (1.0 mL), and methanol (5.0 mL) was stirred at room temperature for 2 hours under a hydrogen atmosphere. The reaction mixture was filtered through Celite, and the filtrate was concentrated under reduced pressure to give the title compound (0.141 g).

### Reference Example F-1

### 2-bromo-5-(difluoromethyl)aniline

To a mixture of 4-bromo-3-nitrobenzaldehyde (1.00 g) and toluene (10 mL), bis(2-methoxyethyl)aminosulfur trifluoride (1.92 g) was added at room temperature, and the mixture was stirred at the same temperature for 2 hours. The reaction mixture was poured into a saturated aqueous sodium hydrogen carbonate solution (20 mL) under ice cooling, and the mixture was extracted with ethyl acetate. The extract was washed with saturated brine, dried over anhydrous sodium sulfate, and concentrated under reduced pressure.

To a mixture of the residue and DMF (10 mL), tetrahydroxydiboron (1.17 g) and 4,4'-dipyridyl (0.003 g) were added under water cooling, and the mixture was stirred under water cooling for 5 minutes. Water was added to the reaction mixture, and the mixture was extracted with ethyl acetate. The extract was washed with water and saturated brine, dried over anhydrous sodium sulfate, and concentrated under reduced pressure. The residue was purified by Method A (eluent: n-hexane/ethyl acetate = 98/2 to 80/20) to give the title compound (0.840 g).

### Reference Example G-1

### (R)-3-amino-1-(3-methylbenzyl)-3,4-dihydroquinolin-2(1H)-one

To a mixture of 2-bromoaniline (0.200 g), 3-methylbenzaldehyde (0.140 g), and methanol (1.0 mL), decaborane (0.043 g) was added at room temperature, and the mixture was stirred at the same temperature for 20 minutes. APS was added to the reaction mixture, and the mixture was stirred at room temperature for 30 minutes. The mixture was filtered, and the filtrate was concentrated under reduced pressure. The residue was purified by silica gel column chromatography (eluent: n-hexane/ethyl acetate = 98/2 to 85/15) to give 2-bromo-N-(3-methylbenzyl)aniline (0.294 g).

Under an argon atmosphere, to a mixture of methyl (S)-2-((tert-butoxycarbonyl)amino)-3-iodopropanoate (0.526 g) and DMF (1.2 mL), zinc powder (0.153 g) was added at room temperature, and the mixture was stirred at the same temperature for 30 minutes. To the reaction mixture, a mixture of the above 2-bromo-N-(3-methylbenzyl)aniline (0.294 g) and DMF (1.2 mL), palladium(II) acetate (0.012 g), and Xphos (0.051 g) were added at room temperature, and the mixture was stirred at 45°C for 1 hour. After the reaction mixture was allowed to cool to room temperature, a saturated aqueous ammonium chloride solution and ethyl acetate were added, and the mixture was stirred at the same temperature for 10 minutes. The mixture was filtered through Celite, and an organic layer of the filtrate was separated. The organic layer was washed with water and saturated brine, dried over anhydrous sodium sulfate, and concentrated under reduced pressure.

To a mixture of the residue, ethanol (0.88 mL), and toluene (1.5 mL), methanesulfonic acid (0.614 g) was added at room temperature, and the mixture was stirred at 65°C for 1 hour and at room temperature overnight. Ethyl acetate, toluene, and water were added to the reaction mixture, and an aqueous layer was separated. A saturated aqueous sodium hydrogen carbonate solution was added to the aqueous layer, and the mixture was extracted with ethyl acetate. The extract was concentrated under reduced pressure to give the title compound (0.075 g).

### Reference Example G-2

### (R)-3-((3-amino-2-oxo-3,4-dihydroquinolin-1(2H)-yl)methyl)benzonitrile

To a mixture of 2-bromoaniline (0.200 g), 3-formylbenzonitrile (0.198 g), and methanol (2.0 mL), decaborane (0.043 g) was added under water cooling, and the mixture was stirred at room temperature for 30 minutes. APS was added to the reaction mixture, and the mixture was stirred at room temperature for 20 minutes. The mixture was filtered, and the filtrate was concentrated under reduced pressure. The residue was purified by APS column chromatography (eluent: n-hexane/ethyl acetate = 100/0 to 70/30) to give 3-(((2-bromophenyl)amino)methyl)benzonitrile (0.242 g).

Under an argon atmosphere, to a mixture of methyl (S)-2-((tert-butoxycarbonyl)amino)-3-iodopropanoate (0.413 g), THF (0.48 mL), and DMF (0.96 mL), zinc powder (0.120 g) was added under water cooling, and the mixture was stirred at room temperature for 1 hour. To the reaction mixture, a mixture of the above 3-(((2-bromophenyl)amino)methyl)benzonitrile (0.240 g) and THF (0.96 mL), palladium(II) acetate (0.009 g), and Xphos (0.040 g) were added at room temperature, and the mixture was stirred at 40°C for 2 hours. A saturated aqueous ammonium chloride solution, water, and n-hexane/ethyl acetate (1/1) were added to the reaction mixture, and the mixture was stirred at room temperature for 10 minutes. The mixture was filtered through Celite, and an organic layer of the filtrate was separated. The organic layer was washed with water, and then was concentrated under reduced pressure.

To a mixture of the residue, ethanol (0.72 mL), and toluene (1.2 mL), methanesulfonic acid (0.241 g) was added at room temperature, and the mixture was stirred at 70°C for 30 minutes. The reaction mixture was concentrated under reduced pressure. Ethyl acetate, toluene, and water were added to the residue, and an aqueous layer was separated. A saturated aqueous sodium hydrogen carbonate solution was added to the aqueous layer, and the mixture was extracted with ethyl acetate. The extract was washed with water and saturated brine, and concentrated under reduced pressure to give the title compound (0.043 g).

### Reference Example G-3

### (R)-3-amino-1-(3-methoxybenzyl)-3,4-dihydroquinolin-2(1H)-one

Reference Example G-3 was synthesized by the same method as in Reference Example G-1, except that 3-methoxybenzaldehyde was used instead of 3-methylbenzaldehyde.

### Reference Example G-4

### (R)-3-amino-1-(5-(difluoromethyl)-2-fluorobenzyl)-3,4-dihydroquinolin-2(1H)-one

To a mixture of 2-bromoaniline (0.100 g), Reference Example A-1 (0.111 g), and methanol (2.0 mL), decaborane (0.021 g) was added under ice cooling, and the mixture was stirred at room temperature for 13 hours. APS was added to the reaction mixture, and the mixture was stirred at room temperature for 10 minutes. The mixture was filtered, and the filtrate was concentrated under reduced pressure. The residue was purified by Method A (eluent: n-hexane/ethyl acetate = 98/2 to 85/15) to give 2-bromo-N-(5-(difluoromethyl)-2-fluorobenzyl)aniline (0.188 g).

Under an argon atmosphere, to a mixture of methyl (S)-2-((tert-butoxycarbonyl)amino)-3-iodopropanoate (0.215 g) and DMF (2.0 mL), zinc powder (0.078 g) was added at room temperature, and the mixture was stirred at the same temperature for 1 hour. To the reaction mixture, the above 2-bromo-N-(5-(difluoromethyl)-2-fluorobenzyl)aniline (0.180 g), palladium(II) acetate (0.006 g), and Xphos (0.026 g) were added at room temperature, and the mixture was stirred at the same temperature for 8 hours. A saturated aqueous ammonium chloride solution and ethyl acetate were added to the reaction mixture, and the mixture was stirred at the same temperature for 10 minutes. The mixture was filtered through Celite, and an organic layer of the filtrate was separated. The organic layer was washed with water and saturated brine, dried over anhydrous sodium sulfate, and concentrated under reduced pressure.

To a mixture of the residue and DCM (3.0 mL), TFA (1.24 g) was added under ice cooling, and the mixture was stirred at room temperature for 37 hours. To the reaction mixture, a 5 mol/L aqueous sodium hydroxide solution (2.2 mL) was added under ice cooling, and the mixture was extracted with DCM The extract was concentrated under reduced pressure. The residue was purified by APS column chromatography (eluent: n-hexane/ethyl acetate/methanol = 49/49/2 to 0/98/2 to 0/86/14) to give the title compound (0.120 g).

### Reference Example G-5

### (R)-3-amino-1-((2,2-difluorobenzo[d][1,3]dioxol-4-yl)methyl)-3,4-dihydroquinolin-2(1H)-one

To a mixture of 2-bromoaniline (0.104 g), potassium carbonate (0.070 g), and DMF (1.5 mL), 4-(bromomethyl)-2,2-difluorobenzo[d][1,3]dioxole (0.127 g) was added at room temperature, and the mixture was stirred at 80°C for 17 hours. After the reaction mixture was allowed to cool to room temperature, a saturated aqueous ammonium chloride solution was added, and the mixture was extracted with n-hexane/ethyl acetate (1/1). The extract was washed with water and saturated brine, dried over anhydrous magnesium sulfate, and concentrated under reduced pressure. The residue was purified by silica gel column chromatography (eluent: n-hexane/ethyl acetate = 100/0 to 90/10) to give 2-bromo-N-((2,2-difluorobenzo[d][1,3]dioxol-4-yl)methyl)aniline (0.127 g).

Under an argon atmosphere, to a mixture of zinc powder (0.053 g) and DMF (0.48 mL), a mixture of methyl (S)-2-((tert-butoxycarbonyl)amino)-3-iodopropanoate (0.183 g) and DMF (0.24 mL) was added at room temperature, and the mixture was stirred at the same temperature for 1 hour. To the reaction mixture, a mixture of the above 2-bromo-N-((2,2-difluorobenzo[d][1,3]dioxol-4-yl)methyl)aniline (0.127 g) and DMF (0.48 mL), palladium(II) acetate (0.004 g), and Xphos (0.018 g) were added at room temperature, and the mixture was stirred at 80°C for 15 minutes and at room temperature for 2 hours. To the reaction mixture, water, a saturated aqueous ammonium chloride solution, and n-hexane/ethyl acetate (1/1) were added under ice cooling, and the mixture was stirred at room temperature for 10 minutes. The mixture was filtered through Celite, and an organic layer of the filtrate was separated. The organic layer was washed with water and saturated brine, dried over anhydrous magnesium sulfate, and concentrated under reduced pressure.

To a mixture of the residue, ethanol (0.37 mL), and toluene (0.55 mL), methanesulfonic acid (0.107 g) was added at room temperature, and the mixture was stirred at 80°C for 1 hour. To the reaction mixture, a 5 mol/L aqueous sodium hydroxide solution (0.223 mL) was added under ice cooling, and the mixture was extracted with DCM. The extract was concentrated under reduced pressure. The residue was purified by silica gel column chromatography (eluent: n-hexane/ethyl acetate/methanol = 50/50/0 to 0/100/0 to 0/80/20) to give the title compound (0.052 g).

### Reference Example G-6

### (R)-3-amino-7-methyl-1-(3-(trifluoromethyl)benzyl)-3,4-dihydroquinolin-2(1H)-one

Reference Example G-6 was synthesized by the same method as in Reference Example G-2, except that 2-bromo-5-methylaniline was used instead of 2-bromoaniline and 3-(trifluoromethyl)benzaldehyde was used instead of 3-formylbenzonitrile.

### Reference Example G-7

### (R)-3-amino-7-methoxy-1-(3-(trifluoromethyl)benzyl)-3,4-dihydroquinolin-2(1H)-one

Reference Example G-7 was synthesized by the same method as in Reference Example G-1, except that 2-bromo-5-methoxyaniline was used instead of 2-bromoaniline and 3-(trifluoromethyl)benzaldehyde was used instead of 3-methylbenzaldehyde.

### Reference Example G-8

### (R)-3-amino-2-oxo-1-(3-(trifluoromethyl)benzyl)-1,2,3,4-tetrahydroquinolin-7-carbonitrile

To a mixture of 3-amino-4-bromobenzonitrile (0.100 g), 3-(trifluoromethyl)benzaldehyde (0.088 g), and methanol (2.0 mL), decaborane (0.019 g) was added at room temperature, and the mixture was stirred at the same temperature for 30 minutes. APS was added to the reaction mixture, and the mixture was stirred at room temperature for 10 minutes. The mixture was filtered, and the filtrate was concentrated under reduced pressure. The residue was purified by silica gel column chromatography (eluent: n-hexane/ethyl acetate = 98/2 to 85/15) to give 4-bromo-3-((3-(trifluoromethyl)benzyl)amino)benzonitrile (0.200 g).

Under an argon atmosphere, to a mixture of methyl (S)-2-((tert-butoxycarbonyl)amino)-3-iodopropanoate (0.250 g) and DMF (1.0 mL), zinc powder (0.073 g) was added at room temperature, and the mixture was stirred at the same temperature for 1 hour. To the reaction mixture, a mixture of the above 4-bromo-3-((3-(trifluoromethyl)benzyl)amino)benzonitrile (0.180 g) and DMF (1.5 mL), palladium(II) acetate (0.006 g), and Xphos (0.024 g) were added at room temperature, and the mixture was stirred at 40°C for 2 hours. After the reaction mixture was allowed to cool to room temperature, a saturated aqueous ammonium chloride solution and ethyl acetate were added, and the mixture was stirred at the same temperature for 10 minutes. The mixture was filtered through Celite, and an organic layer of the filtrate was separated. The organic layer was washed with water and saturated brine, dried over anhydrous sodium sulfate, and concentrated under reduced pressure.

To a mixture of the residue, ethanol (0.5 mL), and toluene (1.0 mL), methanesulfonic acid (0.146 g) was added at room temperature, and the mixture was stirred at 70°C for 1 hour. A saturated aqueous sodium hydrogen carbonate solution was added under ice cooling to the reaction mixture, and the mixture was extracted with DCM. The extract was concentrated under reduced pressure. The residue was purified by silica gel column chromatography (eluent: n-hexane/ethyl acetate/methanol = 49/49/2 to 0/98/2 to 0/86/14) to give the title compound (0.110 g).

### Reference Example G-9

### (R)-3-amino-5-fluoro-1-(2-fluoro-5-(trifluoromethyl)benzyl)-3,4-dihydroquinolin-2(1H)-one

Reference Example G-9 was synthesized by the same method as in Reference Example G-4, except that 2-bromo-3-fluoroaniline was used instead of 2-bromoaniline and 2-fluoro-5-(trifluoromethyl)benzaldehyde was used instead of Reference Example A-1.

### Reference Example G-10

### (R)-3-amino-7-(difluoromethyl)-1-(2-fluoro-5-(trifluoromethyl)benzyl)-3,4-dihydroquinolin-2(1H)-one

Reference Example G-10 was synthesized by the same method as in Reference Example G-8, except that Reference Example F-1 was used instead of 3-amino-4-bromobenzonitrile and 2-fluoro-5-(trifluoromethyl)benzaldehyde was used instead of 3-(trifluoromethyl)benzaldehyde.

### Reference Example G-11

### (R)-3-amino-1-(5-(difluoromethyl)-2-fluorobenzyl)-7-fluoro-3,4-dihydroquinolin-2(1H)-one

Reference Example G-11 was synthesized by the same method as in Reference Example G-4, except that 2-bromo-5-fluoroaniline was used instead of 2-bromoaniline.

### Reference Example G-12

### (R)-3-amino-7-chloro-1-(5-(difluoromethyl)-2-fluorobenzyl)-3,4-dihydroquinolin-2(1H)-one

Reference Example G-12 was synthesized by the same method as in Reference Example G-4, except that 2-bromo-5-chloroaniline was used instead of 2-bromoaniline.

### Reference Example G-13

### (R)-3-amino-1-(5-(difluoromethyl)-2-fluorobenzyl)-7-methyl-3,4-dihydroquinolin-2(1H)-one

Reference Example G-13 was synthesized by the same method as in Reference Example G-4, except that 2-bromo-5-methylaniline was used instead of 2-bromoaniline.

### Reference Example G-14

### (R)-3-amino-7-(difluoromethyl)-1-(5-(difluoromethyl)-2-fluorobenzyl)-3,4-dihydroquinolin-2(1H)-one

To a mixture of Reference Example F-1 (0.100 g), Reference Example A-1 (0.086 g), and methanol (2.0 mL), decaborane (0.017 g) was added at room temperature, and the mixture was stirred at the same temperature for 30 minutes. APS was added to the reaction mixture, and the mixture was stirred at room temperature for 10 minutes. The mixture was filtered, and the filtrate was concentrated under reduced pressure. The residue was purified by Method A (eluent: n-hexane/ethyl acetate = 98/2 to 85/15) to give 2-bromo-5-(difluoromethyl)-N-(5-(difluoromethyl)-2-fluorobenzyl)aniline (0.210 g).

Under an argon atmosphere, to a mixture of methyl (S)-2-((tert-butoxycarbonyl)amino)-3-iodopropanoate (0.221 g) and DMF (2.0 mL), zinc powder (0.064 g) was added at room temperature, and the mixture was stirred at the same temperature for 1 hour. To the reaction mixture, a mixture of the above 2-bromo-5-(difluoromethyl)-N-(5-(difluoromethyl)-2-fluorobenzyl)aniline (0.170 g) and DMF (2.0 mL), palladium(II) acetate (0.005 g), and Xphos (0.021 g) were added at room temperature, and the mixture was stirred at 40°C for 2 hours. After the reaction mixture was allowed to cool to room temperature, a saturated aqueous ammonium chloride solution and ethyl acetate were added, and the mixture was stirred at the same temperature for 10 minutes. The mixture was filtered through Celite, and an organic layer of the filtrate was separated. The organic layer was washed with water and saturated brine, dried over anhydrous sodium sulfate, and concentrated under reduced pressure.

To a mixture of the residue, ethanol (1.0 mL), and toluene (2.0 mL), methanesulfonic acid (0.129 g) was added at room temperature, and the mixture was stirred at 70°C for 2 hours. A saturated aqueous sodium hydrogen carbonate solution was added under ice cooling to the reaction mixture, and the mixture was extracted with DCM. The extract was concentrated under reduced pressure. The residue was purified by silica gel column chromatography (eluent: n-hexane/ethyl acetate/methanol = 49/49/2 to 0/98/2 to 0/86/14) to give the title compound (0.065 g).

### Reference Example G-15

### (R)-3-amino-1-(5-(difluoromethyl)-2-fluorobenzyl)-7-(trifluoromethyl)-3,4-dihydroquinolin-2(1H)-one

Reference Example G-15 was synthesized by the same method as in Reference Example G-14, except that 2-bromo-5-(trifluoromethyl)aniline was used instead of Reference Example F-1.

### Reference Example G-16

### (R)-3-amino-1-(5-(difluoromethyl)-2-fluorobenzyl)-7-(hydroxymethyl)-3,4-dihydroquinolin-2(1H)-one

To a mixture of methyl 3-amino-4-bromobenzoate (1.00 g), Reference Example A-1 (0.917 g), and methanol (2.0 mL), decaborane (0.159 g) was added at room temperature, and the mixture was stirred at the same temperature for 30 minutes. APS was added to the reaction mixture, and the mixture was stirred at room temperature for 10 minutes. The mixture was filtered, and the filtrate was concentrated under reduced pressure. The residue was purified by silica gel column chromatography (eluent: n-hexane/ethyl acetate = 98/2 to 85/15) to give methyl 4-bromo-3-((5-(difluoromethyl)-2-fluorobenzyl)amino)benzoate (1.36 g).

To a mixture of the obtained compound (1.36 g) and THF (12 mL), lithium aluminum hydride (1 mol/L in THF) (4.19 mL) was added under ice cooling, and the mixture was stirred under ice cooling for 2 hours. Water (0.16 mL), a 5 mol/L aqueous sodium hydroxide solution (0.16 mL), and water (0.48 mL) were added to the reaction mixture, and the mixture was stirred at room temperature for a while. The mixture was filtered through Celite, and the filtrate was concentrated under reduced pressure to give (4-bromo-3-((5-(difluoromethyl)-2-fluorobenzyl)amino)phenyl)methanol (1.28 g).

Under an argon atmosphere, to a mixture of zinc powder (0.160 g) and DMF (2.0 mL), a mixture of methyl (S)-2-((tert-butoxycarbonyl)amino)-3-iodopropanoate (0.548 g) and DMF (1.0 mL) was added at room temperature, and the mixture was stirred at the same temperature for 1 hour. To the reaction mixture, a mixture of the above (4-bromo-3-((5-(difluoromethyl)-2-fluorobenzyl)amino)phenyl)methanol (0.400 g) and DMF (2.0 mL), palladium(II) acetate (0.012 g), and Xphos (0.053 g) were added at room temperature, and the mixture was stirred at 40°C for 16 hours. After the reaction mixture was allowed to cool to room temperature, water, a saturated aqueous ammonium chloride solution, and n-hexane/ethyl acetate (1/1) were added, and the mixture was stirred at the same temperature for 10 minutes. The mixture was filtered through Celite, and an organic layer of the filtrate was separated. The organic layer was washed with water and saturated brine, dried over anhydrous magnesium sulfate, and concentrated under reduced pressure.

To a mixture of the residue, ethanol (1.0 mL), and toluene (1.5 mL), methanesulfonic acid (0.320 g) was added at room temperature, and the mixture was stirred at 70°C for 1.5 hours. To the reaction mixture, a 5 mol/L aqueous sodium hydroxide solution (0.666 mL) was added under ice cooling, and the mixture was extracted with DCM. The extract was concentrated under reduced pressure. The residue was purified by silica gel column chromatography (eluent: n-hexane/ethyl acetate/methanol = 50/50/0 to 0/100/0 to 0/65/35) to give the title compound (0.228 g).

### Reference Example G-17

### (R)-3-amino-1-(5-(difluoromethyl)-2-fluorobenzyl)-5,7-difluoro-3,4-dihydroquinolin-2(1H)-one

Reference Example G-17 was synthesized by the same method as in Reference Example G-4, except that 2-bromo-3,5-difluoroaniline was used instead of 2-bromoaniline.

### Reference Example G-18

### (R)-3-amino-7-(difluoromethyl)-1-(2-fluoro-5-(trifluoromethoxy)benzyl)-3,4-dihydroquinolin-2(1H)-one

To a mixture of Reference Example F-1 (0.118 g), 2-fluoro-5-(trifluoromethoxy)benzaldehyde (0.116 g), and methanol (1.0 mL), decaborane (0.020 g) was added at room temperature, and the mixture was stirred at the same temperature for 2 hours. APS was added to the reaction mixture, and the mixture was stirred at room temperature for 20 minutes. The mixture was filtered, and the filtrate was concentrated under reduced pressure. The residue was purified by silica gel column chromatography (eluent: n-hexane/ethyl acetate = 98/2 to 85/15) to give 2-bromo-5-(difluoromethyl)-N-(2-fluoro-5-(trifluoromethoxy)benzyl)aniline (0.209 g).

Under an argon atmosphere, to a mixture of zinc powder (0.073 g) and DMF (0.68 mL), a mixture of methyl (S)-2-((tert-butoxycarbonyl)amino)-3-iodopropanoate (0.249 g) and DMF (0.34 mL) was added at room temperature, and the mixture was stirred at the same temperature for 1 hour. To the reaction mixture, a mixture of the above 2-bromo-5-(difluoromethyl)-N-(2-fluoro-5-(trifluoromethoxy)benzyl)aniline (0.209 g) and DMF (0.68 mL), palladium(II) acetate (0.006 g), and Xphos (0.024 g) were added at room temperature, and the mixture was stirred at 40°C for 2 hours and at room temperature for 16 hours. To the reaction mixture, water, a saturated aqueous ammonium chloride solution, and n-hexane/ethyl acetate (1/1) were added under ice cooling, and the mixture was stirred at room temperature for 10 minutes. The mixture was filtered through Celite, and an organic layer of the filtrate was separated. The organic layer was washed with water and saturated brine, dried over anhydrous magnesium sulfate, and concentrated under reduced pressure.

To a mixture of the residue, ethanol (0.52 mL), and toluene (0.77 mL), methanesulfonic acid (0.145 g) was added at room temperature, and the mixture was stirred at 80°C for 1 hour. To the reaction mixture, a 5 mol/L aqueous sodium hydroxide solution (0.300 mL) was added under ice cooling, and the mixture was extracted with DCM. The extract was concentrated under reduced pressure. The residue was purified by silica gel column chromatography (eluent: n-hexane/ethyl acetate/methanol = 50/50/0 to 0/100/0 to 0/80/20) to give the title compound (0.077 g).

### Reference Example G-19

### (R)-3-amino-7-(difluoromethyl)-1-(2-fluoro-5-(pentafluorosulfanyl)benzyl)-3,4-dihydroquinolin-2(1H)-one

Reference Example G-19 was synthesized by the same method as in Reference Example G-18, except that 2-fluoro-5-(pentafluorosulfanyl)benzaldehyde was used instead of 2-fluoro-5-(trifluoromethoxy)benzaldehyde.

### Reference Example G-20

### (R)-3-amino-7-(difluoromethyl)-1-(2-fluoro-5-(1,1,1,3,3,3-hexafluoro-2-hydroxypropan-2-yl)benzyl)-3,4-dihydroquinolin-2(1H)-one

Reference Example G-20 was synthesized by the same method as in Reference Example G-18, except that Reference Example A-11 was used instead of 2-fluoro-5-(trifluoromethoxy)benzaldehyde.

### Reference Example G-21

### (R)-3-amino-1-(3-chloro-2-fluoro-5-(trifluoromethyl)benzyl)-7-fluoro-3,4-dihydroquinolin-2(1H)-one

Reference Example G-21 was synthesized by the same method as in Reference Example G-4, except that 2-bromo-5-fluoroaniline was used instead of 2-bromoaniline and 3-chloro-2-fluoro-5-(trifluoromethyl)benzaldehyde was used instead of Reference Example A-1.

### Reference Example G-22

### (R)-3-amino-1-(3-(trifluoromethyl)benzyl)-3,4-dihydrothieno[3,4-b]pyridin-2(1H)-one

To a mixture of tert-butyl (4-bromothiophen-3-yl)carbamate (0.300 g) and DMF (3.0 mL), sodium hydride (approximately 60%) (0.047 g) and 1-(bromomethyl)-3-(trifluoromethyl)benzene (0.284 g) were added under ice cooling, and the mixture was stirred at room temperature for 1 hour. To the reaction mixture, a saturated aqueous ammonium chloride solution, water, n-hexane, and ethyl acetate were added, and an organic layer was separated. The organic layer was washed with water and saturated brine, dried over anhydrous sodium sulfate, and concentrated under reduced pressure.

To a mixture of the residue and methanol (3.0 mL), hydrogen chloride (4 mol/L in 1,4-dioxane) (1.35 mL) was added at room temperature, and the mixture was stirred at the same temperature for 4 hours. To the reaction mixture, a 5 mol/L aqueous sodium hydroxide solution (1.2 mL) was added under ice cooling, and the mixture was extracted with DCM. The extract was concentrated under reduced pressure. The residue was purified by Method A (eluent: n-hexane/ethyl acetate = 98/2 to 80/20) to give 4-bromo-N-(3-(trifluoromethyl)benzyl)thiophen-3-amine (0.360 g).

Under an argon atmosphere, to a mixture of methyl (S)-2-((tert-butoxycarbonyl)amino)-3-iodopropanoate (0.705 g) and DMF (2.0 mL), zinc powder (0.154 g) was added at room temperature, and the mixture was stirred at the same temperature for 1 hour. To the reaction mixture, a mixture of the above 4-bromo-N-(3-(trifluoromethyl)benzyl)thiophen-3-amine (0.360 g) and DMF (2.0 mL), palladium(II) acetate (0.012 g), and Xphos (0.051 g) were added at room temperature, and the mixture was stirred at 40°C for 2 hours. After the reaction mixture was allowed to cool to room temperature, a saturated aqueous ammonium chloride solution, n-hexane, and ethyl acetate were added, and the mixture was stirred at the same temperature for 10 minutes. The mixture was filtered through Celite, and an organic layer of the filtrate was separated. The organic layer was washed with water and saturated brine, dried over anhydrous sodium sulfate, and concentrated under reduced pressure.

To a mixture of the residue, ethanol (1.0 mL), and toluene (2.0 mL), methanesulfonic acid (0.309 g) was added at room temperature, and the mixture was stirred at 70°C for 1 hour. The reaction mixture was concentrated under reduced pressure. A saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by silica gel column chromatography (eluent: n-hexane/ethyl acetate/methanol = 49/49/2 to 0/98/2 to 0/86/14) to give the title compound (0.260 g).

### Reference Example G-23

### Methyl (R)-2-((tert-butoxycarbonyl)amino)-3-(3-((3-(trifluoromethyl)benzyl)amino)pyridin-4-yl)propanoate

To a mixture of 4-bromopyridin-3-amine (0.300 g), 3-(trifluoromethyl)benzaldehyde (0.302 g), and methanol (3.0 mL), decaborane (0.064 g) was added at room temperature, and the mixture was stirred at the same temperature for 14 hours. APS was added to the reaction mixture, and the mixture was stirred at room temperature for 10 minutes. The mixture was filtered, and the filtrate was concentrated under reduced pressure. The residue was purified by Method A (eluent: n-hexane/ethyl acetate = 90/10 to 50/50) to give 4-bromo-N-(3-(trifluoromethyl)benzyl)pyridin-3-amine (0.180 g).

Under an argon atmosphere, to a mixture of methyl (S)-2-((tertbutoxycarbonyl)amino)-3-iodopropanoate (0.358 g) and DMF (1.0 mL), zinc powder (0.078 g) was added at room temperature, and the mixture was stirred at the same temperature for 1 hour. To the reaction mixture, a mixture of the above 4-bromo-N-(3-(trifluoromethyl)benzyl)pyridin-3-amine (0.180 g) and DMF (1.0 mL), palladium(II) acetate (0.006 g), and Xphos (0.026 g) were added at room temperature, and the mixture was stirred at 40°C for 2 hours. After the reaction mixture was allowed to cool to room temperature, a saturated aqueous ammonium chloride solution and ethyl acetate were added, and the mixture was stirred at the same temperature for 10 minutes. The mixture was filtered through Celite, and an organic layer of the filtrate was separated. The organic layer was washed with water and saturated brine, dried over anhydrous sodium sulfate, and concentrated under reduced pressure. The residue was purified by APS column chromatography (eluent: n-hexane/ethyl acetate = 90/10 to 50/50) to give the title compound (0.049 g).

### Reference Example G-24

### (3R)-3-amino-7-fluoro-1-(1-(2-fluoro-5-(trifluoromethyl)phenyl)ethyl)-3,4-dihydroquinolin-2(1H)-one

Reference Example G-24 was synthesized by the same method as in Reference Example G-4, except that 2-bromo-5-fluoroaniline was used instead of 2-bromoaniline and 1-(2-fluoro-5-(trifluoromethyl)phenyl)ethan-1-one was used instead of Reference Example A-1.

### Reference Example G-25

### (R)-3-amino-1-phenethyl-3,4-dihydroquinolin-2(1H)-one

Reference Example G-25 was synthesized by the same method as in Reference Example G-4, except that 2-phenylacetaldehyde was used instead of Reference Example A-1.

### Reference Example G-26

### (R)-3-amino-1-(3-phenylpropyl)-3,4-dihydroquinolin-2(1H)-one

To a mixture of 3-phenylpropan-1-ol (1.50 g) and DCM (20 mL), sodium hydrogen carbonate (0.928 g) and DMP (5.57 g) were added under ice cooling, and the mixture was stirred at room temperature for 1 hour. A saturated aqueous sodium hydrogen carbonate solution and a 1 mol/L aqueous sodium thiosulfate solution were added under ice cooling to the reaction mixture, and the mixture was extracted with DCM. The extract was concentrated under reduced pressure to give 3-phenylpropanal (1.45 g).

To a mixture of the obtained compound (0.200 g) and methanol (1.0 mL), 2-bromoaniline (0.200 g) and decaborane (0.043 g) were added at room temperature, and the mixture was stirred at the same temperature for 20 minutes. APS was added to the reaction mixture, and the mixture was stirred at room temperature for 10 minutes. The mixture was filtered, and the filtrate was concentrated under reduced pressure. The residue was purified by silica gel column chromatography (eluent: n-hexane/ethyl acetate = 98/2 to 85/15) to give 2-bromo-N-(3-phenylpropyl)aniline (0.337 g).

Under an argon atmosphere, to a mixture of zinc powder (0.167 g) and DMF (2.7 mL), a mixture of methyl (S)-2-((tert-butoxycarbonyl)amino)-3-iodopropanoate (0.573 g) and THF (2.0 mL) was added at room temperature, and the mixture was stirred at the same temperature for 30 minutes. To the reaction mixture, a mixture of the above 2-bromo-N-(3-phenylpropyl)aniline (0.337 g) and THF (1.0 mL), palladium(II) acetate (0.013 g), and Xphos (0.055 g) were added at room temperature, and the mixture was stirred at the same temperature for 30 minutes. A saturated aqueous ammonium chloride solution and ethyl acetate were added to the reaction mixture, and the mixture was stirred at room temperature for 10 minutes. The mixture was filtered through Celite, and an organic layer of the filtrate was separated. The organic layer was washed with water and saturated brine, dried over anhydrous sodium sulfate, and concentrated under reduced pressure.

To a mixture of the residue, ethanol (1.0 mL), and toluene (1.7 mL), methanesulfonic acid (0.335 g) was added at room temperature, and the mixture was stirred at 40°C for 30 minutes, at 75°C for 1 hour, and at room temperature overnight. The reaction mixture was concentrated under reduced pressure. Ethyl acetate, toluene, and water were added to the residue, and an aqueous layer was separated. A saturated aqueous sodium hydrogen carbonate solution was added to the aqueous layer, and the mixture was extracted with ethyl acetate. The extract was dried over anhydrous sodium sulfate and concentrated under reduced pressure to give the title compound (0.203 g).

### Reference Example G-27

### (R)-3-amino-1-(cyclohexylmethyl)-3,4-dihydroquinolin-2(1H)-one

To a mixture of 2-bromoaniline (0.150 g), cyclohexanecarbaldehyde (0.103 g), and methanol (1.7 mL), decaborane (0.032 g) was added under water cooling, and the mixture was stirred at room temperature for 30 minutes. APS was added to the reaction mixture, and the mixture was stirred at room temperature for 15 minutes. The mixture was filtered, and the filtrate was concentrated under reduced pressure. The residue was purified by APS column chromatography (eluent: n-hexane) to give 2-bromo-N-(cyclohexylmethyl)aniline (0.196 g).

Under an argon atmosphere, to a mixture of methyl (S)-2-((tert-butoxycarbonyl)amino)-3-iodopropanoate (0.356 g) and DMF (1.2 mL), zinc powder (0.104 g) was added under water cooling, and the mixture was stirred at room temperature for 1 hour. To the reaction mixture, a mixture of the above 2-bromo-N-(cyclohexylmethyl)aniline (0.193 g) and DMF (0.77 mL), palladium(II) acetate (0.008 g), and Xphos (0.034 g) were added at room temperature, and the mixture was stirred at 40°C for 3 hours. A saturated aqueous ammonium chloride solution, water, and n-hexane/ethyl acetate (1/1) were added to the reaction mixture, and the mixture was stirred at room temperature for 10 minutes. The mixture was filtered through Celite, and an organic layer of the filtrate was separated. The organic layer was washed with water, and then was concentrated under reduced pressure.

To a mixture of the residue, ethanol (0.58 mL), and toluene (0.97 mL), methanesulfonic acid (0.208 g) was added at room temperature, and the mixture was stirred at 70°C for 2 hours. The reaction mixture was concentrated under reduced pressure. A saturated aqueous sodium hydrogen carbonate solution was added to the residue, and the mixture was extracted with ethyl acetate. The extract was concentrated under reduced pressure. The residue was purified by silica gel column chromatography (eluent: ethyl acetate/methanol = 100/0 to 70/30) to give the title compound (0.120 g).

### Reference Example H-1

### Methyl 2-(3-(trifluoromethyl)phenyl)acetate

A mixture of 2-(3-(trifluoromethyl)phenyl)acetic acid (2.02 g), hydrogen chloride (4 mol/L in 1,4-dioxane) (9.89 mL), and methanol (10 mL) was stirred at 60°C for 1 hour. After the reaction mixture was allowed to cool to room temperature, the mixture was concentrated under reduced pressure. A saturated aqueous sodium hydrogen carbonate solution was added to the residue, and the mixture was extracted with DCM. The extract was dried over anhydrous sodium sulfate and concentrated under reduced pressure to give the title compound (1.89 g).

### Reference Example H-2

### Methyl 2-(bromomethyl)-4-(trifluoromethyl)benzoate

Under an argon atmosphere, to a mixture of methyl 2-methyl-4-(trifluoromethyl)benzoate (0.587 g) and carbon tetrachloride (11 mL), NBS (0.575 g) and AIBN (0.013 g) were added at room temperature, and the mixture was stirred at 70°C for 16 hours and at 100°C for 1.5 hours. After the reaction mixture was allowed to cool to room temperature, the mixture was concentrated under reduced pressure. The residue was suspended in n-hexane, and an insoluble material was removed by filtration. The filtrate was concentrated under reduced pressure. The residue was purified by silica gel column chromatography (eluent: n-hexane/ethyl acetate = 100/0 to 97/3) to give the title compound (0.674 g).

### Reference Example H-3

### (6-(trifluoromethyl)pyridin-2-yl)methyl methanesulfonate

To a mixture of (6-(trifluoromethyl)pyridin-2-yl)methanol (0.050 g), TEA (0.043 g), and DCM (2.8 mL), methanesulfonyl chloride (0.039 g) was added under ice cooling, and the mixture was stirred under ice cooling for 30 minutes. At room temperature, the reaction mixture was poured into water, and the mixture was extracted with DCM. The extract was dried over anhydrous sodium sulfate and concentrated under reduced pressure to give the title compound (0.065 g).

### Reference Example I-1

### (S)-6-(hydroxymethyl)piperidin-2-one

To a mixture of (S)-6-oxopiperidine-2-carboxylic acid (1.00 g) and methanol (4.0 mL), thionyl chloride (2.49 g) was added under ice cooling, and the mixture was stirred at 60°C for 8 hours. After the reaction mixture was allowed to cool to room temperature, the mixture was concentrated under reduced pressure. Water was added to the residue, and the mixture was extracted with ethyl acetate. The extract was washed with a saturated aqueous sodium hydrogen carbonate solution, dried over anhydrous sodium sulfate, and concentrated under reduced pressure.

To a mixture of the residue and ethanol (20 mL), sodium borohydride (1.06 g) was added under ice cooling, and the mixture was stirred at room temperature for 65 hours. A small amount of acetic acid was added to the reaction mixture, and the mixture was concentrated under reduced pressure. The residue was suspended in DCM, and an insoluble material was removed by filtration. The filtrate was washed with saturated brine, dried over anhydrous sodium sulfate, and concentrated under reduced pressure to give the title compound (0.783 g).

### Reference Example I-2

### (S)-6-(((tert-butyldiphenylsilyl)oxy)methyl)piperidin-2-one

A mixture of Reference Example I-1 (0.783 g), imidazole (0.825 g), TBDPSCl (1.83 g), DMAP (0.074 g), and DCM (20 mL) was stirred at room temperature for 2 hours. The reaction mixture was poured into water, and the mixture was extracted with DCM. The extract was dried over anhydrous sodium sulfate and concentrated under reduced pressure. The residue was purified by silica gel column chromatography (eluent: n-hexane/ethyl acetate = 100/0 to 0/100) to give the title compound (1.21 g).

### Reference Example I-3

### 1-(tert-butyl) 2-benzyl (2S,5S)-5-(2-ethoxy-2-oxoethyl)pyrrolidine-1,2-dicarboxylate

To a mixture of (S)-5-oxopyrrolidine-1,2-dicarboxylic acid 1-(tert-butyl) 2-benzyl ester (1.00 g) and THF (20 mL), lithium triethylborohydride (1 mol/L in THF) (4.70 mL) was added at -78°C, and the mixture was stirred at the same temperature for 30 minutes. A saturated aqueous sodium hydrogen carbonate solution and water were added to the reaction mixture, and the mixture was extracted with ethyl acetate. The extract was dried over anhydrous sodium sulfate and concentrated under reduced pressure to give (2S)-5-hydroxypyrrolidine-1,2-dicarboxylic acid 1-(tert-butyl) 2-benzyl ester (1.20 g).

To a mixture of diethyl phosphonoacetate (0.439 g) and DMF (3.0 mL), sodium hydride (approximately 60%) (0.085 g) was added under ice cooling, and the mixture was stirred at room temperature for 30 minutes. To the reaction mixture, a mixture of the above (2S)-5-hydroxypyrrolidine-1,2-dicarboxylic acid 1-(tert-butyl) 2-benzyl ester (0.500 g) and DMF (2.0 mL) was added, and the mixture was stirred at room temperature for 30 minutes. Water was added to the reaction mixture, and the mixture was extracted with ethyl acetate. The extract was washed with water and saturated brine, dried over anhydrous sodium sulfate, and concentrated under reduced pressure. The residue was purified by silica gel column chromatography (eluent: n-hexane/ethyl acetate = 95/5 to 70/30) to give the title compound (0.343 g).

### Reference Example I-4

### tert-butyl (2S,5S)-2-(2-ethoxy-2-oxoethyl)-5-formylpyrrolidine-1-carboxylate

A mixture of Reference Example I-3 (0.343 g), 10% Pd/C (0.051 g), and ethanol (3.0 mL) was stirred at room temperature for 2 hours under a hydrogen atmosphere. The reaction mixture was filtered through Celite, and the filtrate was concentrated under reduced pressure.

To a mixture of the residue and THF (3.0 mL), BH₃-THF (0.9 mol/L in THF) (3.89 mL) was added under ice cooling, and the mixture was stirred under ice cooling for 3 hours. Water and saturated brine were added to the reaction mixture, and the mixture was extracted with ethyl acetate. The extract was dried over anhydrous sodium sulfate and concentrated under reduced pressure.

To a mixture of the residue and DCM (10 mL), DMP (1.12 g) was added at room temperature, and the mixture was stirred at the same temperature for 2 hours. A 1 mol/L aqueous sodium thiosulfate solution was added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate = 95/5 to 70/30) to give the title compound (0.242 g).

### Reference Example J-1

### (S)-1-(5-bromo-2-fluorobenzyl)-6-(trifluoromethyl)piperidin-2-one

Reference Example J-1 was synthesized by the same method as in Reference Example C-9, except that 4-bromo-2-(bromomethyl)-1-fluorobenzene was used instead of 2-(bromomethyl)-1-fluoro-4-(trifluoromethyl)benzene.

### Reference Example J-2

### (S)-1-(5-allyl-2-fluorobenzyl)-6-(trifluoromethyl)piperidin-2-one

Under an argon atmosphere, to a mixture of Reference Example J-1 (0.169 g), 2-allyl-4,4,5,5-tetramethyl-1,3,2-dioxaborolane (0.241 g), potassium carbonate (0.330 g), 1,4-dioxane (2.0 mL), and water (0.4 mL), PdCl₂(dppf)-DCM (0.117 g) was added at room temperature, and the mixture was stirred at 80°C for 15 hours under microwave irradiation. Water was added to the reaction mixture, and the mixture was extracted with DCM. The extract was concentrated under reduced pressure. The residue was purified by silica gel column chromatography (eluent: n-hexane/ethyl acetate = 90/10 to 40/60) to give the title compound (0.128 g).

### Reference Example J-3

### (S)-1-(5-(cyclopropylethynyl)-2-fluorobenzyl)-6-(trifluoromethyl)piperidin-2-one

Under an argon atmosphere, to a mixture of Reference Example J-1 (0.169 g), ethynylcyclopropane (0.063 g), cesium carbonate (0.466 g), and MeCN (2.4 mL), Xphos (0.034 g) and bis(acetonitrile)palladium(II) dichloride (0.006 g) were added at room temperature, and the mixture was stirred at 90°C for 15 hours under microwave irradiation. Water was added to the reaction mixture, and the mixture was extracted with DCM. The extract was concentrated under reduced pressure. The residue was purified by silica gel column chromatography (eluent: n-hexane/ethyl acetate = 90/10 to 40/60) to give the title compound (0.138 g).

### Reference Example J-4

### (2S,5S)-tert-butyl 2-(2-ethoxy-2-oxoethyl)-5-(hydroxy(2-(trifluoromethyl)phenyl)methyl)pyrrolidine-1-carboxylate

A mixture of 2-bromobenzotrifluoride (1.13 g), magnesium (0.134 g), iodine (0.063 g), and THF (9.3 mL) was stirred at 60°C for 20 minutes under microwave irradiation to give (2-(trifluoromethyl)phenyl)magnesium bromide (0.5 mol/L in THF) (10 mL).

Under an argon atmosphere, to a mixture of Reference Example I-4 (0.242 g) and THF (5.0 mL), the above (2-(trifluoromethyl)phenyl)magnesium bromide (0.5 mol/L in THF) (8.5 mL) was added at -78°C, and the mixture was stirred at room temperature for 15 minutes. A saturated aqueous ammonium chloride solution was added to the reaction mixture, and the mixture was extracted with ethyl acetate. The extract was dried over anhydrous sodium sulfate and concentrated under reduced pressure. The residue was purified by silica gel column chromatography (eluent: n-hexane/ethyl acetate = 90/10 to 60/40) to give the title compound (0.119 g).

¹H-NMR(CDCl₃)δ ppm: 1.24 (3H, t, J = 7.2 Hz), 1.52 (9H, s), 1.73-2.52 (4H, m), 2.66-3.31 (1H, m), 3.77-5.14 (6H, m), 5.20-5.88 (1H, m), 7.33-8.09 (4H, m)

### Reference Example J-5

### (2S,5S)-tert-butyl 2-(2-ethoxy-2-oxoethyl)-5-(2-(trifluoromethyl)benzyl)pyrrolidine-1-carboxylate

A mixture of Reference Example J-4 (0.119 g) and hydrogen chloride (4 mol/L in 1,4-dioxane) (3.00 mL) was stirred at room temperature for 1 hour. The reaction mixture was concentrated under reduced pressure.

To a mixture of the residue, TEA (0.140 g), and DCM (3.0 mL), triphosgene (0.033 g) was added at room temperature, and the mixture was stirred at the same temperature for 30 minutes. A saturated aqueous sodium hydrogen carbonate solution was added to the reaction mixture, and the mixture was extracted with DCM. The extract was concentrated under reduced pressure. The residue was purified by silica gel column chromatography (eluent: n-hexane/ethyl acetate = 80/20 to 50/50) to give ethyl 2-((1S,5S,7aS)-3-oxo-1-(2-(trifluoromethyl)phenyl)tetrahydro-1H,3H-pyrrolo[1,2-c]oxazol-5-yl)acetate (0.068 g).

A mixture of the obtained compound (0.068 g), 10% Pd/C (0.036 g), and methanol (3.0 mL) was stirred at 50°C for 1 hour under a hydrogen atmosphere. After the reaction mixture was allowed to cool to room temperature, Boc₂O (0.120 g) was added, and the mixture was stirred at the same temperature for 30 minutes. The reaction mixture was filtered through Celite, and the filtrate was concentrated under reduced pressure. The residue was purified by silica gel column chromatography (eluent: n-hexane/ethyl acetate = 95/5 to 70/30) to give the title compound (0.059 g).

### Reference Example J-6

### (2S,5S)-tert-butyl 2-((Z)-3-(((benzyloxy)carbonyl)amino)-4-methoxy-4-oxobut-2-en-1-yl)-5-(2-(trifluoromethyl)benzyl)pyrrolidine-1-carboxylate

To a mixture of Reference Example J-5 (0.059 g) and DCM (3.0 mL), DIBAL-H (1.02 mol/L in n-hexane) (0.418 mL) was added at -78°C, and the mixture was stirred at the same temperature for 30 minutes. Methanol and a 30% aqueous Rochelle salt solution were added to the reaction mixture, and the mixture was stirred at room temperature for 1 hour, and the mixture was extracted with DCM. The extract was dried over anhydrous sodium sulfate and concentrated under reduced pressure to give (2S,5S)-tert-butyl 2-(2-oxoethyl)-5-(2-(trifluoromethyl)benzyl)pyrrolidine-1-carboxylate (0.053 g).

To a mixture of N-benzyloxycarbonyl-2-phosphonoglycine trimethyl ester (0.071 g) and DCM (1.0 mL), DBU (0.032 g) was added at room temperature, and the mixture was stirred at the same temperature for 10 minutes. A mixture of the above (2S,5S)-tert-butyl 2-(2-oxoethyl)-5-(2-(trifluoromethyl)benzyl)pyrrolidine-1-carboxylate (0.053 g) and DCM (1.0 mL) was added, and the mixture was stirred at room temperature for 30 minutes. Water was added to the reaction mixture, and the mixture was extracted with DCM. The extract was concentrated under reduced pressure. The residue was purified by silica gel column chromatography (eluent: n-hexane/ethyl acetate = 80/20 to 50/50) to give the title compound (0.072 g).

### Reference Example J-7

### (S)-6-(((tert-butyldiphenylsilyl)oxy)methyl)-1-(2-fluoro-5-(trifluoromethyl)benzyl)piperidin-2-one

To a mixture of Reference Example I-2 (1.21 g) and DMF (24 mL), sodium hydride (approximately 60%) (0.145 g) was added under ice cooling, and the mixture was stirred at room temperature for 30 minutes. 2-(bromomethyl)-1-fluoro-4-(trifluoromethyl)benzene (1.02 g) was added to the reaction mixture, and the mixture was stirred at room temperature for 4 hours. The reaction mixture was poured into a saturated aqueous ammonium chloride solution, and the mixture was extracted with ethyl acetate. The extract was washed with saturated brine, dried over anhydrous sodium sulfate, and concentrated under reduced pressure. The residue was purified by silica gel column chromatography (eluent: n-hexane/ethyl acetate = 100/0 to 80/20) to give the title compound (0.684 g).

### Reference Example J-8

### (S)-2-fluoro-5-(trifluoromethyl)benzyl (S)-1-(2-fluoro-5-(trifluoromethyl)benzyl)-6-oxopiperidine-2-carboxylate

To a mixture of (S)-6-oxopiperidine-2-carboxylic acid (0.500 g) and DMF (3.0 mL), sodium hydride (approximately 60%) (0.385 g) was added under ice cooling, and the mixture was stirred under ice cooling for 20 minutes. 2-(Bromomethyl)-1-fluoro-4-(trifluoromethyl)benzene (1.84 g) was added to the reaction mixture under ice cooling, and the mixture was stirred at room temperature for 2 hours and at 60°C for 4 hours. After the reaction mixture was allowed to cool to room temperature, a saturated aqueous ammonium chloride solution was added, and the mixture was extracted with ethyl acetate. The extract was washed with saturated brine, dried over anhydrous sodium sulfate, and concentrated under reduced pressure. The residue was purified by silica gel column chromatography (eluent: n-hexane/ethyl acetate = 100/0 to 60/40) to give the title compound (0.194 g).

### Reference Example J-9

### (S)-1-(2-fluoro-5-(trifluoromethyl)benzyl)-6-(hydroxymethyl)piperidin-2-one

To a mixture of Reference Example J-8 (0.194 g) and ethanol (1.0 mL), sodium borohydride (0.074 g) was added under ice cooling, and the mixture was stirred at room temperature for 3 hours. Water and a saturated aqueous ammonium chloride solution were added to the reaction mixture, and the mixture was extracted with ethyl acetate. The extract was dried over anhydrous sodium sulfate and concentrated under reduced pressure. The residue was purified by silica gel column chromatography (eluent: n-hexane/ethyl acetate = 95/5 to 30/70) to give the title compound (0.097 g).

### Reference Example J-10

### (S)-1-(2-fluoro-5-(trifluoromethyl)benzyl)-6-(((trimethylsilyl)oxy)methyl)piperidin-2-one

To a mixture of Reference Example J-9 (0.097 g), TEA (0.096 g), and THF (1.0 mL), TMSCl (0.069 g) was added under ice cooling, and the mixture was stirred at room temperature for 1.5 hours. A saturated aqueous sodium hydrogen carbonate solution was added to the reaction mixture, and the mixture was extracted with DCM. The extract was concentrated under reduced pressure to give the title compound (0.097 g).

### Reference Example J-11

### tert-butyl ((3R,6S)-6-(((tert-butyldiphenylsilyl)oxy)methyl)-1-(2-fluoro-5-(trifluoromethyl)benzyl)-2-oxopiperidin-3-yl)carbamate

To a mixture of Reference Example J-7 (1.32 g) and THF (20 mL), LDA (1.01 mol/L in THF/n-hexane) (4.80 mL) was added at -78°C, and the mixture was stirred at the same temperature for 10 minutes. DPPA (1.33 g) was added to the reaction mixture at -78°C, and the mixture was stirred at the same temperature for 45 minutes. A mixture of Boc₂O (0.741 g) and THF (10 mL) was added to the reaction mixture at -78°C, and the mixture was stirred at the same temperature for 10 minutes and under ice cooling for 1 hour. Water was added to the reaction mixture, and the mixture was stirred at 50°C for 1 hour. After the reaction mixture was allowed to cool to room temperature, a saturated aqueous ammonium chloride solution was added, and the mixture was extracted with ethyl acetate. The extract was dried over anhydrous sodium sulfate and concentrated under reduced pressure. The residue was purified by silica gel column chromatography (eluent: n-hexane/ethyl acetate = 95/5 to 70/30) to give the title compound (0.897 g).

### Reference Example J-12

### tert-butyl ((3R,6S)-6-(azidomethyl)-1-(2-fluoro-5-(trifluoromethyl)benzyl)-2-oxopiperidin-3-yl)carbamate

To a mixture of Reference Example J-11 (0.897 g) and THF (15 mL), TBAF (1 mol/L in THF) (4.85 mL) was added, and the mixture was stirred at room temperature for 20 minutes. A saturated aqueous ammonium chloride solution and water were added to the reaction mixture, and the mixture was extracted with ethyl acetate. The extract was dried over anhydrous sodium sulfate and concentrated under reduced pressure. The residue was sequentially purified by silica gel column chromatography (eluent: n-hexane/ethyl acetate = 95/5 to 60/40) and silica gel column chromatography (eluent: n-hexane/ethyl acetate = 70/30 to 30/70) to give tert-butyl ((3R,6S)-1-(2-fluoro-5-(trifluoromethyl)benzyl)-6-(hydroxymethyl)-2-oxopiperidin-3-yl)carbamate (0.308 g).

Under an argon atmosphere, to a mixture of the obtained compound (0.020 g), TEA (0.019 g), and DCM (1.5 mL), methanesulfonyl chloride (0.012 g) was added under ice cooling, and the mixture was stirred at room temperature for 15 minutes. Water was added to the reaction mixture, and the mixture was extracted with DCM. The extract was dried over anhydrous magnesium sulfate and concentrated under reduced pressure. A mixture of the residue, sodium azide (0.012 g), and DMSO (1.0 mL) was stirred at 80°C for 2 hours. After allowing the reaction mixture to cool to room temperature, water was added, and the mixture was extracted with ethyl acetate. The extract was dried over anhydrous magnesium sulfate and concentrated under reduced pressure. The residue was purified by silica gel column chromatography (eluent: n-hexane/ethyl acetate = 80/20 to 0/100) to give the title compound (0.020 g).

### Reference Example J-13

### 1-(2-fluoro-5-(trifluoromethyl)benzyl)piperidine-2,6-dione

A mixture of piperidine-2,6-dione (3.17 g), 2-(bromomethyl)-1-fluoro-4-(trifluoromethyl)benzene (7.92 g), potassium carbonate (4.65 g), and acetone (40 mL) was refluxed for 14 hours. After the reaction mixture was allowed to cool to room temperature, the mixture was filtered, and the filtrate was concentrated under reduced pressure. The residue was purified by silica gel column chromatography (eluent: n-hexane/ethyl acetate = 100/0 to 75/25) to give the title compound (7.73 g).

### Reference Example J-14

### Methyl 2-(1-(2-fluoro-5-(trifluoromethyl)benzyl)-6-oxopiperidin-2-yl)acetate

Under an argon atmosphere, to a mixture of Reference Example J-13 (7.73 g) and DCM (100 mL), DIBAL-H (1.03 mol/L in n-hexane) (34.5 mL) was added at -78°C, and the mixture was stirred at the same temperature for 1.5 hours. Methanol and a saturated aqueous Rochelle salt solution were added to the reaction mixture under ice cooling, and the mixture was stirred at room temperature for 30 minutes, and the mixture was extracted with DCM. The extract was dried over anhydrous sodium sulfate and concentrated under reduced pressure to give 1-(2-fluoro-5-(trifluoromethyl)benzyl)-6-hydroxypiperidin-2-one (7.39 g).

Under an argon atmosphere, to a mixture of triethyl phosphonoacetate (10.2 g) and THF (80 mL), sodium hydride (approximately 60%) (2.03 g) was added under ice cooling, and the mixture was stirred under ice cooling for 50 minutes. A mixture of the above 1-(2-fluoro-5-(trifluoromethyl)benzyl)-6-hydroxypiperidin-2-one (7.39 g) and THF (20 mL) was added under ice cooling, and the mixture was stirred at room temperature for 1 hour. A saturated aqueous ammonium chloride solution and water were added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate = 70/30 to 20/80) to give the title compound (2.18 g).

### Reference Example J-15

### 6-(2-((tert-butyldiphenylsilyl)oxy)ethyl)-1-(2-fluoro-5-(trifluoromethyl)benzyl)piperidin-2-one

Under an argon atmosphere, to a mixture of Reference Example J-14 (2.18 g), methanol (10 mL), and THF (10 mL), sodium borohydride (0.685 g) was added under ice cooling, and the mixture was stirred at room temperature for 1 hour. Sodium borohydride (0.913 g) was further added under ice cooling, and the reaction mixture was stirred at room temperature for 30 minutes. A saturated aqueous ammonium chloride solution was added to the reaction mixture, and the mixture was extracted with DCM. The extract was concentrated under reduced pressure.

Under an argon atmosphere, to a mixture of the residue, imidazole (0.821 g), DMAP (0.074 g), and DCM (20 mL), TBDPSCl (1.99 g) was added at room temperature, and the mixture was stirred at the same temperature for 15 minutes. Water was added to the reaction mixture, and the mixture was extracted with DCM. The extract was concentrated under reduced pressure. The residue was purified by silica gel column chromatography (eluent: n-hexane/ethyl acetate = 90/10 to 60/40) to give the title compound (2.58 g).

### Reference Example J-16

### tert-butyl ((3R*,6S*)-6-(2-((tert-butyldiphenylsilyl)oxy)ethyl)-1-(2-fluoro-5-(trifluoromethyl)benzyl)-2-oxopiperidin-3-yl)carbamate

Under an argon atmosphere, to a mixture of Reference Example J-15 (1.50 g) and THF (20 mL), LDA (1.01 mol/L in THF/n-hexane) (2.93 mL) was added at -78°C, and the mixture was stirred at the same temperature for 25 minutes. DPPA (0.814 g) was added to the reaction mixture at -78°C, and the mixture was stirred at the same temperature for 30 minutes. A mixture of Boc₂O (0.822 g) and THF (5.0 mL) was added to the reaction mixture at -78°C, and the mixture was stirred at the same temperature for 40 minutes. Water was added to the reaction mixture under ice cooling, and the mixture was stirred at 50°C for 30 minutes. After the reaction mixture was allowed to cool to room temperature, a saturated aqueous ammonium chloride solution was added, and the mixture was extracted with ethyl acetate. The extract was dried over anhydrous sodium sulfate and concentrated under reduced pressure. The residue was purified by silica gel column chromatography (eluent: n-hexane/ethyl acetate = 100/0 to 60/40) to give the title compound (0.539 g).

### Reference Example K-1

### Methyl 5-bromo-6-((tert-butyldimethylsilyl)oxy)picolinate

Under an argon atmosphere, to a mixture of methyl 5-bromo-6-oxo-1,6-dihydropyridine-2-carboxylate (3.04 g), TEA (5.30 g), and DCM (60 mL), tertbutyldimethylsilyl trifluoromethanesulfonate (10.4 g) was added under ice cooling, and the mixture was stirred at room temperature for 30 minutes. Water was added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate = 100/0 to 95/5) to give the title compound (1.49 g).

### Reference Example L-1

### Methyl 3-(5-bromo-6-((tert-butyldimethylsilyl)oxy)pyridin-2-yl)-3-oxo-2-(3-(trifluoromethyl)phenyl)propanoate

Under an argon atmosphere, to a mixture of Reference Example K-1 (1.49 g) and THF (20 mL), lithium bis(trimethylsilyl)amide (1 mol/L in THF) (8.61 mL) was added, followed by a mixture of Reference Example H-1 (1.31 g) and THF (10 mL) at -78°C, and the mixture was stirred under ice cooling for 2 hours. A saturated aqueous ammonium chloride solution and water were added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate = 95/5 to 90/10) to give the title compound (0.469 g).

### Reference Example L-2

### 3-bromo-6-(1,3-dihydroxy-2-(3-(trifluoromethyl)phenyl)propyl)pyridin-2(1H)-one

To a mixture of Reference Example L-1 (0.847 g) and methanol (10 mL), sodium borohydride (0.241 g) was added under ice cooling, and the mixture was stirred at room temperature for 20 minutes. The reaction mixture was concentrated under reduced pressure. A saturated aqueous ammonium chloride solution was added to the residue, and the mixture was extracted with DCM. The extract was concentrated under reduced pressure.

To a mixture of the residue and methanol (10 mL), hydrogen chloride (4 mol/L in 1,4-dioxane) (1.99 mL) was added at room temperature, and the mixture was stirred at the same temperature for 15 minutes. The reaction mixture was concentrated under reduced pressure. The residue was purified by silica gel column chromatography (eluent: n-hexane/ethyl acetate = 50/50 to 0/100) to give the title compound (0.376 g).

### Reference Example L-3

### 6-bromo-5-oxo-2-(3-(trifluoromethyl)phenyl)-1,2,3,5-tetrahydroindolizin-1-yl acetate

Under an argon atmosphere, to a mixture of Reference Example L-2 (0.376 g), triphenylphosphine (0.503 g), and THF (6.0 mL), DEAD (2.2 mol/L in toluene) (0.654 mL) was added under ice cooling, and the mixture was stirred at room temperature for 15 minutes. The reaction mixture was concentrated under reduced pressure. The residue was purified by silica gel column chromatography (eluent: n-hexane/ethyl acetate = 70/30 to 0/100) to give 6-bromo-1-hydroxy-2-(3-(trifluoromethyl)phenyl)-2,3-dihydroindolizin-5(1H)-one (0.359 g).

A mixture of the obtained compound (0.359 g), acetic anhydride (0.147 g), TEA (0.194 g), DMAP (0.012 g), and DCM (5.0 mL) was stirred at room temperature for 20 minutes. Water was added to the reaction mixture, and the mixture was extracted with DCM. The extract was dried over anhydrous sodium sulfate and concentrated under reduced pressure. The residue was sequentially purified by silica gel column chromatography (eluent: n-hexane/ethyl acetate = 80/20 to 50/50), silica gel column chromatography (eluent: n-hexane/ethyl acetate = 90/10 to 60/40), and ODS column chromatography (eluent: water/MeCN = 70/30 to 10/90) to give the title compound (0.069 g).

### Reference Example M-1

### (R)-tert-butyl (7-(difluoromethyl)-2-oxo-1,2,3,4-tetrahydroquinolin-3-yl)carbamate

Under an argon atmosphere, to a mixture of zinc powder (0.324 g) and DMF (4.0 mL), TMSCl (0.049 g) was added at room temperature, and the mixture was stirred at the same temperature for 20 minutes. A mixture of methyl (S)-2-((tert-butoxycarbonyl)amino)-3-iodopropanoate (1.48 g) and THF (2.0 mL) was added to the reaction mixture under water cooling, and the mixture was stirred under water cooling for 30 minutes. Palladium(II) acetate (0.025 g) and Xphos (0.107 g), followed by a mixture of Reference Example F-1 (0.500 g) and THF (4.0 mL), were added to the reaction mixture under water cooling, and the mixture was stirred at 40°C for 2 hours. Acetic acid (0.811 g) was added to the reaction mixture, and the mixture was stirred at 60°C for 1 hour. After the reaction mixture was allowed to cool to room temperature, water was added, and the mixture was extracted with n-hexane/ethyl acetate (1/1). The extract was dried over anhydrous sodium sulfate and concentrated under reduced pressure. The residue was purified by silica gel column chromatography (eluent: n-hexane/ethyl acetate = 100/0 to 60/40) to give the title compound (0.452 g).

### Reference Example M-2

### tert-butyl (3-bromothiophen-2-yl)carbamate

To a mixture of 3-bromothiophene-2-carboxylic acid (0.300 g) and tert-butanol (3.0 mL), TEA (0.293 g) and DPPA (0.598 g) were added, and the mixture was stirred at 60°C for 4 hours. After the reaction mixture was allowed to cool to room temperature, a 2 mol/L aqueous sodium hydroxide solution (2.5 mL) was added, and the mixture was extracted with ethyl acetate. The extract was washed with saturated brine, dried over anhydrous sodium sulfate, and concentrated under reduced pressure. The residue was purified by silica gel column chromatography (eluent: n-hexane/ethyl acetate = 98/2 to 80/20) to give the title compound (0.275 g).

### Reference Example M-3

### (R)-5-amino-4,7-dihydrothieno[2,3-b]pyridin-6(5H)-one

Under an argon atmosphere, to a mixture of methyl (S)-2-((tert-butoxycarbonyl)amino)-3-iodopropanoate (0.089 g) and DMF (0.65 mL), zinc powder (0.026 g) was added at room temperature, and the mixture was stirred at the same temperature for 1 hour. A mixture of Reference Example M-2 (0.050 g) and DMF (1.3 mL), palladium(II) acetate (0.002 g), and Xphos (0.009 g) were added to the reaction mixture at room temperature, and the mixture was stirred at 40°C for 30 minutes and at room temperature for 15 hours. Water, a saturated aqueous ammonium chloride solution, and n-hexane/ethyl acetate (1/1) were added to the reaction mixture, and the mixture was stirred at room temperature for 10 minutes. The mixture was filtered through Celite, and the organic layer of the filtrate was separated. The organic layer was washed with water and saturated brine, dried over anhydrous magnesium sulfate, and concentrated under reduced pressure.

To a mixture of the residue, ethanol (1.0 mL), and toluene (0.5 mL), methanesulfonic acid (0.052 g) was added at room temperature, and the mixture was stirred at 80°C for 45 minutes. Methanesulfonic acid (0.294 g) was added to the reaction mixture at room temperature, and the mixture was stirred at 80°C for 4.5 hours. A 5 mol/L aqueous sodium hydroxide solution (0.720 mL) was added to the reaction mixture under ice cooling, and the mixture was extracted with DCM. The extract was concentrated under reduced pressure. The residue was purified by APS column chromatography (eluent: n-hexane/ethyl acetate/methanol = 100/0/0 to 0/100/0 to 0/80/20) to give the title compound (0.008 g).

### Reference Example M-4

### (R)-tert-butyl (6-oxo-4,5,6,7-tetrahydrothieno[2,3-b]pyridin-5-yl)carbamate

To a mixture of Reference Example M-3 (0.008 g) and DCM (0.5 mL), Boc₂O (0.016 g) was added, and the mixture was stirred at room temperature for 1.5 hours. A saturated aqueous ammonium chloride solution was added to the reaction mixture, and the mixture was extracted with DCM. The extract was concentrated under reduced pressure. The residue was purified by Method A (eluent: n-hexane/ethyl acetate = 100/0 to 50/50) to give the title compound (0.003 g).

### Reference Example M-5

### tert-butyl (2-bromothiophen-3-yl)carbamate

Using 2-bromothiophene-3-carboxylic acid instead of 3-bromothiophene-2-carboxylic acid, Reference Example M-5 was synthesized by the same method as in Reference Example M-2.

### Reference Example M-6

### (R)-6-amino-6,7-dihydrothieno[3,2-b]pyridin-5(4H)-one

Using Reference Example M-5 instead of Reference Example M-2, Reference Example M-6 was synthesized by the same method as in Reference Example M-3.

### Reference Example M-7

### (R)-tert-butyl (5-oxo-4,5,6,7-tetrahydrothieno[3,2-b]pyridin-6-yl)carbamate

Using Reference Example M-6 instead of Reference Example M-3, Reference Example M-7 was synthesized by the same method as in Reference Example M-4.

### Reference Example M-8

### tert-butyl (2-methylthiazol-5-yl)carbamate

Using 2-methylthiazole-5-carboxylic acid instead of 3-bromothiophene-2-carboxylic acid, Reference Example M-8 was synthesized by the same method as in Reference Example M-2.

### Reference Example M-9

### tert-Butyl (4-bromo-2-methylthiazol-5-yl)carbamate

A mixture of Reference Example M-8 (0.420 g), NBS (0.349 g), and DMF (20 mL) was stirred at room temperature for 1 hour. The reaction mixture was poured into a saturated aqueous sodium hydrogen carbonate solution, and the mixture was extracted with ethyl acetate. The extract was dried over anhydrous sodium sulfate and concentrated under reduced pressure. The residue was purified by silica gel column chromatography (eluent: n-hexane/ethyl acetate = 100/0 to 60/40) to give the title compound (0.513 g).

### Reference Example M-10

### (R)-methyl 2-((tert-butoxycarbonyl)amino)-3-(5-((tert-butoxycarbonyl)amino)-2-methylthiazol-4-yl)propanoate

Under an argon atmosphere, to a mixture of zinc powder (0.252 g) and DMF (4.0 mL), TMSCl (0.038 g) was added at room temperature, and the mixture was stirred at the same temperature for 20 minutes. A mixture of methyl (S)-2-((tert-butoxycarbonyl)amino)-3-iodopropanoate (1.15 g) and THF (2.0 mL) was added to the reaction mixture under water cooling, and the mixture was stirred under water cooling for 30 minutes. Palladium(II) acetate (0.020 g) and Xphos (0.083 g), followed by a mixture of Reference Example M-9 (0.513 g) and THF (4.0 mL), were added to the reaction mixture under water cooling, and the mixture was stirred at 40°C for 12 hours. After allowing the reaction mixture to cool to room temperature, water was added, and the mixture was extracted with n-hexane/ethyl acetate (1/1). The extract was dried over anhydrous sodium sulfate and concentrated under reduced pressure. The residue was purified by column chromatography on silica gel (eluent: n-hexane/ethyl acetate = 100/0 to 60/40) to give the title compound (0.651 g).

### Reference Example M-11

### tert-butyl (R)-(2-methyl-5-oxo-4,5,6,7-tetrahydrothiazolo[5,4-b]pyridin-6-yl)carbamate

A mixture of Reference Example M-10 (0.156 g), a 4 mol/L aqueous lithium hydroxide solution (0.282 mL), and THF (3.6 mL) was stirred at room temperature for 2 hours. The reaction mixture was poured into 2 mol/L hydrochloric acid, and the mixture was extracted with DCM. The extract was dried over anhydrous sodium sulfate and concentrated under reduced pressure.

A mixture of the residue, hydrogen chloride (4 mol/L in 1,4-dioxane) (0.470 mL), and 1,4-dioxane (3.0 mL) was stirred at 50°C for 3 hours. After the reaction mixture was allowed to cool to room temperature, the mixture was concentrated under reduced pressure to give (R)-2-amino-3-(5-amino-2-methylthiazol-4-yl)propanoic acid dihydrochloride (0.103 g).

To a mixture of the obtained compound (0.030 g), TEA (0.055 g), 4-(dimethylamino)pyridine N-oxide hydrate (0.302 g), and MeCN (10 mL), Boc₂O (0.060 g) was added under ice cooling, and the mixture was stirred at room temperature for 13 hours. The reaction mixture was concentrated under reduced pressure. The residue was purified by silica gel column chromatography (eluent: n-hexane/ethyl acetate = 100/0 to 25/75) to give the title compound (0.017 g).

### Reference Example M-12

### (Z)-4-((2-hydroxyphenyl)amino)-4-oxobut-2-enoic acid

A mixture of furan-2,5-dione (2.00 g), 2-aminophenol (2.23 g), and toluene (22 mL) was stirred at room temperature for 12 hours. An insoluble material was collected by filtration, and the obtained solid was washed with toluene and then dried under reduced pressure to give the title compound (6.07 g).

### Reference Example M-13

### Ethyl 2-(3-oxo-3,4-dihydro-2H-benzo[b][1,4]oxazin-2-yl)acetate

A mixture of Reference Example M-12 (1.00 g), TEA (0.910 g), and ethanol (30 mL) was refluxed for 3 hours. After the reaction mixture was allowed to cool to room temperature, the mixture was concentrated under reduced pressure. The residue was purified by silica gel column chromatography (eluent: n-hexane/ethyl acetate = 100/0 to 50/50) to give the title compound (0.388 g).

### Reference Example M-14

### (Z)-4-((2-hydroxy-4-nitrophenyl)amino)-4-oxobut-2-enoic acid

Reference Example M-14 was synthesized by the same method as in Reference Example M-12, except that 2-amino-5-nitrophenol was used instead of 2-aminophenol.

### Reference Example M-15

### Ethyl 2-(7-nitro-3-oxo-3,4-dihydro-2H-benzo[b][1,4]oxazin-2-yl)acetate

Reference Example M-15 was synthesized by the same method as in Reference Example M-13, except that Reference Example M-14 was used instead of Reference Example M-12.

### Reference Example N-1

### (3-bromo-2-((2-fluoro-5-(trifluoromethyl)benzyl)amino)phenyl)methanol

To a mixture of 2-amino-3-bromobenzoic acid (2.00 g), 2-fluoro-5-(trifluoromethyl)benzaldehyde (2.13 g), and methanol (19 mL), decaborane (0.339 g) was added under water cooling, and the mixture was stirred at room temperature for 20 hours. Water and a saturated aqueous ammonium chloride solution were added to the reaction mixture, and the mixture was extracted with DCM. The extract was concentrated under reduced pressure. n-Hexane/DCM (1/1) was added to the residue, and the mixture was filtered. The filtrate was concentrated under reduced pressure. The residue was purified by silica gel column chromatography (eluent: n-hexane/ethyl acetate = 95/5 to 70/30) to give 3-bromo-2-((2-fluoro-5-(trifluoromethyl)benzyl)amino)benzoic acid (1.70 g).

To a mixture of the obtained compound (1.70 g) and THF (8.7 mL), BH₃-THF (0.9 mol/L in THF) (14.4 mL) was added at room temperature, and the mixture was stirred at the same temperature for 18 hours. A saturated aqueous sodium hydrogen carbonate solution was added to the reaction mixture, and the mixture was extracted with ethyl acetate. The extract was washed with water and saturated brine, dried over anhydrous magnesium sulfate, and concentrated under reduced pressure. The residue was purified by Method A (eluent: n-hexane/ethyl acetate = 98/2 to 85/15) to give the title compound (0.274 g).

### Reference Example N-2

### (R)-3-amino-1-(2-fluoro-5-(trifluoromethyl)benzyl)-8-(hydroxymethyl)-3,4-dihydroquinolin-2(1H)-one

Under an argon atmosphere, to a mixture of zinc powder (0.104 g) and DMF (1.3 mL), a mixture of methyl (S)-2-((tert-butoxycarbonyl)amino)-3-iodopropanoate (0.358 g) and DMF (0.65 mL) was added, and the mixture was stirred at room temperature for 1 hour. To the reaction mixture, a mixture of Reference Example N-1 (0.274 g) and DMF (1.3 mL), palladium(II) acetate (0.008 g), and Xphos (0.035 g) were added at room temperature, and the mixture was stirred at 40°C for 5 hours and at room temperature for 14 hours. Water, a saturated aqueous ammonium chloride solution, and n-hexane/ethyl acetate (1/1) were added to the reaction mixture, and the mixture was stirred at room temperature for 10 minutes. The mixture was filtered through Celite, and an organic layer of the filtrate was separated. The organic layer was washed with water and saturated brine, dried over anhydrous magnesium sulfate, and concentrated under reduced pressure.

To a mixture of the residue, ethanol (0.65 mL), and toluene (1.0 mL), methanesulfonic acid (0.209 g) was added at room temperature, and the mixture was stirred at 70°C for 1.5 hours. To the reaction mixture, a 5 mol/L aqueous sodium hydroxide solution (0.435 mL) was added under ice cooling, and the mixture was extracted with DCM. The extract was concentrated under reduced pressure. The residue was purified by APS column chromatography (eluent: n-hexane/ethyl acetate/methanol = 50/50/0 to 0/100/0 to 0/70/30) to give the title compound (0.040 g).

### Reference Example N-3

### Ethyl 2-(4-(2-fluoro-5-(trifluoromethyl)benzyl)-7-nitro-3-oxo-3,4-dihydro-2H-benzo[b][1,4]oxazin-2-yl)acetate

A mixture of Reference Example M-15 (0.357 g), potassium carbonate (0.264 g), 2-(bromomethyl)-1-fluoro-4-(trifluoromethyl)benzene (0.360 g), and DMF (13 mL) was stirred at 60°C for 1.5 hours under microwave irradiation. The reaction mixture was poured into water, and the mixture was extracted with ethyl acetate. The extract was washed with saturated brine, dried over anhydrous sodium sulfate, and concentrated under reduced pressure. The residue was purified by silica gel column chromatography (eluent: n-hexane/ethyl acetate = 100/0 to 60/40) to give the title compound (0.632 g).

### Reference Example N-4

### Ethyl 2-(7-amino-4-(2-fluoro-5-(trifluoromethyl)benzyl)-3-oxo-3,4-dihydro-2H-benzo[b][1,4]oxazin-2-yl)acetate

Under an argon atmosphere, to a mixture of Reference Example N-3 (0.316 g) and THF (5.6 mL), 10% Pd/C (0.077 g) was added, and the mixture was stirred at 50°C for 1.5 hours under a hydrogen atmosphere. After the reaction mixture was allowed to cool to room temperature, the mixture was filtered through Celite. The filtrate was concentrated under reduced pressure. The residue was purified by silica gel column chromatography (eluent: n-hexane/ethyl acetate = 100/0 to 40/60) to give the title compound (0.211 g).

### Reference Example N-5

### 1-(2-fluoro-5-(trifluoromethyl)benzyl)quinoxalin-2(1H)-one

To a mixture of quinoxalin-2(1H)-one (0.731 g), potassium carbonate (1.38 g), and DMF (5.0 mL), 2-(bromomethyl)-1-fluoro-4-(trifluoromethyl)benzene (1.67 g) was added, and the mixture was stirred at room temperature for 1.5 hours. Water and a saturated aqueous ammonium chloride solution were added to the reaction mixture. An insoluble material was collected by filtration and washed with water. The obtained solid was dissolved in DCM, and the mixture was dried over anhydrous magnesium sulfate and then concentrated under reduced pressure. The residue was purified by silica gel column chromatography (eluent: n-hexane/ethyl acetate = 100/0 to 70/30) to give the title compound (0.997 g).

¹H-NMR(CDCl₃)δ ppm: 5.56 (2H, s), 7.19-7.44 (4H, m), 7.48-7.63 (2H, m), 7.94 (1H, dd, J = 1.6, 8.0 Hz), 8.43 (1H, s)

### Reference Example N-6

### 3-amino-1-(2-fluoro-5-(trifluoromethyl)benzyl)quinoxalin-2(1H)-one

Under an argon atmosphere, a mixture of Reference Example N-5 (0.258 g), cerium(IV) ammonium nitrate (0.658 g), trimethylsilyl azide (0.277 g), water (0.029 g), and ethyl acetate (8.0 mL) was stirred at room temperature for 16 hours. A saturated aqueous sodium hydrogen carbonate solution was added to the reaction mixture, and the mixture was extracted with DCM. The extract was concentrated under reduced pressure. The residue was suspended in a mixed solvent of n-hexane/DCM/methanol, and an insoluble material was collected by filtration. The obtained solid was washed with n-hexane/DCM (1/1) and then dried under reduced pressure to give the title compound (0.114 g).

### Reference Example N-7

### 1-(4-(2-fluoro-5-(trifluoromethyl)benzyl)-7-nitro-3-oxo-3,4-dihydroquinoxalin-2-yl)urea

To a mixture of Reference Example N-6 (0.051 g) and DCM (0.5 mL), triphosgene (0.044 g) and TEA (0.045 g) were added under ice cooling, and the mixture was stirred under ice cooling for 20 minutes. Ammonium chloride (0.080 g) and DIPEA (0.194 g) were added to the reaction mixture under ice cooling, and the mixture was stirred at room temperature for 1.5 hours. MeCN (0.5 mL) was added to the reaction mixture, and the mixture was stirred at room temperature for 1 hour. A saturated aqueous ammonium chloride solution, MTBE, and n-hexane were added to the reaction mixture, and an insoluble material was collected by filtration. The obtained solid was washed with water and MTBE and then dried under reduced pressure to give 1-(4-(2-fluoro-5-(trifluoromethyl)benzyl)-3-oxo-3,4-dihydroquinoxalin-2-yl)urea (0.039 g).

To a mixture of the obtained compound (0.027 g) and sulfuric acid (0.5 mL), nitric acid (0.005 g) was added under ice cooling, and the mixture was stirred under ice cooling for 1 hour. Nitric acid (0.002 g) was added to the reaction mixture, and the mixture was stirred under ice cooling for 1 hour. Water was added to the reaction mixture, and an insoluble material was collected by filtration. The obtained solid was washed with water and then dried under reduced pressure to give the title compound (0.023 g).

¹H-NMR(DMSO-d6)δ ppm: 5.58 (2H, s), 7.40-7.84 (5H, m), 8.17 (1H, dd, J = 2.8, 9.2 Hz), 8.60 (1H, br), 8.69 (1H, br), 8.75 (1H, d, J = 2.8 Hz)

### Reference Example N-8

### 1-(7-amino-4-(2-fluoro-5-(trifluoromethyl)benzyl)-3-oxo-3,4-dihydroquinoxalin-2-yl)urea

To a mixture of Reference Example N-7 (0.022 g), ammonium chloride (0.028 g), water (0.5 mL), and ethanol (2.0 mL), iron powder (0.029 g) was added at room temperature, and the mixture was stirred at 70°C for 2.5 hours. After the reaction mixture was allowed to cool to room temperature, water was added, and the mixture was extracted with DCM. The extract was concentrated under reduced pressure. The residue was purified by APS column chromatography (eluent: n-hexane/ethyl acetate = 50/50 to 0/100) to give the title compound (0.014 g).

The structural formulas of the reference examples are shown in the following tables.

**[Table 1]**

| Ref. No. | Str. | Ref. No. | Str. |
|---|---|---|---|
| A-1 | 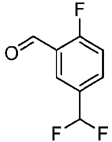 | A-2 | 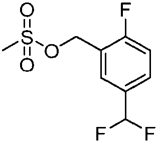 |
| A-3 | 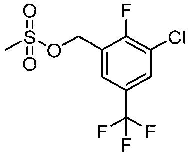 | A-4 | 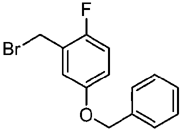 |
| A-5 | 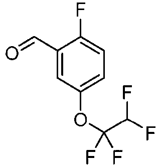 | A-6 | 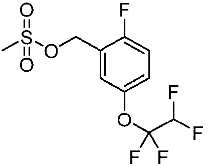 |
| A-7 | 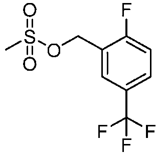 | A-8 | 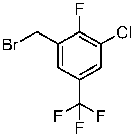 |
| A-9 | 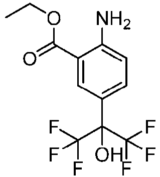 | A-10 | 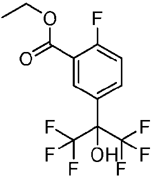 |
| A-11 | 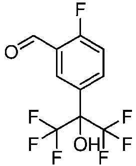 | A-12 | 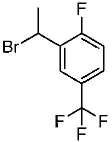 |
| B-1 | 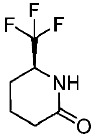 | B-2 | 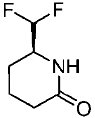 |

**[Table 2]**

| Ref. No. | Str. | Ref. No. | Str. |
|---|---|---|---|
| B-3 | 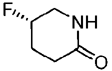 | B-4 | 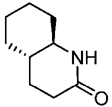 |
| C-1 | 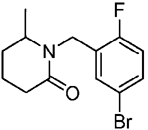 | C-2 | 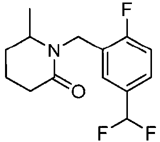 |
| C-3 | 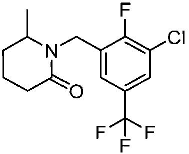 | C-4 | 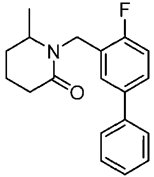 |
| C-5 | 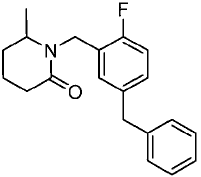 | C-6 | 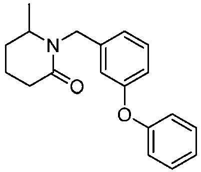 |
| C-7 | 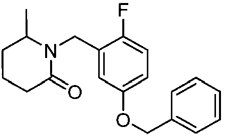 | C-8 | 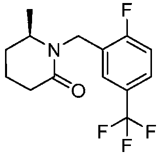 |
| C-9 | 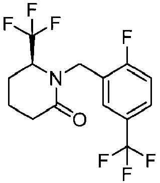 | C-10 | 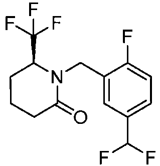 |
| C-11 | 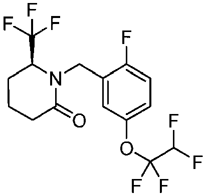 | C-12 | 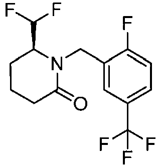 |

**[Table 3]**

| Ref. No. | Str. | Ref. No. | Str. |
|---|---|---|---|
| C-13 | 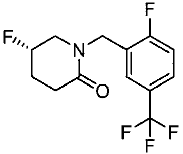 | C-14 | 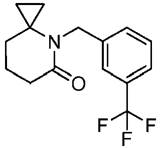 |
| C-15 | 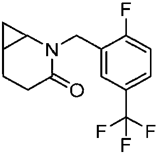 | C-16 | 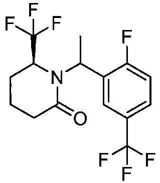 |
| C-17 | 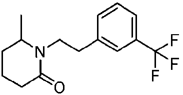 | C-18 | 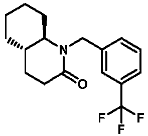 |
| D-1 | 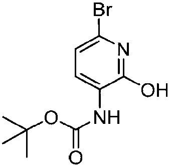 | D-2 | 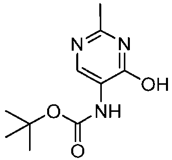 |
| D-3 | 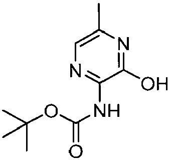 | E-1 | 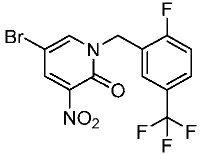 |
| E-2 | 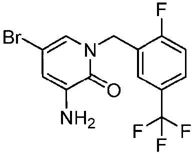 | E-3 | 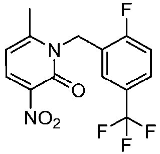 |
| E-4 | 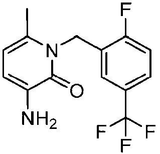 | E-5 | 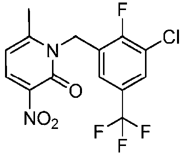 |

**[Table 4]**

| Ref. No. | Str. | Ref. No. | Str. |
|---|---|---|---|
| E-6 | 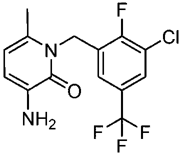 | E-7 | 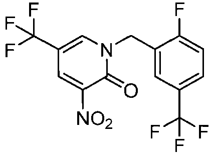 |
| E-8 | 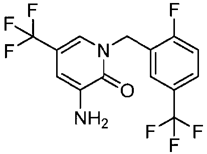 | F-1 | 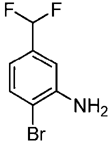 |
| G-1 | 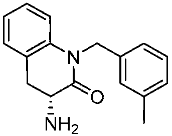 | G-2 | 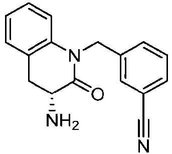 |
| G-3 | 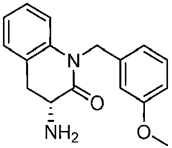 | G-4 | 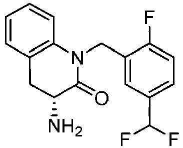 |
| G-5 | 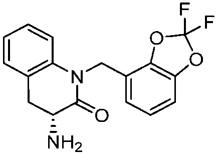 | G-6 | 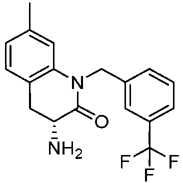 |
| G-7 | 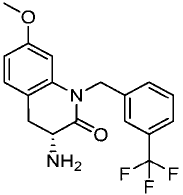 | G-8 | 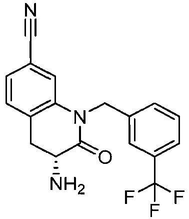 |
| G-9 | 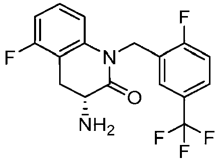 | G-10 | 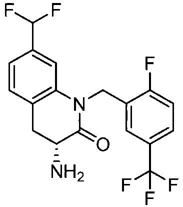 |

**[Table 5]**

| Ref. No. | Str. | Ref. No. | Str. |
|---|---|---|---|
| G-11 | 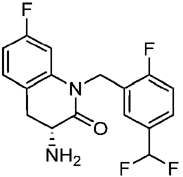 | G-12 | 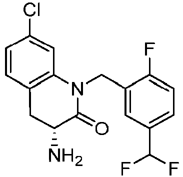 |
| G-13 | 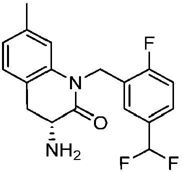 | G-14 | 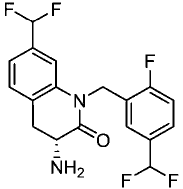 |
| G-15 | 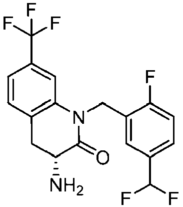 | G-16 | 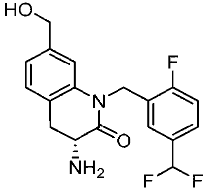 |
| G-17 | 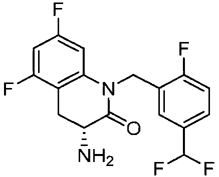 | G-18 | 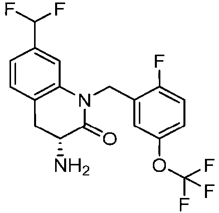 |
| G-19 | 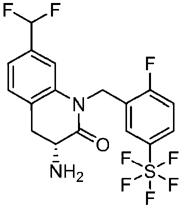 | G-20 | 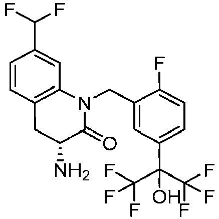 |
| G-21 | 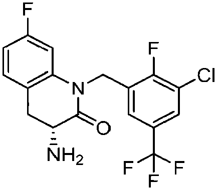 | G-22 | 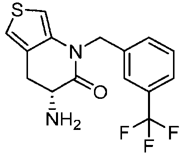 |

**[Table 6]**

| Ref. No. | Str. | Ref. No. | Str. |
|---|---|---|---|
| G-23 | 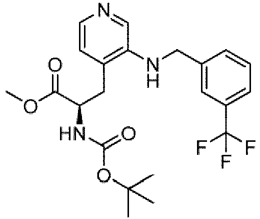 | G-24 | 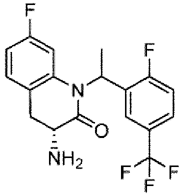 |
| G-25 | 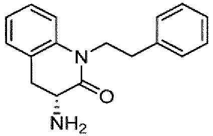 | G-26 | 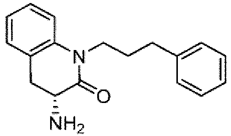 |
| G--27 | 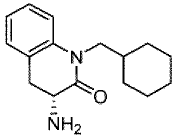 | | |

**[Table 7]**

| Ref. No. | Str. | Ref. No. | Str. |
|---|---|---|---|
| H-1 | 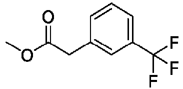 | H-2 | 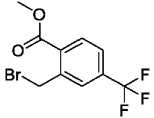 |
| H-3 | 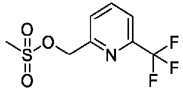 | I-1 | 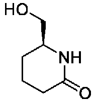 |
| I-2 | 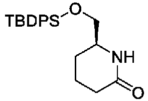 | I-3 | 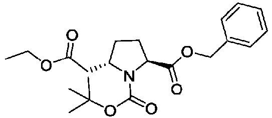 |
| I-4 | 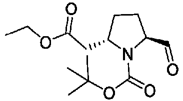 | J-1 | 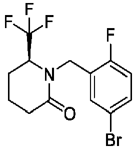 |

**[Table 8]**

| Ref. No. | Str. | Ref. No. | Str. |
|---|---|---|---|
| J-2 | 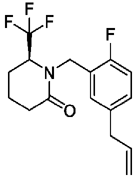 | J-3 | 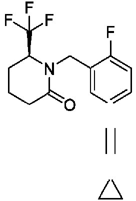 |
| J-4 | 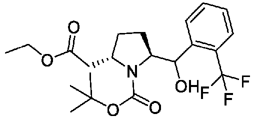 | J-5 | 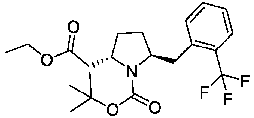 |
| J-6 | 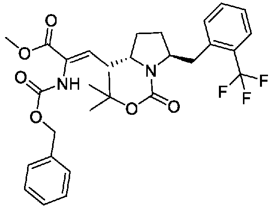 | J-7 | 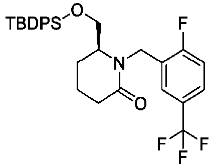 |
| J-8 | 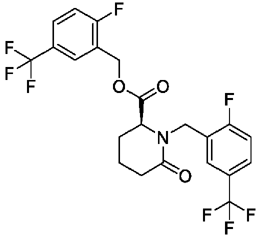 | J-9 | 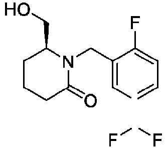 |
| J-10 | 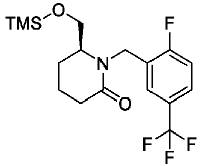 | J-11 | 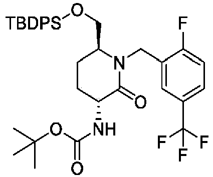 |
| J-12 | 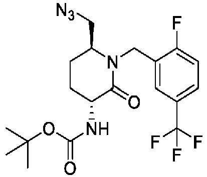 | J-13 | 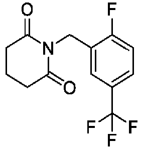 |

**[Table 9]**

| Ref. No. | Str. | Ref. No. | Str. |
|---|---|---|---|
| J-14 | 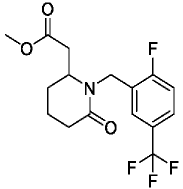 | J-15 | 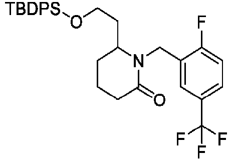 |
| J-16 | 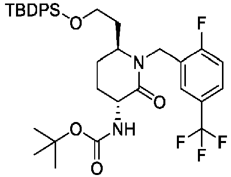 | K-1 | 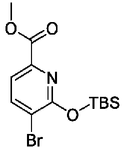 |
| L-1 | 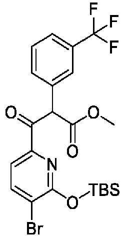 | L-2 | 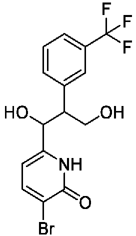 |
| L-3 | 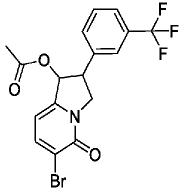 | M-1 | 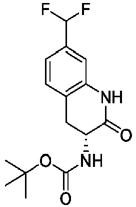 |
| M-2 | 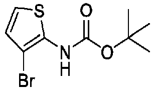 | M-3 | 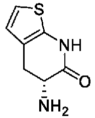 |
| M-4 | 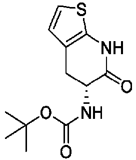 | M-5 | 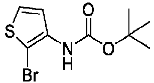 |

**[Table 10]**

| Ref. No. | Str. | Ref. No. | Str. |
|---|---|---|---|
| M-6 | 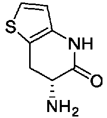 | M-7 | 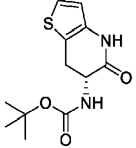 |
| M-8 | 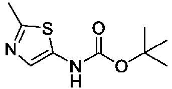 | M-9 | 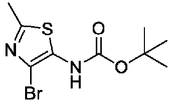 |
| M-10 | 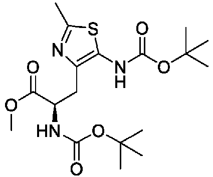 | M-11 | 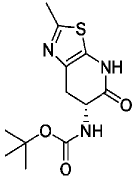 |
| M-12 | 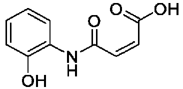 | M-13 | 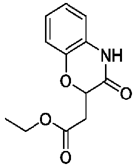 |
| M-14 | 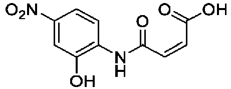 | M-15 | 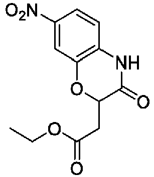 |
| N-1 | 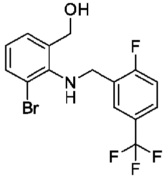 | N-2 | 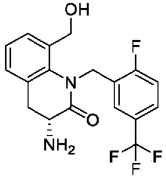 |
| N-3 | 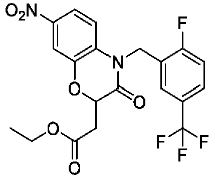 | N-4 | 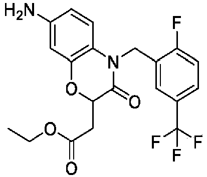 |

**[Table 11]**

| Ref. No. | Str. | Ref. No. | Str. |
|---|---|---|---|
| N-5 | 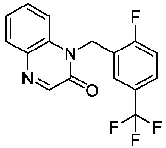 | N-6 | 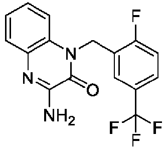 |
| N-7 | 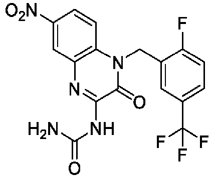 | N-8 | 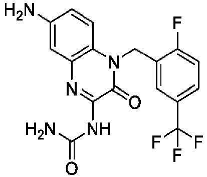 |

The stereochemical notations of Reference Examples B-4, C-18, and J-16 in the tables indicate relative configurations.

### Example A-1

### 1-((3R^{*},6R^{*})-1-(5-bromo-2-fluorobenzyl)-6-methyl-2-oxopiperidin-3-yl)urea

To a mixture of Reference Example C-1 (0.687 g) and THF (11 mL), LDA (1.07 mol/L in THF/n-hexane) (3.21 mL) was added at -78°C, and the mixture was stirred at the same temperature for 15 minutes. DPPA (0.945 g) was added to the reaction mixture at - 78°C, and the mixture was stirred at the same temperature for 1 hour. A mixture of Boc₂O (0.599 g) and THF (5.5 mL) was added to the reaction mixture at -78°C, and the mixture was stirred at the same temperature for 10 minutes and then under ice cooling for 1 hour. Water was added to the reaction mixture, and the mixture was stirred at 50°C for 1 hour. After the reaction mixture was allowed to cool to room temperature, a saturated aqueous ammonium chloride solution was added, and the mixture was extracted with ethyl acetate. The extract was washed with water and saturated brine, dried over anhydrous magnesium sulfate, and concentrated under reduced pressure. The residue was purified by Method A (eluent: n-hexane/ethyl acetate = 90/10 to 70/30) to give tert-butyl ((3R*,6R*)-1-(5-bromo-2-fluorobenzyl)-6-methyl-2-oxopiperidin-3-yl)carbamate (0.446 g).

To a mixture of the obtained compound (0.214 g) and DCM (2.1 mL), TFA (1.17 g) was added at room temperature, and the mixture was stirred at the same temperature for 40 minutes. To the reaction mixture, a 5 mol/L aqueous sodium hydroxide solution (2.06 mL) was added under ice cooling, and the mixture was extracted with DCM. The extract was concentrated under reduced pressure.

To a mixture of the residue, water (0.210 mL), and THF (2.1 mL), potassium cyanate (0.055 g) and acetic acid (0.032 g) were added under ice cooling, and the mixture was stirred at room temperature for 2 hours. Water was added to the reaction mixture, and the mixture was extracted with DCM. The extract was concentrated under reduced pressure. The residue was purified by silica gel column chromatography (eluent: n-hexane/ethyl acetate/methanol = 30/70/0 to 0/100/0 to 0/95/5) to give the title compound (0.113 g).

### Example A-2

### 1-((3R^{*},6R^{*})-1-(5-(difluoromethyl)-2-fluorobenzyl)-6-methyl-2-oxopiperidin-3-yl)urea

To a mixture of Reference Example C-2 (0.067 g) and THF (1.2 mL), LDA (1.07 mol/L in THF/n-hexane) (0.346 mL) was added at -78°C, and the mixture was stirred at the same temperature for 20 minutes. DPPA (0.102 g) was added to the reaction mixture at - 78°C, and the mixture was stirred at the same temperature for 45 minutes. A mixture of Boc₂O (0.065 g) and THF (0.6 mL) was added to the reaction mixture at -78°C, and the mixture was stirred at the same temperature for 10 minutes and then under ice cooling for 1 hour. Water was added to the reaction mixture, and the mixture was stirred at 50°C for 1 hour. After the reaction mixture was allowed to cool to room temperature, a saturated aqueous ammonium chloride solution was added, and the mixture was extracted with ethyl acetate. The extract was dried over anhydrous magnesium sulfate and concentrated under reduced pressure. The residue was purified by Method A (eluent: n-hexane/ethyl acetate = 90/10 to 70/30) to give tert-butyl ((3R^{*},6R^{*})-1-(5-(difluoromethyl)-2-fluorobenzyl)-6-methyl-2-oxopiperidin-3-yl)carbamate (0.032 g).

To a mixture of the obtained compound (0.031 g) and DCM (0.5 mL), TFA (0.183 g) was added at room temperature, and the mixture was stirred at the same temperature for 30 minutes. To the reaction mixture, a 5 mol/L aqueous sodium hydroxide solution (0.320 mL) was added under ice cooling, and the mixture was extracted with DCM. The extract was concentrated under reduced pressure.

To a mixture of the residue, water (0.050 mL), and THF (0.5 mL), potassium cyanate (0.009 g) and acetic acid (0.005 g) were added at room temperature, and the mixture was stirred at the same temperature for 1 hour. Water was added to the reaction mixture, and the mixture was extracted with DCM. The extract was concentrated under reduced pressure. The residue was suspended in MTBE, and an insoluble material was collected by filtration. The obtained solid was dried under reduced pressure to give the title compound (0.020 g).

### Example A-3

### 1-((3R^{*},6R^{*})-1-(3-chloro-2-fluoro-5-(trifluoromethyl)benzyl)-6-methyl-2-oxopiperidin-3-yl)urea

Example A-3 was synthesized by the same method as in Example A-2, except that Reference Example C-3 was used instead of Reference Example C-2.

### Example A-4

### 1-((3R^{*},6R^{*})-1-((4-fluoro-[1,1'-biphenyl]-3-yl)methyl)-6-methyl-2-oxopiperidin-3-yl)urea

Example A-4 was synthesized by the same method as in Example A-1, except that Reference Example C-4 was used instead of Reference Example C-1.

### Example A-5

### 1-((3R^{*},6R^{*})-1-(5-benzyl-2-fluorobenzyl)-6-methyl-2-oxopiperidin-3-yl)urea

Example A-5 was synthesized by the same method as in Example A-1, except that Reference Example C-5 was used instead of Reference Example C-1.

### Example A-6

### 1-((3R^{*},6R^{*})-6-methyl-2-oxo-1-(3-phenoxybenzyl)piperidin-3-yl)urea

Example A-6 was synthesized by the same method as in Example A-2, except that Reference Example C-6 was used instead of Reference Example C-2.

### Example A-7

### 1-((3R^{*},6R^{*})-1-(2-fluoro-5-(phenylethynyl)benzyl)-6-methyl-2-oxopiperidin-3-yl)urea

Under an argon atmosphere, to a mixture of Example A-1 (0.035 g), phenylacetylene (0.030 g), TEA (0.040 g), and DMF (1.0 mL), bis(di-tert-butyl(4-dimethylaminophenyl)phosphine)dichloropalladium(II) (0.003 g) was added at room temperature, and the mixture was stirred at 80°C for 17 hours. After the reaction mixture was allowed to cool to room temperature, a saturated aqueous ammonium chloride solution and water were added, and the mixture was extracted with diethyl ether. The extract was washed with water and saturated brine, dried over anhydrous magnesium sulfate, and concentrated under reduced pressure. The residue was successively purified by silica gel column chromatography (eluent: n-hexane/ethyl acetate/methanol = 30/70/0 to 0/100/0 to 0/95/5) and silica gel column chromatography (eluent: DCM/methanol = 100/0 to 90/10) to give the title compound (0.007 g).

### Example A-8

### 1-((3R^{*},6R^{*})-1-(2-fluoro-5-(pyridin-4-ylethynyl)benzyl)-6-methyl-2-oxopiperidin-3-yl)urea

Example A-8 was synthesized by the same method as in Example A-7, except that 4-ethynylpyridine was used instead of phenylacetylene.

### Example A-9

### 1-((3R^{*},6R^{*})-1-(2-fluoro-5-(pyridin-3-ylethynyl)benzyl)-6-methyl-2-oxopiperidin-3-yl)urea

Example A-9 was synthesized by the same method as in Example A-7, except that 3-ethynylpyridine was used instead of phenylacetylene.

### Example A-10

### 1-((3R^{*},6R^{*})-1-(5-(cyclopropylethynyl)-2-fluorobenzyl)-6-methyl-2-oxopiperidin-3-yl)urea

Under an argon atmosphere, to a mixture of Example A-1 (0.034 g), ethynylcyclopropane (0.012 g), cesium carbonate (0.091 g), and MeCN (0.7 mL), Xphos (0.007 g) and bis(acetonitrile)palladium(II) chloride (0.001 g) were added at room temperature, and the mixture was stirred at 90°C for 12 hours under microwave irradiation. Water was added to the reaction mixture, and the mixture was extracted with DCM. The extract was concentrated under reduced pressure. The residue was suspended in MTBE, and the insoluble material was collected by filtration. The obtained solid was purified by silica gel column chromatography (eluent: DCM/methanol = 100/0 to 90/10) to give the title compound (0.003 g).

### Example A-11

### 1-((3R^{*},6R^{*})-1-(5-(benzyloxy)-2-fluorobenzyl)-6-methyl-2-oxopiperidin-3-yl)urea

Using Reference Example C-7 instead of Reference Example C-1, Example A-11 was synthesized by the same method as in Example A-1.

### Example A-12

### 1-((3R^{*},6R^{*})-1-(2-fluoro-5-phenethylbenzyl)-6-methyl-2-oxopiperidin-3-yl)urea

A mixture of Example A-7 (0.007 g), 10% Pd/C (0.007 g), THF (0.5 mL), and methanol (0.5 mL) was stirred under a hydrogen atmosphere at room temperature for 1 hour. The reaction mixture was filtered through Celite, and the filtrate was concentrated under reduced pressure. The residue was purified by silica gel column chromatography (eluent: ethyl acetate/methanol = 100/0 to 80/20) to give the title compound (0.004 g).

### Example A-13

### 1-((3R,6R)-1-(2-fluoro-5-(trifluoromethyl)benzyl)-6-methyl-2-oxopiperidin-3-yl)urea

To a mixture of Reference Example C-8 (0.350 g) and THF (6.0 mL), LDA (1.07 mol/L in THF/n-hexane) (1.47 mL) was added at -78°C, and the mixture was stirred at the same temperature for 30 minutes. DPPA (0.433 g) was added to the reaction mixture at - 78°C, and the mixture was stirred at the same temperature for 20 minutes. A mixture of Boc₂O (0.396 g) and THF (3.0 mL) was added to the reaction mixture at -78°C, and the mixture was stirred at the same temperature for 15 minutes. Water was added to the reaction mixture at room temperature, and the mixture was stirred at the same temperature for 1 hour. A saturated aqueous ammonium chloride solution, water, and a saturated aqueous sodium hydrogen carbonate solution were added to the reaction mixture, and the mixture was extracted with ethyl acetate. The extract was concentrated under reduced pressure. The residue was purified by silica gel column chromatography (eluent: n-hexane/ethyl acetate = 90/10 to 70/30) to give tert-butyl ((3R,6R)-1-(2-fluoro-5-(trifluoromethyl)benzyl)-6-methyl-2-oxopiperidin-3-yl)carbamate (0.226 g).

Hydrogen chloride (4 mol/L in 1,4-dioxane) (0.685 mL) was added to a mixture of the obtained compound (0.123 g) and DCM (2.0 mL) at room temperature at room temperature, and the mixture was stirred at the same temperature for 1 hour. A saturated aqueous sodium hydrogen carbonate solution was added to the reaction mixture, and the mixture was extracted with ethyl acetate. The extract was concentrated under reduced pressure. The residue was purified by APS column chromatography (eluent: n-hexane/ethyl acetate/methanol = 60/40/0 to 0/100/0 to 0/70/30) to give (3R,6R)-3-amino-1-(2-fluoro-5-(trifluoromethyl)benzyl)-6-methylpiperidin-2-one (0.050 g).

A mixture of the obtained compound (0.050 g), water (0.059 mL), methanol (0.5 mL), and THF (0.5 mL) was added with potassium cyanate (0.017 g) and acetic acid (0.012 g) at room temperature, and the mixture was stirred at the same temperature for 2 hours. Water was added to the reaction mixture, and the mixture was extracted with ethyl acetate. The extract was concentrated under reduced pressure. The residue was purified by APS column chromatography (eluent: n-hexane/ethyl acetate/methanol = 70/30/0 to 0/100/0 to 0/32/68) to give the title compound (0.027 g).

### Example A-14

### 1-((3R,6S)-1-(2-fluoro-5-(trifluoromethyl)benzyl)-2-oxo-6-(trifluoromethyl)piperidin-3-yl)urea

To a mixture of Reference Example C-9 (0.174 g) and THF (2.5 mL), LDA (1.07 mol/L in THF/n-hexane) (0.711 mL) was added at -78°C, and the mixture was stirred at the same temperature for 20 minutes. DPPA (0.209 g) was added to the reaction mixture at - 78°C, and the mixture was stirred at the same temperature for 45 minutes. A mixture of Boc₂O (0.133 g) and THF (1.3 mL) was added to the reaction mixture at -78°C, and the mixture was stirred at the same temperature for 10 minutes and under ice cooling for 1 hour. Water was added to the reaction mixture, and the mixture was stirred at 50°C for 1 hour. After allowing the reaction mixture to cool to room temperature, a saturated aqueous ammonium chloride solution was added, and the mixture was extracted with ethyl acetate. The extract was dried over anhydrous magnesium sulfate and concentrated under reduced pressure. The residue was purified by Method A (eluent: n-hexane/ethyl acetate = 90/10 to 70/30) to give tert-butyl ((3R,6S)-1-(2-fluoro-5-(trifluoromethyl)benzyl)-2-oxo-6-(trifluoromethyl)piperidin-3-yl)carbamate (0.144 g).

A mixture of the obtained compound (0.050 g) and DCM (0.5 mL) was added with TFA (0.249 g) at room temperature, and the mixture was stirred at the same temperature for 45 minutes. A 5 mol/L aqueous sodium hydroxide solution (0.435 mL) was added to the reaction mixture under ice cooling, and the mixture was extracted with DCM. The extract was concentrated under reduced pressure.

A mixture of the residue, water (0.050 mL), and THF (0.5 mL) was added with potassium cyanate (0.012 g) and acetic acid (0.007 g) at room temperature, and the mixture was stirred at the same temperature for 1.5 hours. Water was added to the reaction mixture, and the mixture was extracted with DCM. The extract was concentrated under reduced pressure. The residue was purified by silica gel column chromatography (eluent: n-hexane/ethyl acetate/methanol = 30/70/0 to 0/100/0 to 0/95/5) to give the title compound (0.010 g).

### Example A-15

### 1-((3R,6S)-1-(5-(difluoromethyl)-2-fluorobenzyl)-2-oxo-6-(trifluoromethyl)piperidin-3-yl)urea

To a mixture of Reference Example C-10 (0.109 g) and THF (1.7 mL), LDA (1.07 mol/L in THF/n-hexane) (0.469 mL) was added at -78°C, and the mixture was stirred at the same temperature for 15 minutes. DPPA (0.138 g) was added to the reaction mixture at - 78°C, and the mixture was stirred at the same temperature for 45 minutes. A mixture of Boc₂O (0.088 g) and THF (0.85 mL) was added to the reaction mixture at -78°C, and the mixture was stirred at the same temperature for 10 minutes and under ice cooling for 50 minutes. Water was added to the reaction mixture, and the mixture was stirred at 50°C for 1 hour. After allowing the reaction mixture to cool to room temperature, a saturated aqueous ammonium chloride solution was added, and the mixture was extracted with ethyl acetate. The extract was dried over anhydrous magnesium sulfate and concentrated under reduced pressure. The residue was purified by Method A (eluent: n-hexane/ethyl acetate = 90/10 to 70/30) to give tert-butyl ((3R,6S)-1-(5-(difluoromethyl)-2-fluorobenzyl)-2-oxo-6-(trifluoromethyl)piperidin-3-yl)carbamate (0.086 g).

A mixture of the obtained compound (0.025 g) and DCM (0.5 mL) was added with TFA (0.129 g) at room temperature, and the mixture was stirred at the same temperature for 40 minutes. A 5 mol/L aqueous sodium hydroxide solution (0.230 mL) was added to the reaction mixture under ice cooling, and the mixture was extracted with DCM. The extract was concentrated under reduced pressure.

A mixture of the residue, water (0.050 mL), and THF (0.5 mL) was added with potassium cyanate (0.006 g) and acetic acid (0.004 g) under ice cooling, and the mixture was stirred at room temperature for 2.5 hours. Water was added to the reaction mixture, and the mixture was extracted with DCM. The extract was concentrated under reduced pressure. The residue was purified by silica gel column chromatography (eluent: n-hexane/ethyl acetate/methanol = 30/70/0 to 0/100/0 to 0/95/5) to give the title compound (0.017 g).

### Example A-16

### 1-((3R,6S)-1-(2-fluoro-5-(1,1,2,2-tetrafluoroethoxy)benzyl)-2-oxo-6-(trifluoromethyl)piperidin-3-yl)urea

To a mixture of Reference Example C-11 (0.165 g) and THF (1.4 mL), LDA (1.07 mol/L in THF/n-hexane) (0.591 mL) was added at -78°C, and the mixture was stirred at the same temperature for 20 minutes. DPPA (0.174 g) was added to the reaction mixture at - 78°C, and the mixture was stirred at the same temperature for 50 minutes. A mixture of Boc₂O (0.110 g) and THF (0.7 mL) was added to the reaction mixture at -78°C, and the mixture was stirred at the same temperature for 15 minutes and under ice cooling for 1 hour. Water was added to the reaction mixture, and the mixture was stirred at 50°C for 2 hours. After allowing the reaction mixture to cool to room temperature, a saturated aqueous ammonium chloride solution was added, and the mixture was extracted with ethyl acetate. The extract was dried over anhydrous magnesium sulfate and concentrated under reduced pressure. The residue was purified by Method A (eluent: n-hexane/ethyl acetate = 90/10 to 70/30) to give tert-butyl ((3R,6S)-1-(2-fluoro-5-(1,1,2,2-tetrafluoroethoxy)benzyl)-2-oxo-6-(trifluoromethyl)piperidin-3-yl)carbamate (0.098 g).

A mixture of the obtained compound (0.097 g) and DCM (0.97 mL) was added with TFA (0.437 g) at room temperature, and the mixture was stirred at the same temperature for 30 minutes. A 5 mol/L aqueous sodium hydroxide solution (0.765 mL) was added to the reaction mixture under ice cooling, and the mixture was extracted with DCM. The extract was concentrated under reduced pressure.

A mixture of the residue, water (0.097 mL), and THF (0.97 mL) was added with potassium cyanate (0.021 g) and acetic acid (0.012 g) under ice cooling, and the mixture was stirred under ice cooling for 40 hours. Water was added to the reaction mixture, and the mixture was extracted with DCM. The extract was concentrated under reduced pressure. The residue was purified by silica gel column chromatography (eluent: ethyl acetate/methanol = 100/0 to 95/5) to give the title compound (0.070 g).

### Example A-17

### 1-((3R,6S)-6-(difluoromethyl)-1-(2-fluoro-5-(trifluoromethyl)benzyl)-2-oxopiperidin-3-yl)urea

To a mixture of Reference Example C-12 (0.152 g) and THF (2.3 mL), LDA (1.07 mol/L in THF/n-hexane) (0.656 mL) was added at -78°C, and the mixture was stirred at the same temperature for 20 minutes. DPPA (0.193 g) was added to the reaction mixture at - 78°C, and the mixture was stirred at the same temperature for 45 minutes. A mixture of Boc₂O (0.123 g) and THF (1.2 mL) was added to the reaction mixture at -78°C, and the mixture was stirred at the same temperature for 10 minutes and under ice cooling for 1 hour. Water was added to the reaction mixture, and the mixture was stirred at 50°C for 1 hour. After allowing the reaction mixture to cool to room temperature, a saturated aqueous ammonium chloride solution was added, and the mixture was extracted with ethyl acetate. The extract was dried over anhydrous magnesium sulfate and concentrated under reduced pressure. The residue was purified by Method A (eluent: n-hexane/ethyl acetate = 90/10 to 70/30) to give tert-butyl ((3R,6S)-6-(difluoromethyl)-1-(2-fluoro-5-(trifluoromethyl)benzyl)-2-oxopiperidin-3-yl)carbamate (0.085 g).

A mixture of the obtained compound (0.027 g) and DCM (0.5 mL) was added with TFA (0.140 g) at room temperature, and the mixture was stirred at the same temperature for 20 minutes. A 5 mol/L aqueous sodium hydroxide solution (0.245 mL) was added to the reaction mixture under ice cooling, and the mixture was extracted with DCM. The extract was concentrated under reduced pressure.

A mixture of the residue, water (0.050 mL), and THF (0.5 mL) was added with potassium cyanate (0.007 g) and acetic acid (0.004 g) at room temperature, and the mixture was stirred at the same temperature for 19 hours. Water was added to the reaction mixture, and the mixture was extracted with DCM. The extract was concentrated under reduced pressure. The residue was suspended in diethyl ether, and the insoluble material was collected by filtration. The obtained solid was dried under reduced pressure to give the title compound (0.012 g).

### Example A-18

### 1-((3R,5S)-5-fluoro-1-(2-fluoro-5-(trifluoromethyl)benzyl)-2-oxopiperidin-3-yl)urea

Using Reference Example C-13 instead of Reference Example C-2, Example A-18 was synthesized by the same method as in Example A-2.

### Example A-19

### tert-butyl (2-((3R*,5S*)-1-(2-fluoro-5-(trifluoromethyl)benzyl)-6-oxo-5-ureidopiperidin-3-yl)ethyl)carbamate

A mixture of Example B-3 (0.010 g), platinum(IV) oxide (0.020 g), and methanol (2.0 mL) was stirred under a hydrogen atmosphere at room temperature for 1 hour and at 50°C for 2 hours. After allowing the reaction mixture to cool to room temperature, the mixture was filtered through Celite, and the filtrate was concentrated under reduced pressure. The residue was purified by silica gel column chromatography (eluent: n-hexane/ethyl acetate/methanol = 49/49/2 to 0/98/2 to 0/86/14) to give the title compound (0.004 g).

### Example A-20

### 1-(5-oxo-4-(3-(trifluoromethyl)benzyl)-4-azaspiro[2.5]octan-6-yl)urea

Using Reference Example C-14 instead of Reference Example C-1, Example A-20 was synthesized by the same method as in Example A-1.

### Example A-21

### 1-((1R^{*},4R^{*},6S^{*})-2-(2-fluoro-5-(trifluoromethyl)benzyl)-3-oxo-2-azabicyclo[4.1.0]heptan-4-yl)urea

Using Reference Example C-15 instead of Reference Example C-1, Example A-21 was synthesized by the same method as in Example A-1.

### Example A-22

### 1-((3R,6S)-1-(1-(2-fluoro-5-(trifluoromethyl)phenyl)ethyl)-2-oxo-6-(trifluoromethyl)piperidin-3-yl)urea

Using Reference Example C-16 instead of Reference Example C-1, Example A-22 was synthesized by the same method as in Example A-1.

### Example A-23

### 1-((3R^{*},6S^{*})-6-methyl-2-oxo-1-(3-(trifluoromethyl)phenethyl)piperidin-3-yl)urea

Using Reference Example C-17 instead of Reference Example C-8, Example A-23 was synthesized by the same method as in Example A-13.

### Example A-24

### 1-((3R,6S)-1-(2-fluoro-5-(trifluoromethyl)benzyl)-2-oxo-6-(trifluoromethyl)piperidin-3-yl)-3-methylurea

To a mixture of Reference Example C-9 (0.174 g) and THF (2.5 mL), LDA (1.07 mol/L in THF/n-hexane) (0.711 mL) was added at -78°C, and the mixture was stirred at the same temperature for 20 minutes. DPPA (0.209 g) was added to the reaction mixture at - 78°C, and the mixture was stirred at the same temperature for 45 minutes. A mixture of Boc₂O (0.133 g) and THF (1.3 mL) was added to the reaction mixture at -78°C, and the mixture was stirred at the same temperature for 10 minutes and under ice cooling for 1 hour. Water was added to the reaction mixture, and the mixture was stirred at 50°C for 1 hour. After allowing the reaction mixture to cool to room temperature, a saturated aqueous ammonium chloride solution was added, and the mixture was extracted with ethyl acetate. The extract was dried over anhydrous magnesium sulfate and concentrated under reduced pressure. The residue was purified by Method A (eluent: n-hexane/ethyl acetate = 90/10 to 70/30) to give tert-butyl ((3R,6S)-1-(2-fluoro-5-(trifluoromethyl)benzyl)-2-oxo-6-(trifluoromethyl)piperidin-3-yl)carbamate (0.144 g).

A mixture of the obtained compound (0.046 g) and DCM (0.5 mL) was added with TFA (0.229 g) at room temperature, and the mixture was stirred at the same temperature for 30 minutes. A 5 mol/L aqueous sodium hydroxide solution (0.400 mL) was added to the reaction mixture under ice cooling, and the mixture was extracted with DCM. The extract was concentrated under reduced pressure.

To a mixture of the residue, TEA (0.030 g), and DCM (1.0 mL), triphosgene (0.030 g) was added under ice cooling, and the mixture was stirred at room temperature for 30 minutes. Methylamine (2 mol/L in THF) (0.502 mL) was added to the reaction mixture at room temperature, and the mixture was stirred at the same temperature for 2 hours. Methylamine (2 mol/L in THF) (1.00 mL) was added to the reaction mixture at room temperature, and the mixture was stirred at the same temperature for 30 minutes. Water was added to the reaction mixture, and the mixture was extracted with DCM. The extract was concentrated under reduced pressure. The residue was purified by silica gel column chromatography (eluent: n-hexane/ethyl acetate/methanol = 30/70/0 to 0/100/0 to 0/95/5) to give the title compound (0.006 g).

### Example A-25

### (S)-N-((3R,6S)-1-(2-fluoro-5-(trifluoromethyl)benzyl)-2-oxo-6-(trifluoromethyl)piperidin-3-yl)-2-methoxypropanamide

To a mixture of Reference Example C-9 (0.174 g) and THF (2.5 mL), LDA (1.07 mol/L in THF/n-hexane) (0.711 mL) was added at -78°C, and the mixture was stirred at the same temperature for 20 minutes. DPPA (0.209 g) was added to the reaction mixture at - 78°C, and the mixture was stirred at the same temperature for 45 minutes. A mixture of Boc₂O (0.133 g) and THF (1.3 mL) was added to the reaction mixture at -78°C, and the mixture was stirred at the same temperature for 10 minutes and under ice cooling for 1 hour. Water was added to the reaction mixture, and the mixture was stirred at 50°C for 1 hour. After allowing the reaction mixture to cool to room temperature, a saturated aqueous ammonium chloride solution was added, and the mixture was extracted with ethyl acetate. The extract was dried over anhydrous magnesium sulfate and concentrated under reduced pressure. The residue was purified by Method A (eluent: n-hexane/ethyl acetate = 90/10 to 70/30) to give tert-butyl ((3R,6S)-1-(2-fluoro-5-(trifluoromethyl)benzyl)-2-oxo-6-(trifluoromethyl)piperidin-3-yl)carbamate (0.144 g).

A mixture of the obtained compound (0.046 g) and DCM (0.5 mL) was added with TFA (0.228 g) at room temperature, and the mixture was stirred at the same temperature for 30 minutes. A 5 mol/L aqueous sodium hydroxide solution (0.400 mL) was added to the reaction mixture under ice cooling, and the mixture was extracted with DCM. The extract was concentrated under reduced pressure.

To a mixture of the residue, (S)-2-methoxypropanoic acid (0.014 g), DIPEA (0.019 g), and MeCN (0.5 mL), HATU (0.046 g) was added at room temperature, and the mixture was stirred at the same temperature for 1 hour. Water was added to the reaction mixture, and the mixture was extracted with DCM. The extract was concentrated under reduced pressure. The residue was purified by Method A (eluent: n-hexane/ethyl acetate/methanol = 50/50/0 to 0/100/0 to 0/95/5) to give the title compound (0.034 g).

### Example A-26

### 1-cyclopropyl-3-((3R,6S)-6-(difluoromethyl)-1-(2-fluoro-5-(trifluoromethyl)benzyl)-2-oxopiperidin-3-yl)urea

Using Reference Example C-12 instead of Reference Example C-9 and cyclopropylamine instead of methylamine, Example A-26 was synthesized by the same method as in Example A-24.

### Example A-27

### N-((3R,6S)-6-(difluoromethyl)-1-(2-fluoro-5-(trifluoromethyl)benzyl)-2-oxopiperidin-3-yl)acetamide

To a mixture of Reference Example C-12 (0.152 g) and THF (2.3 mL), LDA (1.07 mol/L in THF/n-hexane) (0.656 mL) was added at -78°C, and the mixture was stirred at the same temperature for 20 minutes. DPPA (0.193 g) was added to the reaction mixture at - 78°C, and the mixture was stirred at the same temperature for 45 minutes. A mixture of Boc₂O (0.123 g) and THF (1.2 mL) was added to the reaction mixture at -78°C, and the mixture was stirred at the same temperature for 10 minutes and under ice cooling for 1 hour. Water was added to the reaction mixture, and the mixture was stirred at 50°C for 1 hour. After allowing the reaction mixture to cool to room temperature, a saturated aqueous ammonium chloride solution was added, and the mixture was extracted with ethyl acetate. The extract was dried over anhydrous magnesium sulfate and concentrated under reduced pressure. The residue was purified by Method A (eluent: n-hexane/ethyl acetate = 90/10 to 70/30) to give tert-butyl ((3R,6S)-6-(difluoromethyl)-1-(2-fluoro-5-(trifluoromethyl)benzyl)-2-oxopiperidin-3-yl)carbamate (0.085 g).

A mixture of the obtained compound (0.018 g) and DCM (0.5 mL) was added with TFA (0.091 g) at room temperature, and the mixture was stirred at the same temperature for 1.5 hours. A 5 mol/L aqueous sodium hydroxide solution (0.160 mL) was added to the reaction mixture under ice cooling, and the mixture was extracted with DCM. The extract was concentrated under reduced pressure.

To a mixture of the residue, TEA (0.012 g), and DCM (0.5 mL), acetyl chloride (0.005 g) was added at room temperature, and the mixture was stirred at the same temperature for 45 minutes. Water was added to the reaction mixture, and the mixture was extracted with DCM. The extract was concentrated under reduced pressure. The residue was purified by silica gel column chromatography (eluent: n-hexane/ethyl acetate/methanol = 30/70/0 to 0/100/0 to 0/95/5) to give the title compound (0.011 g).

### Example A-28

### N-((3R,6S)-6-(difluoromethyl)-1-(2-fluoro-5-(trifluoromethyl)benzyl)-2-oxopiperidin-3-yl)cyclopropanecarboxamide

Using cyclopropanecarbonyl chloride instead of acetyl chloride, Example A-28 was synthesized by the same method as in Example A-27.

### Example A-29

### 1-((3R^{*},4aS^{*},8aR^{*})-(2-oxo-1-(3-(trifluoromethyl)benzyl)decahydroquinolin-3-yl)urea

Using Reference Example C-18 instead of Reference Example C-9, Example A-29 was synthesized by the same method as in Example A-14.

### Example B-1

### 1-(5-bromo-1-(2-fluoro-5-(trifluoromethyl)benzyl)-2-oxo-1,2-dihydropyridin-3-yl)urea

To a mixture of Reference Example E-2 (0.140 g) and DCM (3.0 mL), triphosgene (0.114 g) and TEA (0.116 g) were added under ice cooling, and the mixture was stirred under ice cooling for 30 minutes. Ammonium chloride (0.205 g) and DIPEA (0.496 g) were added to the reaction mixture under ice cooling, and the mixture was stirred at room temperature for 30 minutes. Water was added to the reaction mixture, and the mixture was stirred at room temperature for 10 minutes. The insoluble material was collected by filtration, and the obtained solid was washed with water and MTBE and then dried under reduced pressure to give the title compound (0.130 g).

### Example B-2

### 3-(1-(2-fluoro-5-(trifluoromethyl)benzyl)-6-oxo-5-ureido-1,6-dihydropyridin-3-yl)propanoic acid

To a mixture of Example B-1 (0.100 g), tri-o-tolylphosphine (0.015 g), TEA (0.099 g), benzyl acrylate (0.079 g), and MeCN (1.0 mL), palladium(II) acetate (0.006 g) was added at room temperature, and the mixture was stirred at 80°C for 1 hour and then stirred at 140°C for 10 minutes under microwave irradiation. A saturated aqueous ammonium chloride solution was added to the reaction mixture, and the mixture was extracted with ethyl acetate. The extract was washed with saturated brine, dried over anhydrous sodium sulfate, and concentrated under reduced pressure. The residue was suspended in MTBE, and the insoluble material was collected by filtration. The obtained solid was dried under reduced pressure to give benzyl (E)-3-(1-(2-fluoro-5-(trifluoromethyl)benzyl)-6-oxo-5-ureido-1,6-dihydropyridin-3-yl) acrylate (0.067 g).

A mixture of the obtained compound (0.065 g), 10% Pd/C (0.030 g), THF (2.0 mL), and methanol (2.0 mL) was stirred under a hydrogen atmosphere at room temperature for 1 hour. The reaction mixture was filtered through Celite, and the filtrate was concentrated under reduced pressure. The residue was suspended in MTBE, and the insoluble material was collected by filtration. The obtained solid was dried under reduced pressure to give the title compound (0.044 g).

### Example B-3

### tert-butyl (2-(1-(2-fluoro-5-(trifluoromethyl)benzyl)-6-oxo-5-ureido-1,6-dihydropyridin-3-yl)ethyl)carbamate

To a mixture of Example B-2 (0.042 g), DMF (0.5 mL), and tert-butyl alcohol (1.0 mL), TEA (0.021 g) and DPPA (0.043 g) were added at room temperature, and the mixture was stirred at 60°C for 1 hour. After allowing the reaction mixture to cool to room temperature, a 1 mol/L aqueous sodium hydroxide solution was added, and the mixture was extracted with ethyl acetate. The extract was washed with saturated brine, dried over anhydrous sodium sulfate, and concentrated under reduced pressure.

To a mixture of the residue and THF (1.0 mL), Boc₂O (0.046 g) was added at room temperature, and the mixture was stirred at the same temperature for 30 minutes. The reaction mixture was concentrated under reduced pressure. The residue was purified by Method A (eluent: n-hexane/ethyl acetate/methanol = 50/50/0 to 0/100/0 to 0/88/12) to give the title compound (0.015 g).

### Example B-4

### 1-(1-(2-fluoro-5-(trifluoromethyl)benzyl)-6-methyl-2-oxo-1,2-dihydropyridin-3-yl)urea

To a mixture of Reference Example E-4 (0.055 g) and DCM (1.0 mL), triphosgene (0.054 g) and TEA (0.056 g) were added under ice cooling, and the mixture was stirred under ice cooling for 30 minutes. Ammonium chloride (0.098 g) and DIPEA (0.237 g) were added to the reaction mixture under ice cooling, and the mixture was stirred at room temperature for 30 minutes. Water was added to the reaction mixture, and the mixture was stirred at room temperature for 10 minutes. The insoluble material was collected by filtration, and the obtained solid was washed with water and MTBE and then dried under reduced pressure to give the title compound (0.048 g).

### Example B-5

### 1-(1-(3-chloro-2-fluoro-5-(trifluoromethyl)benzyl)-6-methyl-2-oxo-1,2-dihydropyridin-3-yl)urea

To a mixture of Reference Example E-6 (0.040 g) and DCM (1.0 mL), triphosgene (0.035 g) and TEA (0.036 g) were added under ice cooling, and the mixture was stirred under ice cooling for 30 minutes. Ammonium chloride (0.064 g) and DIPEA (0.154 g) were added to the reaction mixture under ice cooling, and the mixture was stirred at room temperature for 30 minutes. Water was added to the reaction mixture, and the mixture was stirred at room temperature for 10 minutes. The insoluble material was collected by filtration, and the obtained solid was washed with water and MTBE and then dried under reduced pressure to give the title compound (0.026 g).

### Example B-6

### 1-(1-(2-fluoro-5-(trifluoromethyl)benzyl)-2-oxo-5-(trifluoromethyl)-1,2-dihydropyridin-3-yl)urea

Using Reference Example E-8 instead of Reference Example E-2, Example B-6 was synthesized by the same method as in Example B-1.

### Example B-7

### 1-(6-cyano-1-(2-fluoro-5-(trifluoromethyl)benzyl)-2-oxo-1,2-dihydropyridin-3-yl)urea

To a mixture of Reference Example D-1 (0.402 g) and MeCN (6.0 mL), potassium carbonate (0.330 g) and 2-(bromomethyl)-1-fluoro-4-(trifluoromethyl)benzene (0.460 g) were added at room temperature, and the mixture was stirred at the same temperature for 4.5 hours. Water was added to the reaction mixture, and the mixture was extracted with ethyl acetate. The extract was washed with saturated brine, dried over anhydrous sodium sulfate, and concentrated under reduced pressure. The residue was purified by Method B (eluent: n-hexane/ethyl acetate = 100/0 to 85/15) to give tert-butyl (6-bromo-1-(2-fluoro-5-(trifluoromethyl)benzyl)-2-oxo-1,2-dihydropyridin-3-yl)carbamate (0.035 g).

Under an argon atmosphere, to a mixture of the obtained compound (0.034 g), zinc cyanide (II) (0.017 g), zinc powder (0.001 g), and DMF (1.0 mL), bis(tri-tert-butylphosphine)palladium(0) (0.004 g) was added at room temperature, and the mixture was stirred at 100°C for 18 hours. After allowing the reaction mixture to cool to room temperature, the mixture was filtered through Celite. The filtrate was concentrated under reduced pressure. The residue was purified by silica gel column chromatography (eluent: n-hexane/ethyl acetate = 100/0 to 80/20) to give tert-butyl (6-cyano-1-(2-fluoro-5-(trifluoromethyl)benzyl)-2-oxo-1,2-dihydropyridin-3-yl)carbamate (0.014 g).

A mixture of the obtained compound (0.013 g) and DCM (0.5 mL) was added with TFA (0.071 g) at room temperature, and the mixture was stirred at the same temperature for 1.5 hours. A 5 mol/L aqueous sodium hydroxide solution (0.125 mL) was added to the reaction mixture under ice cooling, and the mixture was extracted with DCM. The extract was concentrated under reduced pressure.

To a mixture of the residue, TEA (0.009 g), and DCM (0.5 mL), triphosgene (0.009 g) was added under ice cooling, and the mixture was stirred under ice cooling for 30 minutes. Ammonium chloride (0.017 g) and DIPEA (0.040 g) were added to the reaction mixture under ice cooling, and the mixture was stirred at room temperature for 30 minutes. Water was added to the reaction mixture, and the mixture was stirred at room temperature for 10 minutes. The insoluble material was collected by filtration, and the obtained solid was washed with water and MTBE and then dried under reduced pressure to give the title compound (0.005 g).

### Example B-8

### 1-(1-(3-chloro-2-fluoro-5-(trifluoromethyl)benzyl)-2-methyl-6-oxo-1,6-dihydropyrimidin-5-yl)urea

To a mixture of Reference Example D-2 (0.067 g) and DMF (1.3 mL), sodium hydride (approximately 60%) (0.018 g) was added under ice cooling, and the mixture was stirred at room temperature for 20 minutes. Reference Example A-3 (0.100 g) was added to the reaction mixture under ice cooling, and the mixture was stirred at room temperature for 1 hour and at 50°C for 1.5 hours. Reference Example A-3 (0.046 g) was added to the reaction mixture at room temperature, and the mixture was stirred at 50°C for 1 hour. A saturated aqueous ammonium chloride solution was added to the reaction mixture under ice cooling, and the mixture was extracted with n-hexane/ethyl acetate (1/1). The extract was washed with water and saturated brine, dried over anhydrous sodium sulfate, and concentrated under reduced pressure. The residue was purified by silica gel column chromatography (eluent: n-hexane/ethyl acetate = 90/10 to 30/70) to give tert-butyl (1-(3-chloro-2-fluoro-5-(trifluoromethyl)benzyl)-2-methyl-6-oxo-1,6-dihydropyrimidin-5-yl)carbamate (0.053 g).

A mixture of the obtained compound (0.050 g) and DCM (0.5 mL) was added with TFA (0.261 g) at room temperature, and the mixture was stirred at the same temperature for 1 hour. A 5 mol/L aqueous sodium hydroxide solution (0.460 mL) was added to the reaction mixture under ice cooling, and the mixture was extracted with DCM. The extract was concentrated under reduced pressure.

To a mixture of the residue, TEA (0.035 g), and DCM (0.5 mL), triphosgene (0.034 g) was added under ice cooling, and the mixture was stirred under ice cooling for 30 minutes. Ammonium chloride (0.061 g), DIPEA (0.148 g), and DCM (0.5 mL) were added to the reaction mixture under ice cooling, and the mixture was stirred at room temperature for 1.5 hours. Water was added to the reaction mixture, and the mixture was extracted with DCM. The extract was concentrated under reduced pressure. The residue was suspended in MTBE, and the insoluble material was collected by filtration. The obtained solid was washed with MTBE and then dried under reduced pressure to give the title compound (0.005 g).

### Example B-9

### 1-(4-(3-chloro-2-fluoro-5-(trifluoromethyl)benzyl)-5-methyl-3-oxo-3,4-dihydropyrazin-2-yl)urea

Using Reference Example D-3 instead of Reference Example D-2, Example B-9 was synthesized by the same method as in Example B-8.

### Example B-10

### 1-(1-(3-chloro-2-fluoro-5-(trifluoromethyl)benzyl)-6-methyl-2-oxo-1,2-dihydropyridin-3-yl)-3-methylurea

To a mixture of Reference Example E-6 (0.033 g) and DCM (1.0 mL), triphosgene (0.032 g) and TEA (0.030 g) were added under ice cooling, and the mixture was stirred at room temperature for 30 minutes. Methylamine (2 mol/L in THF) (0.986 mL) was added to the reaction mixture at room temperature, and the mixture was stirred at the same temperature for 1 hour. A saturated aqueous sodium hydrogen carbonate solution and water were added to the reaction mixture, and the mixture was extracted with DCM. The extract was concentrated under reduced pressure. The residue was purified by silica gel column chromatography (eluent: n-hexane/ethyl acetate = 98/2 to 45/55) to give the title compound (0.008 g).

### Example B-11

### 1-(1-(3-chloro-2-fluoro-5-(trifluoromethyl)benzyl)-6-methyl-2-oxo-1,2-dihydropyridin-3-yl)-3-cyclopropylurea

Using cyclopropylamine instead of methylamine, Example B-11 was synthesized by the same method as in Example B-10.

### Example C-1

### (R)-1-(1-(3-methylbenzyl)-2-oxo-1,2,3,4-tetrahydroquinolin-3-yl)urea

To a mixture of Reference Example G-1 (0.050 g), acetic acid (0.012 g), and methanol (0.15 mL), a mixture of potassium cyanate (0.018 g) and water (0.068 mL) was added at room temperature, and the mixture was stirred at the same temperature for 1 hour. Water was added to the reaction mixture, and the mixture was stirred at room temperature for 20 minutes. The insoluble material was collected by filtration, and the obtained solid was washed with a mixed solvent of water/THF/methanol (10/1/1) and then dried under reduced pressure to give the title compound (0.010 g).

### Example C-2

### (R)-1-(1-(3-cyanobenzyl)-2-oxo-1,2,3,4-tetrahydroquinolin-3-yl)urea

To a mixture of Reference Example G-2 (0.043 g), water (0.055 mL), potassium cyanate (0.016 g), methanol (0.256 mL), and THF (0.383 mL), acetic acid (0.011 g) was added under ice cooling, and the mixture was stirred at room temperature for 13 hours. THF (0.256 mL), methanol (0.192 mL), and water (1.92 mL) were added to the reaction mixture, and the mixture was stirred at room temperature for 40 minutes. The insoluble material was collected by filtration, and the obtained solid was washed with ethanol/n-heptane (1/4) and then dried under reduced pressure to give the title compound (0.026 g).

### Example C-3

### (R)-1-(1-(3-methoxybenzyl)-2-oxo-1,2,3,4-tetrahydroquinolin-3-yl)urea

Using Reference Example G-3 instead of Reference Example G-1, Example C-3 was synthesized by the same method as in Example C-1.

### Example C-4

### (R)-1-(1-(5-(difluoromethyl)-2-fluorobenzyl)-2-oxo-1,2,3,4-tetrahydroquinolin-3-yl)urea

To a mixture of Reference Example G-4 (0.120 g), water (0.067 mL), and THF (1.0 mL), potassium cyanate (0.040 g) and methanesulfonic acid (0.038 g) were added at room temperature, and the mixture was stirred at the same temperature for 1 hour. A saturated aqueous sodium hydrogen carbonate solution, methanol, and water were added to the reaction mixture, and the mixture was extracted with DCM. The extract was concentrated under reduced pressure. The residue was suspended in ethyl acetate, and the insoluble material was collected by filtration. The obtained solid was dried under reduced pressure to give the title compound (0.058 g).

### Example C-5

### (R)-1-(1-((2,2-difluorobenzo[d][1,3]dioxol-4-yl)methyl)-2-oxo-1,2,3,4-tetrahydroquinolin-3-yl)urea

To a mixture of Reference Example G-5 (0.052 g), water (0.071 mL), and THF (0.71 mL), potassium cyanate (0.023 g), acetic acid (0.016 g), and THF (0.5 mL) were added at room temperature, and the mixture was stirred at the same temperature for 17 hours. Water, a saturated aqueous sodium hydrogen carbonate solution, and MTBE were added to the reaction mixture, and the insoluble material was collected by filtration. The obtained solid was washed with water and MTBE and then dried under reduced pressure to give the title compound (0.037 g).

### Example C-6

### (R)-1-(7-methyl-2-oxo-1-(3-(trifluoromethyl)benzyl)-1,2,3,4-tetrahydroquinolin-3-yl)urea

Using Reference Example G-6 instead of Reference Example G-2, Example C-6 was synthesized by the same method as in Example C-2.

### Example C-7

### (R)-1-(7-methoxy-2-oxo-1-(3-(trifluoromethyl)benzyl)-1,2,3,4-tetrahydroquinolin-3-yl)urea

Using Reference Example G-7 instead of Reference Example G-1, Example C-7 was synthesized by the same method as in Example C-1.

### Example C-8

### (R)-1-(7-cyano-2-oxo-1-(3-(trifluoromethyl)benzyl)-1,2,3,4-tetrahydroquinolin-3-yl)urea

Using Reference Example G-8 instead of Reference Example G-5, Example C-8 was synthesized by the same method as in Example C-5.

### Example C-9

### (R)-1-(5-fluoro-1-(2-fluoro-5-(trifluoromethyl)benzyl)-2-oxo-1,2,3,4-tetrahydroquinolin-3-yl)urea

To a mixture of Reference Example G-9 (0.085 g), water (0.043 mL), and THF (1.0 mL), potassium cyanate (0.025 g) and methanesulfonic acid (0.024 g) were added at room temperature, and the mixture was stirred at the same temperature for 1 hour. A saturated aqueous sodium hydrogen carbonate solution and water were added to the reaction mixture, and the mixture was stirred at room temperature for 10 minutes. The insoluble material was collected by filtration, and the obtained solid was washed with water and ethyl acetate and then dried under reduced pressure to give the title compound (0.064 g).

### Example C-10

### (R)-1-(7-(difluoromethyl)-1-(2-fluoro-5-(trifluoromethyl)benzyl)-2-oxo-1,2,3,4-tetrahydroquinolin-3-yl)urea

To a mixture of Reference Example G-10 (0.079 g), water (0.037 mL), and THF (0.5 mL), potassium cyanate (0.021 g) and acetic acid (0.013 g) were added at room temperature, and the mixture was stirred at the same temperature for 14 hours. A saturated aqueous sodium hydrogen carbonate solution and water were added to the reaction mixture, and the mixture was stirred at room temperature for 10 minutes. The insoluble material was collected by filtration, and the obtained solid was washed with water and MTBE and then dried under reduced pressure to give the title compound (0.052 g).

### Example C-11

### (R)-1-(1-(5-(difluoromethyl)-2-fluorobenzyl)-7-fluoro-2-oxo-1,2,3,4-tetrahydroquinolin-3-yl)urea

To a mixture of Reference Example G-11 (0.110 g), water (0.059 mL), and THF (1.0 mL), potassium cyanate (0.034 g) and methanesulfonic acid (0.033 g) were added at room temperature, and the mixture was stirred at the same temperature for 1 hour. A saturated aqueous sodium hydrogen carbonate solution and water were added to the reaction mixture, and the mixture was stirred at room temperature for 10 minutes. The insoluble material was collected by filtration, and the obtained solid was washed with water and MTBE and then dried under reduced pressure to give the title compound (0.100 g).

### Example C-12

### (R)-1-(7-chloro-1-(5-(difluoromethyl)-2-fluorobenzyl)-2-oxo-1,2,3,4-tetrahydroquinolin-3-yl)urea

To a mixture of Reference Example G-12 (0.150 g), water (0.076 mL), and THF (1.5 mL), potassium cyanate (0.045 g) and methanesulfonic acid (0.043 g) were added at room temperature, and the mixture was stirred at the same temperature for 1 hour. A saturated aqueous sodium hydrogen carbonate solution and water were added to the reaction mixture, and the mixture was extracted with DCM. The extract was concentrated under reduced pressure. The residue was suspended in ethyl acetate, and the insoluble material was collected by filtration. The obtained solid was dried under reduced pressure to give the title compound (0.090 g).

### Example C-13

### (R)-1-(1-(5-(difluoromethyl)-2-fluorobenzyl)-7-methyl-2-oxo-1,2,3,4-tetrahydroquinolin-3-yl)urea

To a mixture of Reference Example G-13 (0.099 g), water (0.053 mL), and THF (1.0 mL), potassium cyanate (0.031 g) and methanesulfonic acid (0.030 g) were added at room temperature, and the mixture was stirred at the same temperature for 1 hour. A saturated aqueous sodium hydrogen carbonate solution and water were added to the reaction mixture, and the mixture was stirred at room temperature for 10 minutes. The insoluble material was collected by filtration, and the obtained solid was washed with water and MTBE and then dried under reduced pressure to give the title compound (0.083 g).

### Example C-14

### (R)-1-(7-(difluoromethyl)-1-(5-(difluoromethyl)-2-fluorobenzyl)-2-oxo-1,2,3,4-tetrahydroquinolin-3-yl)urea

To a mixture of Reference Example G-14 (0.065 g), water (0.032 mL), and THF (0.5 mL), potassium cyanate (0.019 g) and acetic acid (0.011 g) were added at room temperature, and the mixture was stirred at the same temperature for 1 hour. A saturated aqueous sodium hydrogen carbonate solution and water were added to the reaction mixture, and the mixture was stirred at room temperature for 10 minutes. The insoluble material was collected by filtration, and the obtained solid was washed with water and MTBE and then dried under reduced pressure to give the title compound (0.045 g).

### Example C-15

### (R)-1-(1-(5-(difluoromethyl)-2-fluorobenzyl)-2-oxo-7-(trifluoromethyl)-1,2,3,4-tetrahydroquinolin-3-yl)urea

To a mixture of Reference Example G-15 (0.039 g), water (0.018 mL), and THF (0.5 mL), potassium cyanate (0.011 g) and acetic acid (0.006 g) were added at room temperature, and the mixture was stirred at the same temperature for 14 hours. A saturated aqueous sodium hydrogen carbonate solution and water were added to the reaction mixture, and the mixture was stirred at room temperature for 10 minutes. The insoluble material was collected by filtration, and the obtained solid was washed with water and MTBE and then dried under reduced pressure to give the title compound (0.033 g).

### Example C-16

### (R)-1-(1-(5-(difluoromethyl)-2-fluorobenzyl)-7-(hydroxymethyl)-2-oxo-1,2,3,4-tetrahydroquinolin-3-yl)urea

To a mixture of Reference Example G-16 (0.050 g), water (0.026 mL), and THF (0.5 mL), potassium cyanate (0.015 g) and acetic acid (0.009 g) were added under ice cooling, and the mixture was stirred under ice cooling for 1 hour. Water and diethyl ether were added to the reaction mixture, and the insoluble material was collected by filtration. The obtained solid was washed with water and diethyl ether and then dried under reduced pressure to give the title compound (0.035 g).

### Example C-17

### (R)-1-(1-(5-(difluoromethyl)-2-fluorobenzyl)-5,7-difluoro-2-oxo-1,2,3,4-tetrahydroquinolin-3-yl)urea

To a mixture of Reference Example G-17 (0.150 g), water (0.076 mL), and THF (1.0 mL), potassium cyanate (0.044 g) and methanesulfonic acid (0.042 g) were added at room temperature, and the mixture was stirred at the same temperature for 2 hours. A saturated aqueous sodium hydrogen carbonate solution and water were added to the reaction mixture, and the mixture was stirred at room temperature for 10 minutes. The insoluble material was collected by filtration, and the obtained solid was washed with water and MTBE and then dried under reduced pressure to give the title compound (0.040 g).

### Example C-18

### (R)-1-(7-(difluoromethyl)-1-(2-fluoro-5-(trifluoromethoxy)benzyl)-2-oxo-1,2,3,4-tetrahydroquinolin-3-yl)urea

To a mixture of Reference Example G-18 (0.077 g), water (0.084 mL), and THF (0.84 mL), potassium cyanate (0.028 g) and acetic acid (0.019 g) were added at room temperature, and the mixture was stirred at the same temperature for 1 hour. Water and MTBE were added to the reaction mixture, and the insoluble material was collected by filtration. The obtained solid was washed with water and MTBE and then dried under reduced pressure to give the title compound (0.030 g).

### Example C-19

### (R)-1-(7-(difluoromethyl)-1-(2-fluoro-5-(pentafluorosulfanyl)benzyl)-2-oxo-1,2,3,4-tetrahydroquinolin-3-yl)urea

To a mixture of Reference Example G-19 (0.063 g), water (0.070 mL), and THF (0.7 mL), potassium cyanate (0.023 g) and acetic acid (0.016 g) were added at room temperature, and the mixture was stirred at the same temperature for 2 hours. Water was added to the reaction mixture, and the insoluble material was collected by filtration. The obtained solid was washed with water and MTBE and then dried under reduced pressure to give the title compound (0.026 g).

### Example C-20

### (R)-1-(7-(difluoromethyl)-1-(2-fluoro-5-(1,1,1,3,3,3-hexafluoro-2-hydroxypropan-2-yl)benzyl)-2-oxo-1,2,3,4-tetrahydroquinolin-3-yl)urea

To a mixture of Reference Example G-20 (0.036 g), water (0.050 mL), and THF (0.5 mL), potassium cyanate (0.012 g) and acetic acid (0.008 g) were added at room temperature, and the mixture was stirred at the same temperature for 19 hours. Water was added to the reaction mixture, and the mixture was extracted with DCM. The extract was concentrated under reduced pressure. The residue was purified by silica gel column chromatography (eluent: n-hexane/ethyl acetate/methanol = 40/60/0 to 0/100/0 to 0/90/10) to give the title compound (0.032 g).

### Example C-21

### (R)-1-(1-(3-chloro-2-fluoro-5-(trifluoromethyl)benzyl)-7-fluoro-2-oxo-1,2,3,4-tetrahydroquinolin-3-yl)urea

Using Reference Example G-21 instead of Reference Example G-12, Example C-21 was synthesized by the same method as in Example C-12.

### Example C-22

### (R)-1-(2-oxo-1-(3-(trifluoromethyl)benzyl)-1,2,3,4-tetrahydrothieno[3,4-b]pyridin-3-yl)urea

Using Reference Example G-22 instead of Reference Example G-14, Example C-22 was synthesized by the same method as in Example C-14.

### Example C-23

### (R)-1-(2-oxo-1-(3-(trifluoromethyl)benzyl)-1,2,3,4-tetrahydro-1,7-naphthyridin-3-yl)urea

To a mixture of Reference Example G-23 (0.049 g), methanol (0.5 mL), and THF (0.5 mL), a 4 mol/L aqueous lithium hydroxide solution (0.081 mL) was added under ice cooling, and the mixture was stirred under ice cooling for 30 minutes. A 1 mol/L hydrochloric acid (0.4 mL) was added to the reaction mixture, and the mixture was extracted with DCM. The extract was concentrated under reduced pressure.

To a mixture of the residue and THF (1.0 mL), HATU (0.062 g) and TEA (0.022 g) were added at room temperature, and the mixture was stirred at the same temperature for 1 hour. A saturated aqueous ammonium chloride solution was added to the reaction mixture, and the mixture was extracted with DCM. The extract was concentrated under reduced pressure. The residue was purified by Method A (eluent: n-hexane/ethyl acetate = 70/30 to 0/100) to give tert-butyl (R)-(2-oxo-1-(3-(trifluoromethyl)benzyl)-1,2,3,4-tetrahydro-1,7-naphthyridin-3-yl)carbamate (0.028 g).

To a mixture of the obtained compound (0.028 g) and DCM (1.0 mL), TFA (0.152 g) was added under ice cooling, and the mixture was stirred at room temperature for 2 hours. The reaction mixture was concentrated under reduced pressure. A saturated aqueous sodium hydrogen carbonate solution was added to the residue, and the mixture was extracted with DCM. The extract was concentrated under reduced pressure.

To a mixture of the residue, water (0.012 mL), and THF (1.0 mL), potassium cyanate (0.007 g) and acetic acid (0.004 g) were added at room temperature, and the mixture was stirred at the same temperature for 2 hours. A saturated aqueous sodium hydrogen carbonate solution and water were added to the reaction mixture, and the mixture was stirred at room temperature for 10 minutes. The insoluble material was collected by filtration, and the obtained solid was washed with water and MTBE and then dried under reduced pressure to give the title compound (0.010 g).

### Example C-24

### 1-((3R)-7-fluoro-1-(1-(2-fluoro-5-(trifluoromethyl)phenyl)ethyl)-2-oxo-1,2,3,4-tetrahydroquinolin-3-yl)urea

Using Reference Example G-24 instead of Reference Example G-20, Example C-24 was synthesized by the same method as in Example C-20.

### Example C-25

### (R)-1-(2-oxo-1-phenethyl-1,2,3,4-tetrahydroquinolin-3-yl)urea

Using Reference Example G-25 instead of Reference Example G-12, Example C-25 was synthesized by the same method as in Example C-12.

### Example C-26

### (R)-1-(2-oxo-1-(3-phenylpropyl)-1,2,3,4-tetrahydroquinolin-3-yl)urea

Using Reference Example G-26 instead of Reference Example G-2, Example C-26 was synthesized by the same method as in Example C-2.

### Example C-27

### (R)-1-(1-(cyclohexylmethyl)-2-oxo-1,2,3,4-tetrahydroquinolin-3-yl)urea

Using Reference Example G-27 instead of Reference Example G-2, Example C-27 was synthesized by the same method as in Example C-2.

### Example C-28

### (R)-1-(1-(5-(difluoromethyl)-2-fluorobenzyl)-2-oxo-7-(trifluoromethyl)-1,2,3,4-tetrahydroquinolin-3-yl)-3-methylurea

To a mixture of Reference Example G-15 (0.030 g) and DCM (1.0 mL), triphosgene (0.023 g) and TEA (0.023 g) were added under ice cooling, and the mixture was stirred at room temperature for 30 minutes. Methylamine (2 mol/L in THF) (0.058 mL) was added to the reaction mixture under ice cooling, and the mixture was stirred at room temperature for 1 hour. Methylamine (2 mol/L in THF) (0.773 mL) was added to the reaction mixture at room temperature, and the mixture was stirred at the same temperature for 10 minutes. A saturated aqueous sodium hydrogen carbonate solution and water were added to the reaction mixture, and the mixture was extracted with DCM. The extract was concentrated under reduced pressure. The residue was suspended in MTBE, and the insoluble material was collected by filtration. The obtained solid was washed with MTBE and then dried under reduced pressure to give the title compound (0.025 g).

### Example C-29

### (R)-1-(2-fluoro-5-(trifluoromethyl)benzyl)-2-oxo-3-ureido-1,2,3,4-tetrahydroquinolin-7-carboxylic acid

Under an argon atmosphere, to a mixture of methyl (S)-2-((tert-butoxycarbonyl)amino)-3-iodopropanoate (2.15 g) and DMF (7.8 mL), zinc powder (0.625 g) was added at room temperature, and the mixture was stirred at the same temperature for 1 hour. A mixture of methyl 3-amino-4-bromobenzoate (1.00 g) and DMF (3.9 mL), palladium(II) acetate (0.049 g), and Xphos (0.207 g) were added to the reaction mixture at room temperature, and the mixture was stirred at 40°C for 5 hours. Acetic acid (0.783 g) was added to the reaction mixture, and the mixture was stirred at 70°C for 1 hour. After allowing the reaction mixture to cool to room temperature, water and n-hexane/ethyl acetate (1/1) were added, and the mixture was stirred at room temperature for 10 minutes. The insoluble material was removed by filtration, and the organic layer of the filtrate was separated. The organic layer was washed with water and a saturated brine and then concentrated under reduced pressure. The residue was suspended in n-hexane/ethyl acetate (2/1), and the insoluble material was collected by filtration. The obtained solid was washed with water and n-hexane and then dried under reduced pressure to give methyl (R)-3-((tert-butoxycarbonyl)amino)-2-oxo-1,2,3,4-tetrahydroquinolin-7-carboxylate (0.619 g).

To a mixture of the obtained compound (0.300 g) and DMF (3.0 mL), 2-(bromomethyl)-1-fluoro-4-(trifluoromethyl)benzene (0.265 g) and potassium carbonate (0.194 g) were added under ice cooling, and the mixture was stirred under ice cooling for 30 minutes and at room temperature for 6 hours. A saturated aqueous ammonium chloride solution and water were added to the reaction mixture under ice cooling, and the mixture was extracted with n-hexane/ethyl acetate (1/1). The extract was washed with water and saturated brine, dried over anhydrous magnesium sulfate, and concentrated under reduced pressure. The residue was suspended in n-hexane, and the insoluble material was collected by filtration. The obtained solid was washed with n-hexane and then dried under reduced pressure to give methyl (R)-3-((tert-butoxycarbonyl)amino)-1-(2-fluoro-5-(trifluoromethyl)benzyl)-2-oxo-1,2,3,4-tetrahydroquinolin-7-carboxylate (0.390 g).

To a mixture of the obtained compound (0.206 g) and DCM (2.0 mL), TFA (0.946 g) was added at room temperature, and the mixture was stirred at the same temperature for 45 minutes. A 5 mol/L aqueous sodium hydroxide solution (1.66 mL) was added to the reaction mixture under ice cooling, and the mixture was extracted with DCM. The extract was concentrated under reduced pressure.

To a mixture of the residue, water (0.2 mL), and THF (2.0 mL), potassium cyanate (0.037 g) and acetic acid (0.026 g) were added under ice cooling, and the mixture was stirred under ice cooling for 45 minutes. THF (2.0 mL) was added to the reaction mixture, and the mixture was stirred under ice cooling for 2 hours and at room temperature for 65 hours. Water was added to the reaction mixture, and the insoluble material was collected by filtration. The obtained solid was washed with water and then dried under reduced pressure to give methyl (R)-1-(2-fluoro-5-(trifluoromethyl)benzyl)-2-oxo-3-ureido-1,2,3,4-tetrahydroquinolin-7-carboxylate (0.173 g).

To a mixture of the obtained compound (0.051 g), methanol (0.5 mL), and THF (0.5 mL), a 4 mol/L aqueous lithium hydroxide solution (0.116 mL) was added under ice cooling, and the mixture was stirred at room temperature for 50 minutes and at 50°C for 1 hour. After allowing the reaction mixture to cool to room temperature, a 1 mol/L hydrochloric acid (0.465 mL) and water were added, and the insoluble material was collected by filtration. The obtained solid was washed with water and then dried under reduced pressure to give the title compound (0.036 g).

### Example C-30

### (R)-1-(2-fluoro-5-(trifluoromethyl)benzyl)-2-oxo-3-ureido-1,2,3,4-tetrahydroquinolin-7-carboxamide

To a mixture of Example C-29 (0.030 g), ammonium chloride (0.038 g), HATU (0.032 g), and MeCN (2.0 mL), TEA (0.093 g) was added, and the mixture was stirred at room temperature for 1 hour. A saturated aqueous sodium hydrogen carbonate solution and MTBE were added to the reaction mixture, and the insoluble material was collected by filtration. The obtained solid was washed with water and MTBE and then dried under reduced pressure to give the title compound (0.018 g).

### Example D-1

### 1-((3R,6S)-1-(5-allyl-2-fluorobenzyl)-2-oxo-6-(trifluoromethyl)piperidin-3-yl)urea

To a mixture of Reference Example J-2 (0.127 g) and THF (2.0 mL), LDA (1.07 mol/L in THF/n-hexane) (0.565 mL) was added at -78°C, and the mixture was stirred at the same temperature for 15 minutes. DPPA (0.166 g) was added to the reaction mixture at - 78°C, and the mixture was stirred at the same temperature for 75 minutes. A mixture of Boc2O (0.105 g) and THF (1.0 mL) was added to the reaction mixture at -78°C, and the mixture was stirred at the same temperature for 10 minutes and under ice cooling for 1 hour. Water was added to the reaction mixture, and the mixture was stirred at 50°C for 1 hour. After allowing the reaction mixture to cool to room temperature, a saturated aqueous ammonium chloride solution was added, and the mixture was extracted with DCM. The extract was concentrated under reduced pressure. The residue was purified by silica gel column chromatography (eluent: n-hexane/ethyl acetate = 95/5 to 70/30) to give tert-butyl ((3R,6S)-1-(5-allyl-2-fluorobenzyl)-2-oxo-6-(trifluoromethyl)piperidin-3-yl)carbamate (0.129 g).

To a mixture of the obtained compound (0.128 g) and DCM (1.3 mL), TFA (0.678 g) was added at room temperature, and the mixture was stirred at the same temperature for 1 hour. A 5 mol/L aqueous sodium hydroxide solution (1.19 mL) was added to the reaction mixture under ice cooling, and the mixture was extracted with DCM. The extract was concentrated under reduced pressure.

To a mixture of the residue, water (0.130 mL), and THF (1.3 mL), potassium cyanate (0.032 g) and acetic acid (0.019 g) were added under ice cooling, and the mixture was stirred at room temperature for 21 hours. Water was added to the reaction mixture, and the mixture was extracted with DCM. The extract was concentrated under reduced pressure. The residue was purified by silica gel column chromatography (eluent: n-hexane/ethyl acetate/methanol = 40/60/0 to 0/100/0 to 0/95/5) to give the title compound (0.082 g).

### Example D-2

### 1-((3R,6S)-1-(5-(2-cyclopropylethyl)-2-fluorobenzyl)-2-oxo-6-(trifluoromethyl)piperidin-3-yl)urea

To a mixture of Reference Example J-3 (0.137 g) and THF (2.0 mL), LDA (1.07 mol/L in THF/n-hexane) (0.566 mL) was added at -78°C, and the mixture was stirred at the same temperature for 15 minutes. DPPA (0.167 g) was added to the reaction mixture at - 78°C, and the mixture was stirred at the same temperature for 75 minutes. A mixture of Boc₂O (0.106 g) and THF (1.0 mL) was added to the reaction mixture at -78°C, and the mixture was stirred at the same temperature for 10 minutes and under ice cooling for 1 hour. Water was added to the reaction mixture, and the mixture was stirred at 50°C for 1 hour. After allowing the reaction mixture to cool to room temperature, a saturated aqueous ammonium chloride solution was added, and the mixture was extracted with DCM. The extract was concentrated under reduced pressure. The residue was purified by silica gel column chromatography (eluent: n-hexane/ethyl acetate = 95/5 to 70/30) to give tert-butyl ((3R,6S)-1-(5-(cyclopropylethynyl)-2-fluorobenzyl)-2-oxo-6-(trifluoromethyl)piperidin-3-yl)carbamate (0.098 g).

To a mixture of the obtained compound (0.097 g) and DCM (0.97 mL), TFA (0.487 g) was added at room temperature, and the mixture was stirred at the same temperature for 1 hour. A 5 mol/L aqueous sodium hydroxide solution (0.854 mL) was added to the reaction mixture under ice cooling, and the mixture was extracted with DCM. The extract was concentrated under reduced pressure.

To a mixture of the residue, water (0.097 mL), and THF (0.97 mL), potassium cyanate (0.023 g) and acetic acid (0.013 g) were added under ice cooling, and the mixture was stirred at room temperature for 21 hours. Water was added to the reaction mixture, and the mixture was extracted with DCM. The extract was concentrated under reduced pressure. The residue was sequentially purified by silica gel column chromatography (eluent: n-hexane/ethyl acetate/methanol = 40/60/0 to 0/100/0 to 0/95/5) and Method A (eluent: DCM/methanol = 100/0 to 90/10) to give 1-((3R,6S)-1-(5-(cyclopropylethynyl)-2-fluorobenzyl)-2-oxo-6-(trifluoromethyl)piperidin-3-yl)urea (0.008 g).

A mixture of the obtained compound (0.007 g), 10% Pd/C (0.002 g), THF (0.5 mL), and methanol (0.5 mL) was stirred under a hydrogen atmosphere at room temperature for 2 hours. The reaction mixture was filtered through Celite, and the filtrate was concentrated under reduced pressure. The residue was purified by Method A (eluent: ethyl acetate/methanol = 100/0 to 90/10) to give the title compound (0.004 g).

### Example D-3

### 1-((3S,6R,8aR)-5-oxo-3-(2-(trifluoromethyl)benzyl)octahydroindolizin-6-yl)urea

A mixture of Reference Example J-6 (0.072 g), (1,5-cyclooctadiene)(pyridine)(tricyclohexylphosphine)iridium(I) hexafluorophosphate (0.007 g), and DCM (2.0 mL) was stirred under hydrogen pressure (0.4 MPa) at room temperature for 3 hours. The reaction mixture was purified by silica gel column chromatography (eluent: n-hexane/ethyl acetate = 80/20 to 50/50) to give tert-butyl (2R,5S)-2-(3-(((benzyloxy)carbonyl)amino)-4-methoxy-4-oxobutyl)-5-(2-(trifluoromethyl)benzyl)pyrrolidine-1-carboxylate (0.072 g).

A mixture of the obtained compound (0.072 g), a 4 mol/L aqueous lithium hydroxide solution (0.156 mL), water (0.156 mL), and methanol (2.0 mL) was stirred at room temperature for 1 hour. 2 mol/L hydrochloric acid was added to the reaction mixture, and the mixture was extracted with ethyl acetate. The extract was dried over anhydrous sodium sulfate and concentrated under reduced pressure.

A mixture of the residue and hydrogen chloride (4 mol/L in 1,4-dioxane) (2.00 mL) was stirred at room temperature for 30 minutes. The reaction mixture was concentrated under reduced pressure.

To a mixture of the residue, DIEPA (0.065 g), and DMF (2.0 mL), HATU (0.052 g) was added at room temperature, and the mixture was stirred at the same temperature for 15 minutes. Water was added to the reaction mixture, and the mixture was extracted with ethyl acetate. The extract was washed with water and saturated brine, dried over anhydrous sodium sulfate, and concentrated under reduced pressure. The residue was purified by silica gel column chromatography (eluent: n-hexane/ethyl acetate = 80/20 to 60/40) to give benzyl ((3S,6R,8aR)-5-oxo-3-(2-(trifluoromethyl)benzyl)octahydroindolizin-6-yl)carbamate (0.020 g).

A mixture of the obtained compound (0.020 g), 10% Pd/C (0.006 g), and THF (2.0 mL) was stirred under a hydrogen atmosphere at room temperature for 1 hour. The reaction mixture was filtered through Celite, and the filtrate was concentrated under reduced pressure.

Under an argon atmosphere, potassium cyanate (0.005 g) and acetic acid (0.005 g) were added to a mixture of the residue, water (0.016 g), and THF (1.0 mL) at room temperature, and the mixture was stirred at the same temperature for 3 hours. Water was added to the reaction mixture, and the mixture was extracted with DCM. The extract was concentrated under reduced pressure. The residue was purified by silica gel column chromatography (eluent: ethyl acetate/methanol = 98/2 to 80/20) to give the title compound (0.013 g).

### Example D-4

### 1-((3R,6S)-1-(2-fluoro-5-(trifluoromethyl)benzyl)-6-(hydroxymethyl)-2-oxopiperidin-3-yl)urea

To a mixture of Reference Example J-7 (0.150 g) and THF (2.5 mL), LDA (1.07 mol/L in THF/n-hexane) (0.387 mL) was added at -78°C, and the mixture was stirred at the same temperature for 20 minutes. DPPA (0.114 g) was added to the reaction mixture at - 78°C, and the mixture was stirred at the same temperature for 45 minutes. A mixture of Boc₂O (0.072 g) and THF (1.3 mL) was added to the reaction mixture at -78°C, and the mixture was stirred at the same temperature for 10 minutes and then at room temperature for 3 hours. Water was added to the reaction mixture, and the mixture was stirred at 50°C for 1 hour. After allowing the reaction mixture to cool to room temperature, a saturated aqueous ammonium chloride solution was added, and the mixture was extracted with ethyl acetate. The extract was dried over anhydrous magnesium sulfate and concentrated under reduced pressure. The residue was purified by silica gel column chromatography (eluent: n-hexane/ethyl acetate = 100/0 to 80/20) to give tert-butyl ((3R,6S)-6-(((tert-butyldiphenylsilyl)oxy)methyl)-1-(2-fluoro-5-(trifluoromethyl)benzyl)-2-oxopiperidin-3-yl)carbamate (0.076 g).

A mixture of the obtained compound (0.074 g) and DCM (1.0 mL) was treated with TFA (0.256 g) at room temperature and stirred at the same temperature for 20 minutes. A 5 mol/L aqueous sodium hydroxide solution was added to the reaction mixture under ice cooling, and the mixture was extracted with DCM. The extract was concentrated under reduced pressure.

To a mixture of the residue, water (0.050 mL), and THF (0.5 mL), potassium cyanate (0.010 g) and acetic acid (0.007 g) were added at room temperature, and the mixture was stirred at the same temperature for 19 hours. Water was added to the reaction mixture, and the mixture was extracted with DCM. The extract was concentrated under reduced pressure.

A mixture of the residue, TBAF (1 mol/L in THF) (0.135 mL), and THF (1.0 mL) was stirred at room temperature for 2 hours. The reaction mixture was concentrated under reduced pressure. The residue was sequentially purified by silica gel column chromatography (eluent: n-hexane/ethyl acetate/methanol = 100/0/0 to 0/100/0 to 0/70/30) and PLC (developing solvent: ethyl acetate/methanol = 90/10) to give the title compound (0.001 g).

### Example D-5

### 1-((3R,6S)-1-(2-fluoro-5-(trifluoromethyl)benzyl)-2-oxo-6-(pyrrolidin-1-ylmethyl)piperidin-3-yl)urea

To a mixture of Reference Example J-7 (0.189 g) and THF (1.5 mL), LDA (1.07 mol/L in THF/n-hexane) (0.650 mL) was added at -78°C, and the mixture was stirred at the same temperature for 20 minutes. DPPA (0.191 g) was added to the reaction mixture at - 78°C, and the mixture was stirred at the same temperature for 45 minutes. A mixture of Boc₂O (0.091 g) and THF (1.0 mL) was added to the reaction mixture at -78°C, and the mixture was stirred at the same temperature for 10 minutes and under ice cooling for 1 hour. Water was added to the reaction mixture, and the mixture was stirred at 50°C for 1 hour. After allowing the reaction mixture to cool to room temperature, a saturated aqueous ammonium chloride solution was added, and the mixture was extracted with DCM. The extract was concentrated under reduced pressure. The residue was purified by silica gel column chromatography (eluent: n-hexane/ethyl acetate = 95/5 to 60/40) to give tert-butyl ((3R,6S)-6-(((tert-butyldiphenylsilyl)oxy)methyl)-1-(2-fluoro-5-(trifluoromethyl)benzyl)-2-oxopiperidin-3-yl)carbamate (0.184 g).

To a mixture of the obtained compound (0.184 g) and THF (3.0 mL), TBAF (1 mol/L in THF) (0.559 mL) was added at room temperature, and the mixture was stirred at the same temperature for 30 minutes. Water and a saturated aqueous ammonium chloride solution were added to the reaction mixture, and the mixture was extracted with DCM. The extract was concentrated under reduced pressure. The residue was purified by APS column chromatography (eluent: n-hexane/ethyl acetate = 70/30 to 50/50) to give tert-butyl ((3R,6S)-1-(2-fluoro-5-(trifluoromethyl)benzyl)-6-(hydroxymethyl)-2-oxopiperidin-3-yl)carbamate (0.081 g).

To a mixture of the obtained compound (0.033 g), TEA (0.028 g), and DCM (1.0 mL), methanesulfonyl chloride (0.018 g) was added under ice cooling, and the mixture was stirred at room temperature for 20 minutes. Water and a saturated aqueous sodium hydrogen carbonate solution were added to the reaction mixture, and the mixture was extracted with DCM. The extract was concentrated under reduced pressure.

To a mixture of the residue, pyrrolidine (0.140 g), TBAI (0.029 g), and DMF (1.0 mL), potassium carbonate (0.054 g) was added, and the mixture was stirred at 75°C for 2.5 hours. After allowing the reaction mixture to cool to room temperature, water and a saturated aqueous ammonium chloride solution were added, and the mixture was extracted with n-hexane/ethyl acetate (1/1). The extract was washed with saturated brine, dried over anhydrous sodium sulfate, and concentrated under reduced pressure. The residue was purified by silica gel column chromatography (eluent: n-hexane/ethyl acetate = 88/12 to 0/100) to give tert-butyl ((3R,6S)-1-(2-fluoro-5-(trifluoromethyl)benzyl)-2-oxo-6-(pyrrolidin-1-ylmethyl)piperidin-3-yl)carbamate (0.012 g).

To a mixture of the obtained compound (0.012 g) and DCM (1.0 mL), TFA (0.296 g) was added, and the mixture was stirred at room temperature for 20 minutes. Water and a saturated aqueous sodium hydrogen carbonate solution were added to the reaction mixture, and the mixture was extracted with DCM. The extract was concentrated under reduced pressure.

To a mixture of the residue, water (0.009 mL), and THF (1.0 mL), potassium cyanate (0.003 g) and acetic acid (0.002 g) were added, and the mixture was stirred at room temperature for 2 hours and at 40°C for 30 minutes. Potassium cyanate (0.001 g) was added to the reaction mixture, and the mixture was stirred at 50°C for 1 hour. After allowing the reaction mixture to cool to room temperature, water was added, and the mixture was extracted with DCM. The extract was concentrated under reduced pressure. The extract was concentrated under reduced pressure. The residue was purified by silica gel column chromatography (eluent: ethyl acetate/methanol = 100/0 to 80/20) to give the title compound (0.003 g).

### Example D-6

### 1-((3R,6S)-1-(2-fluoro-5-(trifluoromethyl)benzyl)-6-(methoxymethyl)-2-oxopiperidin-3-yl)urea

To a mixture of Reference Example J-10 (0.097 g) and THF (1.0 mL), LDA (1.07 mol/L in THF/n-hexane) (0.408 mL) was added at -78°C, and the mixture was stirred at the same temperature for 10 minutes. DPPA (0.120 g) was added to the reaction mixture at - 78°C, and the mixture was stirred at the same temperature for 45 minutes. A mixture of Boc₂O (0.067 g) and THF (0.5 mL) was added to the reaction mixture at -78°C, and the mixture was stirred at the same temperature for 10 minutes and under ice cooling for 1 hour. Water was added to the reaction mixture, and the mixture was stirred at 50°C for 1 hour. Acetic acid (0.25 mL) and 2 mol/L hydrochloric acid (0.321 mL) were added to the reaction mixture at room temperature, and the mixture was stirred at the same temperature for 30 minutes. A saturated aqueous sodium hydrogen carbonate solution was added to the reaction mixture, and the mixture was extracted with DCM. The extract was concentrated under reduced pressure. The residue was purified by silica gel column chromatography (eluent: n-hexane/ethyl acetate = 18/82 to 0/100) to give tert-butyl ((3R,6S)-1-(2-fluoro-5-(trifluoromethyl)benzyl)-6-(hydroxymethyl)-2-oxopiperidin-3-yl)carbamate (0.021 g).

A mixture of the obtained compound (0.021 g), methyl iodide (0.014 g), and DMF (0.5 mL) was treated with sodium hydride (approximately 60%) (0.003 g) at room temperature, and the mixture was stirred at the same temperature for 1 hour. Water and a saturated aqueous ammonium chloride solution were added to the reaction mixture, and the mixture was extracted with n-hexane/ethyl acetate (1/1). The extract was washed with a saturated brine, dried over anhydrous sodium sulfate, and concentrated under reduced pressure. The residue was purified by silica gel column chromatography (eluent: n-hexane/ethyl acetate = 88/12 to 0/100) to give tert-butyl ((3R,6S)-1-(2-fluoro-5-(trifluoromethyl)benzyl)-6-(methoxymethyl)-2-oxopiperidin-3-yl)carbamate (0.009 g).

To a mixture of the obtained compound (0.009 g) and DCM (1.0 mL), TFA (0.296 g) was added, and the mixture was stirred at room temperature for 30 minutes. The reaction mixture was concentrated under reduced pressure.

To a mixture of the residue, water (0.008 mL), and THF (1.0 mL), potassium cyanate (0.002 g) and acetic acid (0.003 g) were added, and the mixture was stirred at room temperature for 1.5 hours. Water and a saturated aqueous sodium hydrogen carbonate solution were added to the reaction mixture, and the mixture was extracted with DCM. The extract was concentrated under reduced pressure. The residue was purified by silica gel column chromatography (eluent: ethyl acetate/methanol = 100/0 to 80/20) to give the title compound (0.001 g).

### Example D-7

### 1-((3R,6S)-6-(ethoxymethyl)-1-(2-fluoro-5-(trifluoromethyl)benzyl)-2-oxopiperidin-3-yl)urea

To a mixture of Reference Example J-11 (0.897 g) and THF (15 mL), TBAF (1 mol/L in THF) (4.85 mL) was added, and the mixture was stirred at room temperature for 20 minutes. A saturated aqueous ammonium chloride solution and water were added to the reaction mixture, and the mixture was extracted with ethyl acetate. The extract was dried over anhydrous sodium sulfate and concentrated under reduced pressure. The residue was sequentially purified by silica gel column chromatography (eluent: n-hexane/ethyl acetate = 95/5 to 60/40) and silica gel column chromatography (eluent: n-hexane/ethyl acetate = 70/30 to 30/70) to give tert-butyl ((3R,6S)-1-(2-fluoro-5-(trifluoromethyl)benzyl)-6-(hydroxymethyl)-2-oxopiperidin-3-yl)carbamate (0.308 g).

A mixture of the obtained compound (0.025 g), ethyl iodide (0.012 g), and DMF (0.5 mL) was treated with sodium hydride (approximately 60%) (0.003 g) at room temperature, and the mixture was stirred at the same temperature for 30 minutes. Ethyl iodide (0.005 g) was added to the reaction mixture, and the mixture was stirred at room temperature for 30 minutes. Sodium hydride (approximately 60%) (0.003 g) was added to the reaction mixture, and the mixture was stirred at room temperature for 30 minutes. Water and a saturated aqueous ammonium chloride solution were added to the reaction mixture, and the mixture was extracted with n-hexane/ethyl acetate (1/1). The extract was washed with a saturated brine, dried over anhydrous sodium sulfate, and concentrated under reduced pressure. The residue was purified by silica gel column chromatography (eluent: n-hexane/ethyl acetate = 88/12 to 0/100) to give tert-butyl ((6S)-6-(ethoxymethyl)-1-(2-fluoro-5-(trifluoromethyl)benzyl)-2-oxopiperidin-3-yl)carbamate (0.008 g).

To a mixture of the obtained compound (0.008 g) and DCM (1.0 mL), TFA (0.296 g) was added, and the mixture was stirred at room temperature for 20 minutes. The reaction mixture was concentrated under reduced pressure.

To a mixture of the residue, water (0.006 mL), and THF (1.0 mL), potassium cyanate (0.002 g) and acetic acid (0.002 g) were added, and the mixture was stirred at room temperature for 1.5 hours and at 50°C for 2 hours. After allowing the reaction mixture to cool to room temperature, water and a saturated aqueous sodium hydrogen carbonate solution were added, and the mixture was extracted with DCM. The extract was concentrated under reduced pressure. The residue was purified by silica gel column chromatography (eluent: ethyl acetate/methanol = 100/0 to 80/20) to give the title compound (0.001 g).

### Example D-8

### N-(((2S,5R)-1-(2-fluoro-5-(trifluoromethyl)benzyl)-6-oxo-5-ureidopiperidin-2-yl)methyl)acetamide

A mixture of Reference Example J-12 (0.020 g), 10% Pd/C (0.020 g), and ethyl acetate (2.0 mL) was stirred under a hydrogen atmosphere under ice cooling for 30 minutes and at room temperature for 2 hours. The reaction mixture was filtered through Celite, and the insoluble material was washed with ethyl acetate (2.0 mL). DIPEA (0.035 g) and acetyl chloride (0.017 g) were added to the filtrate under ice cooling, and the mixture was stirred at room temperature for 15 minutes. Water was added to the reaction mixture, and the mixture was extracted with DCM. The extract was concentrated under reduced pressure. The residue was purified by silica gel column chromatography (eluent: n-hexane/ethyl acetate/methanol = 70/30/0 to 0/100/0 to 0/80/20) to give tert-butyl ((3R,6S)-6-(acetamidomethyl)-1-(2-fluoro-5-(trifluoromethyl)benzyl)-2-oxopiperidin-3-yl)carbamate (0.019 g).

A mixture of the obtained compound (0.019 g), hydrogen chloride (4 mol/L in 1,4-dioxane) (1.5 mL), and methanol (1.5 mL) was stirred at room temperature for 40 minutes. The reaction mixture was concentrated under reduced pressure. A saturated aqueous sodium hydrogen carbonate solution was added to the residue, and the mixture was extracted with DCM. The extract was dried over anhydrous magnesium sulfate and concentrated under reduced pressure.

To a mixture of the residue, water (0.015 mL), and THF (1.0 mL), potassium cyanate (0.005 g) and acetic acid (0.005 g) were added at room temperature, and the mixture was stirred at the same temperature for 3 hours. Water was added to the reaction mixture, and the mixture was extracted with DCM 10 times. The combined extracts were concentrated under reduced pressure. The residue was purified by ODS column chromatography (eluent: water/MeCN = 90/10 to 10/90) to give the title compound (0.005 g).

### Example D-9

### 1-((3R,6S)-6-((dimethylamino)methyl)-1-(2-fluoro-5-(trifluoromethyl)benzyl)-2-oxopiperidin-3-yl)urea

To a mixture of Reference Example J-11 (0.897 g) and THF (15 mL), TBAF (1 mol/L in THF) (4.85 mL) was added, and the mixture was stirred at room temperature for 20 minutes. A saturated aqueous ammonium chloride solution and water were added to the reaction mixture, and the mixture was extracted with ethyl acetate. The extract was dried over anhydrous sodium sulfate and concentrated under reduced pressure. The residue was sequentially purified by silica gel column chromatography (eluent: n-hexane/ethyl acetate = 95/5 to 60/40) and silica gel column chromatography (eluent: n-hexane/ethyl acetate = 70/30 to 30/70) to give tert-butyl ((3R,6S)-1-(2-fluoro-5-(trifluoromethyl)benzyl)-6-(hydroxymethyl)-2-oxopiperidin-3-yl)carbamate (0.308 g).

Under an argon atmosphere, DMSO (0.028 g) was added at -78°C to a mixture of oxalyl chloride (0.023 g) and DCM (1.0 mL), and the mixture was stirred at the same temperature for 1 hour. A mixture of the above tert-butyl ((3R,6S)-1-(2-fluoro-5-(trifluoromethyl)benzyl)-6-(hydroxymethyl)-2-oxopiperidin-3-yl)carbamate (0.050 g) and DCM (0.3 mL) was added to the reaction mixture at -78°C, and the mixture was stirred at the same temperature for 30 minutes. TEA (0.060 g) was added to the reaction mixture at - 78°C, and the mixture was stirred at room temperature for 30 minutes. The reaction mixture was concentrated under reduced pressure.

To a mixture of the residue, dimethylamine (2 mol/L in THF) (0.178 mL), and DCM (2.0 mL), sodium triacetoxyborohydride (0.076 g) was added, and the mixture was stirred at room temperature for 1 hour. Water and a saturated aqueous sodium hydrogen carbonate solution were added to the reaction mixture, and the mixture was extracted with DCM. The extract was concentrated under reduced pressure. The residue was purified by APS column chromatography (eluent: n-hexane/ethyl acetate = 88/12 to 0/100) to give tert-butyl ((3R,6S)-6-((dimethylamino)methyl)-1-(2-fluoro-5-(trifluoromethyl)benzyl)-2-oxopiperidin-3-yl)carbamate (0.024 g).

A mixture of the obtained compound (0.024 g) and DCM (1.0 mL) was added with TFA (0.296 g) and stirred at room temperature for 20 minutes. The reaction mixture was concentrated under reduced pressure.

To a mixture of the residue, water (0.019 mL), and THF (1.0 mL), potassium cyanate (0.009 g) and acetic acid (0.006 g) were added, and the mixture was stirred at room temperature for 2.5 hours. Water and a saturated aqueous sodium hydrogen carbonate solution were added to the reaction mixture, and the mixture was extracted with DCM. The extract was concentrated under reduced pressure. The residue was purified by APS column chromatography (eluent: ethyl acetate/methanol = 100/0 to 90/10) to give the title compound (0.007 g).

### Example D-10

### 2-((2S^{*},5R^{*})-1-(2-fluoro-5-(trifluoromethyl)benzyl)-6-oxo-5-ureidopiperidin-2-yl)-N,N-dimethylacetamide

Under an argon atmosphere, to a mixture of Reference Example J-16 (0.384 g) and THF (4.5 mL), TBAF (1 mol/L in THF) (1.14 mL) was added under ice cooling, and the mixture was stirred at room temperature for 20 minutes. The reaction mixture was concentrated under reduced pressure. The residue was purified by silica gel column chromatography (eluent: n-hexane/ethyl acetate = 70/20 to 30/70) to give tert-butyl ((3R^{*},6S^{*})-1-(2-fluoro-5-(trifluoromethyl)benzyl)-6-(2-hydroxyethyl)-2-oxopiperidin-3-yl)carbamate (0.153 g).

Under an argon atmosphere, DMSO (0.053 g) was added at -78°C to a mixture of oxalyl chloride (0.043 g) and DCM (1.0 mL), and the mixture was stirred at the same temperature for 20 minutes. A mixture of the above tert-butyl ((3R*,6S*)-1-(2-fluoro-5-(trifluoromethyl)benzyl)-6-(2-hydroxyethyl)-2-oxopiperidin-3-yl)carbamate (0.098 g) and DCM (1.0 mL) was added to the reaction mixture at -78°C, and the mixture was stirred at the same temperature for 30 minutes. TEA (0.114 g) was added to the reaction mixture at - 78°C, and the mixture was stirred at room temperature for 40 minutes. Water was added to the reaction mixture, and the mixture was extracted with DCM. The extract was concentrated under reduced pressure.

Under an argon atmosphere, a mixture of the residue, 2-methyl-2-butene (0.15 mL), and tert-butanol (1.5 mL) was added with a mixture of sodium chlorite (0.127 g), sodium dihydrogen phosphate (0.135 g), and water (0.375 mL) at room temperature, and the mixture was stirred at the same temperature for 30 minutes. 2 mol/L hydrochloric acid was added to the reaction mixture, and the mixture was extracted with DCM. The extract was dried over anhydrous sodium sulfate and concentrated under reduced pressure to give 2-((2S^{*},5R^{*})-5-((tert-butoxycarbonyl)amino)-1-(2-fluoro-5-(trifluoromethyl)benzyl)-6-oxopiperidin-2-yl)acetic acid (0.101 g).

Under an argon atmosphere, to a mixture of the obtained compound (0.034 g), dimethylamine hydrochloride (0.018 g), DIEPA (0.048 g), and MeCN (1.0 mL), HATU (0.031 g) was added at room temperature, and the mixture was stirred at the same temperature for 45 minutes. Water was added to the reaction mixture, and the mixture was extracted with DCM. The extract was concentrated under reduced pressure. The residue was purified by silica gel column chromatography (eluent: n-hexane/ethyl acetate = 60/40 to 20/80) to give tert-butyl ((3R^{*},6S^{*})-6-(2-(dimethylamino)-2-oxoethyl)-1-(2-fluoro-5-(trifluoromethyl)benzyl)-2-oxopiperidin-3-yl)carbamate (0.028 g).

Under an argon atmosphere, to a mixture of the obtained compound (0.028 g) and DCM (1.0 mL), TFA (0.198 g) was added at room temperature, and the mixture was stirred at the same temperature for 30 minutes. A 5 mol/L aqueous sodium hydroxide solution was added to the reaction mixture under ice cooling, and the mixture was extracted with DCM. The extract was concentrated under reduced pressure.

To a mixture of the residue, water (0.021 mL), and THF (1.0 mL), potassium cyanate (0.006 g) and acetic acid (0.004 g) were added at room temperature, and the mixture was stirred at the same temperature for 12 hours. Water was added to the reaction mixture, and the mixture was extracted with DCM/2-propanol (1/1). The extract was concentrated under reduced pressure. The residue was purified by silica gel column chromatography (eluent: n-hexane/ethyl acetate/methanol = 30/70/0 to 0/100/0 to 85/15) to give the title compound (0.011 g).

### Example E-1

### 1-(1-(3-chloro-2-fluoro-5-(trifluoromethyl)benzyl)-6-(hydroxymethyl)-2-oxo-1,2-dihydropyridin-3-yl)urea

To a mixture of Example B-5 (0.143 g) and 1,4-dioxane (5.0 mL), selenium dioxide (IV) (0.168 g) was added, and the mixture was stirred at 80°C for 6 hours under microwave irradiation. The reaction mixture was filtered through Celite, and the filtrate was concentrated under reduced pressure. The residue was sequentially purified by APS column chromatography (eluent: n-hexane/ethyl acetate/methanol = 100/0/0 to 0/100/0 to 0/90/10) and ODS column chromatography (eluent: water/MeCN = 90/10 to 10/90) to give the title compound (0.025 g).

### Example E-2

### 1-(1-(3-chloro-2-fluoro-5-(trifluoromethyl)benzyl)-6-(methoxymethyl)-2-oxo-1,2-dihydropyridin-3-yl)urea

Under an argon atmosphere, to a mixture of Example E-1 (0.023 g) and DCM (1.0 mL), phosphorus tribromide (0.019 g) was added under ice cooling, and the mixture was stirred for 2.5 hours under ice cooling and for 2 hours at room temperature. Phosphorus tribromide (0.006 g) was added to the reaction mixture under ice cooling, and the mixture was stirred at room temperature for 30 minutes. Methanol was added to the reaction mixture under ice cooling, and the mixture was concentrated under reduced pressure.

Under an argon atmosphere, to a mixture of the residue and methanol (1.0 mL), sodium methoxide (0.010 g) was added at room temperature, and the mixture was stirred at the same temperature for 30 minutes. Water was added to the reaction mixture, and the mixture was extracted with DCM. The extract was dried over anhydrous sodium sulfate and concentrated under reduced pressure. The residue was purified by silica gel column chromatography (eluent: ethyl acetate/methanol = 100/0 to 95/5) to give the title compound (0.014 g).

### Example E-3

### 1-(1-(3-chloro-2-fluoro-5-(trifluoromethyl)benzyl)-6-((dimethylamino)methyl)-2-oxo-1,2-dihydropyridin-3-yl)urea

Under an argon atmosphere, to a mixture of Example E-1 (0.110 g) and DCM (3.0 mL), phosphorus tribromide (0.121 g) was added under ice cooling, and the mixture was stirred under ice cooling for 12 hours. Methanol was added to the reaction mixture, and the mixture was concentrated under reduced pressure to give 1-(6-(bromomethyl)-1-(3-chloro-2-fluoro-5-(trifluoromethyl)benzyl)-2-oxo-1,2-dihydropyridin-3-yl)urea (0.128 g).

A mixture of the obtained compound (0.026 g), dimethylamine hydrochloride (0.014 g), DIPEA (0.036 g), and DMF (1.0 mL) was stirred at room temperature for 2 hours. The reaction mixture was poured into water, and the mixture was extracted with DCM. The extract was dried over anhydrous sodium sulfate and concentrated under reduced pressure. The residue was purified by APS column chromatography (eluent: n-hexane/ethyl acetate = 100/0 to 30/70) to give the title compound (0.003 g).

### Example E-4

### 1-(1-(3-chloro-2-fluoro-5-(trifluoromethyl)benzyl)-2-oxo-6-(pyrrolidin-1-ylmethyl)-1,2-dihydropyridin-3-yl)urea

Under an argon atmosphere, to a mixture of Example E-1 (0.081 g) and DCM (3.0 mL), phosphorus tribromide (0.083 g) was added under ice cooling, and the mixture was stirred at room temperature for 2 hours. Methanol was added to the reaction mixture under ice cooling, and the mixture was concentrated under reduced pressure to give 1-(6-(bromomethyl)-1-(3-chloro-2-fluoro-5-(trifluoromethyl)benzyl)-2-oxo-1,2-dihydropyridin-3-yl)urea (0.093 g).

Under an argon atmosphere, to a mixture of the obtained compound (0.023 g), pyrrolidine (0.011 g), and DMF (1.0 mL), DIPEA (0.033 g) was added under ice cooling, and the mixture was stirred at room temperature for 1 hour. A saturated aqueous sodium hydrogen carbonate solution was added to the reaction mixture, and the mixture was extracted with DCM. The extract was concentrated under reduced pressure. The residue was purified by APS column chromatography (eluent: n-hexane/ethyl acetate = 70/30 to 30/70). The obtained product was suspended in 1 mol/L hydrochloric acid, and the insoluble material was removed by filtration. A 2 mol/L aqueous sodium hydroxide solution was added to the filtrate to make it basic, and the mixture was extracted with DCM. The extract was concentrated under reduced pressure to give the title compound (0.005 g).

### Example E-5

### 1-(6-allyl-1-(3-chloro-2-fluoro-5-(trifluoromethyl)benzyl)-2-oxo-1,2-dihydropyridin-3-yl)urea

To a mixture of Reference Example D-1 (0.712 g), DMF (1.0 mL), and DME (8.0 mL), sodium hydride (approximately 60%) (0.099 g) was added under ice cooling, and the mixture was stirred under ice cooling for 10 minutes. Lithium bromide (0.165 g) was added to the reaction mixture under ice cooling, and the mixture was stirred at room temperature for 15 minutes. Reference Example A-3 (0.581 g) was added to the reaction mixture, and the mixture was stirred at 90°C for 3 hours. The reaction mixture was allowed to cool to room temperature, water was added, and the mixture was extracted with ethyl acetate. The extract was washed with saturated brine, dried over anhydrous magnesium sulfate, and concentrated under reduced pressure. The residue was purified by silica gel column chromatography (eluent: n-hexane/ethyl acetate = 100/0 to 90/10) to give tert-butyl (6-bromo-1-(3-chloro-2-fluoro-5-(trifluoromethyl)benzyl)-2-oxo-1,2-dihydropyridin-3-yl)carbamate (0.516 g).

Under an argon atmosphere, to a mixture of the obtained compound (0.446 g), allyltributylstannane (0.342 g), and DMF (10 mL), tetrakis(triphenylphosphine)palladium(0) (0.052 g) was added at room temperature, and the mixture was stirred at 100°C for 30 minutes. The reaction mixture was allowed to cool to room temperature, water was added, and the mixture was extracted with n-hexane/ethyl acetate (1/1). The extract was dried over anhydrous sodium sulfate and concentrated under reduced pressure. The residue was purified by silica gel column chromatography (eluent: n-hexane/ethyl acetate = 100/0 to 90/10) to give tert-butyl (6-allyl-1-(3-chloro-2-fluoro-5-(trifluoromethyl)benzyl)-2-oxo-1,2-dihydropyridin-3-yl)carbamate (0.269 g).

A mixture of the obtained compound (0.070 g), hydrogen chloride (4 mol/L in 1,4-dioxane) (0.759 mL), and DCM (0.5 mL) was stirred at room temperature for 5 hours. The reaction mixture was concentrated under reduced pressure.

To a mixture of the residue and DCM (1.0 mL), triphosgene (0.045 g) and TEA (0.092 g) were added under ice cooling, and the mixture was stirred under ice cooling for 30 minutes. Ammonium chloride (0.081 g) and DIPEA (0.196 g) were added to the reaction mixture under ice cooling, and the mixture was stirred at room temperature for 30 minutes. The reaction mixture was poured into water, and the mixture was extracted with DCM. The extract was dried over anhydrous sodium sulfate and concentrated under reduced pressure. The residue was purified by silica gel column chromatography (eluent: n-hexane/ethyl acetate = 100/0 to 0/100) to give the title compound (0.038 g).

### Example E-6

### 1-(1-(2-fluoro-5-(trifluoromethyl)benzyl)-2-oxo-6-(pyrrolidin-1-ylmethyl)-1,2-dihydropyridin-3-yl)urea

To a mixture of Example B-4 (0.406 g) and 1,4-dioxane (12 mL), selenium dioxide (IV) (0.443 g) was added, and the mixture was stirred at 80°C for 13 hours. After the reaction mixture was allowed to cool to room temperature, the mixture was filtered through Celite, and the filtrate was concentrated under reduced pressure. The residue was purified by Method B (eluent: n-hexane/ethyl acetate/methanol = 70/30/0 to 0/100/0 to 0/90/10) to give 1-(1-(2-fluoro-5-(trifluoromethyl)benzyl)-6-(hydroxymethyl)-2-oxo-1,2-dihydropyridin-3-yl)urea (0.148 g).

To a mixture of the obtained compound (0.036 g) and DCM (1.5 mL), phosphorus tribromide (0.041 g) was added under ice cooling, and the mixture was stirred at room temperature for 1 hour. Methanol was added to the reaction mixture under ice cooling, and the mixture was concentrated under reduced pressure.

Under an argon atmosphere, to a mixture of the residue, pyrrolidine (0.021 g), and DMF (1.0 mL), DIPEA (0.064 g) was added, and the mixture was stirred at room temperature for 30 minutes. A saturated aqueous ammonium chloride solution was added to the reaction mixture, and the mixture was extracted with n-hexane/ethyl acetate (1/1). The extract was washed with water and saturated brine, dried over anhydrous magnesium sulfate, and concentrated under reduced pressure. The residue was purified by APS column chromatography (eluent: n-hexane/ethyl acetate = 70/30 to 30/70) to give the title compound (0.016 g).

### Example E-7

### 1-(5-oxo-2-(3-(trifluoromethyl)phenyl)-1,2,3,5-tetrahydroindolizin-6-yl)urea

A mixture of Reference Example L-3 (0.069 g), tert-butyl carbamate (0.059 g), cesium carbonate (0.244 g), chloro(2-dicyclohexylphosphino-2',4',6'-triisopropyl-1,1'-biphenyl)[2-(2'-amino-1,1'-biphenyl)]palladium(II) (0.039 g), and 1,4-dioxane (1.5 mL) was stirred at 90°C for 30 minutes under microwave irradiation. 1,4-Dioxane (0.5 mL) was added to the reaction mixture, and the mixture was stirred at 110°C for 30 minutes under microwave irradiation. Water and DCM were added to the reaction mixture, and the mixture was filtered through Celite. The organic layer of the filtrate was separated and concentrated under reduced pressure. The residue was purified by silica gel column chromatography (eluent: n-hexane/ethyl acetate = 80/20 to 50/50) to give acetic acid 6-((tert-butoxycarbonyl)amino)-5-oxo-2-(3-(trifluoromethyl)phenyl)-1,2,3,5-tetrahydroindolizin-1-yl (0.049 g).

A mixture of the obtained compound (0.083 g), potassium carbonate (0.076 g), and methanol (1.0 mL) was stirred at room temperature for 30 minutes. The reaction mixture was concentrated under reduced pressure. The residue was purified by silica gel column chromatography (eluent: n-hexane/ethyl acetate = 80/20 to 40/60) to give tert-butyl (1-hydroxy-5-oxo-2-(3-(trifluoromethyl)phenyl)-1,2,3,5-tetrahydroindolizin-6-yl)carbamate (0.011 g).

Under an argon atmosphere, to a mixture of the obtained compound (0.011 g) and DCM (1.0 mL), TFA (0.031 g) and triethylsilane (0.006 g) were added at room temperature, and the mixture was stirred at the same temperature for 14 hours. TFA (0.031 g) was added to the reaction mixture at room temperature, and the mixture was stirred at the same temperature for 3 hours. A 5 mol/L aqueous sodium hydroxide solution (1.0 mL) was added to the reaction mixture under ice cooling, and the mixture was extracted with DCM. The extract was dried over anhydrous sodium sulfate and concentrated under reduced pressure.

Under an argon atmosphere, to a mixture of the residue and DCM (1.0 mL), triphosgene (0.008 g) and TEA (0.008 g) were added under ice cooling, and the mixture was stirred under ice cooling for 30 minutes. Ammonium chloride (0.015 g) and DIPEA (0.036 g) were added to the reaction mixture under ice cooling, and the mixture was stirred at room temperature for 1 hour. Water was added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate/methanol = 30/70/0 to 0/100/0 to 0/95/5) to give the title compound (0.001 g).

### Example E-8

### N-(1-(3-chloro-2-fluoro-5-(trifluoromethyl)benzyl)-6-methyl-2-oxo-1,2-dihydropyridin-3-yl)-2,2,2-trifluoroacetamide

To a mixture of Reference Example E-6 (0.030 g), DIPEA (0.023 g), and DCM (0.7 mL), trifluoroacetic anhydride (0.017 g) was added under water cooling, and the mixture was stirred at room temperature for 1 hour. The reaction mixture was poured into water, and the mixture was extracted with DCM. The extract was dried over anhydrous sodium sulfate and concentrated under reduced pressure. The residue was sequentially purified by silica gel column chromatography (eluent: n-hexane/ethyl acetate = 100/0 to 0/100) and ODS column chromatography (eluent: water/MeCN = 70/30 to 10/90) to give the title compound (0.028 g).

### Example E-9

### N-(1-(3-chloro-2-fluoro-5-(trifluoromethyl)benzyl)-6-methyl-2-oxo-1,2-dihydropyridin-3-yl)azetidine-1-carboxamide

To a mixture of Reference Example E-6 (0.030 g) and DCM (1.0 mL), triphosgene (0.027 g) and DIPEA (0.027 g) were added under ice cooling, and the mixture was stirred under ice cooling for 30 minutes. Azetidine hydrochloride (0.008 g) and DIPEA (0.116 g) were added to the reaction mixture under ice cooling, and the mixture was stirred at room temperature for 30 minutes. The reaction mixture was poured into water, and the mixture was extracted with DCM. The extract was dried over anhydrous sodium sulfate and concentrated under reduced pressure. The residue was sequentially purified by silica gel column chromatography (eluent: n-hexane/ethyl acetate = 100/0 to 0/100) and ODS column chromatography (eluent: water/MeCN = 70/30 to 10/90) to give the title compound (0.008 g).

### Example F-1

### (R)-1-(7-(difluoromethyl)-1-(2-(hydroxymethyl)-5-(trifluoromethyl)benzyl)-2-oxo-1,2,3,4-tetrahydroquinolin-3-yl)urea

Under an argon atmosphere, to a mixture of Reference Example M-1 (0.151 g) and DMF (2.0 mL), Reference Example H-2 (0.172 g) and potassium carbonate (0.100 g) were added under ice cooling, and the mixture was stirred under ice cooling for 30 minutes and at room temperature for 62 hours. Water and a saturated aqueous ammonium chloride solution were added to the reaction mixture under ice cooling, and the mixture was extracted with n-hexane/ethyl acetate (1/1). The extract was dried over anhydrous sodium sulfate and concentrated under reduced pressure. The residue was purified by silica gel column chromatography (eluent: n-hexane/ethyl acetate = 100/0 to 80/20) to give methyl (R)-2-((3-((tert-butoxycarbonyl)amino)-7-(difluoromethyl)-2-oxo-3,4-dihydroquinolin-1(2H)-yl)methyl)-4-(trifluoromethyl)benzoate (0.217 g).

A mixture of the obtained compound (0.101 g), a 4 mol/L aqueous lithium hydroxide solution (0.191 mL), THF (0.5 mL), and methanol (0.5 mL) was stirred at 60°C for 30 minutes under microwave irradiation. A 2 mol/L hydrochloric acid solution (0.420 mL) was added to the reaction mixture, and the mixture was extracted with DCM. The extract was dried over anhydrous sodium sulfate and concentrated under reduced pressure to give (R)-2-((3-((tert-butoxycarbonyl)amino)-7-(difluoromethyl)-2-oxo-3,4-dihydroquinolin-1(2H)-yl)methyl)-4-(trifluoromethyl)benzoic acid (0.084 g).

Under an argon atmosphere, to a mixture of the obtained compound (0.053 g) and THF (0.75 mL), BH₃-THF (0.93 mol/L in THF) (0.133 mL) was added under ice cooling, and the mixture was stirred at room temperature for 3 hours. Methanol was added to the reaction mixture, and the mixture was stirred at room temperature for 30 minutes. The mixture was concentrated under reduced pressure. The residue was purified by silica gel column chromatography (eluent: n-hexane/ethyl acetate = 90/10 to 50/50) to give (R)-(7-(difluoromethyl)-1-(2-(hydroxymethyl)-5-(trifluoromethyl)benzyl)-2-oxo-1,2,3,4-tetrahydroquinolin-3-yl)carbamic acid tert-butyl ester (0.038 g).

Under an argon atmosphere, to a mixture of the obtained compound (0.038 g) and DCM (0.75 mL), TFA (0.174 g) was added at room temperature, and the mixture was stirred at the same temperature for 1 hour. A 2 mol/L aqueous sodium hydroxide solution was added to the reaction mixture under ice cooling, and the mixture was extracted with DCM. The extract was concentrated under reduced pressure.

Under an argon atmosphere, to a mixture of the residue, water (0.027 g), and THF (0.75 mL), potassium cyanate (0.008 g) and acetic acid (0.005 g) were added under ice cooling, and the mixture was stirred at room temperature for 64 hours. Water was added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate/methanol = 0/100/0 to 0/100/0 to 0/95/5) to give the title compound (0.007 g).

### Example F-2

### (R)-1-(7-(difluoromethyl)-2-oxo-1-((6-(trifluoromethyl)pyridin-2-yl)methyl)-1,2,3,4-tetrahydroquinolin-3-yl)urea

Under an argon atmosphere, to a mixture of Reference Example M-1 (0.030 g) and DMF (2.0 mL), Reference Example H-3 (0.025 g) and potassium carbonate (0.020 g) were added under ice cooling, and the mixture was stirred under ice cooling for 30 minutes and at room temperature for 20 hours. Water and a saturated aqueous ammonium chloride solution were added to the reaction mixture under ice cooling, and the mixture was extracted with n-hexane/ethyl acetate (1/1). The extract was dried over anhydrous sodium sulfate and concentrated under reduced pressure. The residue was purified by silica gel column chromatography (eluent: n-hexane/ethyl acetate = 100/0 to 70/30) to give (R)-(7-(difluoromethyl)-2-oxo-1-((6-(trifluoromethyl)pyridin-2-yl)methyl)-1,2,3,4-tetrahydroquinolin-3-yl)carbamic acid tert-butyl ester (0.024 g).

Under an argon atmosphere, to a mixture of the obtained compound (0.024 g) and DCM (1.0 mL), TFA (0.117 g) was added at room temperature, and the mixture was stirred at the same temperature for 1 hour. To the reaction mixture, a 2 mol/L aqueous sodium hydroxide solution was added under ice cooling, and the mixture was extracted with DCM. The extract was concentrated under reduced pressure.

Under an argon atmosphere, to a mixture of the residue, water (0.019 g), and THF (1.0 mL), potassium cyanate (0.005 g) and acetic acid (0.003 g) were added under ice cooling, and the mixture was stirred at room temperature for 2.5 hours. Water was added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate/methanol = 100/0/0 to 0/100/0 to 0/95/5) to give the title compound (0.012 g).

### Example F-3

### (R)-1-(8-((dimethylamino)methyl)-1-(2-fluoro-5-(trifluoromethyl)benzyl)-2-oxo-1,2,3,4-tetrahydroquinolin-3-yl)urea

To a mixture of Reference Example N-2 (0.039 g), water (0.019 g), and THF (0.5 mL), potassium cyanate (0.011 g) and acetic acid (0.007 g) were added under ice cooling, and the mixture was stirred at room temperature for 18 hours. Water was added to the reaction mixture, and the mixture was extracted with DCM. The residue was suspended in n-hexane/MBTE (1/1), and insoluble material was collected by filtration. The obtained solid was washed with MTBE and then dried under reduced pressure to give (R)-1-(1-(2-fluoro-5-(trifluoromethyl)benzyl)-8-(hydroxymethyl)-2-oxo-1,2,3,4-tetrahydroquinolin-3-yl)urea (0.037 g).

To a mixture of the obtained compound (0.015 g) and DCM (1.0 mL), phosphorus tribromide (0.015 g) was added under ice cooling, and the mixture was stirred under ice cooling for 1 hour. Methanol was added to the reaction mixture, and the mixture was concentrated under reduced pressure.

A mixture of the residue, dimethylamine hydrochloride (0.009 g), DIPEA (0.023 g), and DMF (1.0 mL) was stirred at room temperature for 20 hours. Water was added to the reaction mixture, and the mixture was extracted with DCM. The extract was concentrated under reduced pressure. The residue was purified by APS column chromatography (eluent: n-hexane/ethyl acetate/methanol = 50/50/0 to 0/100/0 to 0/90/10) to give the title compound (0.012 g).

### Example F-4

### (R)-1-(7-(2-fluoro-5-(trifluoromethyl)benzyl)-6-oxo-4,5,6,7-tetrahydrothieno[2,3-b]pyridin-5-yl)urea

Example F-4 was synthesized by the same method as in Example F-2, except that Reference Example M-4 was used instead of Reference Example M-1 and 2-(bromomethyl)-1-fluoro-4-(trifluoromethyl)benzene was used instead of Reference Example H-3.

### Example F-5

### (R)-1-(4-(2-fluoro-5-(trifluoromethyl)benzyl)-5-oxo-4,5,6,7-tetrahydrothieno[3,2-b]pyridin-6-yl)urea

Example F-5 was synthesized by the same method as in Example F-2, except that Reference Example M-7 was used instead of Reference Example M-1 and 2-(bromomethyl)-1-fluoro-4-(trifluoromethyl)benzene was used instead of Reference Example H-3.

### Example F-6

### (R)-1-(4-(2-fluoro-5-(trifluoromethyl)benzyl)-2-methyl-5-oxo-4,5,6,7-tetrahydrothiazolo[5,4-b]pyridin-6-yl)urea

Example F-6 was synthesized by the same method as in Example F-2, except that Reference Example M-11 was used instead of Reference Example M-1 and 2-(bromomethyl)-1-fluoro-4-(trifluoromethyl)benzene was used instead of Reference Example H-3.

### Example F-7

### 2-(4-(2-fluoro-5-(trifluoromethyl)benzyl)-3-oxo-3,4-dihydro-2H-benzo[b][1,4]oxazin-2-yl)acetamide

A mixture of Reference Example M-13 (0.100 g), 2-(bromomethyl)-1-fluoro-4-(trifluoromethyl)benzene (0.120 g), potassium carbonate (0.088 g), and DMF (4.3 mL) was stirred at 60°C for 1.5 hours under microwave irradiation. The reaction mixture was poured into water, and the mixture was extracted with ethyl acetate. The extract was dried over anhydrous sodium sulfate and concentrated under reduced pressure. The residue was purified by silica gel column chromatography (eluent: n-hexane/ethyl acetate = 100/0 to 60/40) to give ethyl 2-(4-(2-fluoro-5-(trifluoromethyl)benzyl)-3-oxo-3,4-dihydro-2H-benzo[b][1,4]oxazin-2-yl)acetate (0.109 g).

To a mixture of the obtained compound (0.098 g), THF (0.5 mL), and methanol (0.5 mL), a 4 mol/L aqueous lithium hydroxide solution (0.179 mL) was added under ice cooling, and the mixture was stirred under ice cooling for 1 hour and at room temperature for 1 hour. To the reaction mixture, 1 mol/L hydrochloric acid and water were added under ice cooling, and the mixture was extracted with DCM. The extract was concentrated under reduced pressure.

To a mixture of the residue and MeCN (2.4 mL), DMT-MM (0.099 g) and 15 mol/L aqueous ammonia (0.024 mL) were added at room temperature, and the mixture was stirred at the same temperature for 30 minutes. A saturated aqueous ammonium chloride solution was added to the reaction mixture, and the mixture was extracted with DCM. The extract was concentrated under reduced pressure. The residue was sequentially purified by silica gel column chromatography (eluent: n-hexane/ethyl acetate = 0/100 to 100/0) and ODS column chromatography (eluent: water/MeCN = 90/10 to 10/90) to give the title compound (0.018 g).

### Example F-8

### tert-butyl (2-(2-amino-2-oxoethyl)-4-(2-fluoro-5-(trifluoromethyl)benzyl)-3-oxo-3,4-dihydro-2H-benzo[b][1,4]oxazin-7-yl)carbamate

To a mixture of Reference Example N-4 (0.105 g), Boc₂O (0.107 g), TEA (0.075 g), and THF (2.5 mL), DMAP (0.015 g) was added at room temperature, and the mixture was stirred at the same temperature for 1 hour. Water was added to the reaction mixture, and the mixture was extracted with DCM. The extract was concentrated under reduced pressure.

A mixture of the residue, potassium carbonate (0.085 g), and methanol (2.5 mL) was stirred at 60°C for 1 hour. After the reaction mixture was allowed to cool to room temperature, water was added, and the mixture was extracted with DCM. The extract was concentrated under reduced pressure.

To a mixture of the residue, THF (0.5 mL), and methanol (0.5 mL), a 4 mol/L aqueous lithium hydroxide solution (0.185 mL) was added at room temperature, and the mixture was stirred at the same temperature for 1 hour. To the reaction mixture, 1 mol/L hydrochloric acid (1.00 mL) and water were added under ice cooling, and the mixture was extracted with DCM. The extract was concentrated under reduced pressure.

To a mixture of the residue and MeCN (2.4 mL), DMT-MM (0.102 g) and 15 mol/L aqueous ammonia (0.025 mL) were added at room temperature, and the mixture was stirred at the same temperature for 30 minutes. A saturated aqueous ammonium chloride solution was added to the reaction mixture, and the mixture was extracted with DCM. The extract was concentrated under reduced pressure. The residue was purified by silica gel column chromatography (eluent: ethyl acetate) and ODS column chromatography (eluent: water/MeCN = 70/30 to 10/90) to give the title compound (0.011 g).

### Example F-9

### 1-(tert-butyl)-3-(1-(2-fluoro-5-(trifluoromethyl)benzyl)-2-oxo-3-ureido-1,2-dihydroquinoxalin-6-yl)urea

To a mixture of Reference Example N-8 (0.013 g), pyridine (0.004 g), and DCM (1.0 mL), 4-nitrophenyl chloroformate (0.007 g) was added at room temperature, and the mixture was stirred at the same temperature for 45 minutes. tert-Butylamine (0.003 g) and DIPEA (0.010 g) were added to the reaction mixture, and the mixture was stirred at room temperature for 1 hour. tert-Butylamine (0.002 g) was added to the reaction mixture, and the mixture was stirred at room temperature for 1.5 hours. A saturated aqueous sodium hydrogen carbonate solution was added to the reaction mixture, and the mixture was extracted with DCM. The extract was concentrated under reduced pressure. The residue was purified by Method A (eluent: n-hexane/ethyl acetate = 50/50 to 100/0) to give the title compound (0.002 g).

The following tables show the structural formulas, physical property values, and TSHR antagonist activity (refer to Test Example 1) of examples.

**[Table 12]**

| Ex. No. | Str. | Phys. data | IC₅₀ |
|---|---|---|---|
| A-1 | 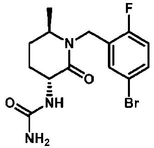 | MS m/z : 358 (M+H)⁺ | B |
| A-2 | 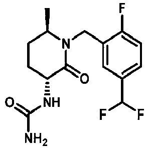 | ¹H-NMR (DMSO-d6) δ ppm : 1.12 (3H, d, J=6.4Hz), 1.48-1.73 (2H, m), 1.93-2.19 (2H, m), 3.41-3.57 (1H, m), 4.02-4.16 (1H, m), 4.53 (1H, d, J=16.0Hz), 4.71 (1H, d, J=16.0Hz), 5.65 (2H, br), 6.26 (1H, d, J=6.4Hz), 7.02 (1H, t, J=55.8Hz), 7.30-7.40 (1H, m), 7.41-7.58 (2H, m) | A |
| | | MS m/z : 330 (M+H)⁺ | |
| A-3 | 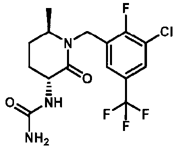 | ¹H-NMR (DMSO-d6) *δ* ppm : 1.14 (3H, d, J=6.4Hz), 1.43-1.82 (2H, m), 1.94-2.19 (2H, m), 3.45-3.64 (1H, m), 4.01-4.23 (1H, m), 4.57 (1H, d, J=16.2Hz), 4.75 (1H, d, J=16.2Hz), 5.62 (2H, br), 6.25 (1H, d, J=6.8Hz), 7.50-7.62 (1H, m), 7.95-8.07 (1H, m) | A |
| | | MS m/z : 382 (M+H)⁺ | |
| A-4 | 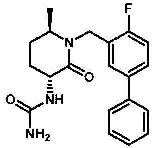 | MS m/z : 356 (M+H)⁺ | B |
| A-5 | 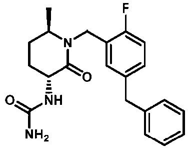 | MS m/z : 370 (M+H)⁺ | A |
| A-6 | 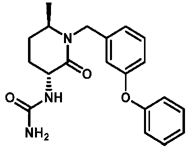 | MS m/z : 354 (M+H)⁺ | B |

**[Table 13]**

| Ex. No. | Str. | Phys. data | IC₅₀ |
|---|---|---|---|
| A-7 | 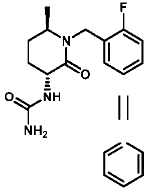 | ¹H-NMR (CDCl₃) δ ppm : 1.20 (3H, d, J=6.4Hz), 1.45-1.82 (2H, m), 1.96-2.10 (1H, m), 2.32-2.52 (1H, m), 3.38-3.54 (1H, m), 4.19-4.32 (1H, m), 4.46 (1H, d, J=15. 6Hz), 4. 76 (2H, br), 4.90 (1H, d, J=15. 6Hz), 5.86-6.24 (1H, m), 7.02 (1H, dd, J=8.6, 9.8Hz), 7.28-7.45 (5H, m), 7.47-7.58 (2H, m) | A |
| | | MS m/z : 380 (M+H)⁺ | |
| A-8 | 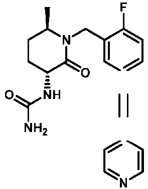 | ¹H-NMR (CDCl₃) *δ* ppm : 1. 23 (3H, d, J=6. 4Hz), 1. 48-1. 82 (2H, m), 2.00-2. 15 (1H, m), 2.40-2.55 (1H, m), 3.40-3.60 (1H, m), 4.14-4.32 (1H, m), 4.54 (1H, d, J=15. 6Hz), 4. 68 (2H, br), 4.83 (1H, d, J=15. 6Hz), 5.74-6.06 (1H, m), 7.06 (1H, dd, J=8.8, 9.6Hz), 7.30-7.49 (4H, m), 8.30-8.90 (2H, m) | A |
| | | MS m/z : 381 (M+H)⁺ | |
| A-9 | 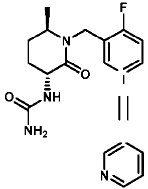 | ¹H-NMR (CDCl₃) δ ppm : 1.23 (3H, d, J=6.0Hz), 1.51-1.80 (2H, m), 2.00-2. 15 (1H, m), 2.40-2.55 (1H, m), 3.41-3.58 (1H, m), 4.17-4.30 (1H, m), 4.52 (1H, d, J=15.8Hz), 4.66 (2H, br), 4.86 (1H, d, J=15. 8Hz), 5.79-5.98 (1H, m), 7.05 (1H, dd, J=8.4, 9.6Hz), 7.21-7.35 (1H, m), 7.36-7.48 (2H, m), 7.77-7.87 (1H, m), 8.56 (1H, dd, J=1.6, 5.2Hz), 8.71-8.82 (1H, m) | A |
| | | MS m/z : 381 (M+H)⁺ | |
| A-10 | 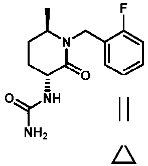 | MS m/z : 344 (M+H)⁺ | B |
| A-11 | 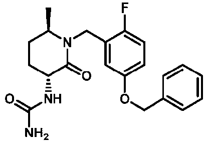 | MS m/z : 386 (M+H)⁺ | A |
| A-12 | 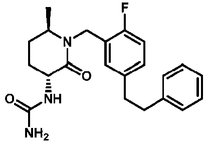 | ¹H-NMR (CDCl₃) *δ* ppm : 1.15 (3H, d, J=6.4Hz), 1. 39-1. 79 (2H, m), 1.93-2.07 (1H, m), 2.36-2.49 (1H, m), 2.77-2.97 (4H, m), 3.27-3.42 (1H, m), 4.08-4.25 (1H, m), 4.38 (1H, d, J=15. 4Hz), 4.71 (2H, br), 4.93 (1H, d, J=15. 4Hz), 5.72-6.20 (1H, m), 6.87-6.97 (2H, m), 6.99-7.05 (1H, m), 7.08-7.20 (3H, m), 7.21-7.30 (2H, m) | A |
| | | MS m/z : 384 (M+H)⁺ | |

**[Table 14]**

| Ex. No. | Str. | Phys. data | IC₅₀ |
|---|---|---|---|
| A-13 | 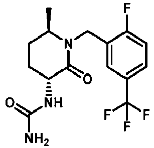 | ¹H-NMR (DMSO-d6) *δ* ppm : 1. 13 (3H, d, J=6.0Hz), 1.44-1.73 (2H, m), 1. 95-2. 17 (2H, m), 3.42-3.56 (1H, m), 4.02-4.15 (1H, m), 4.54 (1H, d, J=16. 0Hz), 4.74 (1H, d, J=16. 0Hz), 5.63 (2H, br), 6.25 (1H, d, J=6.8Hz), 7.39-7.51 (1H, m), 7.53-7.64 (1H, m), 7. 68-7. 78 (1H, m) | A |
| | | MS m/z : 348 (M+H)⁺ | |
| A-14 | 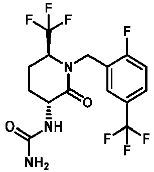 | ¹H-NMR (DMSO-d6) *δ* ppm : 1. 36-1. 58 (1H, m), 1.82-2.04 (1H, m), 2. 06-2. 41 (2H, m), 4.06-4. 26 (1H, m), 4.50 (1H, d, J=15.8Hz), 4. 56-4. 78 (1H, m), 4. 90 (1H, d, J=15. 8Hz), 5.68 (2H, br), 6.34 (1H, d, J=7.2Hz), 7.37-7.53 (1H, m), 7. 54-7. 62 (1H, m), 7.63-7.82 (1H, m) | A |
| | | MS m/z : 402 (M+H)⁺ | |
| A-15 | 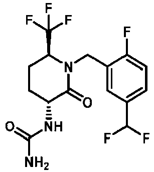 | ¹H-NMR (DMSO-d6) *δ* ppm : 1. 39-1. 59 (1H, m), 1.82-2.01 (1H, m), 2.07-2.39 (2H, m), 4.07-4.20 (1H, m), 4.45 (1H, d, J=15.8Hz), 4.52-4.71 (1H, m), 4.92 (1H, d, J=15. 8Hz), 5.69 (2H, br), 6.34 (1H, d, J=7. 2Hz), 7.03 (1H, t, J=55.6Hz), 7. 30-7. 49 (2H, m), 7.50-7.61 (1H, m) MS m/z : 384 (M+H)⁺ | A |
| A-16 | 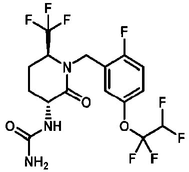 | ¹H-NMR (CDCl₃) *δ* ppm : 1.35-1.51 (1H, m), 2.01-2.29 (2H, m), 2.45-2.62 (1H, m), 3.94-4.08 (1H, m), 4. 20-4. 35 (2H, m), 4. 61 (2H, br), 5.27 (1H, d, J=15. 6Hz), 5. 71-6.12 (2H, m), 7.01-7.21 (3H, m) | A |
| | | MS m/z : 450 (M+H)⁺ | |
| A-17 | 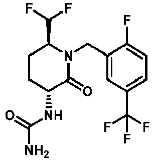 | ¹H-NMR (DMSO-d6) *δ* ppm : 1.34-1.54 (1H, m), 1.75-1.93 (1H, m), 2.05-2.23 (2H, m), 3.93-4.19 (2H, m), 4.50 (1H, d, J=16.0Hz), 4.89 (1H, d, J=16.0Hz), 5.67 (2H, br), 6.05-6. 45 (2H, m), 7.40-7.50 (1H, m), 7.53-7.62 (1H, m), 7. 67-7. 78 (1H, m) | A |
| | | MS m/z : 384 (M+H)⁺ | |
| A-18 | 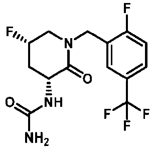 | MS m/z : 352 (M+H)⁺ | C |

**[Table 15]**

| Ex. No. | Str. | Phys. data | IC₅₀ |
|---|---|---|---|
| A-19 | 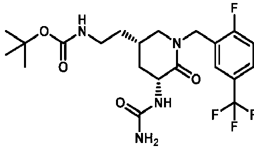 | MS m/z : 477 (M+H)⁺ | A |
| A-20 | 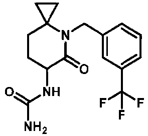 | MS m/z : 342 (M+H)⁺ | B |
| A-21 | 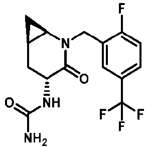 | ¹H-NMR (DMSO-d6) δ ppm : 0.60-0. 81 (2H, m), 1.21-1.40 (1H, m), 1.83-2. 17 (2H, m), 2.72-2.86 (1H, m), 3.93-4.08 (1H, m), 4.58-4.84 (2H, m), 5.62 (2H, br), 6.35 (1H, d, J=7.6Hz), 7.41-7.53 (1H, m), 7.64-7.85 (2H, m) | A |
| | | MS m/z : 346 (M+H)⁺ | |
| A-22 | 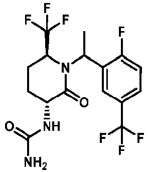 | MS m/z : 416 (M+H)⁺ | C |
| A-23 | 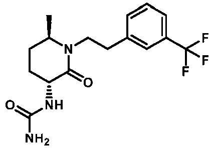 | MS m/z : 344 (M+H)⁺ | B |
| A-24 | 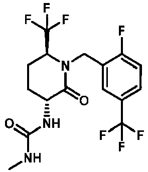 | MS m/z : 416 (M+H)⁺ | A |

**[Table 16]**

| Ex. No. | Str. | Phys. data | IC₅₀ |
|---|---|---|---|
| A-25 | 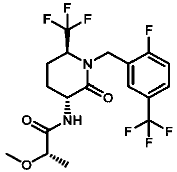 | MS m/z : 445 (M+H)⁺ | C |
| A-26 | 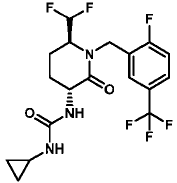 | MS m/z : 424 (M+H)⁺ | B |
| A-27 | 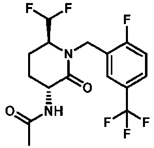 | MS m/z : 383 (M+H)⁺ | B |
| A-28 | 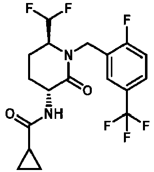 | ¹H-NMR (CDCl₃) *δ* ppm : 0.68-0. 84 (2H, m), 0.85-1.07 (2H, m), 1.35-1.53 (2H, m), 1.91-2.22 (2H, m), 2. 49-2. 65 (1H, m), 3.71-3.89 (1H, m), 4.26-4.36 (1H, m), 4.40 (1H, d, J=15.4Hz), 5.23 (1H, d, J=15. 4Hz), 5. 81 (1H, dt, J=3.9, 55.4Hz), 6. 71-6.93 (1H, m), 7.13-7.25 (1H, m), 7.51-7.65 (2H, m) | A |
| | | MS m/z : 409 (M+H)⁺ | |
| A-29 | 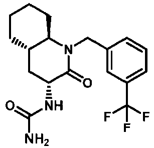 | MS m/z : 370 (M+H)⁺ | C |
| B-1 | 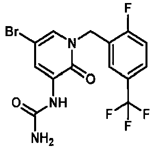 | MS m/z : 408 (M-H)⁻ | A |

**[Table 17]**

| Ex. No. | Str. | Phys. data | IC₅₀ |
|---|---|---|---|
| B-2 | 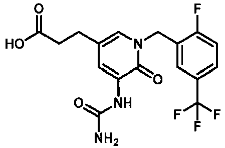 | MS m/z : 402 (M+H)⁺ | C |
| B-3 | 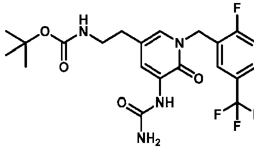 F | ¹H-NMR (DMSO-d6) *δ* ppm : 1.35 (9H, s), 2.42 (2H, t, J=7.3 Hz), 3.04 (2H, dd, J=7.0, 7.1Hz), 5.20 (2H, s), 6.08-6.64 (2H, m), 6.81-6.95 (1H, m), 7.08-7.26 (1H, m), 7.41-7.54 (1H, m), 7.60-7.85 (2H, m), 7.92-8.08 (1H, m), 8. 34 (1H, s) | A |
| | | MS m/z : 473 (M+H)⁺ | |
| B-4 | 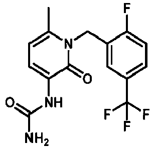 | ¹H-NMR (DMSO-d6) *δ* ppm : 2.27 (3H, s), 5.38 (2H, s), 6.05-6.52 (3H, m), 7.15-7.30 (1H, m), 7.52 (1H, t, J=9.3Hz), 7.73-7.83 (1H, m), 8.00 (1H, d, J=7.6Hz), 8.26 (1H, s) | A |
| | | MS m/z : 344 (M+H)⁺ | |
| B-5 | 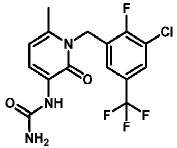 | ¹H-NMR (DMSO-d6) *δ* ppm : 2. 29 (3H, s), 5.39 (2H, s), 6.00-6.67 (3H, m), 6. 80-7. 42 (1H, m), 7. 99 (1H, d, J=7. 6Hz), 8.04-8.12 (1H, m), 8. 24 (1H, s) | A |
| | | MS m/z : 378 (M+H)⁺ | |
| B-6 | 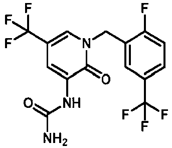 | MS m/z : 398 (M+H)⁺ | A |

**[Table 18]**

| Ex. No. | Str. | Phys. data | IC₅₀ |
|---|---|---|---|
| B-7 | 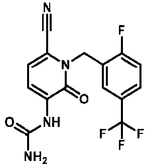 | MS m/z : 353 (M-H)⁻ | A |
| B-8 | 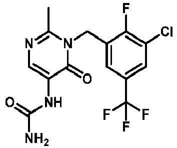 | MS m/z : 379 (M+H)⁺ | A |
| B-9 | 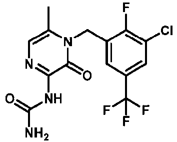 | MS m/z : 379 (M+H)⁺ | B |
| B-10 | 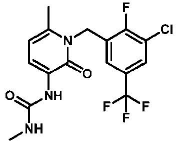 | ¹H-NMR (CDCl₃) δ ppm : 2.27 (3H, s), 2.74-2.89 (3H, m), 4.59-4.99 (1H, m), 5.41 (2H, s), 6.19 (1H, d, J=7. 7Hz), 6.98-7.08 (1H, m), 7.42-7.70 (2H, m), 8.15 (1H, d, J=7.7Hz) | A |
| | | MS m/z : 392 (M+H)⁺ | |
| B-11 | 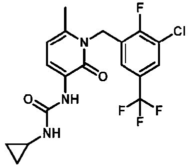 | MS m/z : 418 (M+H)⁺ | A |
| C-1 | 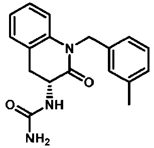 | MS m/z : 310 (M+H)⁺ | B |

**[Table 19]**

| Ex. No. | Str. | Phys. data | IC₅₀ |
|---|---|---|---|
| C-2 | 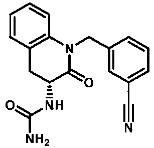 | MS m/z : 321 (M+H)⁺ | c |
| C-3 | 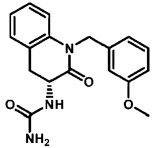 | MS m/z : 326 (M+H)⁺ | C |
| C-4 | 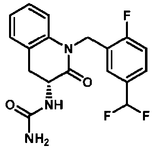 | MS m/z : 362 (M-H)⁻ | A |
| C-5 | 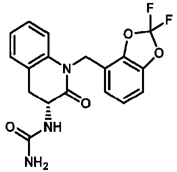 | MS m/z : 376 (M+H)⁺ | A |
| C-6 | 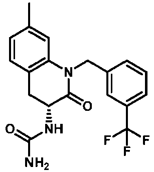 | MS m/z : 378 (M+H)⁺ | A |
| C-7 | 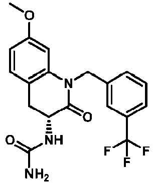 | MS m/z : 392 (M-H)⁻ | A |

**[Table 20]**

| Ex. No. | Str. | Phys. data | IC₅₀ |
|---|---|---|---|
| C-8 | 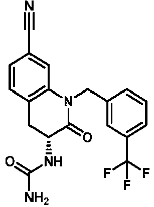 | MS m/z : 389 (M+H)⁺ | B |
| C-9 | 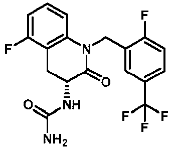 | MS m/z : 398 (M-H)⁻ | A |
| C-10 | 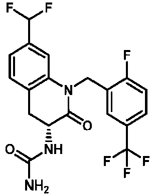 | ¹H-NMR (DMSO-d6) δ ppm : 2.79-2.96 (1H, m), 3.23 (1H, dd, J=5.8, 15.1 Hz), 4.39-4.53 (1H, m), 5.17-5.44 (2H, m), 5.82 (2H, s), 6. 50 (1H, d, J=6. 7Hz), 6.93 (1H, t, J=55.8Hz), 7.18-7.33 (2H, m), 7.37-7.52 (2H, m), 7.56-7.66 (1H, m), 7.69-7.78 (1H, m) | A |
| | | MS m/z : 432 (M+H)⁺ | |
| C-11 | 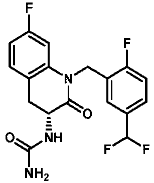 | ¹H-NMR (DMSO-d6) δ ppm : 2.74-2.90 (1H, m), 3.20 (1H, dd, J=5.8, 15.0 Hz), 4.38-4.51 (1H, m), 5.12-5.32 (2H, m), 5.82 (2H, s), 6.48 (1H, d, J=6. 5Hz), 6.77-7.15 (3H, m), 7.24-7.61 (4H, m) | A |
| | | MS m/z : 382 (M+H)⁺ | |
| C-12 | 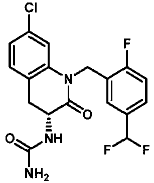 | ¹H-NMR (DMSO-d6) *δ* ppm : 2.76-2.92 (1H, m), 3.20 (1H, dd, J=5.8, 15.2 Hz), 4.38-4.53 (1H, m), 5. 12-5.35 (2H, m), 5.82 (2H, s), 6.48 (1H, d, J=6. 6Hz), 6.80-7.17 (3H, m), 7.25-7.65 (4H, m) | A |
| | | MS m/z : 398 (M+H)⁺ | |
| C-13 | 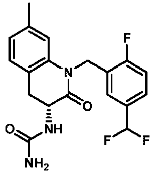 | ¹H-NMR (DMSO-d6) δ ppm : 2.20 (3H, s), 2.71-2.86 (1H, m), 3.16 (1H, dd, J=5.8, 14.9 Hz), 4.32-4.48 (1H, m), 5.03-5.38 (2H, m), 5.81 (2H, s), 6.46 (1H, d, J=6. 4Hz), 6. 74-7. 22 (4H, m), 7.27-7.46 (2H, m), 7.49-7.60 (1H, m) | A |
| | | MS m/z : 378 (M+H)⁺ | |

**[Table 21]**

| Ex. No. | Str. | Phys. data | IC₅₀ |
|---|---|---|---|
| C-14 | 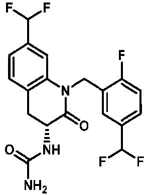 | ¹H-NMR (DMSO-d6) δ ppm : 2.84-3.01 (1H, m), 3.20-3.39 (1H, m), 4.40-4.56 (1H, m), 5.09-5.41 (2H, m), 5.83 (2H, s), 6.49 (1H, d, J=6. 5Hz), 6.75-7.12 (2H, m), 7.14-7.28 (2H, m), 7.32-7.61 (4H, m) | A |
| | | MS m/z : 414 (M+H)⁺ | |
| C-15 | 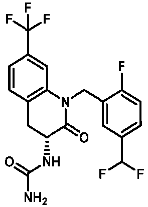 | ¹H-NMR (DMSO-d6) δ ppm : 2.85-3.03 (1H, m), 3.23-3.43 (1H, m), 4.38-4.59 (1H, m), 5.32 (2H, s), 5.84 (2H, s), 6.51 (1H, d, J=6. 6Hz), 6.95 (1H, t, J=55. 7Hz), 7.22-7.64 (6H, m) | A |
| | | MS m/z : 432 (M+H)⁺ | |
| C-16 | 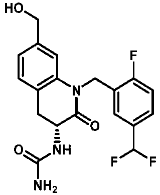 | ¹H-NMR (DMSO-d6) δ ppm : 2.75-2.94 (1H, m), 3.19 (1H, dd, J=5.8, 15.0 Hz), 4.31-4.52 (3H, m), 4.99-5.39 (3H, m), 5.82 (s, 2H), 6.47 (1H, d, J=6.4Hz), 6.77-7.15 (3H, m), 7.18-7.47 (3H, m), 7.50-7.62 (1H, m) | A |
| | | MS m/z : 394 (M+H)⁺ | |
| C-17 | 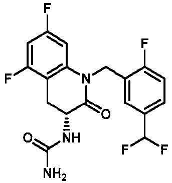 | ¹H-NMR (DMSO-d6) δ ppm : 2.59-2.75 (1H, m), 3.23-3.44 (1H, m), 4.43-4.58 (1H, m), 5.10-5.37 (2H, m), 5.85 (2H, s), 6.52 (1H, d, J=6.4Hz), 6.78-7.14 (3H, m), 7.30-7.46 (2H, m), 7.50-7.61 (1H, m) | A |
| | | MS m/z : 398 (M-H)⁻ | |
| C-18 | 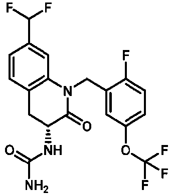 | ¹H-NMR (DMSO-d6) δ ppm : 2.83-3.00 (1H, m), 3.24 (1H, dd, J=6.0, 15.2 Hz), 4.42-4.57 (1H, m), 5.24 (2H, s), 5.83 (2H, s), 6.48 (1H, d, J=6.8Hz), 6.92 (1H, t, J=55. 6Hz), 7. 14-7. 51 (6H, m) | A |
| | | MS m/z : 448 (M+H)⁺ | |
| C-19 | 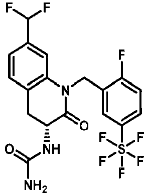 | ¹H-NMR (DMSO-d6) δ ppm : 2.75-2.92 (1H, m), 3.24 (1H, dd, J=6.0, 15.2 Hz), 4.33-4.50 (1H, m), 5.22 (1H, d, J=16.4Hz), 5.47 (1H, d, J=16. 4Hz), 5.82 (2H, s), 6.51 (1H, d, J=6. 8Hz), 6.92 (1H, t, J=55. 6Hz), 7.17-7.28 (1H, m), 7. 32-7. 53 (3H, m), 7. 78-7.97 (2H, m) | A |
| | | MS m/z : 490 (M+H)⁺ | |

**[Table 22]**

| Ex. No. | Str. | Phys. data | IC₅₀ |
|---|---|---|---|
| C-20 | 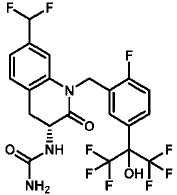 | ¹H-NMR (CDCl₃) 6 ppm : 2.86-3. 05 (1H, m), 3.41 (1H, dd, J=6.0, 15.2 Hz), 4. 35-4. 51 (5H, m), 5.69-6.18 (2H, m), 6.51 (1H, t, J=56.4Hz), 6.92-7.71 (6H, m) MS m/z : 530 (M+H)⁺ | A |
| C-21 | 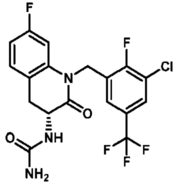 | MS m/z : 432 (M-H)⁻ | A |
| C-22 | 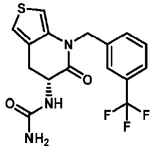 | MS m/z : 370 (M+H)⁺ | A |
| C-23 | 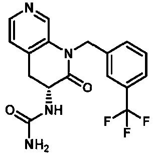 | MS m/z : 363 (M-H)⁻ | B |
| C-24 | 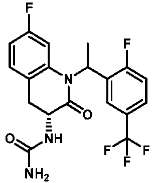 | MS m/z : 414 (M+H)⁺ | B |
| C-25 | 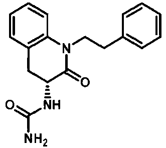 | MS m/z : 308 (M-H)⁻ | B |

**[Table 23]**

| Ex. No. | Str. | Phys. data | IC₅₀ |
|---|---|---|---|
| C-26 | 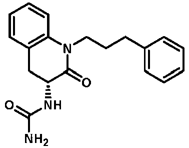 | MS m/z : 324 (M+H)⁺ | c |
| C-27 | 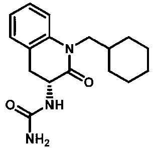 | MS m/z : 302 (M+H)⁺ | C |
| C-28 | 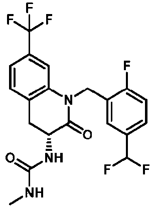 | ¹H-NMR (DMSO-d6) δ ppm : 2.58 (3H, d, J=4. 6Hz), 2.89-3.03 (1H, m), 3.27 (1H, dd, J=5.7, 15.5 Hz), 4.44-4.59 (1H, m), 5.31 (2H, s), 6.20 (1H, q, J=4. 6Hz), 6.44 (1H, d, J=6.8Hz), 6.95 (1H, t, J=55. 7Hz), 7.26-7.61 (6H, m) MS m/z : 446 (M+H)⁺ | A |
| C-29 | 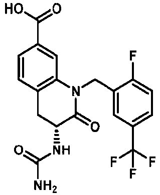 | MS m/z : 426 (M+H)⁺ | C |
| C-30 | 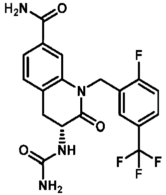 | MS m/z : 425 (M+H)⁺ | B |

**[Table 24]**

| Ex. No. | Str. | Phys. data | IC₅₀ |
|---|---|---|---|
| D-1 | 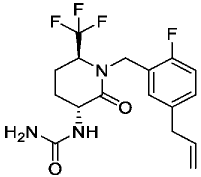 | MS m/z : 374 (M+H)⁺ | A |
| D-2 | 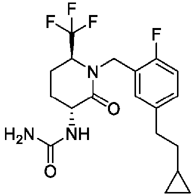 | MS m/z : 402 (M+H)⁺ | A |
| D-3 | 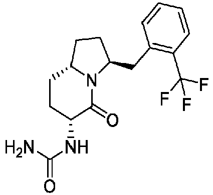 | ¹H-NMR (CDCl₃) δ ppm : 1.39-1.74 (4H, m), 1.79-1.94 (1H, m), 1.95-2.1 0 (1H, m), 2.12-2.29 (1H, m), 2.36-2. 54 (1H, m), 2.85 (1H, dd, J=8.8, 14.0 Hz), 3.30 (1H, dd, J=5.6, 14.0 Hz), 3.66-3.82 (1H, m), 4.16-4.29 (1H, m), 4.47-4.87 (3H, m), 5.79 (1H, br), 7.2 9-7.54 (3H, m), 7.63 (1H, d, J=8.0 H z) | B |
| | | MS m/z : 356 (M+H)⁺ | |
| D-4 | 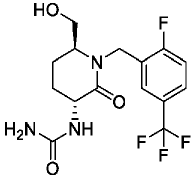 | MS m/z : 364 (M+H)⁺ | B |
| D-5 | 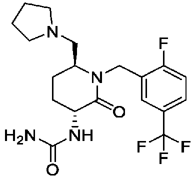 | MS m/z : 417 (M+H)⁺ | C |

**[Table 25]**

| Ex. No. | Str. | Phys. data | IC₅₀ |
|---|---|---|---|
| D-6 | 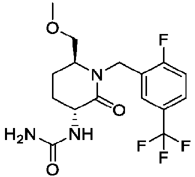 | ¹H-NMR (CDCl₃) δ ppm : 0.60-1.80 (1H, m), 1.83-2.11 (2H, m), 2.42-2.6 1 (1H, m), 3.21 (3H, s), 3.33 (1H, dd, J=4.8, 10.0 Hz), 3.41 (1H, dd, J=4. 0, 10.0 Hz), 3.49-3.68 (1H, m), 4.16-4.30 (1H, m), 4.50 (2H, br), 4.64 (1H, d, J=15.8 Hz), 4.90 (1H, d, J=15.8 H z), 5.76 (1H, br), 7.09-7.20 (1H, m), 7.45-7.59 (2H, m) | A |
| | | MS m/z : 378 (M+H)⁺ | |
| D-7 | 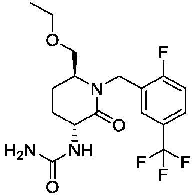 | ¹H-NMR (CDCl₃) δ ppm : 1.11 (3H, t, J=6.8 Hz), 1.40-1.72 (1H, m), 1.82-2.13 (2H, m), 2.44-2.59 (1H, m), 3.2 3-3.70 (5H, m), 4.18-4.31 (1H, m), 4. 54 (2H, br), 4.65 (1H, d, J=15.8 Hz), 4.93 (1H, d, J=15.8 Hz), 5.76-5.92 (1H, m), 7.08-7.21 (1H, m), 7.45-7.6 5 (2H, m) | A |
| | | MS m/z : 392 (M+H)⁺ | |
| D-8 | 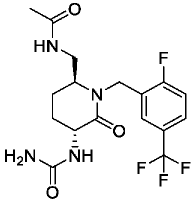 | MS m/z : 405 (M+H)⁺ | C |
| D-9 | 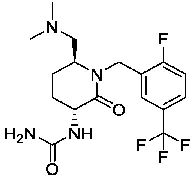 | MS m/z : 391 (M+H)⁺ | C |

**[Table 26]**

| Ex. No. | Str. | Phys. data | IC₅₀ |
|---|---|---|---|
| D-10 | 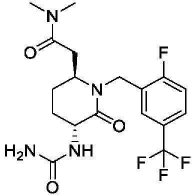 | MS m/z : 419 (M+H)⁺ | C |
| E-1 | 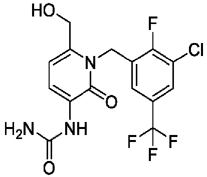 | MS m/z : 394 (M+H)⁺ | A |
| E-2 | 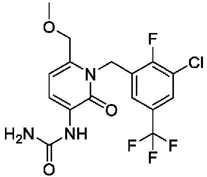 | ¹H-NMR (DMSO-d6) δ ppm : 3.16 (3 H, s), 4.38 (2H, s), 5.34 (2H, s), 6.20 -6.60 (3H, m), 7.16-7.31 (1H, m), 7.9 7-8.13 (2H, m), 8.40 (1H, s) | A |
| | | MS m/z : 408 (M+H)⁺ | |
| E-3 | 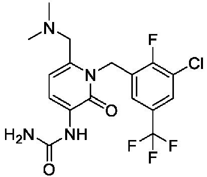 | MS m/z : 421 (M+H)⁺ | A |
| E-4 | 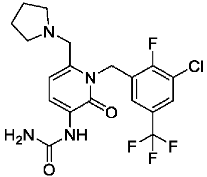 | ¹H-NMR (CD₃OD) δ ppm : 1.40-1.60 (4H, m), 2.25-2.48 (4H, m), 3.56 (2H, s), 5.57 (2H, s), 6.36 (1H, d, J=7.6 Hz), 6.91-7.06 (1H, m), 7.70-7.80 (1 H, m), 8.08 (1H, d, J=7.6 Hz) | A |
| | | MS m/z : 447 (M+H)⁺ | |
| E-5 | 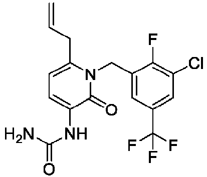 | ¹H-NMR (DMSO-d6) δ ppm : 3.41 (2 H, d, J=6.0 Hz), 4.97-5.17 (2H, m), 5.35 (2H, s), 5.79-5.97 (1H, m), 6.19 (1H, d, J=7.6 Hz), 6.34 (2H, br), 7.1 2-7.28 (1H, m), 7.98-8.11 (2H, m), 8. 28 (1H, s) | A |
| | | MS m/z : 404 (M+H)⁺ | |

**[Table 27]**

| Ex. No. | Str. | Phys. data | IC₅₀ |
|---|---|---|---|
| E-6 | 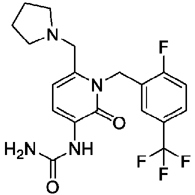 | ¹H-NMR (DMSO-d6) δ ppm : 1.33-1. 56 (4H, m), 2.20-2.40 (4H, m), 3.51 (2H, s), 5.43 (2H, s), 6.15-6.56 (3 H, m), 7.03-7.15 (1H, m), 7.41-7.57 (1H, m), 7.63-7.81 (1H, m), 8.03 (1 H, d, J=7.2 Hz), 8.37 (1H, s) | A |
| | | MS m/z : 413 (M+H)⁺ | |
| E-7 | 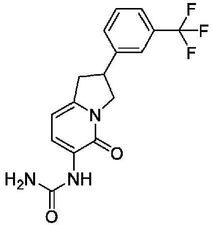 | MS m/z : 338 (M+H)⁺ | B |
| E-8 | 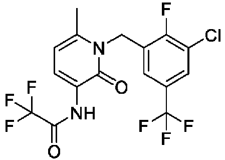 | MS m/z : 431 (M+H)⁺ | B |
| E-9 | 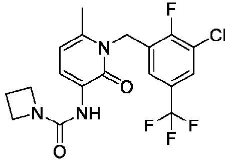 | MS m/z : 418 (M+H)⁺ | A |
| F-1 | 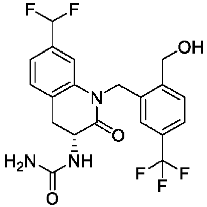 | MS m/z : 444 (M+H)⁺ | B |
| F-2 | 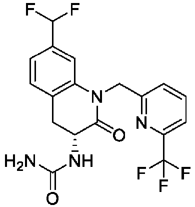 | MS m/z : 413 (M-H)⁻ | A |

**[Table 28]**

| Ex. No. | Str. | Phys. data | IC₅₀ |
|---|---|---|---|
| F-3 | 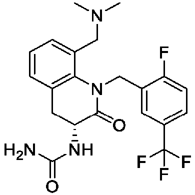 | MS m/z : 439 (M+H)⁺ | B |
| F-4 | 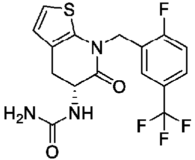 | ¹H-NMR (CDCl₃) δ ppm : 2.55-2. 83 (1H, m), 3.55 (1H, dd, J=6.8, 15.2 Hz), 4.39-4.75 (3H, m), 5. 00-5.20 (2H, m), 5.60-5.90 (1H, m), 6.70-6.85 (2H, m), 7.12-7.2 3 (1H, m), 7.40-7.60 (2H, m) | A |
| | | MS m/z : 388 (M+H)⁺ | |
| F-5 | 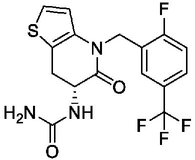 | ¹H-NMR (CDCl₃) δ ppm : 2.70-2. 90 (1H, m), 3.68 (1H, dd, J=7.2, 15.6 Hz), 4.53 (2H, br), 4.60-4. 75 (1H, m), 5.09 (1H, d, J=16.2 Hz), 5.19 (1H, d, J=16.2 Hz), 5. 70-5.90 (1H, m), 6.71 (1H, d, J= 5.2 Hz), 7.05-7.23 (2H, m), 7.40 -7.60 (2H, m) | A |
| | | MS m/z : 388 (M+H)⁺ | |
| F-6 | 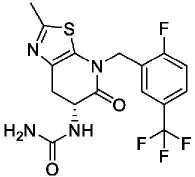 | MS m/z : 403 (M+H)⁺ | B |
| F-7 | 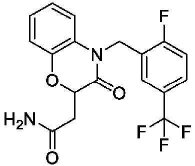 | MS m/z : 366 (M-OH+H)⁺ | C |

**[Table 29]**

| Ex. No. | Str. | Phys. data | IC₅₀ |
|---|---|---|---|
| F-8 | 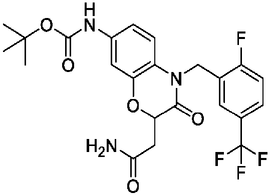 | ¹H-NMR (DMSO-d6) δ ppm : 1.4 5 (9H, s), 2.60-2.81 (2H, m), 5.0 0-5.09 (1H, m), 5.14 (1H, d, J=1 6.6 Hz), 5.30 (1H, d, J=16.6 Hz), 6.86-7.23 (4H, m), 7.40-7.82 (4 H, m), 9.36 (1H, s) | A |
| | | MS m/z : 496 (M-H)⁻ | |
| F-9 | 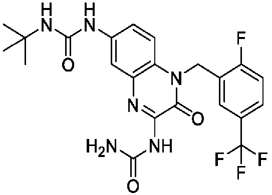 | MS m/z : 495 (M+H)⁺ | A |

The stereochemical notations in Examples A-1 to A-12, A-19, A-21, A-23, A-29, and D-10 represent relative configuration.

### Test Example 1

### Measurement of Antagonist Activity Using TSH-induced cAMP Production in Human TSHR-stably Expressing CHO Cells as an Indicator

A gene sequence of human TSHR (reference number NM_000369.2) was inserted into a multiple cloning site of pcDNA3.1(+). The prepared plasmid vector was introduced into CHO cells by a lipofection method to establish human TSHR stably expressing CHO cells. The obtained cells were seeded in a poly-D-lysine-coated 96-well plate at 5 x 10⁴ cells/well, and cultured for 1 day in F12 medium containing 10% FBS, 400 µg/mL G418, 50 U/mL penicillin, and 50 µg/mL streptomycin under conditions of 37°C and 5% CO₂. After removing a medium, the cells were washed twice with 100 µL per well of an assay buffer (Hanks' Balanced Salt Solution containing 20 mM HEPES and 1 mM IBMX). 30 µL of an assay buffer containing a test compound was added to a well, and the mixture was incubated at room temperature for 15 minutes. Next, 30 µL of an assay buffer containing human TSH (R&D Systems, Inc., final concentration 50 ng/mL) was added, and the mixture was incubated at 37°C for 1 hour. The supernatant was removed, and Lysis and Detection Buffer 2 (Cisbio) was added to prepare a cell lysate by incubating at room temperature for 1 hour. According to the instructions for use of a cAMP Gs HiRange kit (Cisbio), a cell lysate was reacted with d2-labeled cAMP and an anti-cAMP Europium Cryptate labeled antibody (Cisbio) in a 384-well white microplate. Next, a fluorescence intensity ratio (measurement wavelengths 665 nm/620 nm) was measured using a multiplate reader (PHERAstar FSX, BMG LABTECH Japan). A fluorescence intensity ratio of each sample was converted into a cAMP content using a standard curve. A cAMP content was converted into a percentage of a control value, and a cAMP production rate was calculated. Using Prism (Graph Pad Software Inc.), a cAMP production rate was plotted against a test compound concentration, and an IC₅₀ value was calculated. The IC₅₀ of each test compound is shown in the above tables. In the tables, IC₅₀ < 3.0 µM was denoted as A, and 3.0 µM ≤ IC₅₀ < 30 µM was denoted as B. The case where activity was observed but an IC₅₀ value could not be calculated was denoted as C.

As shown in the above tables, it was found that the compound of the present invention had human TSHR antagonist activity.

### [Industrial Applicability]

The compound of the present invention or a pharmacologically acceptable salt thereof are useful as a therapeutic agent for thyroid-related diseases because it has TSHR antagonist activity.

## Claims

1. A compound represented by Formula (A-III): 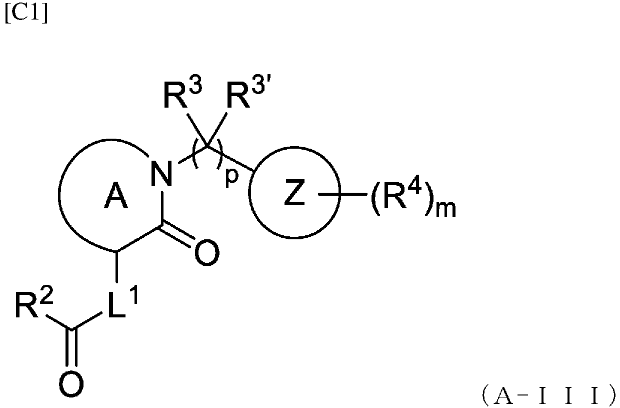 [wherein,
ring A is a group represented by Formula (1), (2), or (3) below: 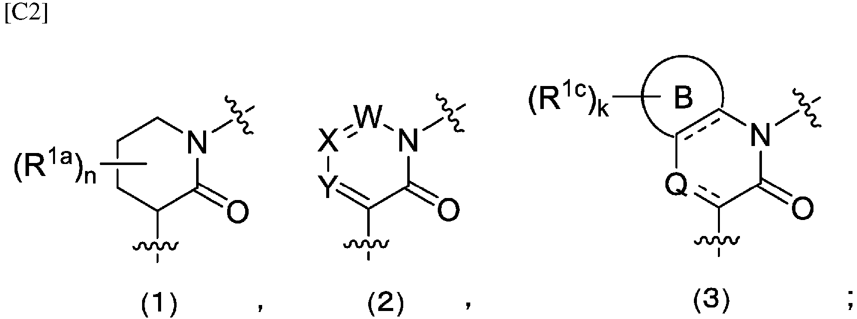
Q is -CH=, -CH₂-, -O-, or -N=;
R^{1a} is a halogen atom, C₁₋₆ alkyl, halo C₁₋₆ alkyl, C₁₋₆ alkoxy C₁₋₆ alkyl, hydroxy C₁₋₆ alkyl, amino C₁₋₆ alkyl, -(C₁₋₆ alkylene)-(3- to 8-membered heterocycloalkyl), which is unsubstituted or substituted with 1 to 6 groups selected from substituent group A, -(C₁₋₆ alkylene)-NH-COO-(C₁₋₆ alkyl), -(C₁₋₆ alkylene)-NH-CO-(C₁₋₆ alkyl), or -(C₁₋₆ alkylene)-CON(C₁₋₆ alkyl)₂;
substituent group A is a group consisting of a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkoxy, C₃₋₈ cycloalkyl C₁₋₆ alkyl, and C₃₋₈ cycloalkyl C₁₋₆ alkoxy;
when substituted with two or more groups selected from substituent group A, each group may be the same as or different from each other;
R^{1c} is a halogen atom, hydroxy, carboxy, cyano, C₁₋₆ alkyl, halo C₁₋₆ alkyl, hydroxy C₁₋₆ alkyl, amino C₁₋₆ alkyl, C₁₋₆ alkoxy, -CONH₂, -NH-COO-(C₁₋₆ alkyl), or -NH-CO-NH-(C₁₋₆ alkyl);
n is an integer of 1 to 3;
when n is 2 or 3, respective R^{1a}s may be the same as or different from each other;
two R^{1a}s may be taken together to form a 3- to 8-membered saturated carbocycle or a 3- to 8-membered saturated heterocycle;
R^{1a} may be taken together with R³ to form a 5- to 8-membered saturated heterocycle or a 5- to 8-membered unsaturated heterocycle;
W, X, and Y are each independently -CR^{1b}= or -N=;
R^{1b} is a hydrogen atom, a halogen atom, cyano, C₁₋₆ alkyl, C₂₋₆ alkenyl, hydroxy C₁₋₆ alkyl, carboxy C₁₋₆ alkyl, amino C₁₋₆ alkyl, halo C₁₋₆ alkyl, C₁₋₆ alkoxy, C₁₋₆ alkoxy C₁₋₆ alkyl, -(C₁₋₆ alkylene)-(3- to 8-membered heterocycloalkyl), or -(C₁₋₆ alkylene)-NH-COO-(C₁₋₆ alkyl);
at least one R^{1b} is other than hydrogen atom;
when two or more R^{1b}s are present, respective R^{1b}s may be the same or different from each other;
R^{1b} may be taken together with R³ to form a 5- to 8-membered saturated heterocycle or a 5- to 8-membered unsaturated heterocycle;
k is an integer of 0 to 3;
when k is 2 or 3, respective R^{1c}s may be the same or different from each other;
ring B is a group represented by the following formula: 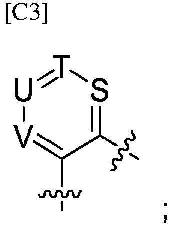
thiazole or thiophene;
S, T, U, and V are each independently -CH=, -CR^{1c}=, or -N=;
[C4] - - -
represents a single bond or a double bond;
ring Z is C₆₋₁₀ aryl, 5- or 6-membered heteroaryl, C₃₋₈ cycloalkyl, or a ring represented by the following formula: 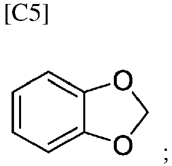
R² is C₁₋₆ alkyl, C₃₋₈ cycloalkyl, C₁₋₆ alkoxy, C₁₋₆ alkoxy C₁₋₆ alkyl, or -NR⁵R^{5'};
provided that, when ring A is a group represented by Formula (2) or Formula (3), R² is -NHR⁵;
R⁵ and R^{5'} are each independently a hydrogen atom, C₁₋₆ alkyl, or C₃₋₈ cycloalkyl;
R³ and R^{3'} are each independently a hydrogen atom or C₁₋₆ alkyl;
R⁴ is a halogen atom, cyano, C₁₋₆ alkyl, C₂₋₆ alkenyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
L¹ is -CR⁶R^{6'}-, -O-, or -NR⁶-;
R⁶ and R^{6'} are each independently a hydrogen atom or C₁₋₆ alkyl;
m is an integer of 0 to 3;
when m is 2 or 3, respective R⁴s may be the same as or different from each other;
provided that, when ring A is a group represented by Formula (2), m is an integer of 1 to 3;
p is an integer of 1 to 3; and
when p is 2 or 3, respective R³ and R^{3'} may be the same as or different from each other]
or a pharmacologically acceptable salt thereof.

2. The compound according to claim 1, which is a compound represented by Formula (A-IV): 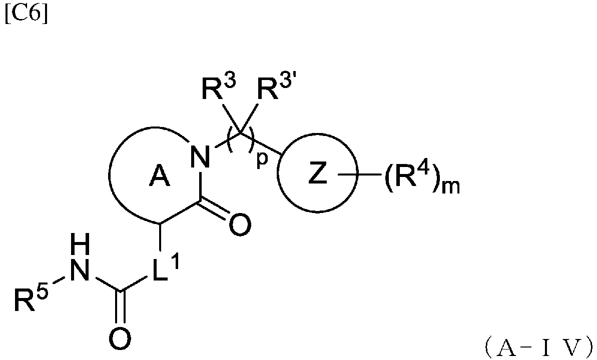 [wherein,
ring A is a group represented by Formula (1), (2), or (3) below: 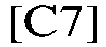 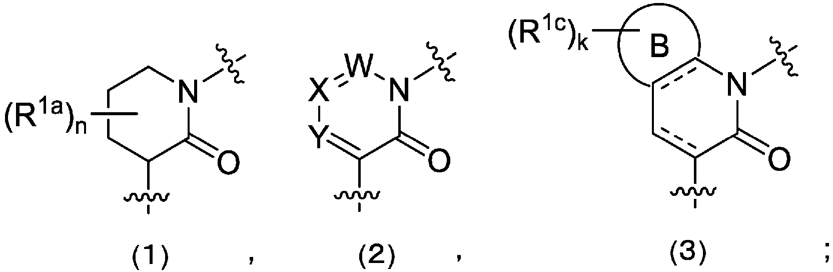
R^{1a} is a halogen atom, C₁₋₆ alkyl, halo C₁₋₆ alkyl, C₁₋₆ alkoxy C₁₋₆ alkyl, hydroxy C₁₋₆ alkyl, amino C₁₋₆ alkyl, -(C₁₋₆ alkylene)-(3- to 8-membered heterocycloalkyl), which is unsubstituted or substituted with 1 to 6 groups selected from substituent group A, -(C₁₋₆ alkylene)-NH-COO-(C₁₋₆ alkyl), -(C₁₋₆ alkylene)-NH-CO-(C₁₋₆ alkyl), or -(C₁₋₆ alkylene)-CON(C₁₋₆ alkyl)₂;
substituent group A is a group consisting of a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkoxy, C₃₋₈ cycloalkyl C₁₋₆ alkyl, and C₃₋₈ cycloalkyl C₁₋₆ alkoxy;
when substituted with two or more groups selected from substituent group A, the respective groups may be the same as or different from each other;
R^{1c} is a halogen atom, hydroxy, carboxy, cyano, C₁₋₆ alkyl, halo C₁₋₆ alkyl, hydroxy C₁₋₆ alkyl, amino C₁₋₆ alkyl, C₁₋₆ alkoxy, -CONH₂, -NH-COO-(C₁₋₆ alkyl), or -NH-CO-NH-(C₁₋₆ alkyl);
n is an integer of 1 to 3;
when n is 2 or 3, respective R^{1a}s may be the same as or different from each other;
two R^{1a}s may be taken together to form a 3- to 8-membered saturated carbocycle or a 3- to 8-membered saturated heterocycle;
R^{1a} may be taken together with R³ to form a 5- to 8-membered saturated heterocycle or a 5- to 8-membered unsaturated heterocycle;
W, X, and Y are each independently -CR^{1b}= or -N=;
R^{1b} is a hydrogen atom, a halogen atom, cyano, C₁₋₆ alkyl, C₂₋₆ alkenyl, hydroxy C₁₋₆ alkyl, carboxy C₁₋₆ alkyl, amino C₁₋₆ alkyl, halo C₁₋₆ alkyl, C₁₋₆ alkoxy, C₁₋₆ alkoxy C₁₋₆ alkyl, -(C₁₋₆ alkylene)-(3- to 8-membered heterocycloalkyl), or -(C₁₋₆ alkylene)-NH-COO-(C₁₋₆ alkyl);
at least one R^{1b} is other than a hydrogen atom;
when two or more R^{1b}s are present, respective R^{1b}s may be the same or different from each other;
k is an integer of 0 to 3;
when k is 2 or 3, respective R^{1c}s may be the same or different from each other;
ring B is a group represented by the following formula: 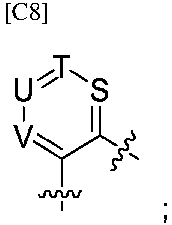
thiazole or thiophene;
S, T, U, and V are each independently -CH=, -CR^{1c}=, or -N=;
[C9] - - -
represents a single bond or a double bond;
ring Z is C₆₋₁₀ aryl, 5- or 6-membered heteroaryl, C₃₋₈ cycloalkyl, or a ring represented by the following formula: 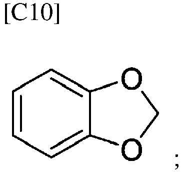
R⁵ is a hydrogen atom, C₁₋₆ alkyl, or C₃₋₈ cycloalkyl;
R³ and R^{3'} are each independently a hydrogen atom or C₁₋₆ alkyl;
R⁴ is a halogen atom, cyano, C₁₋₆ alkyl, C₂₋₆ alkenyl, halo C₁₋₆ alkyl, halohydroxy C₁₋₆ alkyl, C₁₋₆ alkoxy, halo C₁₋₆ alkoxy, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₂₋₆ alkynyl, C₃₋₈ cycloalkyl C₂₋₆ alkynyl, or -SF₅;
L₁ is -CR⁶R^{6'}-, -O-, or -NR⁶-;
R⁶ and R^{6'} are each independently a hydrogen atom or C₁₋₆ alkyl;
m is an integer of 0 to 3;
when m is 2 or 3, respective R⁴s may be the same as or different from each other;
provided that, when ring A is a group represented by Formula (2), m is an integer of 1 to 3;
p is an integer of 1 to 3; and
when p is 2 or 3, respective R³ and R^{3'} may be the same as or different from each other]
or a pharmacologically acceptable salt thereof.

3. The compound according to claim 2, wherein R⁵ is a hydrogen atom, or a pharmacologically acceptable salt thereof.

4. The compound according to claim 3, wherein L¹ is -NH-, or a pharmacologically acceptable salt thereof.

5. The compound according to claim 4, wherein
W is -CR^{1b}=;
R^{1b} is a hydrogen atom, a halogen atom, cyano, C₁₋₆ alkyl, C₂₋₆ alkenyl, hydroxy C₁₋₆ alkyl, carboxy C₁₋₆ alkyl, amino C₁₋₆ alkyl, halo C₁₋₆ alkyl, C₁₋₆ alkoxy C₁₋₆ alkyl, -(C₁₋₆ alkylene)-(3- to 8-membered heterocycloalkyl), or -(C₁₋₆ alkylene)-NH-COO-(C₁₋₆ alkyl);
S, U, and V are each independently -CH= or -CR^{1c}=; and
R^{1c} is a halogen atom, carboxy, cyano, C₁₋₆ alkyl, halo C₁₋₆ alkyl, hydroxy C₁₋₆ alkyl, amino C₁₋₆ alkyl, C₁₋₆ alkoxy, -CONH₂, -NH-COO-(C₁₋₆ alkyl), or -NH-CO-NH-(C₁₋₆ alkyl),
or a pharmacologically acceptable salt thereof.

6. The compound according to claim 5, wherein
X and Y are each independently -CR^{1b}=;
T is -CH= or -CR^{1c}=; and
p is 1 or 2,
or a pharmacologically acceptable salt thereof.

7. The compound according to claim 6, wherein
n is 1 or 2;
k is an integer of 0 to 2,
or a pharmacologically acceptable salt thereof.

8. The compound according to claim 7, wherein
R³ and R^{3'} are hydrogen atoms; and
p is 1,
or a pharmacologically acceptable salt thereof.

9. The compound according to claim 8, wherein
m is an integer of 1 to 3,
or a pharmacologically acceptable salt thereof.

10. A compound selected from the group consisting of compounds below: 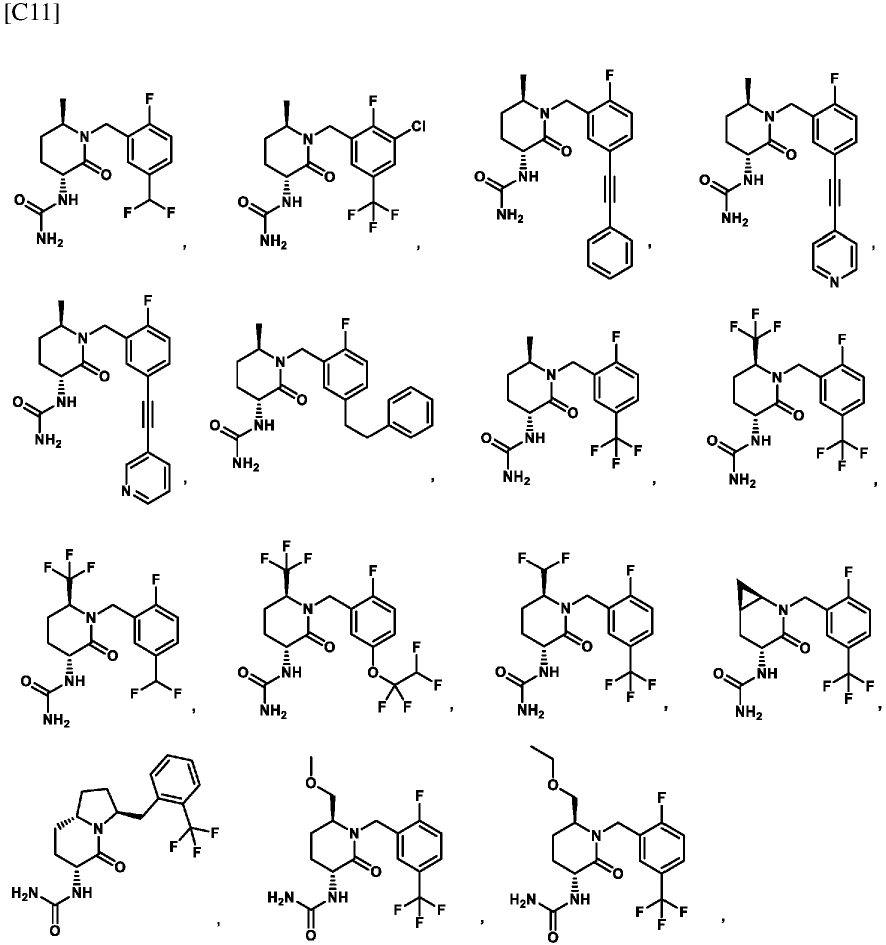 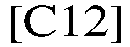 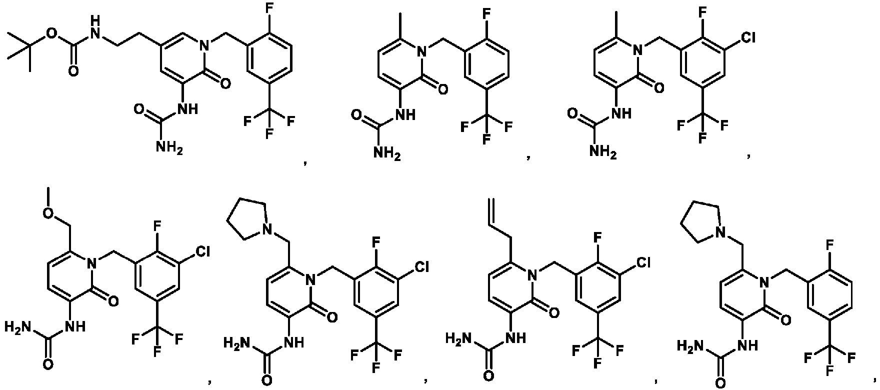 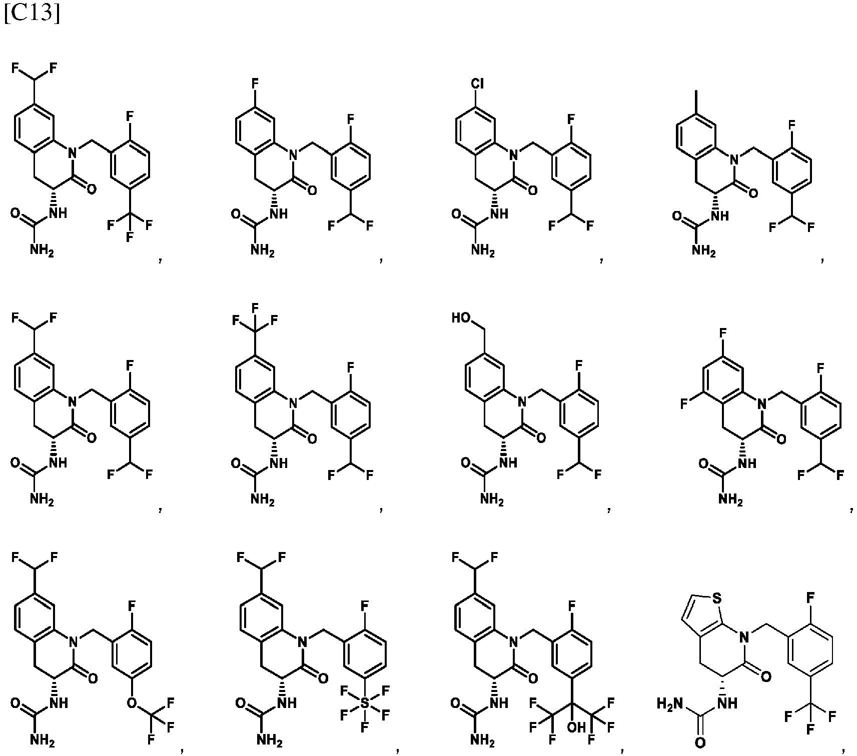 and 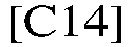 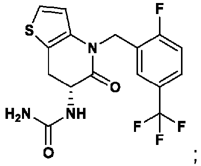 or a pharmacologically acceptable salt thereof.

11. A compound selected from the group consisting of compounds below: 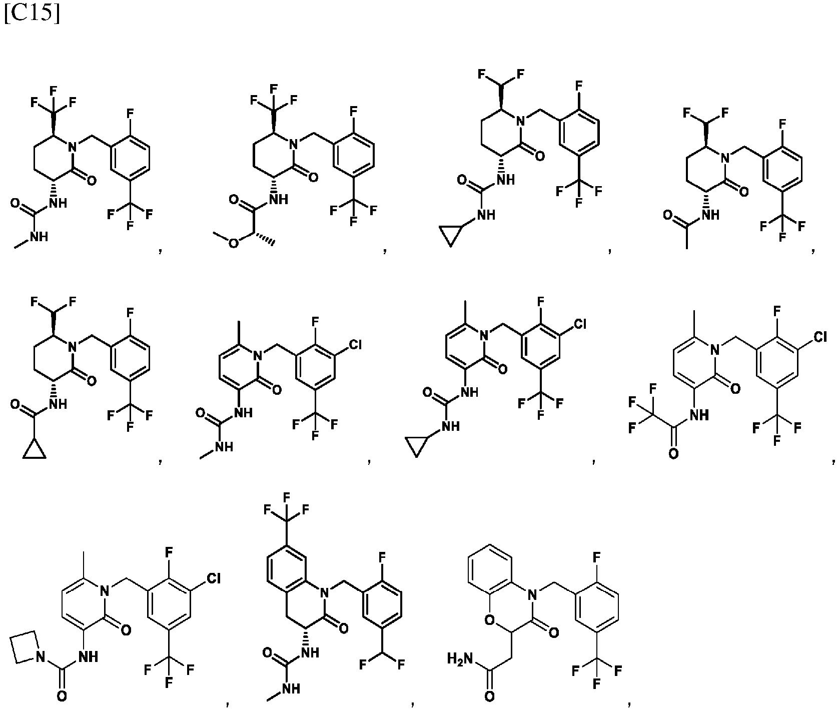 and 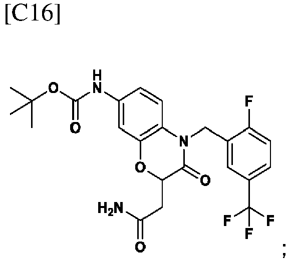 or a pharmacologically acceptable salt thereof.

12. A pharmaceutical composition comprising the compound according to any one of claims 1 to 11 or a pharmacologically acceptable salt thereof, and a pharmaceutical additive.

13. The pharmaceutical composition according to claim 12, which is a pharmaceutical composition for treating thyroid-related diseases.

14. The pharmaceutical composition according to claim 13, wherein the thyroid-related diseases are hyperthyroidism, Graves' disease, or thyroid eye disease.

15. A TSHR inhibitor comprising, as an active ingredient, a compound represented by Formula (A-II): 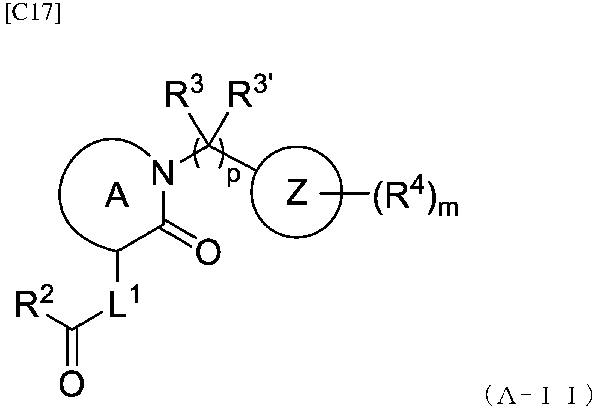 [wherein,
ring A is a group represented by Formula (1), (2), or (3) below: 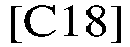 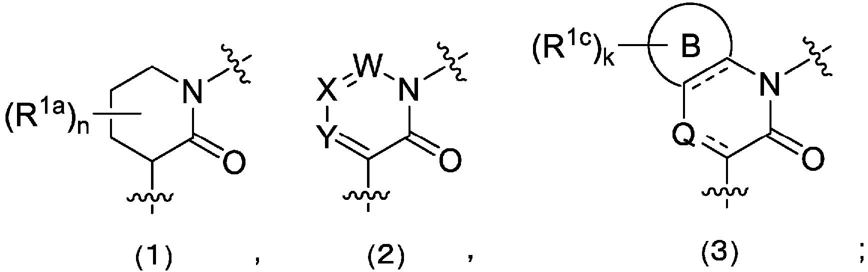
W, X, and Y are each independently -CR^{1b}= or -N=;
Q is -CR^{Q1}=, -CR^{Q1}R^{Q1'}-, -O-, -N=, or -NR^{Q2}-;
R^{Q1} and R^{Q1'} are each independently a hydrogen atom, a halogen atom, hydroxy, C₁₋₆ alkyl, or halo C₁₋₆ alkyl;
R^{Q2} is a hydrogen atom, C₁₋₆ alkyl, or halo C₁₋₆ alkyl;
ring B is 5- or 6-membered heteroaryl or benzene;
R^{1a} and R^{1c} are each independently a halogen atom, hydroxy, carboxy, cyano, C₁₋₆ alkyl, C₁₋₆ alkoxy, halo C₁₋₆ alkyl, C₁₋₆ alkoxy C₁₋₆ alkyl, hydroxy C₁₋₆ alkyl, carboxy C₁₋₆ alkyl, amino C₁₋₆ alkyl, -NR⁷COR⁸, -NR⁷COOR⁹, -NR⁷CONR¹⁰R¹¹, -CONR¹²R¹³, -(C₁₋₆ alkylene)-(3- to 8-membered heterocycloalkyl), which is unsubstituted or substituted with 1 to 6 groups selected from substituent group A, -(C₁₋₆ alkylene)-NR⁷COR⁸, -(C₁₋₆ alkylene)-NR⁷COOR⁹, -(C₁₋₆ alkylene)-NR⁷CONR¹⁰R¹¹, or -(C₁₋₆ alkylene)-CONR¹²R¹³;
R^{1b} is a hydrogen atom, a halogen atom, hydroxy, carboxy, cyano, C₁₋₆ alkyl, C₂₋₆ alkenyl, C₁₋₆ alkoxy, halo C₁₋₆ alkyl, hydroxy C₁₋₆ alkyl, amino C₁₋₆ alkyl, carboxy C₁₋₆ alkyl, -NR⁷COR⁸, -NR⁷COOR⁹, -NR⁷CONR¹⁰R¹¹, -CONR¹²R¹³, -(C₁₋₆ alkylene)-(3- to 8-membered heterocycloalkyl), which is unsubstituted or substituted with 1 to 6 groups selected from substituent group A, -(C₁₋₆ alkylene)-NR⁷COR⁸, -(C₁₋₆ alkylene)-NR⁷COOR⁹, -(C₁₋₆ alkylene)-NR⁷CONR¹⁰R¹¹, or -(C₁₋₆ alkylene)-CONR¹²R¹³;
R⁷ is a hydrogen atom or C₁₋₆ alkyl;
R⁸, R⁹, R¹⁰, R¹¹, R¹², and R¹³ are each independently a hydrogen atom or a group selected from the group consisting of (i) to (v):
(i) C₁₋₆ alkyl which is unsubstituted or substituted with 1 to 6 groups selected from substituent group A,
(ii) C₃₋₈ cycloalkyl which is unsubstituted or substituted with 1 to 6 groups selected from substituent group A,
(iii) C₃₋₈ cycloalkyl C₁₋₆ alkyl which is unsubstituted or substituted with 1 to 6 groups selected from substituent group A,
(iv) C₆₋₁₀ aryl which is unsubstituted or substituted with 1 to 6 groups selected from substituent group A, and
(v) C₆₋₁₀ aryl C₁₋₆ alkyl which is unsubstituted or substituted with 1 to 6 groups selected from substituent group A;
substituent group A is a group consisting of a halogen atom, cyano, C₁₋₆ alkyl, C₁₋₆ alkoxy, halo C₁₋₆ alkyl, halo C₁₋₆ alkoxy, C₃₋₈ cycloalkyl, C₃₋₈ cycloalkoxy, C₃₋₈ cycloalkyl C₁₋₆ alkyl, and C₃₋₈ cycloalkyl C₁₋₆ alkoxy;
when substituted with two or more groups selected from substituent group A, the respective groups may be the same as or different from each other;
R¹⁰ and R¹¹ may be taken together to form a 3- to 10-membered saturated heterocycle or a 3- to 10-membered unsaturated heterocycle;
R¹² and R¹³ may be taken together to form a 3- to 10-membered saturated heterocycle or a 3- to 10-membered unsaturated heterocycle;
n is an integer of 1 to 3;
when n is 2 or 3, respective R^{1a}s may be the same as or different from each other;
when two or more R^{1b}s are present, respective R^{1b}s may be the same or different from each other;
k is an integer of 0 to 3;
when k is 2 or 3, respective R^{1c}s may be the same or different from each other;
two R^{1a}s may be taken together to form a 3- to 8-membered saturated carbocycle or a 3- to 8-membered saturated heterocycle;
[C19] - - -
represents a single bond or a double bond;
ring Z is C₆₋₁₀ aryl, a 5- to 10-membered heteroaryl, or C₃₋₈ cycloalkyl;
R² is C₁₋₆ alkyl, halo C₁₋₆ alkyl, C₁₋₆ alkoxy C₁₋₆ alkyl, C₃₋₈ cycloalkyl, a 3- to 8-membered heterocycloalkyl, or -NR⁵R^{5'};
provided that, when ring A is a group represented by Formula (2) or Formula (3), R² is halo C₁₋₆ alkyl, a 3- to 8-membered heterocycloalkyl, or -NHR⁵;
R⁵ and R^{5'} are each independently a hydrogen atom, C₁₋₆ alkyl, or C₃₋₈ cycloalkyl;
R³ and R^{3'} are each independently a hydrogen atom or C₁₋₆ alkyl;
R⁴ is a halogen atom, cyano, C₁₋₆ alkyl, C₂₋₆ alkenyl, C₁₋₆ alkoxy, halo C₁₋₆ alkyl, halo C₁₋₆ alkoxy, C₃₋₈ cycloalkyl, C₃₋₈ cycloalkyl C₁₋₆ alkyl, C₆₋₁₀ aryl, C₆₋₁₀ aryl C₁₋₆ alkyl, C₆₋₁₀ aryl C₂₋₆ alkynyl, C₆₋₁₀ aryloxy, C₆₋₁₀ aryl C₁₋₆ alkoxy, 5- or 6-membered heteroaryl C₁₋₆ alkyl, 5- or 6-membered heteroaryl C₂₋₆ alkynyl, C₃₋₈ cycloalkyl C₂₋₆ alkynyl, halohydroxy C₁₋₆ alkyl, or -SF₅;
R^{1a}, R^{1b}, or R^{1c} may be taken together with R³ to form a 5- to 8-membered saturated heterocycle or a 5- to 8-membered unsaturated heterocycle;
L₁ is -CR⁶R^{6'}-, -O-, or -NR⁶-;
R⁶ and R^{6'} are each independently a hydrogen atom or C₁₋₆ alkyl;
m is an integer of 0 to 3;
when m is 2 or 3, respective R⁴s may be the same as or different from each other;
provided that, when ring A is a group represented by Formula (2), m is an integer of 1 to 3;
p is an integer of 0 to 3; and
when p is 2 or 3, respective R³ and R^{3'} may be the same as or different from each other]
or a pharmacologically acceptable salt thereof.
